# EUROPEAN PATENT APPLICATION

(11) **EP 1 304 325 A1**
(43) Date of publication of application: **23.04.2003**
(21) Application number: 01951975.0
(22) Date of filing: 23.07.2001
(51) Int. Cl.: C07D 231/40, C07D 401/12, C07D 403/12, C07D 405/12, C07D 409/12, C07D 413/12, C07D 417/12, A01N 43/56, A01N 43/80, A01N 43/82, A01N 43/84, A01N 43/88

(54) **5-(M-CYANOBENZYLAMINO)PYRAZOLE DERIVATIVES**

(30) Priority: 25.07.2000 JP 2000223651
(71) Applicant: Sankyo Company, Limited, Tokyo 103-8426 (JP)
(72) Inventor: ITO, Hiroyuki, c/o SANKYO COMPANY, LIMITED, Yasu-gun, Shiga 520-2342 (JP); IMAI, Chiaki, c/o SANKYO COMPANY, LIMITED, Yasu-gun, Shiga 520-2342 (JP); TAKADA, Takeshi, c/o SANKYO COMPANY, LIMITED, Yasu-gun, Shiga 520-2342 (JP); TANAKA, Harukazu, c/o SANKYO COMPANY, LIMITED, Yasu-gun, Shiga 520-2342 (JP); OHNISHI, Tohru, c/o SANKYO COMPANY, LIMITED, ,Yasu-gun, Shiga 520-2342 (JP)
(74) Representative: Gibson, Christian John Robert
(86) International application number: JP0106346
(87) International publication number: WO02008196

(57) **Abstract**

Provided is a 5-(m-cyanobenzylamino)pyrazole derivative represented by the following formula: 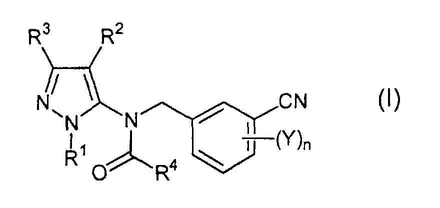 wherein:
R¹ represents a C₁₋₆ alkyl group, a C₃₋₇ cycloalkyl group or a phenyl group;
R² represents a hydrogen atom or a C₁₋₆ alkyl group;
R³ represents a C₁₋₆ alkyl, etc.,
R⁴ represents a hydrogen atom, a halogen atom, a C₁₋₆ alkyl group, etc.,
Y represents a C₁₋₆ alkyl group, etc.; and
n is an integer of 0 to 4;
or a salt thereof.

## Description

### [TECHNICAL FIELD]

The present invention relates to 5-(m-cyanobenzylamino)pyrazole derivatives, salts thereof, and agricultural chemicals containing them as an effective ingredient.

### [BACKGROUND ART]

5-(Substituted amino)pyrazole derivatives are described in Japanese Patent Application Kokai No. Sho 57-167972 and they are known to be usable as herbicides. This publication however does not include any description of 5-(m-cyanobenzylamino)pyrazole derivatives having a 5-amino group to which a m-cyanobenzyl group is bonded. In addition, the fact that 5-(substituted amino)pyrazole derivatives are usable as an agricultural or horticultural fungicide is not known.

An object of the present invention is to develop novel agricultural or horticultural fungicides capable of controlling, at a low application rate, late blight on tomatoes (*Phytophtora infestans*) and downy mildew on grape vines (*Plasmopara viticola*) which, among plant fungal diseases, sometimes cause serious damage to agricultural or horticultural crops.

### [Disclosure of the Invention]

The present inventors carried out an extensive investigation on 5-aminopyrazole derivatives. As a result, it has been found that 5-(m-cyanobenzylamino)pyrazole derivatives having a 5-amino group to which a m-cyanobenzyl group is bonded exhibit excellent fungicidal activities, specifically against various plant diseases, leading to the completion of the present invention.

According to the present invention, there is thus provided a 5-(m-cyanobenzylamino)pyrazole derivative represented by the following formula (I): 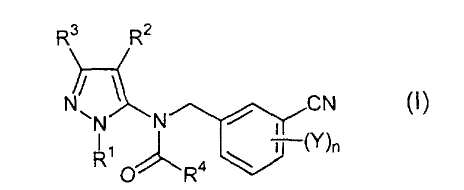 wherein:
R¹ represents a C₁₋₆ alkyl group, a C₃₋₇ cycloalkyl group or a phenyl group;
R² represents a hydrogen atom or a C₁₋₆ alkyl group;
R³ represents a C₁₋₆ alkyl group, a cyano-C₁₋₆ alkyl group, a hydroxy-C₁₋₆ alkyl group, a (C₁₋₆ alkoxy)-C₁₋₆ alkyl group, a (C₂₋₇ aliphatic acyloxy)-C₁₋₆ alkyl group, a (C₁₋₆ alkylamino)-C₁₋₆ alkyl group, a di(C₁₋₆ alkyl)amino-C₁₋₆ alkyl group, a (5- or 6-membered nitrogen-containing saturated heterocyclyl)-C₁₋₆ alkyl group (the heterocyclyl moiety of said heterocyclylalkyl group may additionally include one ring oxygen atom or NH group), a substituted or unsubstituted C₃₋₇ cycloalkyl-C₁₋₆ alkyl group (the substituent(s) of the cycloalkyl moiety of said cycloalkylalkyl group is (are) one C₁₋₆ alkyl group or 1 to 3 halogen atoms which may be the same or different, and the cycloalkyl moiety of said cycloalkylalkyl group may be interrupted by one oxygen atom), a C₃₋₇ cycloalkenyl-C₁₋₆ alkyl group, a halo-C₁₋₆ alkyl group (said halogen substituent(s) is (are) 1 to 6 halogen atoms which may be the same or different), a substituted or unsubstituted C₇₋₉ aralkyl group (the substituent(s) of the aryl moiety of said aralkyl group is (are) 1 to 5 substituents which are selected from the below-described substituent group A and may be the same or different), a substituted or unsubstituted heteroaralkyl group (the substituent(s) of the heteroaryl moiety of said heteroaralkyl group is (are) 1 to 5 substituents which are selected from the below-described substituent group B and may be the same or different) , a C₃₋₇ cycloalkyl group, a C₃₋₆ alkenyl group, a C₂₋₇ aliphatic acyl group or a substituted or unsubstituted phenyl group (the substituent(s) of said phenyl group is (are) 1 to 5 substituents which are selected from the below-described substituent group A and may be the same or different),
R⁴ represents a hydrogen atom, a halogen atom, a C₁₋₆ alkyl group, a cyano-C₁₋₆ alkyl group, a hydroxy-C₁₋₆ alkyl group, a halo-C₁₋₆ alkyl group (said halogen substituent(s) is (are) 1 to 3 halogen atoms which may be the same or different), a (C₁₋₆ alkoxy)-C₁₋₆ alkyl group, a {(C₁₋₆ alkoxy)-C₁₋₆ alkoxy}-C₁₋₆ alkyl group, a (C₃₋₆ alkenyloxy)-C₁₋₆ alkyl group, a (substituted or unsubstituted C₂₋₇ aliphatic acyloxy)-C₁₋₆ alkyl group (said substituent is a C₁₋₆ alkoxy group), a (substituted or unsubstituted C₂₋₇ alkoxycarbonyloxy)-C₁₋₆ alkyl group (said substituent is a C₁₋₆ alkoxy group), a (substituted or unsubstituted phenoxy)-C₁₋₆ alkyl group (said substituent(s) is (are) 1 to 5 substituents which are selected from the below-described substituent group A and may be the same or different), a (substituted or unsubstituted benzyloxy)-C₁₋₆ alkyl group (said substituent(s) is (are) 1 to 5 substituents which are selected from the below-described substituent group A and may be the same or different), a (substituted or unsubstituted heteroaryloxy)-C₁₋₆ alkyl group (said substituent(s) is (are) 1 to 3 substituents which are selected from the below-described substituent group B and may be the same or different), a (substituted or unsubstituted C₁₋₆ alkylamino)-C₁₋₆ alkyl group (said substituent is a phenyl group or a C₁₋₆ alkoxy group), a (C₃₋₆ alkenylamino)-C₁₋₆ alkyl group, a (phenylamino)-C₁₋₆ alkyl group, an {N-(C₁₋₆ alkyl)anilino}-C₁₋₆ alkyl group, a di(C₁₋₆ alkyl)amino-C₁₋₆ alkyl group, a (substituted or unsubstituted 5- or 6-membered nitrogen-containing saturated heterocyclyl)-C₁₋₆ alkyl group (said substituent(s) is (are) 1 or 2 C₁₋₆ alkyl groups which may be the same or different and the heterocyclyl moiety of said heterocyclylalkyl group may additionally include one ring oxygen atom or NH group), a (substituted or unsubstituted C₁₋₆ alkylthio)-C₁₋₆ alkyl group (said substituent is a phenyl group or a C₁₋₆ alkoxy group), a (C₃₋₆ alkenylthio)-C₁₋₆ alkyl group, a (substituted or unsubstituted phenylthio)-C₁₋₆ alkyl group (said substituent(s) is (are) 1 to 5 substituents which are selected from the below-described substituent group A and may be the same or different), a (substituted or unsubstituted heteroarylthio)-C₁₋₆ alkyl group (said substituent(s) is (are) 1 to 3 substituents which are selected from the below-described substituent group B and may be the same or different), a (C₂₋₇ alkoxycarbonyl)-C₁₋₆ alkyl group, a substituted or unsubstituted C₇₋₉ aralkyl group (said substituent(s) is (are) 1 to 5 substituents which are selected from the below-described substituent group A and may be the same or different), a substituted or unsubstituted heteroaralkyl group (said substituent(s) is (are) 1 to 3 substituents which are selected from the below-described substituent group B and may be the same or different), a C₃₋₇ cycloalkyl group, a substituted or unsubstituted phenyl group (the substituent(s) of said phenyl group is (are) 1 to 5 substituents which are selected from the below-described substituent group A and may be the same or different), a substituted or unsubstituted heteroaryl group (said substituent(s) is (are) 1 to 3 substituents which are selected from the below-described substituent group B and may be the same or different), a C₃₋₆ alkenyl group, a C₂₋₇ alkoxycarbonyl group, a C₁₋₆ alkoxy group, a (C₁₋₆ alkoxy)-C₁₋₆ alkoxy group, a (C₁₋₆ alkylamino)-C₁₋₆ alkoxy group, a di(C₁₋₆ alkyl)amino-C₁₋₆ alkoxy group, a substituted or unsubstituted heteroaralkyloxy group (said substituent(s) is (are) 1 to 3 substituents which are selected from the below-described substituent group B and may be the same or different), a C₃₋₇ cycloalkoxy group, a C₃₋₆ alkenyloxy group, a substituted or unsubstituted phenoxy group (said substituent(s) is (are) 1 to 5 substituents which are selected from the below-described substituent group A and may be the same or different), a substituted or unsubstituted benzyloxy group (said substituent(s) is (are) 1 to 5 substituents which are selected from the below-described substituent group A and may be the same or different), a substituted or unsubstituted heteroaryloxy group (said substituent(s) is (are) 1 to 3 substituents which are selected from the below-described substituent group B and may be the same or different), a substituted or unsubstituted C₁₋₆ alkylamino group (said substituent is a phenyl group or a C₁₋₆ alkoxy group), a C₃₋₆ alkenylamino group, a di(C₁₋₆ alkyl)amino group, a substituted or unsubstituted 5- or 6-membered nitrogen-containing saturated heterocyclic group (said substituent(s) is (are) 1 or 2 C₁₋₆ alkyl groups which may be the same or different, and said heterocyclic group may additionally include one ring oxygen atom or NH group), a substituted or unsubstituted C₁₋₆ alkylthio group (said substituent is a phenyl group or a C₁₋₆ alkoxy group), a C₃₋₆ alkenylthio group, a substituted or unsubstituted phenylthio group (said substituent(s) is (are) 1 to 5 substituents which are selected from the below-described substituent group A and may be the same or different), or a substituted or unsubstituted heteroarylthio group (said substituent(s) is (are) 1 to 3 substituents which are selected from the below-described substituent group B and may be the same or different);
Y represents a C₁₋₆ alkyl group, a C₁₋₆ alkoxy group, a cyano group, a nitro group, a halogen atom, or a C₂₋₇ alkoxycarbonyl group;
n is an integer of 0 to 4, with the proviso that when n is 2 to 4, the groups Y may be the same or different;
substituent group A consists of C₁₋₆ alkyl groups, halo-C₁₋₆ alkyl groups (said halogen substituent(s) is (are) 1 to 3 halogen atoms which may be the same or different), C₁₋₆ alkoxy groups, a cyano group, a nitro group, halogen atoms, C₂₋₇ alkoxycarbonyl groups, C₂₋₇ alkylcarbonylamino groups and C₁₋₃ alkylenedioxy groups; and
substituent group B consists of C₁₋₆ alkyl groups, halo-C₁₋₆ alkyl groups (said halogen substituent(s) is (are) 1 to 3 halogen atoms which may be the same or different), C₁₋₆ alkoxy groups, a cyano group, a phenyl group, halogen atoms, C₂₋₇ alkoxycarbonyl groups, and an oxo group;
or a salt thereof; and an agricultural chemical containing the derivative or salt as an effective ingredient.

In the present invention, the "C₁₋₆ alkyl group" represents, for example, a linear or branched alkyl group having 1 to 6 carbon atoms such as methyl, ethyl, propyl, isopropyl, butyl, isobutyl, s-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl, preferably a linear or branched alkyl group having 1 to 5 carbon atoms (C₁₋₅ alkyl group) . As R³, the "C₁₋₆ alkyl group" is more preferably a linear or branched alkyl group having 4 to 5 carbon atoms (C₄₋₅ alkyl group), still more preferably a branched alkyl group having 4 or 5 carbon atoms (branched C₄₋₅ alkyl group), of which the isobutyl or neopentyl group is particularly preferred and the isobutyl group is most preferred. As the other substituents and the substituent group A of R³, a linear or branched alkyl group having 1 to 4 carbon atoms (C₁₋₄ alkyl group) is more preferred, of which a linear or branched alkyl group having 1 to 3 carbon atoms is still more preferred, the methyl or ethyl group (C₁₋₂ alkyl group) is particularly preferred, and the methyl group is most preferred.

In the present invention, the "C₃₋₇ cycloalkyl group" represents, for example, a cyclopropyl, cyclobutyl, cyclopentyl, cyclohexyl or cycloheptyl group. As the substituent for the alkyl group of R³, the cyclopropyl, cyclobutyl, cyclopentyl or cyclohexyl (C₃₋₆ cycloalkyl group) is preferred, of which the cyclobutyl, cyclopentyl or cyclohexyl (C₄₋₆ cycloalkyl group) is more preferred and the cyclobutyl or cyclopentyl group is still more preferred. As R⁴, the cyclopropyl or cyclobutyl group is preferred, while as the other substituents, the cyclohexyl group is preferred.

In the present invention, the "cyano-C₁₋₆ alkyl group" represents, for example, the above-described "C₁₋₆ alkyl group" substituted with 1 or 2 cyano groups, such as cyanomethyl, dicyanomethyl, 1-cyanoethyl, 2-cyanoethyl, 3-cyanopropyl, 3,3-dicyanopropyl, 4-cyanobutyl, or 3-cyano-2-methylpropyl, of which the cyanomethyl or 2-cyanoethyl group is preferred and the cyanomethyl group is more preferred.

In the present invention, the "hydroxy-C₁₋₆ alkyl group" represents, for example, the above-described "C₁₋₆ alkyl group" substituted with 1 or 2 hydroxyl groups, such as hydroxymethyl, dihydroxymethyl, 1-hydroxyethyl, 2-hydroxyethyl, 3-hydroxypropyl, 3,3-dihydroxypropyl, 4-hydroxybutyl, or 3-hydroxy-2-methylpropyl, of which the hydroxymethyl, hydroxyethyl or hydroxypropyl group is preferred, the hydroxymethyl or 2-hydroxyethyl group is more preferred, and the hydroxymethyl group is still more preferred.

In the present invention, the "C₁₋₆ alkoxy group" or "C₁₋₆ alkoxy moiety of the (C₁₋₆ alkoxy)-C₁₋₆ alkyl group" represents, for example, a linear or branched C₁₋₆ alkoxy group such as methoxy, ethoxy, propoxy, isopropoxy, butoxy, isobutoxy, s-butoxy, t-butoxy, pentyloxy, isopentyloxy, 2-methylbutoxy, neopentyloxy, 1-ethylpropoxy, hexyloxy, (4-methylpentyl)oxy, (3-methylpentyl)oxy, (2-methylpentyl)oxy, (1-methylpentyl)oxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy or 2-ethylbutoxy, of which a linear or branched alkoxy group having 1 to 4 carbon atoms (C₁₋₄ alkoxy group) is preferred, the methoxy, ethoxy or isopropoxy group is more preferred, the methoxy or ethoxy group is still more preferred, and the methoxy group is particularly preferred.

In the present invention, the "(C₁₋₆ alkoxy)-C₁₋₆ alkyl group" represents, for example, the above-described "C₁₋₆ alkyl group" substituted with one above-described "C₁₋₆ alkoxy group", such as methoxymethyl, ethoxymethyl, propoxymethyl, isopropoxymethyl, butoxymethyl, isobutoxymethyl, s-butoxymethyl, t-butoxymethyl, pentyloxymethyl, (isopentyloxy)methyl, (2-methylbutoxy)methyl, (neopentyloxy)methyl, (1-ethylpropoxy)methyl, 2-methoxyethyl, 2-ethoxyethyl, 2-propoxyethyl, 1-isopropoxyethyl, 1-butoxyethyl, 2-isobutoxyethyl, 2-t-butoxyethyl, 3-methoxypropyl, 3-ethoxypropyl, 3-propoxypropyl, 3-isopropoxypropyl, 1-methoxybutyl, 4-ethoxybutyl or 5-methoxypentyl, of which an alkyl group which has 1 or 2 carbon atoms and is substituted with one linear or branched alkoxy group having 1 to 4 carbon atoms {(C₁₋₄ alkoxy)-C₁₋₂ alkyl} is preferred, the methoxymethyl, ethoxymethyl or methoxyethyl group is more preferred, the methoxymethyl or 2-methoxyethyl group is still more preferred, and the methoxymethyl group is particularly preferred.

In the present invention, the "C₂₋₇ aliphatic acyloxy moiety of the (C₂₋₇ aliphatic acyloxy)-C₁₋₆ alkyl group" represents, for example, a linear or branched aliphatic acyloxy group having 2 to 7 carbon atoms such as acetoxy, propionyloxy, butyryloxy, isobutyryloxy, pentanoyloxy, isobutyryloxy, isopentanoyloxy, 2-methylbutanoyloxy, pivaloyloxy or hexanoyloxy; a cyclic aliphatic acyloxy group having 4 to 7 carbon atoms such as cyclopropylcarbonyloxy, cyclobutylcarbonyloxy or cyclopentylcarbonyloxy; or an unsaturated aliphatic acyloxy group having 4 to 7 carbon atoms such as 2-methyl-1-butenoyloxy or 2-methyl-2-butenoyloxy, of which a linear or branched aliphatic acyloxy group having 2 to 6 carbon atoms, a cyclic aliphatic acyloxy group having 4 to 6 carbon atoms or an unsaturated aliphatic acyloxy group having 5 or 6 carbon atoms is preferred. As R³, the acetoxy group is more preferred, while as R⁴, the butyryloxy, isobutyryloxy, pivaloyloxy, cyclopropylcarbonyloxy, 2-methyl-1-butenoyloxy or 2-methyl-2-butenoyloxy group is more preferred, with the butyryloxy, isobutyryloxy, pivaloyloxy or cyclopropylcarbonyloxy group being still more preferred.

In the present invention, the "(C₂₋₇ aliphatic acyloxy)-C₁₋₆ alkyl group" represents, for example, the above-described "C₁₋₆ alkyl group" substituted with one above-described "C₂₋₇ aliphatic acyloxy" moiety, such as acetoxymethyl, propionyloxymethyl, butyryloxymethyl, isobutyryloxymethyl, pentanoyloxymethyl, isopentanoyloxymethyl, 2-methylbutanoyloxymethyl, pivaloyloxymethyl, hexanoyloxymethyl, 1-(acetoxy)ethyl, 2-(propionyloxy)ethyl, 1-(butyryloxy)ethyl, 1-(acetoxy)propyl, cyclopropylcarbonyloxymethyl, cyclobutylcarbonyloxymethyl, cyclopentylcarbonyloxymethyl, (2-methyl-1-butenoyl)oxymethyl, (2-methyl-2-butenoyl)oxymethyl or methoxyacetoxymethyl, preferably an alkyl group which has 1 or 2 carbon atoms and is substituted with one linear or branched aliphatic acyloxy group having 2 to 6 carbon atoms, cyclic aliphatic acyloxy group having 4 to 6 carbon atoms or unsaturated aliphatic acyloxy group having 5 or 6 carbon atoms. As R³, the acetoxymethyl group is more preferred, while as R⁴, the butyryloxymethyl, isobutyryloxymethyl, pivaloyloxymethyl, cyclopropylcarbonyloxymethyl, 2-methyl-1-butenoyloxymethyl or 2-methyl-2-butenoyloxymethyl group is more preferred, of which the butyryloxymethyl, isobutyryloxymethyl, pivaloyloxymethyl or cyclopropylcarbonyloxymethyl group is still more preferred.

In the present invention, the "C₁₋₆ alkylamino group" or the C₁₋₆ alkylamino moiety of the (C₁₋₆ alkylamino)-C₁₋₆ alkyl group" represents, for example, an amino group substituted with one above-described "C₁₋₆ alkyl group", such as methylamino, ethylamino, propylamino, isopropylamino, butylamino, isobutylamino, t-butylamino or hexylamino, of which the amino group substituted with one linear or branched alkyl group having 1 to 4 carbon atoms is preferred.

In the present invention, the "(C₁₋₆ alkylamino)-C₁₋₆ alkyl group" represents, for example, the above-described "C₁₋₆ alkyl group" substituted with one above-described "C₁₋₆ alkylamino group", such as methylaminomethyl, ethylaminomethyl, propylaminomethyl, isopropylaminomethyl, butylaminomethyl, isobutylaminomethyl, t-butylaminomethyl, hexylaminomethyl, 2-(methylamino)ethyl, 2-(ethylamino)ethyl, 2-(propylamino)ethyl, 2-(butylamino)ethyl, 1-(dimethylamino)ethyl, 2-(dimethylamino)ethyl, 3-(methylamino)propyl, 3-(ethylamino)propyl, 3-(dimethylamino)propyl, 4-(methylamino)butyl, or 6-(methylamino)hexyl, of which the methylaminomethyl, ethylaminomethyl, propylaminomethyl, isopropylaminomethyl, butylaminomethyl, isobutylaminomethyl or t-butylaminomethyl group is preferred.

In the present invention, the "di(C₁₋₆ alkyl)amino group" or the di(C₁₋₆ alkyl)amino moiety of the "di(C₁₋₆ alkyl)amino-C₁₋₆ alkyl group" represents, for example, an amino group substituted with two above-described "C₁₋₆ alkyl groups" which may be the same or different, such as dimethylamino, diethylamino, ethylmethylamino, dipropylamino or dihexylamino, of which the dimethylamino, diethylamino or ethylmethylamino group is preferred, and the dimethylamino group is more preferred.

In the present invention, the "di(C₁₋₆ alkyl)amino-C₁₋₆ alkyl group" represents, for example, the above-described "C₁₋₆ alkyl group" substituted with one above-described "di(C₁₋₆ alkyl)amino group", such as dimethylaminomethyl, diethylaminomethyl, ethylmethylaminomethyl, dipropylaminomethyl, dihexylaminomethyl, 1-(dimethylamino)ethyl, 2-(dimethylamino)ethyl, 3-(dimethylamino)propyl or 6-(dimethylamino)hexyl, of which the dimethylaminomethyl, diethylaminomethyl or ethylmethylaminomethyl group is preferred, and the dimethylaminomethyl group is more preferred.

In the present invention, the "5- or 6-membered nitrogen-containing saturated heterocyclic moiety (said heterocyclic moiety may additionally include one ring oxygen atom or NH group) in the (5- or 6-membered nitrogen-containing saturated heterocyclyl)-C₁₋₆ alkyl group (the heterocyclic moiety of said heterocyclylalkyl group may additionally include one ring oxygen atom or NH group)" represents, for example, a heterocyclic group having a 5- or 6-membered ring formed by one nitrogen atom and 4 or 5 methylene groups, or a heterocyclic group which may have, between two methylene groups thereof adjacent each other, one oxygen atom, such as a 1-pyrrolidinyl, piperidino, 1-piperazinyl or morpholino group. As R³, the 1-pyrrolidinyl or piperidino group is preferred, while as the other substituent, the piperidino or morpholino group is preferred, with the morpholino group being more preferred.

In the present invention, the "(5- or 6-membered nitrogen-containing saturated heterocyclyl)-C₁₋₆ alkyl group (the heterocyclic moiety of said heterocyclylalkyl group may additionally include one oxygen atom or NH group)" represents, for example, the above-described "C₁₋₆ alkyl group" substituted with one above-described "5- or 6-membered nitrogen-containing saturated heterocyclyl group (said heterocyclyl group may additionally include one ring oxygen atom or NH group)", such as 1-pyrrolidinylmethyl, piperidinomethyl, 1-piperazinylmethyl, morpholinomethyl, 1-(1-pyrrolidinyl)ethyl, 2-piperidinoethyl, 2-(1-piperazinyl)ethyl, 2-(morpholino)ethyl, 3-piperidinopropyl, 2-(1-piperazinyl)propyl, 3-(morpholino)propyl, 4-(morpholino)butyl, 5-(morpholino)pentyl or 6-(morpholino)hexyl. As R³, the 1-pyrrolidinylmethyl or piperidinomethyl group is preferred, while as the other substituent, the piperidinomethyl or morpholinomethyl group is preferred, with the morpholinomethyl group being more preferred.

In the present invention, the "substituted or unsubstituted C₃₋₇-cycloalkyl-C₁₋₆ alkyl group (the substituent(s) of the cycloalkyl moiety of said cycloalkylalkyl group is (are) one C₁₋₆ alkyl group or 1 to 3 halogen atoms which may be the same or different, and the cycloalkyl moiety of said cycloalkylalkyl group may be interrupted by one oxygen atom)" represents, for example, the above-described "C₁₋₆ alkyl group" which is substituted with the above-described "C₃₋₇ cycloalkyl group" which may be substituted with the above-described one "C₁₋₆ alkyl group" or the above-described 1 to 3 "halogen atoms" which may be the same or different, or with a "C₃₋₇ cycloalkyl group" which may be interrupted by one oxygen atom, such as cyclopropylmethyl, cyclobutylmethyl, cyclopentylmethyl, cyclohexylmethyl, cycloheptylmethyl, 2-cyclopropylethyl, 2-cyclobutylethyl, 2-cyclopentylethyl, 3-cyclobutylpropyl, 3-cyclopentylpropyl, tetrahydrofuranylmethyl, tetrahydropyranylmethyl, (methylcyclobutyl)methyl, (methylcyclopentyl)methyl, (fluorocyclopentyl)methyl, or (difluorocyclopentyl)methyl, of which a C₁₋₂ alkyl group substituted with a C₃₋₆ cycloalkyl group which may be substituted with one C₁₋₂ alkyl group or fluorine atoms, or with a tetrahydrofuranyl group or a teterahydropyranyl group is preferred, the cyclobutylmethyl, (methylcyclobutyl)methyl, tetrahydrofuranylmethyl, cyclopentylmethyl, (methylcyclopentyl)methyl, (fluorocyclopentyl)methyl, (difluorocyclopentyl)methyl, tetrahydropyranylmethyl or cyclohexylmethyl group is more preferred, the cyclobutylmethyl, (methylcyclobutyl)methyl, tetrahydrofuranylmethyl, cyclopentylmethyl, (fluorocyclopentyl)methyl, (difluorocyclopentyl)methyl, tetrahydropyranylmethyl or cyclohexylmethyl group is still more preferred, and the cyclobutylmethyl, tetrahydrofuranylmethyl or cyclopentylmethyl group is particularly preferred.

In the present invention, the "C₃₋₇ cycloalkenyl" moiety of the "C₃₋₇ cycloalkenyl-C₁₋₆ alkyl group" represents a cyclopropenyl, cyclobutenyl, cyclopentenyl, cyclohexenyl or cycloheptynyl group, preferably the cyclopentenyl, cyclohexenyl or cycloheptynyl group, more preferably the cyclopentenyl group.

In the present invention, the "C₃₋₇ cycloalkenyl-C₁₋₆ alkyl group" represents, for example, the above-described "C₁₋₆ alkyl group" substituted with one above-described "C₃₋₇ cycloalkenyl group", such as cyclobutenylmethyl, cyclopentenylmethyl, cyclohexenylmethyl, 2-cyclobutenylethyl, 2-cyclopentenylethyl, 3-cyclobutenylpropyl or 3-cyclopentenylpropyl, of which a C₁₋₂ alkyl group substituted with one cyclopentenyl, cyclohexenyl or cycloheptynyl group is preferred, the cyclopentenylmethyl or cyclohexenylmethyl group is more preferred, and the cyclopentenylmethyl group is still more preferred.

In the present invention, the "halogen atom" represents a fluorine, chlorine, bromine or iodine atom, preferably a fluorine, chlorine or bromine atom, more preferably a fluorine or chlorine atom, still more preferably a fluorine atom.

In the present invention, the "halo-C₁₋₆ alkyl group (said halogen substituent(s) is (are) 1 to 6 halogen atoms which may be the same or different) represents, for example, the above-described "C₁₋₆ alkyl group" substituted with the above-described 1 to 3 "halogen atoms" which may be the same or different, such as trifluoromethyl, trichloromethyl, difluoromethyl, dichloromethyl, dibromomethyl, fluoromethyl, chloromethyl, bromomethyl, iodomethyl, 2,2,2-trichloroethyl, 2,2,2-trifluoroethyl, 2-bromoethyl, 2-chloroethyl, 2-fluoroethyl, 3-chloropropyl, 3,3,3-trifluoropropyl, 4-fluorobutyl, 3-fluoro-2-methylpropyl, 3,3,3-trifluoro-2-methylpropyl, or 6,6,6-trichlorohexyl. As R³, a C₁₋₄ alkyl group substituted with 1 to 3 chlorine atoms or fluorine atoms which are the same are preferred, of which the chloromethyl or 3,3,3-trifluoro-2-methylpropyl group is more preferred, and the 3,3,3-trifluoro-2-methylpropyl group is still more preferred. As R⁴, the trifluoromethyl, trichloromethyl, difluoromethyl, dichloromethyl, fluoromethyl, chloromethyl or bromomethyl group is preferred.

In the present invention, the "C₂₋₇ alkoxycarbonyl group" represents, for example, a linear or branched alkoxycarbonyl group having 2 to 7 carbon atoms such as methoxycarbonyl, ethoxycarbonyl, propoxycarbonyl, isopropoxycarbonyl, butoxycarbonyl, 2-methylbutoxycarbonyl, isobutoxycarbonyl, t-butoxycarbonyl, pentyloxycarbonyl or hexyloxycarbonyl, preferably the linear or branched alkoxycarbonyl groups having 2 to 5 carbon atoms, more preferably the methoxycarbonyl or ethoxycarbonyl group, still more preferably the methoxycarbonyl group.

In the present invention, the "C₂₋₇ alkylcarbonylamino group" represents, for example, a linear or branched alkylcarbonylamino group having 2 to 7 carbon atoms such as acetylamino, propionylamino, butyrylamino, isobutyrylamino, pentanoylamino, isobutyrylamino, isopentanoylamino, 2-methylbutanoylamino, pivaloylamino or hexanoylamino, preferably a linear or branched alkylcarbonylamino group having 2 to 4 carbon atoms, more preferably the acetylamino group.

In the present invention, the "C₁₋₃ alkylenedioxy group" represents a methylenedioxy, ethylenedioxy or trimethylenedioxy group, preferably the methylenedioxy group.

In the present invention, the "substituted or unsubstituted C₇₋₉ aralkyl group (the substituent(s) of the aryl moiety of said aralkyl group is (are) 1 to 5 substituents which are selected from the below-described substituent group A and may be the same or different)" represents, for example, a benzyl, methylbenzyl, ethylbenzyl, propylbenzyl, isopropylbenzyl, dimethylbenzyl, trimethylbenzyl, trifluoromethylbenzyl, methoxybenzyl, dimethoxybenzyl, trimethoxybenzyl, ethoxybenzyl, isopropoxybenzyl, methylenedioxybenzyl, ethylenedioxybenzyl, cyanobenzyl, nitrobenzyl, chlorobenzyl, dichlorobenzyl, fluorobenzyl, difluorobenzyl, chloro-methylbenzyl, fluoro-methylbenzyl, methoxycarbonylbenzyl, chloro-methoxybenzyl, acetylaminobenzyl, 1-phenylethyl, 2-phenylethyl or 3-phenylpropyl group, preferably the benzyl, methylbenzyl, isopropylbenzyl, methoxybenzyl, cyanobenzyl, nitrobenzyl, chlorobenzyl or methoxycarbonylbenzyl group, more preferably the benzyl group.

In the present invention, the "substituted or unsubstituted heteroaralkyl group (the substituent(s) of the heteroaryl moiety of said heteroaralkyl group is (are) 1 to 5 substituents which are selected from the below-described substituent group B and may be the same or different)" represents, for example, a 2-pyridylmethyl, (3-methyl-2-pyridyl)methyl, (6-methyl-2-pyridyl)methyl, (3-methoxy-2-pyridyl)methyl, (6-chloro-2-pyridyl)methyl, 3-pyridylmethyl, 1-pyrazolylmethyl, (4-methyl-1-pyrazolyl)methyl, 3-isoxazolylmethyl, (5-methyl-3-isoxazolyl)methyl, (4,5-dimethyl-3-isoxazolyl)methyl, (4-chloro-5-phenyl-3-isoxazolyl)methyl, (5-methoxycarbonyl-3-isoxazolyl)methyl, {5-methyl-2-(1,3,4-thiadiazolyl)}methyl, 2-pyrazinylmethyl, 6-quinolylmethyl, 8-quinolylmethyl, 4-quinazolylmethyl, 2-pyrimidinylmethyl, (4-methyl-2-pyrimidinyl)methyl, (3,5-dimethyl-2-pyrimidinyl)methyl, 4-pyrimidinylmethyl, (2,6-dimethyl-4-pyrimidinyl)methyl, (6-methyl-2-isopropyl-4-pyrimidinyl)methyl, 3-benzoisoxazolylmethyl, 2-furylmethyl, (5-methyl-2-furyl)methyl, (3,5-dimethyl-2-furyl)methyl, 3-furylmethyl, (2-methyl-3-furyl)methyl, 2-thienylmethyl, (5-methyl-2-thienyl)methyl, (3,5-dimethyl-2-thienyl)methyl, 3-thienylmethyl or (2-methyl-3-thienyl)methyl group, preferably the 2-furylmethyl, 3-furylmethyl, 2-thienylmethyl or 3-thienylmethyl group, more preferably the 3-thienylmethyl group.

In the present invention, the "C₃₋₆ alkenyl group" represents a linear or branched alkenyl group having 3 to 6 carbon atoms such as allyl, 1-propenyl, 2-methyl-1-propenyl, 2-methyl-2-propenyl, 3-butenyl, 2-methyl-2-butenyl or 4-pentenyl, preferably a linear or branched alkenyl group having 3 to 5 carbon atoms (C₃₋₅ alkenyl), more preferably the allyl, 2-methyl-1-propenyl, 2-methyl-2-propenyl or 3-butenyl group. As R³, the 3-butenyl group is still more preferred, while as R⁴, the allyl, 2-methyl-1-propenyl or 2-methyl-2-propenyl group is still more preferred.

In the present invention, the "aliphatic C₂₋₇ acyl group" represents, for example, a linear or branched aliphatic acyl group having 2 to 7 carbon atoms such as acetyl, propionyl, butyryl, isobutyryl, pentanoyl, isobutyryl, isopentanoyl, 2-methylbutanoyl, pivaloyl or hexanoyl; a cyclic aliphatic acyl group having 4 to 7 carbon atoms such as cyclopropylcarbonyl, cyclobutylcarbonyl or cyclopentylcarbonyl; or an unsaturated aliphatic acyl group having 4 to 7 carbon atoms such as 2-methyl-1-butenoyl or 2-methyl-2-butenoyl, of which a linear or branched aliphatic acyl group having 2 to 6 carbon atoms, a cyclic aliphatic acyl group having 4 to 6 carbon atoms or an unsaturated aliphatic acyl group having 5 or 6 carbon atoms is preferred, a linear or branched aliphatic acyl group having 2 to 4 carbon atoms is more preferred, and the acetyl group is still more preferred.

In the present invention, the "substituted or unsubstituted phenyl group (the substituent(s) of said phenyl group is (are) 1 to 5 substituents which are selected from the below-described substituent group A and may be the same or different)" represents, for example, a phenyl, methylphenyl, ethylphenyl, propylphenyl, isopropylphenyl, dimethylphenyl, trimethylphenyl, trifluoromethylphenyl, methoxyphenyl, dimethoxyphenyl, trimethoxyphenyl, ethoxyphenyl, isopropoxyphenyl, methylenedioxyphenyl, ethylenedioxyphenyl, cyanophenyl, nitrophenyl, chlorophenyl, dichlorophenyl, fluorophenyl, difluorophenyl, chloro-methylphenyl, fluoro-methylphenyl, methoxycarbonylphenyl, chloro-methoxyphenyl or acetylaminophenyl group, preferably the phenyl, methylphenyl, isopropylphenyl, methoxyphenyl, cyanophenyl, nitrophenyl, chlorophenyl, fluorophenyl or methoxycarbonylphenyl group. As R³, the 3-fluorophenyl or phenyl group is more preferred, while as R⁴, the 3-methoxyphenyl or phenyl group is more preferred.

In the present invention, the "{(C₁₋₆-alkoxy)-C₁₋₆ alkoxy}-C₁₋₆ alkyl group" represents, for example, a methoxymethoxymethyl, 2-methoxyethoxymethyl, 3-methoxypropoxymethyl, 4-methoxybutoxymethyl, 5-methoxypentyloxymethyl, 2-(methoxymethoxy)ethyl, 2-(2-methoxyethoxy)ethyl, 2-(3-propoxy)ethyl, 1-(4-methoxybutoxy)ethyl, 3-(methoxymethoxy)propyl, 3-(2-methoxyethoxy)propyl, 3-(3-methoxypropoxy)propyl, 1-(methoxymethoxy)butyl, 4-(2-methoxyethoxy)butyl or 5-(methoxymethoxy)pentyl group, preferably the above-described "(C₁₋₄ alkoxy)-C₁₋₂ alkyl group" substituted, at the alkoxy moiety thereof, with an alkoxy group having 1 or 2 carbon atoms, more preferably the methoxymethoxymethyl, (2-methoxy)ethoxymethyl or 2-(methoxymethoxy)ethyl group, still more preferably the (2-methoxy)ethoxymethyl group.

In the present invention, the "C₃₋₆ alkenyloxy group" or "C₃₋₆ alkenyloxy moiety of the "(C₃₋₆ alkenyloxy)-C₁₋₆ alkyl group" represents, for example, a linear or branched alkenyloxy group having 3 to 6 carbon atoms, such as allyloxy, 1-propenyloxy, 2-methyl-1-propenyloxy, 2-methyl-2-propenyloxy, 3-butenyloxy, 2-methyl-2-butenyloxy or 4-pentenyloxy, preferably the allyloxy group.

In the present invention, the "(C₃₋₆ alkenyloxy)-C₁₋₆ alkyl group" represents, for example, the above-described "C₁₋₆ alkyl group" substituted with one above-described "C₃₋₆ alkenyloxy group", such as allyloxymethyl, 1-propenyloxymethyl, 2-methyl-1-propenyloxymethyl, 2-methyl-2-propenyloxymethyl, 3-butenyloxymethyl, 2-methyl-2-butenyloxymethyl, 4-pentenyloxymethyl, 2-(allyloxy)ethyl, 2-(1-propenyloxy)ethyl, 2-(2-methyl-l-propenyloxy)ethyl, 2-(2-methyl-2-propenyloxy)ethyl, 2-(3-butenyloxy)ethyl, 2-(2-methyl-2-butenyloxy)ethyl, 2-(4-pentenyloxy)ethyl, 3-(allyloxy)propyl, 4-(allyloxy)butyl, or 6-(allyloxy)hexyl group, preferably the allyloxymethyl or 2-allyloxyethyl group, more preferably the 2-allyloxyethyl group.

In the present invention, the "(substituted or unsubstituted C₂₋₇ aliphatic acyloxy)-C₁₋₆ alkyl group (said substituent is a C₁₋₆ alkoxy group)" represents, in addition to the above-described "(C₂₋₇ aliphatic acyloxy)-C₁₋₆ alkyl group", the above-described "(C₂₋₇ aliphatic acyloxy)-C₁₋₆ alkyl group" substituted with one above-described "C₁₋₆ alkoxy group", such as methoxyacetoxymethyl, ethoxyacetoxymethyl, propoxyacetoxymethyl, butoxyacetoxymethyl, pentyloxyacetoxymethyl, hexyloxyacetoxymethyl, 3-methoxybutyryloxymethyl, 2-(methoxyacetoxy)ethyl, 3-(methoxyacetoxy)propyl, or 4-(methoxyacetoxy)butyl, preferably the butyryloxymethyl, isobutyryloxymethyl, pivaloyloxymethyl, cyclopropylcarbonyloxymethyl, 2-methyl-1-butenoyloxymethyl, 2-methyl-2-butenoylcxymethyl, methoxyacetoxymethyl, 2-methoxybutyryloxymethyl or ethoxyacetoxymethyl, more preferably the butyryloxymethyl, isobutyryloxymethyl, pivaloyloxymethyl, cyclopropylcarbonyloxymethyl or methoxyacetoxymethyl group.

In the present invention, the "substituted or unsubstituted C₂₋₇ alkoxycarbonyloxy moiety of the (substituted or unsubstituted C₂₋₇ alkoxycarbonyloxy)-C₁₋₆ alkyl group (said substituent is a C₁₋₆ alkoxy group)" represents, for example, a methoxycarbonyloxy, ethoxycarbonyloxy, propoxycarbonyloxy, isopropoxycarbonyloxy, butoxycarbonyloxy, 2-methylbutoxycarbonyloxy, isobutoxycarbonyloxy, t-butoxycarbonyloxy, pentyloxycarbonyloxy, hexyloxycarbonyloxy, methoxymethoxycarbonyloxy, 2-methoxyethoxycarbonyloxy, 3-methoxypropoxycarbonyloxy, 4-methoxybutoxycarbonyloxy, 5-methoxypentyloxycarbonyloxy or 6-methoxyhexyloxycarbonyloxy group, preferably the methoxycarbonyloxy, ethoxycarbonyloxy, methoxymethoxycarbonyloxy or 2-methoxyethoxycarbonyloxy group, more preferably the methoxycarbonyloxy or 2-methoxyethoxycarbonyloxy group.

In the present invention, the "(substituted or unsubstituted C₂₋₇ alkoxycarbonyloxy)-C₁₋₆ alkyl group (said substituent is a C₁₋₆ alkoxy group)" represents, in addition to the above-described "(C₂₋₇ alkoxycarbonyloxy)-C₁₋₆ alkyl group", for example, a C₂₋₇ alkoxycarbonyloxy group substituted with one C₁₋₆ alkoxy group such as methoxymethoxycarbonyloxymethyl, 2-methoxyethoxycarbonyloxymethyl, 3-methoxypropoxycarbonyloxymethyl, 4-methoxybutoxycarbonyloxymethyl, 5-methoxypentyloxycarbonyloxymethyl, 6-methoxyhexyloxycarbonyloxymethyl, 2-(methoxymethoxycarbonyloxy)ethyl, 2-(2-methoxyethoxycarbonyloxy)ethyl or 2-(ethoxymethoxycarbonyloxy)ethyl, preferably the methoxycarbonyloxymethyl, ethoxycarbonyloxymethyl, 2-(methoxycarbonyloxy)ethyl, methoxymethoxycarbonyloxymethyl or 2-methoxyethoxycarbonyloxymethyl group, more preferably the methoxycarbonyloxymethyl or 2-methoxyethoxycarbonyloxymethyl group.

In the present invention, the "(phenoxy)-C₁₋₆ alkyl group" represents, for example, the above-described "C₁₋₆ alkyl group" substituted with one phenoxy group, such as phenoxymethyl, 1-phenoxyethyl, 2-phenoxyethyl, 3-phenoxypropyl, 4-phenoxybutyl, 5-phenoxypentyl or 6-phenoxyhexyl, preferably the phenoxymethyl or 1-phenoxyethyl group.

In the present invention, the "substituted or unsubstituted phenoxy group (said substituent(s) is (are) 1 to 5 substituents which are selected from the below-described substituent group A and may be the same or different)" or "the substituted or unsubstituted phenoxy moiety (said substituent(s) is (are) 1 to 5 substituents which are selected from the below-described substituent group A and may be the same or different) of the (substituted or unsubstituted phenoxy)-C₁₋₆ alkyl group (said substituent(s) is (are) 1 to 5 substituents which are selected from the below-described substituent group A and may be the same or different)" represents, for example, a phenoxy, methylphenoxy, ethylphenoxy, propylphenoxy, isopropylphenoxy, dimethylphenoxy, trimethylphenoxy, trifluoromethylphenoxy, methoxyphenoxy, dimethoxyphenoxy, trimethoxyphenoxy, ethoxyphenoxy, isopropoxyphenoxy, methylenedioxyphenoxy, ethylenedioxyphenoxy, cyanophenoxy, nitrophenoxy, chlorophenoxy, dichlorophenoxy, fluorophenoxy, difluorophenoxy, chloro-methylphenoxy, fluoro-methylphenoxy, methoxycarbonylphenoxy, chloro-methoxyphenoxy or acetylaminophenoxy group, preferably the phenoxy, 2-fluorophenoxy, 3-fluorophenoxy, 4-fluorophenoxy, 2,4-dichlorophenoxy, 3-trifluoromethylphenoxy, 2-methylphenoxy, 3-methoxyphenoxy, 4-methoxyphenoxy, 3-cyanophenoxy, or 4-cyanophenoxy group, more preferably the phenoxy, 2-fluorophenoxy, 4-fluorophenoxy, 4-methoxyphenoxy or 3-cyanophenoxy group.

In the present invention, the "(substituted or unsubstituted phenoxy)-C₁₋₆ alkyl group (said substituent(s) is (are) 1 to 5 substituents which are selected from the below-described substituent group A and may be the same or different)" represents, for example, a phenoxymethyl, methylphenoxymethyl, ethylphenoxymethyl, propylphenoxymethyl, isopropylphenoxymethyl, dimethylphenoxymethyl, trimethylphenoxymethyl, trifluoromethylphenoxymethyl, methoxyphenoxymethyl, dimethoxyphenoxymethyl, trimethoxyphenoxymethyl, ethoxyphenoxymethyl, isopropoxyphenoxymethyl, methylenedioxyphenoxymethyl, ethylenedioxyphenoxymethyl, cyanophenoxymethyl, nitrophenoxymethyl, chlorophenoxymethyl, dichlorophenoxymethyl, fluorophenoxymethyl, difluorophenoxymethyl, chloro-methylphenoxymethyl, fluoro-methylphenoxymethyl, methoxycarbonylphenoxymethyl, chloro-methoxyphenoxymethyl, acetylaminophenoxymethyl, 1-phenoxyethyl, 1-(methylphenoxy)ethyl, 1-(trifluoromethylphenoxy)ethyl, 1-(methoxyphenoxy)ethyl, 1-(dimethoxyphenoxy)ethyl, 1-(trimethoxyphenoxy)ethyl, 1-(isopropoxyphenoxy)ethyl, 1-(methylenedioxyphenoxy)ethyl, 1-(cyanophenoxy)ethyl, 1-(chlorophenoxy)ethyl, 1-(dichlorophenoxy)ethyl, 1-(fluorophenoxy)ethyl, 1-(methoxycarbonylphenoxy)ethyl, or 1-(chloro-methoxyphenoxy)ethyl group, preferably the phenoxymethyl, 2-fluorophenoxymethyl, 3-fluorophenoxymethyl, 4-fluorophenoxymethyl, 2,4-dichlorophenoxymethyl, 3-trifluoromethylphenoxymethyl, 2-methylphenoxymethyl, 3-methoxyphenoxymethyl, 4-methoxyphenoxymethyl, 3-cyanophenoxymethyl or 4-cyanophenoxymethyl group, more preferably the phenoxymethyl, 2-fluorophenoxymethyl, 4-fluorophenoxymethyl, 4-methoxyphenoxymethyl or 3-cyanophenoxymethyl group.

In the present invention, the "substituted or unsubstituted benzyloxy group (said substituent(s) is (are) 1 to 5 substituents which are selected from the below-described substituent group A and may be the same or different)" or "substituted or unsubstituted benzyloxy moiety (said substituent(s) is (are) 1 to 5 substituents which are selected from the below-described substituent group A and may be the same or different)" of the "(substituted or unsubstituted benzyloxy)-C₁₋₆ alkyl group (said substituent(s) is (are) 1 to 5 substituents which are selected from the below-described substituent group A and may be the same or different)" represents, for example, a benzyloxy, methylbenzyloxy, ethylbenzyloxy, propylbenzyloxy, isopropylbenzyloxy, dimethylbenzyloxy, trimethylbenzyloxy, trifluoromethylbenzyloxy, methoxybenzyloxy, dimethoxybenzyloxy, trimethoxybenzyloxy, ethoxybenzyloxy, isopropoxybenzyloxy, methylenedioxybenzyloxy, ethylenedioxybenzyloxy, cyanobenzyloxy, nitrobenzyloxy, chlorobenzyloxy, dichlorobenzyloxy, fluorobenzyloxy, difluorobenzyloxy, chloro-methylbenzyloxy, fluoro-methylbenzyloxy, methoxycarbonylbenzyloxy, chloro-methoxybenzyloxy, or acetylaminobenzyloxy group, preferably the benzyloxy, methylbenzyloxy, isopropylbenzyloxy, cyanobenzyloxy, nitrobenzyloxy, chlorobenzyloxy or methoxycarbonylbenzyloxy group, more preferably the benzyloxy group.

In the present invention, the "(substituted or unsubstituted benzyloxy)-C₁₋₆ alkyl group (said substituent(s) is (are) 1 to 5 substituents which are selected from the below-described substituent group A and may be the same or different)" represents, for example, a benzyloxymethyl, methylbenzyloxymethyl, ethylbenzyloxymethyl, propylbenzyloxymethyl, isopropylbenzyloxymethyl, dimethylbenzyloxymethyl, trimethylbenzyloxymethyl, trifluoromethylbenzyloxymethyl, methoxybenzyloxymethyl, dimethoxybenzyloxymethyl, trimethoxybenzyloxymethyl, ethoxybenzyloxymethyl, isopropoxybenzyloxymethyl, methylenedioxybenzyloxymethyl, ethylenedioxybenzyloxymethyl, cyanobenzyloxymethyl, nitrobenzyloxymethyl, chlorobenzyloxymethyl, dichlorobenzyloxymethyl, fluorobenzyloxymethyl, difluorobenzyloxymethyl, chloro-methylbenzyloxymethyl, fluoro-methylbenzyloxymethyl, methoxycarbonylbenzyloxymethyl, chloro-methoxybenzyloxymethyl or acetylaminobenzyloxymethyl group, preferably the benzyloxymethyl, methylbenzyloxymethyl, isopropylbenzyloxymethyl, cyanobenzyloxymethyl, nitrobenzyloxymethyl, chlorobenzyloxymethyl or methoxycarbonylbenzyloxymethyl group, more preferably the benzyloxymethyl group.

In the present invention, the "substituted or unsubstituted heteroaryloxy group (said substituent(s) is (are) 1 to 3 substituents which are selected from the below-described substituent group B and may be the same or different)" or the "substituted or unsubstituted heteroaryloxy moiety (said substituent(s) is (are) 1 to 3 substituents which are selected from the below-described substituent group B and may be the same or different)" of the "(substituted or unsubstituted heteroaryloxy)-C₁₋₆ alkyl group (said substituent(s) is (are) 1 to 3 substituents which are selected from the below-described substituent group B and may be the same or different)" represents, for example, a 2-pyridyloxy, (3-methyl-2-pyridyl)oxy, (6-methyl-2-pyridyl)oxy, (3-methoxy-2-pyridyl)oxy, (6-chloro-2-pyridyl)oxy, 3-pyridyloxy, 1-pyrazolyloxy, (4-methyl-1-pyrazolyl)oxy, 3-isoxazolyloxy, (5-methyl-3-isoxazolyl)oxy, (4,5-dimethyl-3-isoxazolyl)oxy, (4-chloro-5-phenyl-3-isoxazolyl)oxy, (5-methoxycarbonyl-3-isoxazolyl)oxy, {5-methyl-2-(1,3,4-thiadiazolyl)}oxy, 2-pyrazinyloxy, 6-quinolyloxy, 8-quinolyloxy, 4-quinazolyloxy, 2-pyrimidinyloxy, (4-methyl-2-pyrimidinyl)oxy, (3,5-dimethyl-2-pyrimidinyl)oxy, 4-pyrimidinyloxy, (2,6-dimethyl-4-pyrimidinyl)oxy, (6-methyl-2-isopropyl-4-pyrimidinyl)oxy, or 3-benzoisoxazolyloxy, preferably the 2-pyridyloxy, (3-methyl-2-pyridyl)oxy, (6-methyl-2-pyridyl)oxy, 3-pyridyloxy or (4-methyl-1-pyrazolyl)oxy, more preferably the 2-pyridyloxy, (3-methyl-2-pyridyl)oxy or 3-pyridyloxy group, still more preferably the 2-pyridyloxy group.

In the present invention, the "(substituted or unsubstituted heteroaryloxy)-C₁₋₆ alkyl group (said substituent(s) is (are) 1 to 3 substituents which are selected from the below-described substituent group B and may be the same or different)" represents, for example, 2-pyridyloxymethyl, (3-methyl-2-pyridyl)oxymethyl, (6-methyl-2-pyridyl)oxymethyl, (3-methoxy-2-pyridyl)oxymethyl, (4-chloro-2-pyridyl)oxymethyl, (3,5-dichloro-2-pyridyl)oxymethyl, (4-trifluoromethyl-2-pyridyl)oxymethyl, (3-cyano-6-methyl-2-pyridyl)oxymethyl, (4,6-dimethyl-3-cyano-2-pyridyl)oxymethyl, 3-pyridyloxymethyl, (2-chloro-3-pyridyl)oxymethyl, (5-chloro-3-pyridyl)oxymethyl, 1-pyrazolyloxymethyl, (4-methyl-1-pyrazolyl)oxymethyl, 5-pyrazolyloxymethyl, (4-methyl-5-pyrazolyl)oxymethyl, 3-isoxazolyloxymethyl, (5-methyl-3-isoxazolyl)oxymethyl, (5-isopropyl-3-isoxazolyl)oxymethyl, (4,5-dimethyl-3-isoxazolyl)oxymethyl, (4-fluoro-5-methyl-3-isoxazolyl)oxymethyl, (4-chloro-5-methyl-3-isoxazolyl)oxymethyl, (4-chloro-5-phenyl-3-isoxazolyl)oxymethyl, (5-methoxycarbonyl-3-isoxazolyl)oxymethyl, 2-(1,3,4-thiadiazolyl)oxymethyl, (5-methyl-2-(1,3,4-thiadiazolyl)}oxymethyl, 2-pyrazinyloxymethyl, 6-quinolyloxymethyl, 8-quinolyloxymethyl, 4-quinazolyloxymethyl 2-pyrimidinyloxymethyl, (4-methyl-2-pyrimidinyl)oxymethyl, (3,5-dimethyl-2-pyrimidinyl)oxymethyl, 4-pyrimidinyloxymethyl, (2,6-dimethyl-4-pyrimidinyl)oxymethyl, (6-methyl-2-isopropyl-4-pyrimidinyl)oxymethyl, or 3-benzoisoxazolyloxymethyl, preferably the 2-pyridyloxymethyl, (3-methyl-2-pyridyl)oxymethyl, (6-methyl-2-pyridyl)oxymethyl, (3-methoxy-2-pyridyl)oxymethyl,(2-chloro-3-pyridyl)oxymethyl, (5-chloro-3-pyridyl)oxymethyl, 1-pyrazolyloxymethyl, (4-methyl-1-pyrazolyl)oxymethyl, 3-isoxazolyloxymethyl, (5-methyl-3-isoxazolyl)oxymethyl, (4,5-dimethyl-3-isoxazolyl)oxymethyl, {5-methyl-2-(1,3,4-thiadiazolyl)}oxymethyl, 6-quinolyloxymethyl, (4-methyl-2-pyrimidinyl)oxymethyl or 4-pyrimidinyloxymethyl group, more preferably, the 2-pyridyloxymethyl, 1-pyrazolyloxymethyl, 3-isoxazolyloxymethyl, (5-methyl-3-isoxazolyl)oxymethyl or 6-quinolyloxymethyl group, still more preferably the 2-pyridyloxymethyl group.

In the present invention, the "(substituted or unsubstituted C₁₋₆ alkylamino)-C₁₋₆ alkyl group (said substituent is a phenyl group or a C₁₋₆ alkoxy group)" represents, for example, a methylaminomethyl, benzylaminomethyl, methoxymethylaminomethyl, ethoxymethylaminomethyl, hexyloxymethylaminomethyl, ethylaminomethyl, phenethylaminomethyl, 2-methoxyethylaminomethyl, 2-ethoxyethylaminomethyl, propylaminomethyl, isopropylaminomethyl, butylaminomethyl, isobutylaminomethyl, t-butylaminomethyl, hexylaminomethyl, 2-(methylamino)ethyl, 2-(benzylamino)ethyl, 2-(methoxymethylamino)ethyl, 2-(ethoxymethylamino)ethyl, 2-(ethylamino)ethyl, 2-(propylamino)ethyl, 2-(butylamino)ethyl, 1-(dimethylamino)ethyl, 2-(dimethylamino)ethyl, 3-(methylamino)propyl, 3-(ethylamino)propyl, 3-(dimethylamino)propyl, 4-(methylamino)butyl or 6-(methylamino)hexyl group, preferably the methylaminomethyl, benzylaminomethyl, methoxymethylaminomethyl, ethylaminomethyl, propylaminomethyl, isopropylaminomethyl, butylaminomethyl, isobutylaminomethyl or t-butylaminomethyl group, more preferably, the methylaminomethyl group.

In the present invention, the "C₃₋₆ alkenylamino group" or the C₃₋₆ alkenylamino moiety of the "(C₃₋₆ alkenylamino)-C₁₋₆ alkyl group" represents, for example, a linear or branched alkenylamino group having 1 to 6 carbon atoms, such as allylamino, 1-propenylamino, 2-methyl-1-propenylamino, 2-methyl-2-propenylamino, 3-butenylamino, 2-methyl-2-butenylamino or 4-pentenylamino group, preferably the allylamino group.

In the present invention, the "(C₃₋₆ alkenylamino)-C₁₋₆ alkyl group" represents, for example, the above-described "C₁₋₆ alkyl group" substituted with one above-described "C₃₋₆ alkenylamino group", such as allylaminomethyl, 1-propenylaminomethyl, 2-methyl-1-propenylaminomethyl, 2-methyl-2-propenylaminomethyl, 3-butenylaminomethyl, 2-methyl-2-butenylaminomethyl, 4-pentenylaminomethyl, 2-(allylamino)ethyl, 3-(allylamino)propyl, 4-(allylamino)butyl or 6-(allylamino)hexyl group, preferably the allylaminomethyl group.

In the present invention, the "(phenylamino)-C₁₋₆ alkyl group" represents, for example, the above-described "C₁₋₆ alkyl group" substituted with one phenylamino group, such as a phenylaminomethyl, 1-(phenylamino)ethyl, 2-(phenylamino)ethyl, 3-(phenylamino)propyl, 4-(phenylamino)butyl or 6-(phenylamino)hexyl group, preferably the phenylaminomethyl group.

In the present invention, the "N-(C₁₋₆ alkyl)anilino moiety of the {N-(C₁₋₆ alkyl)anilino}-C₁₋₆ alkyl group" represents, for example, an anilino group to which one above-described "C₁₋₆ alkyl group" is bonded at the nitrogen atom thereof, such as N-methylanilino, N-ethylanilino, N-propylanilino, N-isopropylanilino, N-butylanilino or N-hexylanilino group, preferably the N-methylanilino group.

In the present invention, the "{N-(C₁₋₆ alkyl)anilino}-C₁₋₆ alkyl group" represents, for example, the above-described "C₁₋₆ alkyl group" substituted with the above-described one "N-(C₁₋₆ alkyl)anilino group", such as N-methylanilinomethyl, N-ethylanilinomethyl, 1-(N-methylanilino)ethyl, 2-(N-methylanilino)ethyl, 3-(N-methylanilino)propyl or 6-(N-methylanilino)hexyl group, preferably the N-methylanilinomethyl group.

In the present invention, the "substituted or unsubstituted 5- or 6-membered nitrogen-containing saturated heterocyclic group (said substituent(s) is (are) 1 or 2 C₁₋₆ alkyl groups which may be the same or different, and said heterocyclic group may additionally include one ring oxygen atom or NH group" or the "substituted or unsubstituted 5- or 6-membered nitrogen-containing saturated heterocyclic moiety (said substituent(s) is (are) 1 or 2 C₁₋₆ alkyl groups which may be the same or different and said heterocyclic moiety may additionally include one ring oxygen atom or NH group) of the (substituted or unsubstituted 5- or 6-membered nitrogen-containing saturated heterocyclic)-C₁₋₆ alkyl group (said substituent(s) is (are) 1 or 2 C₁₋₆ alkyl groups which may be the same or different and the heterocyclic moiety of said heterocyclylalkyl may additionally include one ring oxygen atom or NH group)" represents, for example, the 1-pyrrolidinyl, piperidino, 1-piperazinyl, morpholino or 2,6-dimethylmorpholino group, preferably the piperidino, morpholino or 2,6-dimethylmorpholino group, more preferably the morpholino group.

In the present invention, the "(substituted or unsubstituted 5- or 6-membered nitrogen-containing saturated heterocyclyl)-C₁₋₆ alkyl group (said substituent(s) is (are) 1 or 2 C₁₋₆ alkyl groups which may be the same or different and the heterocyclic moiety of said heterocyclylalkyl group may additionally include one ring oxygen atom or NH group)" represents, for example, the 1-pyrrolidinylmethyl, 2,5-dimethyl-1-pyrrolidinylmethyl, piperidinomethyl, 2,6-dimethylpiperidinomethyl, 1-piperazinylmethyl, 2,6-dimethyl-1-piperazinylmethyl, morpholinomethyl, 3-methylmorpholinomethyl or 2,6-dimethylmorpholinomethyl group, preferably the 1-pyrrolidinylmethyl, piperidinomethyl or 6-dimethylmorpholinomethyl group.

In the present invention, the "substituted or unsubstituted C₁₋₆-alkylthio group (said substituent is a phenyl or C₁₋₆ alkoxy group)" or the "substituted or unsubstituted C₁₋₆ alkylthio moiety (said substituent is a phenyl or C₁₋₆ alkoxy group)" of the "(substituted or unsubstituted C₁₋₆ alkylthio)-C₁₋₆ alkyl group (said substituent is a phenyl or C₁₋₆ alkoxy group)" represents, for example, a methylthio, benzylthio, methoxymethylthio, ethoxymethylthio, hexyloxymethylthio, ethylthio, phenethylthio, 2-methoxyethylthio, 2-ethoxyethylthio, propylthio, isopropylthio, butylthio, isobutylthio, t-butylthio, or hexylthio group, preferably the methylthio, benzylthio, methoxymethylthio, ethylthio, propylthio, isopropylthio, butylthio, isobutylthio, or t-butylthio group, more preferably the methylthiomethyl group.

In the present invention, the "(substituted or unsubstituted C₁₋₆ alkylthio)-C₁₋₆ alkyl group (said substituent is a phenyl or C₁₋₆ alkoxy group)" represents, for example, a methylthiomethyl, benzylthiomethyl, methoxymethylthiomethyl, ethoxymethylthiomethyl, hexyloxymethylthiomethyl, ethylthiomethyl, phenethylthiomethyl, 2-methoxyethylthiomethyl, 2-ethoxyethylthiomethyl, propylthiomethyl, isopropylthiomethyl, butylthiomethyl, isobutylthiomethyl, t-butylthiomethyl, hexylthiomethyl, 2-(methylthio)ethyl, 2-(benzylthio)ethyl, 2-(methoxymethylthio)ethyl, 2-(ethoxymethylthio)ethyl, 2-(ethylthio)ethyl, 2-(propylthio)ethyl, 2-(butylthio)ethyl, 1-(dimethylthio)ethyl, 2-(dimethylthio)ethyl, 3-(methylthio)propyl, 3-(ethylthio)propyl, 3-(dimethylthio)propyl, 4-(methylthio)butyl or 6-(methylthio)hexyl group, preferably the methylthiomethyl, benzylthiomethyl, methoxymethylthiomethyl, ethylthiomethyl, propylthiomethyl, isopropylthiomethyl, butylthiomethyl, isobutylthiomethyl, or t-butylthiomethyl group, more preferably the methylthiomethyl group.

In the present invention, the "C₃₋₆ alkenylthio group" or the C₃₋₆ alkenylthio moiety of the "(C₃₋₆ alkenylthio)-C₁₋₆ alkyl group" represents a linear or branched alkenylthio group having 1 to 6 carbon atoms, such as allylthio, 1-propenylthio, 2-methyl-1-propenylthio, 2-methyl-2-propenylthio, 3-butenylthio, 2-methyl-2-butenylthio, or 4-pentenylthio group, preferably the allylthio group.

In the present invention, the "(C₃₋₆ alkenylthio)-C₁₋₆ alkyl group" represents, for example, the above-described "C₁₋₆ alkyl group" substituted with one above-described "C₃₋₆ alkenylthio group", such as the allylthiomethyl, 1-propenylthiomethyl, 2-methyl-1-propenylthiomethyl, 2-methyl-2-propenylthiomethyl, 3-butenylthiomethyl, 2-methyl-2-butenylthiomethyl, 4-pentenylthiomethyl, 2-(allylthio)ethyl, 3-(allylthio)propyl, 4-(allylthio)butyl or 6-(allylthio)hexyl group, preferably the allylthiomethyl group.

In the present invention, the "substituted or unsubstituted phenylthio group (said substituent(s) is (are) 1 to 5 substituents which are selected from the below-described substituent group A and may be the same or different)" or the "substituted or unsubstituted phenylthio moiety (said substituent(s) is (are) 1 to 5 substituents which are selected from the below-described substituent group A and may be the same or different) of the (substituted or unsubstituted phenylthio)-C₁₋₆ alkyl group (said substituent(s) is (are) 1 to 5 substituents which are selected from the below-described substituent group A and may be the same or different)" represents, for example, a phenylthio, methylphenylthio, ethylphenylthio, propylphenylthio, isopropylphenylthio, dimethylphenylthio, trimethylphenylthio, trifluoromethylphenylthio, methoxyphenylthio, dimethoxyphenylthio, trimethoxyphenylthio, ethoxyphenylthio, isopropoxyphenylthio, methylenedioxyphenylthio, ethylenedioxyphenylthio, cyanophenylthio, nitrophenylthio, chlorophenylthio, dichlorophenylthio, fluorophenylthio, difluorophenylthio, chloro-methylphenylthio, fluoro-methylphenylthio, methoxycarbonylphenylthio, chloro-methoxyphenylthio, or acetylaminophenylthio group, preferably the phenylthio, methylphenylthio, isopropylphenylthio, methoxyphenylthio, cyanophenylthio, nitrophenylthio, chlorophenylthio, or methoxycarbonylphenylthio group.

In the present invention, the "(substituted or unsubstituted phenylthio)-C₁₋₆ alkyl group (said substituent(s) is (are) 1 to 5 substituents which are selected from the below-described substituent group A and may be the same or different)" represents, for example, a phenylthiomethyl, methylphenylthiomethyl, ethylphenylthiomethyl, propylphenylthiomethyl, isopropylphenylthiomethyl, dimethylphenylthiomethyl, trimethylphenylthiomethyl, trifluoromethylphenylthiomethyl, methoxyphenylthiomethyl, dimethoxyphenylthiomethyl, trimethoxyphenylthiomethyl, ethoxyphenylthiomethyl, isopropoxyphenylthiomethyl, methylenedioxyphenylthiomethyl, ethylenedioxyphenylthiomethyl, cyanophenylthiomethyl, nitrophenylthiomethyl, chlorophenylthiomethyl, dichlorophenylthiomethyl, fluorophenylthiomethyl, difluorophenylthiomethyl, chloro-methylphenylthiomethyl, fluoro-methylphenylthiomethyl, methoxycarbonylphenylthiomethyl, chloro-methoxyphenylthiomethyl, acetylaminophenylthiomethyl, 1-phenylthioethyl, 1-(methylphenylthio)ethyl, 1-(trifluoromethylphenylthio)ethyl, 1-(methoxyphenylthio) ethyl, 1-(dimethoxyphenylthio)ethyl, 1-(trimethoxyphenylthio)ethyl, 1-(isopropoxyphenylthio)ethyl, 1-(methylenedioxyphenylthio)ethyl, 1-(cyanophenylthio)ethyl, 1-(chlorophenylthio)ethyl, 1-(dichlorophenylthio)ethyl, 1-(fluorophenylthio)ethyl, 1-(methoxycarbonylphenylthio)ethyl or 1-(chloro-methoxyphenylthio)ethyl group, preferably, the phenylthiomethyl, methylphenylthiomethyl, isopropylphenylthiomethyl, methoxyphenylthiomethyl, cyanophenylthiomethyl, nitrophenylthiomethyl, chlorophenylthiomethyl or methoxycarbonylphenylthiomethyl group.

In the present invention, the "substituted or unsubstituted heteroarylthio group (said substituent(s) is (are) 1 to 3 substituents which are selected from the below-described substituent group B and may be the same or different)" or the "substituted or unsubstituted heteroarylthio moiety (said substituent(s) is (are) 1 to 3 substituents which are selected from the below-described substituent group B and may be the same or different) of the (substituted or unsubstituted heteroarylthio)-C₁₋₆ alkyl group (said substituent(s) is (are) 1 to 3 substituents which are selected from the below-described substituent group B and may be the same or different)" represents, for example, a 2-oxo-1-pyridylthio, 2-pyridylthio, 2-pyrimidinylthio, 2-imidazolylthio, 1-methyl-2-imidazolylthio, 2-(1,3,4-thiadiazolyl)thio, 5-methyl-2-(1,3,4-thiadiazolyl)thio, 6-trifluoromethyl-2-pyridylthio, 3-methoxy-2-pyridylthio, 3-cyano-2-pyridylthio, 4-chloro-5-phenyl-3-isoxazolylthio, or 5-methoxycarbonyl-3-isoxazolylthio group, preferably the 2-oxo-1-pyridylthio, 2-pyridylthio, 1-methyl-2-imidazolylthio, or 5-methyl-2-(1,3,4-thiadiazolyl)thio group, more preferably the 2-oxo-1-pyridylthio, 1-methyl-2-imidazolylthio, or 5-methyl-2-(1,3,4-thiadiazolyl)thio group.

In the present invention, the "(substituted or unsubstituted heteroarylthio)-C₁₋₆ alkyl group (said substituent(s) is (are) 1 to 3 substituents which are selected from the below-described substituent group B and may be the same or different)" represents, for example, a 1-pyridylthiomethyl, (2-oxo-1-pyridyl)thiomethyl, 2-pyridylthiomethyl, 2-pyrimidinylthiomethyl, 2-imidazolylthiomethyl, (1-methyl-2-imidazolyl)thiomethyl, 2-(1,3,4-thiadiazolyl)thiomethyl, {5-methyl-2-(1,3,4-thiadiazolyl)}thiomethyl, (6-trifluoromethyl-2-pyridyl)thiomethyl, (3-methoxy-2-pyridyl)thiomethyl, (3-cyano-2-pyridyl)thiomethyl, (4-chloro-5-phenyl-3-isoxazolyl)thiomethyl, 2-pyrimidylthiomethyl or (5-methoxycarbonyl-3-isoxazolyl)thiomethyl group, preferably the (2-oxo-1-pyridyl)thiomethyl, 2-pyridylthiomethyl, (1-methyl-2-imidazolyl)thiomethyl, 2-pyrimidylthiomethyl or {5-methyl-2-(1,3,4-thiadiazolyl)}thiomethyl group, more preferably, the (1-methyl-2-imidazolyl)thiomethyl, 2-pyrimidylthiomethyl or {5-methyl-2-(1,3,4-thiadiazolyl)}thiomethyl group, still more preferably the (1-methyl-2-imidazolyl)thiomethyl or {5-methyl-2-(1,3,4-thiadiazolyl)}thiomethyl group.

In the present invention, the "(C₂₋₇ alkoxycarbonyl)-C₁₋₆ alkyl group" represents, for example, the above-described "C₁₋₆ alkyl group" substituted with one above-described "C₂₋₇ alkoxycarbonyl group", such as the methoxycarbonylmethyl, 1-(methoxycarbonyl)ethyl, 2-(methoxycarbonyl)ethyl, 3-(methoxycarbonyl)propyl, 6-(methoxycarbonyl)hexyl, ethoxycarbonylmethyl, 2-(ethoxycarbonyl)ethyl, isopropoxycarbonylmethyl or hexyloxycarbonylmethyl group, preferably the methoxycarbonylmethyl, 2-(methoxycarbonyl)ethyl, ethoxycarbonylmethyl or 2-(ethoxycarbonyl)ethyl group, more preferably the 2-(methoxycarbonyl)ethyl group.

In the present invention, the "substituted or unsubstituted heteroaralkyl group (said substituent(s) is (are) 1 to 3 substituents which are selected from the below-described substituent group B and may be the same or different)" represents for example, 2-pyridylmethyl, (2-oxo-1-pyridyl)methyl, (3-methyl-2-oxo-1-pyridyl)methyl, (5-methyl-2-oxo-1-pyridyl)methyl, (3-methoxy-2-oxo-1-pyridyl)methyl, (3,5-dichloro-2-oxo-1-pyridyl)methyl, (5-trifluoromethyl-2-oxo-1-pyridyl)methyl, (4-oxo-1-pyridyl)methyl, (3,5-dichloro-4-oxo-1-pyridyl)methyl, 2-pyridylmethyl, 3-pyridylmethyl, 4-pyridylmethyl, 1-pyrazolylmethyl, (4-methyl-1-pyrazolyl)methyl, (3,5-dimethyl-1-pyrazolyl)methyl, (4-bromo-1-pyrazolyl)methyl, 1-imidazolylmethyl, (2-methyl-1-imidazolyl)methyl, (4,5-dichloro-1-imidazolyl)methyl, {4,5-di(methoxycarbonyl)-1-imidazolyl}methyl, 3-isoxazolylmethyl, (5-methyl-3-isoxazolyl)methyl, 1-(1,2,4-triazolyl)methyl, 2-pyrimidinylmethyl, (2-oxo-1-pyrimidinyl)methyl, or 2-furylmethyl group, preferably the (2-oxo-1-pyridyl)methyl, 2-pyridylmethyl, 3-pyridylmethyl, (3,5-dimethyl-1-pyrazolyl)methyl, (4-bromo-1-pyrazolyl)methyl or (5-methyl-3-isoxazolyl)methyl group, more preferably the 2-pyridylmethyl, 3-pyridylmethyl or (5-methyl-3-isoxazolyl)methyl group, still more preferably the 2-pyridylmethyl group.

In the present invention, the "substituted or unsubstituted heteroaryl group (the substituent(s) is (are) 1 to 3 substituents which are selected from the below-described substituent group B and may be the same or different)" represents, for example, a 2-furyl, 2-thienyl, 5-(1,2,3-thiadiazolyl), 4-methyl-5-(1,2,3-thiadiazolyl), 4-pyridyl, 2,6-dichloro-4-pyridyl, 1-imidazolyl, 4,5-dichloro-1-imidazolyl, 2-pyrrolyl or 1-methyl-2-pyrrolyl group, preferably the 2-furyl, 2-thienyl, 2,6-dichloro-4-pyridyl or 1-methyl-2-pyrrolyl group.

In the present invention, the "(C₁₋₆ alkoxy)-C₁₋₆ alkoxy group" represents, for example, the above-described "C₁₋₆ alkoxy group" substituted with the above-described one "C₁₋₆ alkoxy group", such as methoxymethoxy, 2-methoxyethoxy, 3-methoxypropoxy, 4-methoxybutoxy, 5-methoxypentyloxy, ethoxymethoxy, (2-ethoxy)ethoxy, propoxymethoxy, isopropoxymethoxy, butoxymethoxy, isobutoxymethoxy, t-butoxymethoxy, pentyloxymethoxy or hexyloxymethoxy group, preferably the above-described "C₁₋₂ alkoxy group" substituted, at the alkoxy moiety therof, with 1 to 3 alkoxy groups, more preferably the methoxymethoxy, (2-methoxy)ethoxy or ethoxymethoxy group, more preferably, the (2-methoxy)ethoxy group.

In the present invention, the "(C₁₋₆ alkylamino)-C₁₋₆ alkoxy group" represents, for example, a methylaminomethoxy, benzylaminomethoxy, methoxymethylaminomethoxy, ethoxymethylaminomethoxy, hexyloxymethylaminomethoxy, ethylaminomethoxy, phenethylaminomethoxy, 2-methoxyethylaminomethoxy, 2-ethoxyethylaminomethoxy, propylaminomethoxy, isopropylaminomethoxy, butylaminomethoxy, isobutylaminomethoxy, t-butylaminomethoxy, hexylaminomethoxy, 2-(methylamino)ethoxy, 2-(benzylamino)ethoxy, 2-(methoxymethylamino)ethoxy, 2-(ethoxymethylamino)ethoxy, 2-(ethylamino)ethoxy, 2-(propylamino)ethoxy, 2-(butylamino)ethoxy, 1-(dimethylamino)ethoxy, 2-(dimethylamino)ethoxy, 3-(methylamino)propoxy, 3-(ethylamino)propoxy, 3-(dimethylamino)propoxy, 4-(methylamino)butoxy or 6-(methylamino)hexyloxy group, preferably, the methylaminomethoxy, benzylaminomethoxy, methoxymethylaminomethoxy, ethylaminomethoxy, propylaminomethoxy, isopropylaminomethoxy, butylaminomethoxy, isobutylaminomethoxy or t-butylaminomethoxy group, more preferably, the methylaminomethoxy or ethylaminomethoxy group, still more preferably, the methylaminomethoxy group.

In the present invention, the "di(C₁₋₆ alkyl)amino-C₁₋₆ alkoxy group" represents, for example, the above-described "C₁₋₆ alkoxy group" substituted with the above-described one "di(C₁₋₆ alkyl)amino group", such as the dimethylaminomethoxy, diethylaminomethoxy, ethylmethylaminomethoxy, dipropylaminomethoxy, dihexylaminomethoxy, 1-(dimethylamino)ethoxy, 2-(dimethylamino)ethoxy, 3-(dimethylamino)propoxy, or 6-(dimethylamino)hexyloxy group, preferably the dimethylaminomethoxy, diethylaminomethoxy or ethylmethylaminomethoxy group, more preferably the dimethylaminomethoxy group.

In the present invention, the "substituted or unsubstituted heteroaralkyloxy group (said substituent(s) is (are) 1 to 3 substituents which are selected from the below-described substituent group B and may be the same or different) represents, for example, a 2-pyridylmethoxy, 3-pyridylmethoxy, 4-pyridylmethoxy, 1-pyrazolylmethoxy, 1-imidazolylmethoxy, 3-isoxazolylmethoxy, 1-(1,2,4-triazolyl)methoxy, 2-pyrimidinylmethoxy, 2-furylmethoxy, (6-trifluoromethyl-2-pyridyl)methoxy, (5-phenyl-2-methyl-3-furyl)methoxy, (6-chloro-2-pyridyl)methoxy, (5-cyano-3-methyl-1-pyrazolyl)methoxy or (5-methoxycarbonyl-1-pyrazolyl)methoxy group, preferably, the 2-pyridylmethoxy group.

In the present invention, the "C₃₋₇ cycloalkoxy group" represents, for example, a cyclopropoxy, cyclobutoxy, cyclopentyloxy, cyclohexyloxy or cycloheptyloxy group, preferably the cyclopropoxy, cyclobutoxy, or cyclohexyloxy group, more preferably the cyclopropoxy group.

In the present invention, the "substituted or unsubstituted C₁₋₆ alkylamino group (said substituent is a phenyl or C₁₋₆ alkoxy group)" represents, for example, the methylamino, benzylamino, methoxymethylamino, ethoxymethylamino, hexyloxymethylamino, ethylamino, phenethylamino, 2-methoxyethylamino, 2-ethoxyethylamino, propylamino, isopropylamino, butylamino, isobutylamino, t-butylamino or hexylamino group, preferably the methylamino, benzylamino, methoxymethylamino, ethylamino, 2-methoxyethylamino, propylamino, isopropylamino, butylamino, isobutylamino or t-butylamino group.
(1) In the present invention, R¹ is preferably a C₁₋₄ alkyl group, a cyclohexyl group or a phenyl group; more preferably, a C₁₋₂ alkyl group;
   still more preferably, a methyl group.
(2) In the present invention, R² is preferably a hydrogen atom or a C₁₋₂ alkyl group;
   more preferably, a hydrogen atom or a methyl group; still more preferably, a hydrogen atom.
(3) In the present invention, R³ is preferably a C₁₋₅ alkyl, cyanomethyl, 2-cyanoethyl, hydroxymethyl, 2-hydroxyethyl, methoxymethyl, ethoxymethyl, 2-methoxyethyl, acetoxymethyl, methylaminomethyl, ethylaminomethyl, propylaminomethyl, isopropylaminomethyl, butylaminomethyl, isobutylaminomethyl, t-butylaminomethyl, dimethylaminomethyl, diethylaminomethyl, ethylmethylaminomethyl, 1-pyrrolidinylmethyl, piperidinomethyl, (C₁₋₂ alkyl-C₃₋₆ cycloalkyl)-C₁₋₂ alkyl, (fluoro-C₃₋₆ cycloalkyl)-C₁₋₂ alkyl, (difluoro-C₃₋₆ cycloalkyl)-C₁₋₂ alkyl, tetrahydrofuranyl-C₁₋₂ alkyl, tetrahydropyranyl-C₁₋₂ alkyl, halo-C₁₋₄ alkyl (said halogen substituent(s) is (are) 1 to 3 fluorine atoms or chlorine atoms which are the same), benzyl, methylbenzyl, isopropylbenzyl, methoxybenzyl, cyanobenzyl, nitrobenzyl, chlorobenzyl, methoxycarbonylbenzyl, 3-thienylmethyl, acetyl, cyclohexyl, 3-butenyl, phenyl, methylphenyl, isopropylphenyl, methoxyphenyl, cyanophenyl, nitrophenyl, chlorophenyl, fluorophenyl or methoxycarbonylphenyl group;
   more preferably, a C₄₋₅ alkyl group, cyclobutylmethyl, (methylcyclobutyl)methyl, tetrahydrofuranylmethyl, cyclopentylmethyl, (methylcyclopentyl)methyl, (fluorocyclopentyl)methyl, (difluorocyclopentyl)methyl, tetrahydropyranylmethyl, cyclohexylmethyl, cyclopentenylmethyl, chloromethyl, 1,1-difluoroisobutyl, 3,3,3-trifluoro-2-trifluoromethylpropyl, 3,3,3-trifluoro-2-methylpropyl, 3,3,3-trifluoro-2,2-dimethylpropyl or cyclopentyl group;
   still more preferably, a branched C₄₋₅ alkyl, cyclobutylmethyl, (methylcyclobutyl)methyl, tetrahydrofuranyl, cyclopentylmethyl, (fluorocyclopentyl)methyl, (difluorocyclopentyl)methyl, tetrahydropyranylmethyl, cyclohexylmethyl, cyclopent-2-enylmethyl, cyclopent-3-enylmethyl, 1,1-difluoroisobutyl, 3,3,3-trifluoro-2-trifluoromethylpropyl, 3,3,3-trifluoro-2-methylpropyl, or 3,3,3-trifluoro-2,2-dimethylpropyl group;
   particularly preferably, the isobutyl, cyclobutylmethyl, tetrahydrofuranylmethyl, cyclopentylmethyl, 3,3,3-trifluoro-2-methylpropyl or 3,3,3-trifluoro-2,2-dimethylpropyl group.
(4) In the present invention, R⁴ is preferably a hydrogen, fluorine or chlorine atom, or a C₁₋₄ alkyl, cyanomethyl, 2-cyanoethyl, hydroxymethyl, hydroxyethyl, hydroxypropyl, trifluoromethyl, trichloromethyl, difluoromethyl, dichloromethyl, fluoromethyl, chloromethyl, bromomethyl, (C₁₋₄ alkoxy)-C₁₋₂ alkyl, methoxymethoxymethyl, (2-methoxy)ethoxymethyl, 2-(methoxymethoxy)ethyl, allyloxymethyl, 2-allyloxyethyl, butyryloxymethyl, isobutyryloxymethyl, pivaloyloxymethyl, cyclopropylcarbonyloxymethyl, 2-methyl-1-butenoyloxymethyl, 2-methyl-2-butenoyloxymethyl, methoxyacetoxymethyl, 2-methoxybutyryloxymethyl, ethoxyacetoxymethyl, methoxycarbonyloxymethyl, ethoxycarbonyloxymethyl, 2-(methoxycarbonyloxy)ethyl, methoxymethoxycarbonyloxymethyl, 2-methoxyethoxycarbonyloxymethyl, phenoxymethyl, 2-fluorophenoxymethyl, 3-fluorophenoxymethyl, 4-fluorophenoxymethyl, 2,4-dichlorophenoxymethyl, 3-trifluoromethylphenoxymethyl, 2-methylphenoxymethyl, 3-methoxyphenoxymethyl, 4-methoxyphenoxymethyl, 3-cyanophenoxymethyl, 4-cyanophenoxymethyl, benzyloxy, methylbenzyloxy, isopropylbenzyloxy, cyanobenzyloxy, nitrobenzyloxy, chlorobenzyloxy, methoxycarbonylbenzyloxy, 2-pyridyloxymethyl, (3-methyl-2-pyridyl)oxymethyl, (6-methyl-2-pyridyl)oxymethyl, (3-methoxy-2-pyridyl)oxymethyl, (2-chloro-3-pyridyl)oxymethyl, (5-chloro-3-pyridyl)oxymethyl, 1-pyrazolyloxymethyl, (4-methyl-1-pyrazolyl)oxymethyl, 3-isoxazolyloxymethyl, (5-methyl-3-isoxazolyl)oxymethyl, (4,5-dimethyl-3-isoxazolyl)oxymethyl, {5-methyl-2-(1,3,4-thiadiazolyl)}oxymethyl, 6-quinolyloxymethyl, (4-methyl-2-pyrimidinyl)oxymethyl, 4-pyrimidinyloxymethyl, methylaminomethyl, benzylaminomethyl, methoxymethylaminomethyl, ethylaminomethyl, propylaminomethyl, isopropylaminomethyl, butylaminomethyl, isobutylaminomethyl, t-butylaminomethyl, allylaminomethyl, phenylaminomethyl, N-methylanilinomethyl, dimethylaminomethyl, diethylaminomethyl, ethylmethylaminomethyl, piperidinomethyl, morpholinomethyl, 2,6-dimethylmorpholinomethyl, methylthiomethyl, benzylthiomethyl, methoxymethylthiomethyl, ethylthiomethyl, propylthiomethyl, isopropylthiomethyl, butylthiomethyl, isobutylthiomethyl, t-butylthiomethyl, allylthiomethyl, phenylthiomethyl, methylphenylthiomethyl, isopropylphenylthiomethyl, methoxyphenylthiomethyl, cyanophenylthiomethyl, nitrophenylthiomethyl, chlorophenylthiomethyl, methoxycarbonylphenylthiomethyl, (2-oxo-1-pyridyl)thiomethyl, 2-pyridylthiomethyl, (1-methyl-2-imidazolyl)thiomethyl, 2-pyrimidylthiomethyl, {5-methyl-2-(1,3,4-thiadiazolyl)}thiomethyl, methoxycarbonylmethyl, 2-(methoxycarbonyl)ethyl, ethoxycarbonylmethyl, 2-(ethoxycarbonyl)ethyl, benzyl, methylbenzyl, isopropylbenzyl, methoxybenzyl, cyanobenzyl, nitrobenzyl, chlorobenzyl, fluorobenzyl, methoxycarbonylbenzyl, 2-pyridylmethyl, (2-oxo-1-pyridyl)methyl, (3-methyl-2-oxo-1-pyridyl)methyl, (5-methyl-2-oxo-1-pyridyl)methyl, (3-methoxy-2-oxo-1-pyridyl)methyl, (3,5-dichloro-2-oxo-1-pyridyl)methyl, (5-trifluoromethyl-2-oxo-1-pyridyl)methyl, (4-oxo-1-pyridyl)methyl, (3,5-dichloro-4-oxo-1-pyridyl)methyl, 2-pyridylmethyl, 3-pyridylmethyl, 4-pyridylmethyl, 1-pyrazolylmethyl, (4-methyl-1-pyrazolyl)methyl, (3,5-dimethyl-1-pyrazolyl)methyl, (4-bromo-1-pyrazolyl)methyl, 1-imidazolylmethyl, (2-methyl-1-imidazolyl)methyl, (4,5-dichloro-1-imidazolyl)methyl, {4,5-di(methoxycarbonyl)-1-imidazolyl}methyl, 3-isoxazolylmethyl, (5-methyl-3-isoxazolyl)methyl, 1-(1,2,4-triazolyl)methyl, 2-pyrimidinylmethyl, (2-oxo-1-pyrimidinyl)methyl, 2-furylmethyl, C₃₋₆ cycloalkyl, phenyl, methylphenyl, isopropylphenyl, methoxyphenyl, cyanophenyl, nitrophenyl, chlorophenyl, methoxycarbonylphenyl, 2-furyl, 2-thienyl, 2,6-dichloro-4-pyridyl, 1-methyl-2-pyrrolyl, C₃₋₅ alkenyl, methoxycarbonyl, ethoxycarbonyl, C₁₋₄ alkoxy, methoxymethoxy, (2-methoxy)ethoxy, ethoxymethoxy, methylaminomethoxy, ethylaminomethoxy, dimethylaminomethoxy, diethylaminomethoxy, ethylmethylaminomethoxy, 2-pyridylmethoxy, 3-pyridylmethoxy, 4-pyridylmethoxy, 1-pyrazolylmethoxy, 1-imidazolylmethoxy, 3-isoxazolylmethoxy, 1-(1,2,4-triazolyl)methoxy, 2-pyrimidinylmethoxy, 2-furylmethoxy, (6-trifluoromethyl-2-pyridyl)methoxy, (5-phenyl-2-methyl-3-furyl)methoxy, (6-chloro-2-pyridyl)methoxy, (5-cyano-3-methyl-1-pyrazolyl)methoxy, (5-methoxycarbonyl-1-pyrazolyl)methoxy, cyclopropoxy, cyclobutoxy, cyclohexyloxy, allyloxy, phenoxy, 2-fluorophenoxy, 3-fluorophenoxy, 4-fluorophenoxy, 2,4-dichlorophenoxy, 3-trifluoromethylphenoxy, 2-methylphenoxy, 3-methoxyphenoxy, 4-methoxyphenoxy, 3-cyanophenoxy, 4-cyanophenoxy, benzyloxy, methylbenzyloxy, isopropylbenzyloxy, cyanobenzyloxy, nitrobenzyloxy, chlorobenzyloxy, methoxycarbonylbenzyloxy, 2-pyridyloxy, (3-methyl-2-pyridyl)oxy, (6-methyl-2-pyridyl)oxy, (3-methoxy-2-pyridyl)oxy, (6-chloro-2-pyridyl)oxy, 3-pyridyloxy, 1-pyrazolyloxy, (4-methyl-1-pyrazolyl)oxy, 3-isoxazolyloxy, (5-methyl-3-isoxazolyl)oxy, (4,5-dimethyl-3-isoxazolyl)oxy, (4-chloro-5-phenyl-3-isoxazolyl)oxy, (5-methoxycarbonyl-3-isoxazolyl)oxy, {5-methyl-2-(1,3,4-thiadiazolyl))oxy, 2-pyrazinyloxy, 6-quinolyloxy, 8-quinolyloxy, 4-quinazolyloxy, 2-pyrimidinyloxy, (4-methyl-2-pyrimidinyl)oxy, (3,5-dimethyl-2-pyrimidinyl)oxy, 4-pyrimidinyloxy, (2,6-dimethyl-4-pyrimidinyl)oxy, (6-methyl-2-isopropyl-4-pyrimidinyl)oxy, 3-benzoisoxazolyloxy, methylaminomethyl, benzylaminomethyl, methoxymethylaminomethyl, ethylaminomethyl, propylaminomethyl, isopropylaminomethyl, butylaminomethyl, isobutylaminomethyl, t-butylaminomethyl, allylamino, dimethylamino, diethylamino, ethylmethylamino, piperidino, morpholino, 2,6-dimethylmorpholino, methylthio, benzylthio, methoxymethylthio, ethylthio, propylthio, isopropylthio, butylthio, isobutylthio, t-butylthio, allylthio, phenylthio, methylphenylthio, isopropylphenylthio, methoxyphenylthio, cyanophenylthio, nitrophenylthio, chlorophenylthio, methoxycarbonylphenylthio, 2-oxo-1-pyridylthio, 2-pyridylthio, 1-methyl-2-imidazolylthio or 5-methyl-2-(1,3,4-thiadiazolyl)thio group;
   more preferably, a C₁₋₂ alkyl, methoxymethyl, ethoxymethyl, 2-methoxyethyl, butyryloxymethyl, isobutyryloxymethyl, pivaloyloxymethyl, cyclopropylcarbonyloxymethyl, methoxyacetoxymethyl, methoxycarbonyloxymethyl, 2-methoxyethoxycarbonyloxymethyl, phenoxymethyl, 2-pyridyloxymethyl, 1-pyrazolyloxymethyl, 3-isoxazolyloxymethyl, (5-methyl-3-isoxazolyl)oxymethyl, 6-quinolyloxymethyl, (1-methyl-2-imidazolyl)thiomethyl, 2-pyrimidylthiomethyl, {5-methyl-2-(1,3,4-thiadiazolyl)}thiomethyl, methoxybenzyl, fluorobenzyl, (2-oxo-1-pyridyl)methyl, 2-pyridylmethyl, 3-pyridylmethyl, (3,5-dimethyl-1-pyrazolyl)methyl, (4-bromo-1-pyrazolyl)methyl, (5-methyl-3-isoxazolyl)methyl, cyclopropyl, cyclobutyl, methoxy, ethoxy, isopropoxy, 2-pyridylmethoxy, phenoxy, 2-pyridyloxy, (3-methyl-2-pyridyl)oxy, (6-methyl-2-pyridyl)oxy, 3-pyridyloxy or (4-methyl-1-pyrazolyl)oxy group;
   still more preferably, a C₁₋₂ alkyl, methoxymethyl, cyclopropylcarbonyloxymethyl, phenoxymethyl, 2-pyridyloxymethyl, (1-methyl-2-imidazolyl)thiomethyl, {5-methyl-2-(1,3,4-thiadiazolyl)}thiomethyl, fluorobenzyl, 2-pyridylmethyl, 3-pyridylmethyl, (5-methyl-3-isoxazolyl)methyl, cyclopropyl, cyclobutyl, methoxy, ethoxy, isopropoxy, 2-pyridylmethoxy, phenoxy, 2-pyridyloxy, (3-methyl-2-pyridyl)oxy, or 3-pyridyloxy group;
   particularly preferably, the methyl, methoxymethyl, 2-pyridyloxymethyl, methoxy, phenoxy, or 2-pyridyloxy group.
(5) In the present invention, Y preferably represents the methyl group, ethyl group, methoxy group, ethoxy group, cyano group, nitro group, fluorine atom, chlorine atom, bromine atom, methoxycarbonyl group or ethoxycarbonyl group, and
   n is 0 or 1, preferably 0.
When the compound (I) of the present invention has, in the molecule thereof, a hydroxyl group, it can be converted into the corresponding alkali metal salt, alkaline earth metal salt or ammonium salt. When it has, in the molecule thereof, a base portion, it can be converted into the corresponding salt such as sulfate, hydrochloride, nitrate or phosphate. Such salts are encompassed in the present invention, provided that they are usable as an agricultural or horticultural fungicide.

In the present invention, the "alkali metal salt" means, for example, a sodium salt, potassium salt or lithium salt, preferably a sodium salt or potassium salt.

In the present invention, the "alkaline earth metal salt" means, for example, a calcium salt or magnesium salt, preferably a calcium salt.

Hydrates of the compounds of the present invention are also encompassed in the present invention.

Some of the compounds of the present invention have an asymmetric carbon. In this case, the invention according to the present application encompasses a pure optically active substance or a mixture of several optically active substances in any ratio.

The compounds of formula (I) of the present invention preferably have:
(a1) as R¹, a C₁₋₄ alkyl, cyclohexyl or phenyl group;
(a2) as R², a hydrogen atom or C₁₋₂ alkyl group;
(a3) as R³, a C₁₋₅ alkyl, cyanomethyl, 2-cyanoethyl, hydroxymethyl, 2-hydroxyethyl, methoxymethyl, ethoxymethyl, 2-methoxyethyl, acetoxymethyl, methylaminomethyl, ethylaminomethyl, propylaminomethyl, isopropylaminomethyl, butylaminomethyl, isobutylaminomethyl, t-butylaminomethyl, dimethylaminomethyl, diethylaminomethyl, ethylmethylaminomethyl, 1-pyrrolidinylmethyl, piperidinomethyl, (C₁₋₂ alkyl-C₃₋₆ cycloalkyl)-C₁₋₂ alkyl, (fluoro-C₃₋₆ cycloalkyl)-C₁₋₂ alkyl, (difluoro-C₃₋₆ cycloalkyl)-C₁₋₂ alkyl, tetrahydrofuranyl-C₁₋₂ alkyl, tetrahydropyranyl-C₁₋₂ alkyl, halo-C₁₋₄ alkyl (said halogen substituent(s) is (are) 1 to 3 fluorine atoms or chlorine atoms which are the same), benzyl, methylbenzyl, isopropylbenzyl, methoxybenzyl, cyanobenzyl, nitrobenzyl, chlorobenzyl, methoxycarbonylbenzyl, 3-thienylmethyl, acetyl, cyclohexyl, 3-butenyl, phenyl, methylphenyl, isopropylphenyl, methoxyphenyl, cyanophenyl, nitrophenyl, chlorophenyl, fluorophenyl or methoxycarbonylphenyl group;
(a4) as R⁴, a hydrogen, fluorine or chlorine atom, or a C₁₋ ₄ alkyl, cyanomethyl, 2-cyanoethyl, hydroxymethyl, hydroxyethyl, hydroxypropyl, trifluoromethyl, trichloromethyl, difluoromethyl, dichloromethyl, fluoromethyl, chloromethyl, bromomethyl, (C₁₋₄ alkoxy)-C₁₋₂ alkyl, methoxymethoxymethyl, (2-methoxy)ethoxymethyl, 2-(methoxymethoxy)ethyl, allyloxymethyl, 2-allyloxyethyl, butyryloxymethyl, isobutyryloxymethyl, pivaloyloxymethyl, cyclopropylcarbonyloxymethyl, 2-methyl-1-butenoyloxymethyl, 2-methyl-2-butenoyloxymethyl, methoxyacetoxymethyl, 2-methoxybutyryloxymethyl, ethoxyacetoxymethyl, methoxycarbonyloxymethyl, ethoxycarbonyloxymethyl, 2-(methoxycarbonyloxy)ethyl, methoxymethoxycarbonyloxymethyl, 2-methoxyethoxycarbonyloxymethyl, phenoxymethyl, 2-fluorophenoxymethyl, 3-fluorophenoxymethyl, 4-fluorophenoxymethyl, 2,4-dichlorophenoxymethyl, 3-trifluoromethylphenoxymethyl, 2-methylphenoxymethyl, 3-methoxyphenoxymethyl, 4-methoxyphenoxymethyl, 3-cyanophenoxymethyl, 4-cyanophenoxymethyl, benzyloxy, methylbenzyloxy, isopropylbenzyloxy, cyanobenzyloxy, nitrobenzyloxy, chlorobenzyloxy, methoxycarbonylbenzyloxy, 2-pyridyloxymethyl, (3-methyl-2-pyridyl)oxymethyl, (6-methyl-2-pyridyl)oxymethyl, (3-methoxy-2-pyridyl)oxymethyl, (2-chloro-3-pyridyl)oxymethyl, (5-chloro-3-pyridyl)oxymethyl, 1-pyrazolyloxymethyl, (4-methyl-1-pyrazolyl)oxymethyl, 3-isoxazolyloxymethyl, (5-methyl-3-isoxazolyl)oxymethyl, (4,5-dimethyl-3-isoxazolyl)oxymethyl, {5-methyl-2-(1,3,4-thiadiazolyl)}oxymethyl, 6-quinolyloxymethyl, (4-methyl-2-pyrimidinyl)oxymethyl, 4-pyrimidinyloxymethyl, methylaminomethyl, benzylaminomethyl, methoxymethylaminomethyl, ethylaminomethyl, propylaminomethyl, isopropylaminomethyl, butylaminomethyl, isobutylaminomethyl, t-butylaminomethyl, allylaminomethyl, phenylaminomethyl, N-methylanilinomethyl, dimethylaminomethyl, diethylaminomethyl, ethylmethylaminomethyl, piperidinomethyl, morpholinomethyl, 2,6-dimethylmorpholinomethyl, methylthiomethyl, benzylthiomethyl, methoxymethylthiomethyl, ethylthiomethyl, propylthiomethyl, isopropylthiomethyl, butylthiomethyl, isobutylthiomethyl, t-butylthiomethyl, allylthiomethyl, phenylthiomethyl, methylphenylthiomethyl, isopropylphenylthiomethyl, methoxyphenylthiomethyl, cyanophenylthiomethyl, nitrophenylthiomethyl, chlorophenylthiomethyl, methoxycarbonylphenylthiomethyl, (2-oxo-1-pyridyl)thiomethyl, 2-pyridylthiomethyl, (1-methyl-2-imidazolyl)thiomethyl, 2-pyrimidylthiomethyl, {5-methyl-2-(1,3,4-thiadiazolyl)}thiomethyl, methoxycarbonylmethyl, 2-(methoxycarbonyl)ethyl, ethoxycarbonylmethyl, 2-(ethoxycarbonyl)ethyl, benzyl, methylbenzyl, isopropylbenzyl, methoxybenzyl, cyanobenzyl, nitrobenzyl, chlorobenzyl, fluorobenzyl, methoxycarbonylbenzyl, 2-pyridylmethyl, (2-oxo-1-pyridyl)methyl, (3-methyl-2-oxo-1-pyridyl)methyl, (5-methyl-2-oxo-1-pyridyl)methyl, (3-methoxy-2-oxo-1-pyridyl)methyl, (3,5-dichloro-2-oxo-1-pyridyl)methyl, (5-trifluoromethyl-2-oxo-1-pyridyl)methyl, (4-oxo-1-pyridyl)methyl, (3,5-dichloro-4-oxo-1-pyridyl)methyl, 2-pyridylmethyl, 3-pyridylmethyl, 4-pyridylmethyl, 1-pyrazolylmethyl, (4-methyl-1-pyrazolyl)methyl, (3,5-dimethyl-1-pyrazolyl)methyl, (4-bromo-1-pyrazolyl)methyl, 1-imidazolylmethyl, (2-methyl-1-imidazolyl)methyl, (4,5-dichloro-1-imidazolyl)methyl, {4,5-di(methoxycarbonyl)-1-imidazolyl}methyl, 3-isoxazolylmethyl, (5-methyl-3-isoxazolyl)methyl, 1-(1,2,4-triazolyl)methyl, 2-pyrimidinylmethyl, (2-oxo-1-pyrimidinyl)methyl, 2-furylmethyl, C₃₋₆ cycloalkyl, phenyl, methylphenyl, isopropylphenyl, methoxyphenyl, cyanophenyl, nitrophenyl, chlorophenyl, methoxycarbonylphenyl, 2-furyl, 2-thienyl, 2,6-dichloro-4-pyridyl, 1-methyl-2-pyrrolyl, C₃₋₅ alkenyl, methoxycarbonyl, ethoxycarbonyl, C₁₋₄ alkoxy, methoxymethoxy, (2-methoxy)ethoxy, ethoxymethoxy, methylaminomethoxy, ethylaminomethoxy, dimethylaminomethoxy, diethylaminomethoxy, ethylmethylaminomethoxy, 2-pyridylmethoxy, 3-pyridylmethoxy, 4-pyridylmethoxy, 1-pyrazolylmethoxy, 1-imidazolylmethoxy, 3-isoxazolylmethoxy, 1-(1,2,4-triazolyl)methoxy, 2-pyrimidinylmethoxy, 2-furylmethoxy, (6-trifluoromethyl-2-pyridyl)methoxy, (5-phenyl-2-methyl-3-furyl)methoxy, (6-chloro-2-pyridyl)methoxy, (5-cyano-3-methyl-1-pyrazolyl)methoxy, (5-methoxycarbonyl-1-pyrazolyl)methoxy, cyclopropoxy, cyclobutoxy, cyclohexyloxy, allyloxy, phenoxy, 2-fluorophenoxy, 3-fluorophenoxy, 4-fluorophenoxy, 2,4-dichlorophenoxy, 3-trifluoromethylphenoxy, 2-methylphenoxy, 3-methoxyphenoxy, 4-methoxyphenoxy, 3-cyanophenoxy, 4-cyanophenoxy, benzyloxy, methylbenzyloxy, isopropylbenzyloxy, cyanobenzyloxy, nitrobenzyloxy, chlorobenzyloxy, methoxycarbonylbenzyloxy, 2-pyridyloxy, (3-methyl-2-pyridyl)oxy, (6-methyl-2-pyridyl)oxy, (3-methoxy-2-pyridyl)oxy, (6-chloro-2-pyridyl)oxy, 3-pyridyloxy, 1-pyrazolyloxy, (4-methyl-1-pyrazolyl)oxy, 3-isoxazolyloxy, (5-methyl-3-isoxazolyl)oxy, (4,5-dimethyl-3-isoxazolyl)oxy, (4-chloro-5-phenyl-3-isoxazolyl)oxy, (5-methoxycarbonyl-3-isoxazolyl)oxy, {5-methyl-2-(1,3,4-thiadiazolyl)}oxy, 2-pyrazinyloxy, 6-quinolyloxy, 8-quinolyloxy, 4-quinazolyloxy, 2-pyrimidinyloxy, (4-methyl-2-pyrimidinyl)oxy, (3,5-dimethyl-2-pyrimidinyl)oxy, 4-pyrimidinyloxy, (2,6-dimethyl-4-pyrimidinyl)oxy, (6-methyl-2-isopropyl-4-pyrimidinyl)oxy, 3-benzoisoxazolyloxy, methylaminomethyl, benzylaminomethyl, methoxymethylaminomethyl, ethylaminomethyl, propylaminomethyl, isopropylaminomethyl, butylaminomethyl, isobutylaminomethyl, t-butylaminomethyl, allylamino, dimethylamino, diethylamino, ethylmethylamino, piperidino, morpholino, 2,6-dimethylmorpholino, methylthio, benzylthio, methoxymethylthio, ethylthio, propylthio, isopropylthio, butylthio, isobutylthio, t-butylthio, allylthio, phenylthio, methylphenylthio, isopropylphenylthio, methoxyphenylthio, cyanophenylthio, nitrophenylthio, chlorophenylthio, methoxycarbonylphenylthio, 2-oxo-1-pyridylthio, 2-pyridylthio, 1-methyl-2-imidazolylthio or 5-methyl-2-(1,3,4-thiadiazolyl)thio group;
(a5) as Y, a methyl group, ethyl group, methoxy group, ethoxy group, cyano group, nitro group, fluorine atom, chlorine atom, bromine atom, methoxycarbonyl group, or ethoxycarbonyl group; and
as n, 0 or 1.

The compounds of formula (I) of the invention more preferably have:
(b1) as R¹, a C₁₋₂ alkyl group;
(b2) as R², a hydrogen atom or methyl group;
(b3) as R³, a C₄₋₅ alkyl, cyclobutylmethyl, (methylcyclobutyl)methyl, tetrahydrofuranylmethyl, cyclopentylmethyl, (methylcyclopentyl)methyl, (fluorocyclopentyl)methyl, (difluorocyclopentyl)methyl, tetrahydropyranylmethyl, cyclohexylmethyl, cyclopentenylmethyl, chloromethyl, 1,1-difluoroisobutyl, 3,3,3-trifluoro-2-trifluoromethylpropyl, 3,3,3-trifluoro-2-methylpropyl, 3,3,3-trifluoro-2,2-dimethylpropyl or cyclopentyl group;
(b4) as R⁴, a C₁₋₂ alkyl, methoxymethyl, ethoxymethyl, 2-methoxyethyl, butyryloxymethyl, isobutyryloxymethyl, pivaloyloxymethyl, cyclopropylcarbonyloxymethyl, methoxyacetoxymethyl, methoxycarbonyloxymethyl, 2-methoxyethoxycarbonyloxymethyl, phenoxymethyl, 2-pyridyloxymethyl, 1-pyrazolyloxymethyl, 3-isoxazolyloxymethyl, (5-methyl-3-isoxazolyl)oxymethyl, 6-quinolyloxymethyl, (1-methyl-2-imidazolyl)thiomethyl, 2-pyrimidylthiomethyl, {5-methyl-2-(1,3,4-thiadiazolyl)}thiomethyl, methoxybenzyl, fluorobenzyl, (2-oxo-1-pyridyl)methyl, 2-pyridylmethyl, 3-pyridylmethyl, (3,5-dimethyl-1-pyrazolyl)methyl, (4-bromo-1-pyrazolyl)methyl, (5-methyl-3-isoxazolyl)methyl, cyclopropyl, cyclobutyl, methoxy, ethoxy, isopropoxy, 2-pyridylmethoxy, phenoxy, 2-pyridyloxy, (3-methyl-2-pyridyl)oxy, (6-methyl-2-pyridyl)oxy, 3-pyridyloxy or (4-methyl-1-pyrazolyl)oxy group;
(b5) as n, 0.

The compounds of formula (I) of the invention still more preferably have:
(c1) as R¹, a methyl group;
(C2) as R², a hydrogen atom;
(C3) as R³, a branched C₄₋₅ alkyl, cyclobutylmethyl, (methylcyclobutyl)methyl, tetrahydrofuranyl, cyclopentylmethyl, (fluorocyclopentyl)methyl, (difluorocyclopentyl)methyl, tetrahydropyranylmethyl, cyclohexylmethyl, cyclopent-2-enylmethyl, cyclopent-3-enylmethyl, 1,1-difluoroisobutyl, 3,3,3-trifluoro-2-trifluoromethylpropyl, 3,3,3-trifluoro-2-methylpropyl, or 3,3,3-trifluoro-2,2-dimethylpropyl group;
(c4) as R⁴, a C₁₋₂ alkyl, methoxymethyl, cyclopropylcarbonyloxymethyl, phenoxymethyl, 2-pyridyloxymethyl, (1-methyl-2-imidazolyl)thiomethyl, {5-methyl-2-(1,3,4-thiadiazolyl)}thiomethyl, fluorobenzyl, 2-pyridylmethyl, 3-pyridylmethyl, (5-methyl-3-isoxazolyl)methyl, cyclopropyl, cyclobutyl, methoxy, ethoxy, isopropoxy, 2-pyridylmethoxy, phenoxy, 2-pyridyloxy, (3-methyl-2-pyridyl)oxy, or 3-pyridyloxy group; and
(c5) as n, 0.

The compounds of formula (I) of the invention more preferably have:
(d1) as R¹, a methyl group;
(d2) as R², a hydrogen atom;
(d3) as R³, an isobutyl, cyclobutylmethyl, tetrahydrofuranylmethyl, cyclopentylmethyl, 3,3,3-trifluoro-2-methylpropyl or 3,3,3-trifluoro-2,2-dimethylpropyl group;
(d4) as R⁴, a methyl, methoxymethyl, 2-pyridyloxymethyl, methoxy, phenoxy or 2-pyridyloxy group;
(d5) as n, 0.

The typical compounds of the present invention will be exemplified below in Table 1, but the present invention is not limited to or by them.

In the table, the following abbreviations are used: "Me" for methyl, "Et" for ethyl, "Pr" for propyl, "iPr" for isopropyl, "cPr" for cyclopropyl, "Bu" for butyl, "iBu" for isobutyl, "tBu" for tert-butyl, "cBu" for cyclobutyl, "Pent" for pentyl, "iPent" for isopentyl, "neoPent" for neopentyl, "cPent" for cyclopentyl, "Hex" for hexyl, "cHex" for cyclohexyl, "Ph" for phenyl", "2-Me-Ph" for 2-methylphenyl, "2,4-Cl₂-Ph" for 2,4-dichlorophenyl, "Bn" for benzyl, "3-Pyr" for 3-pyridyl, "2-Fur" for 2-furyl, "2-Thi" for 2-thienyl, "Thidz" for 5-(1,2,3-thiadiazolyl", "2-Thida" for 2-(1,3,4-thiadiazolyl), "1-Triz" for 1-(1,2,4-triazolyl), "1-Pyrd" for 1-pyrrolidinyl, "1-Pip" for piperidino, "4-Mor" for morpholino, "5-Me-3-Isox" for 5-methyl-3-isoxazolyl, "1-Pyza" for 1-pyrazolyl, "2-oxo-1-Pyr" for 2-oxo-1-pyridyl, "6-Quino" for 6-quinolyl, "4-Quina" for 4-quinazolinyl, "2-Pym" for 2-pyrimidinyl, "2-Imid" for 2-imidazolyl, "4-Pyz" for 4-pyrazinyl, "2-Pyrr" for 2-pyrrolyl, "3-Bisox" for 3-benzoisoxazolyl, "CN" for cyano, "Ac" for acetyl, "(3,4-OCH₂O-)-Ph" for 3,4-methylenedioxyphenyl, "Nor" for bicyclo[2.2.1]hept-2-yl, "cPent-3-en" for 3-cyclopentenyl, "2-Thf" for tetrahyrofuran-2-yl, "Thp" for tetrahydropyran-4-yl, "2-Thio" for 2-thienyl, "Np" for naphthyl, "5-Me" in "(Y)ₙ" for methyl bonded to the 5-position of benzyl, and "H" in "(Y)ₙ" for n=0.

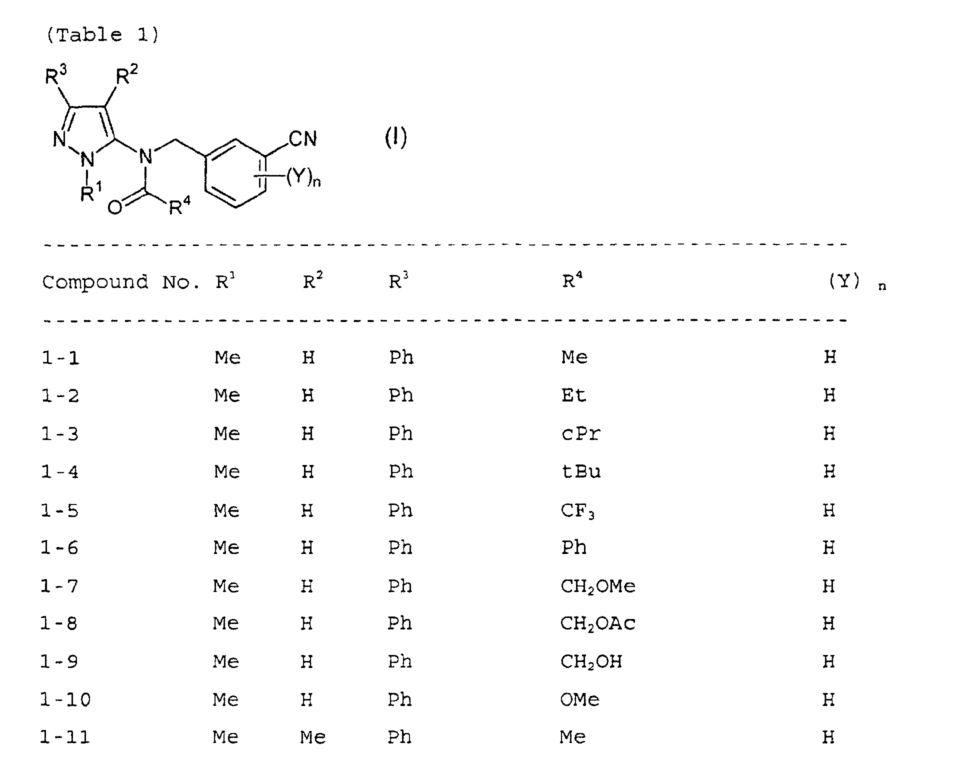

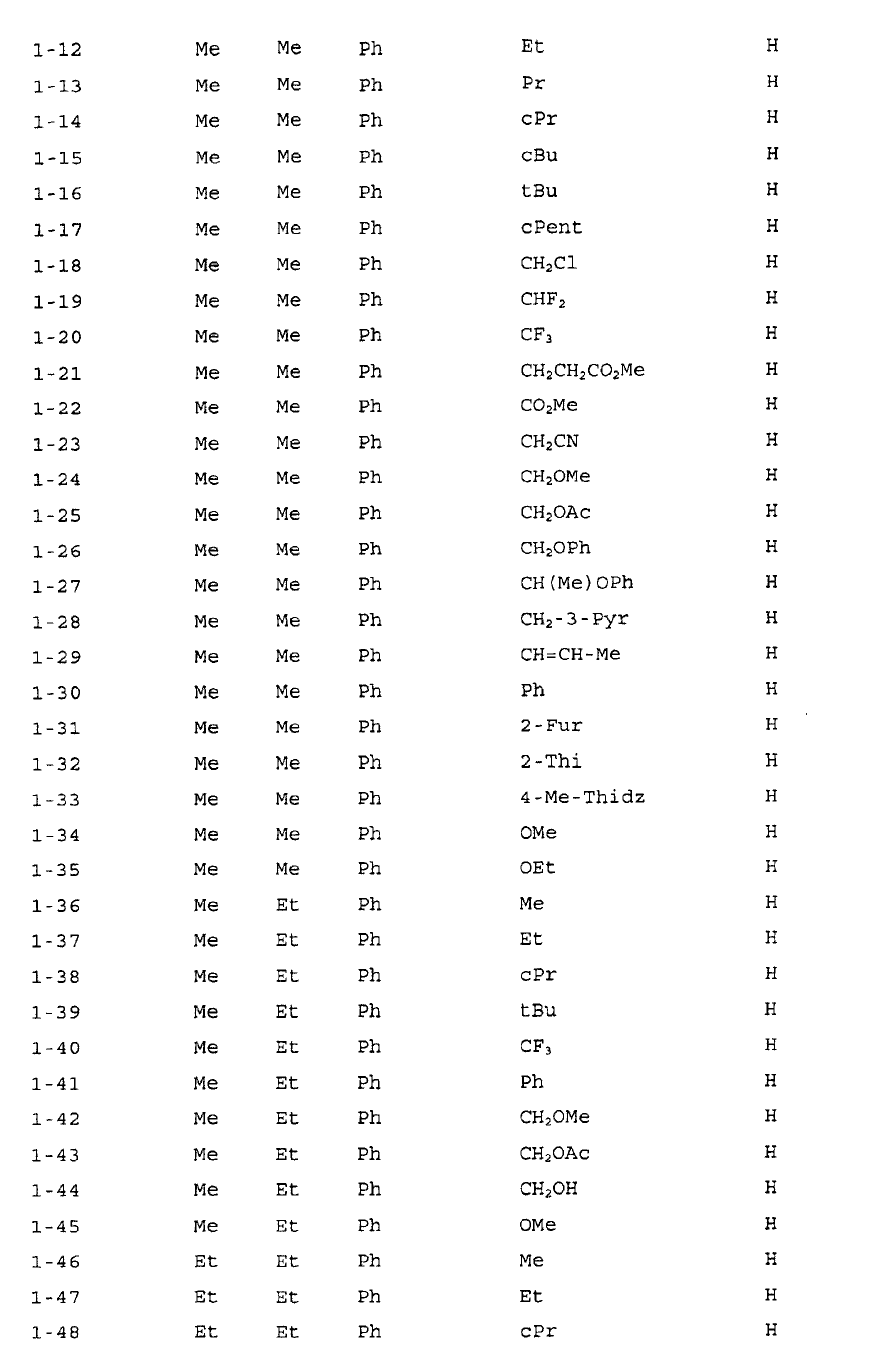

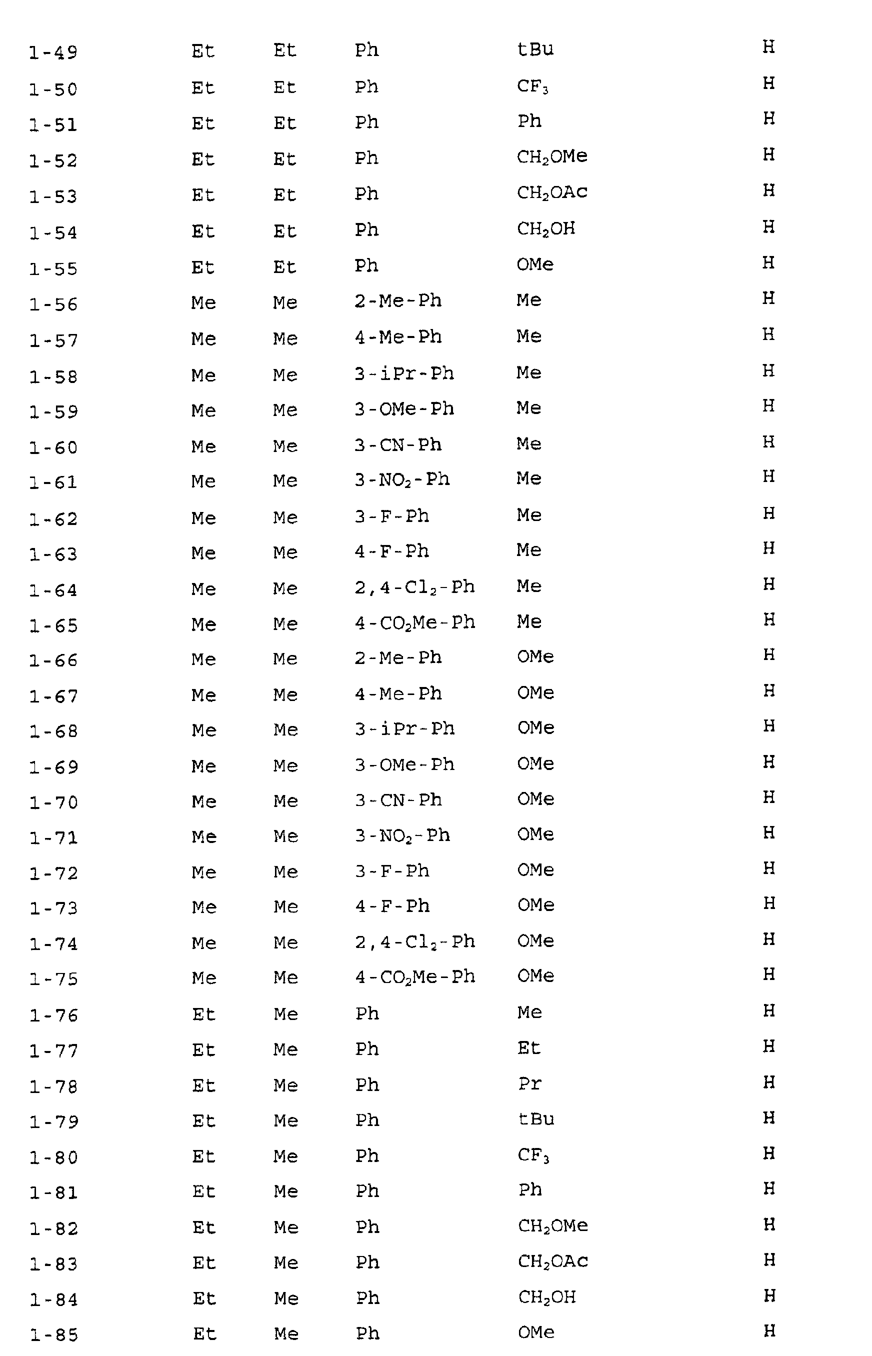

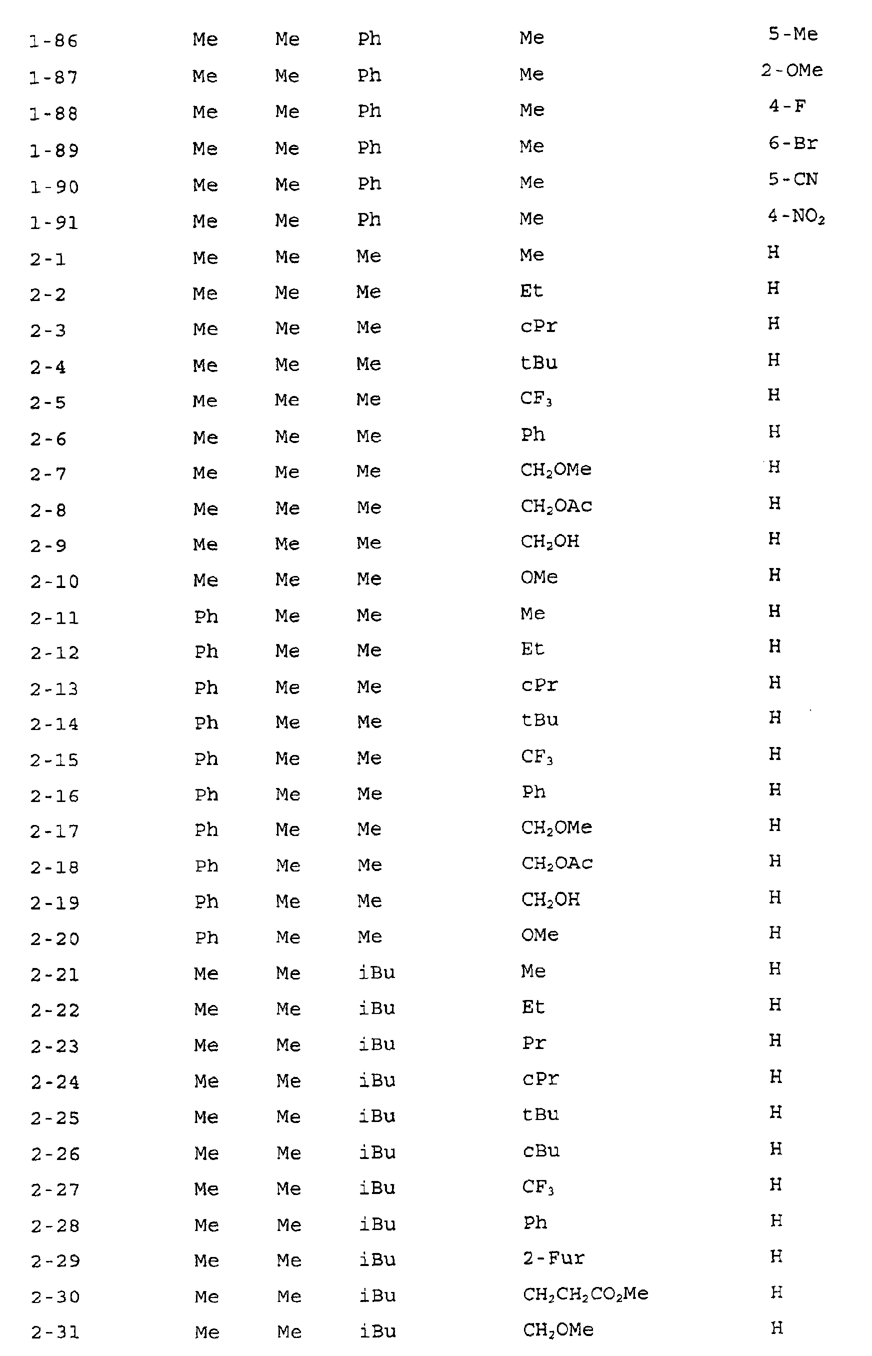

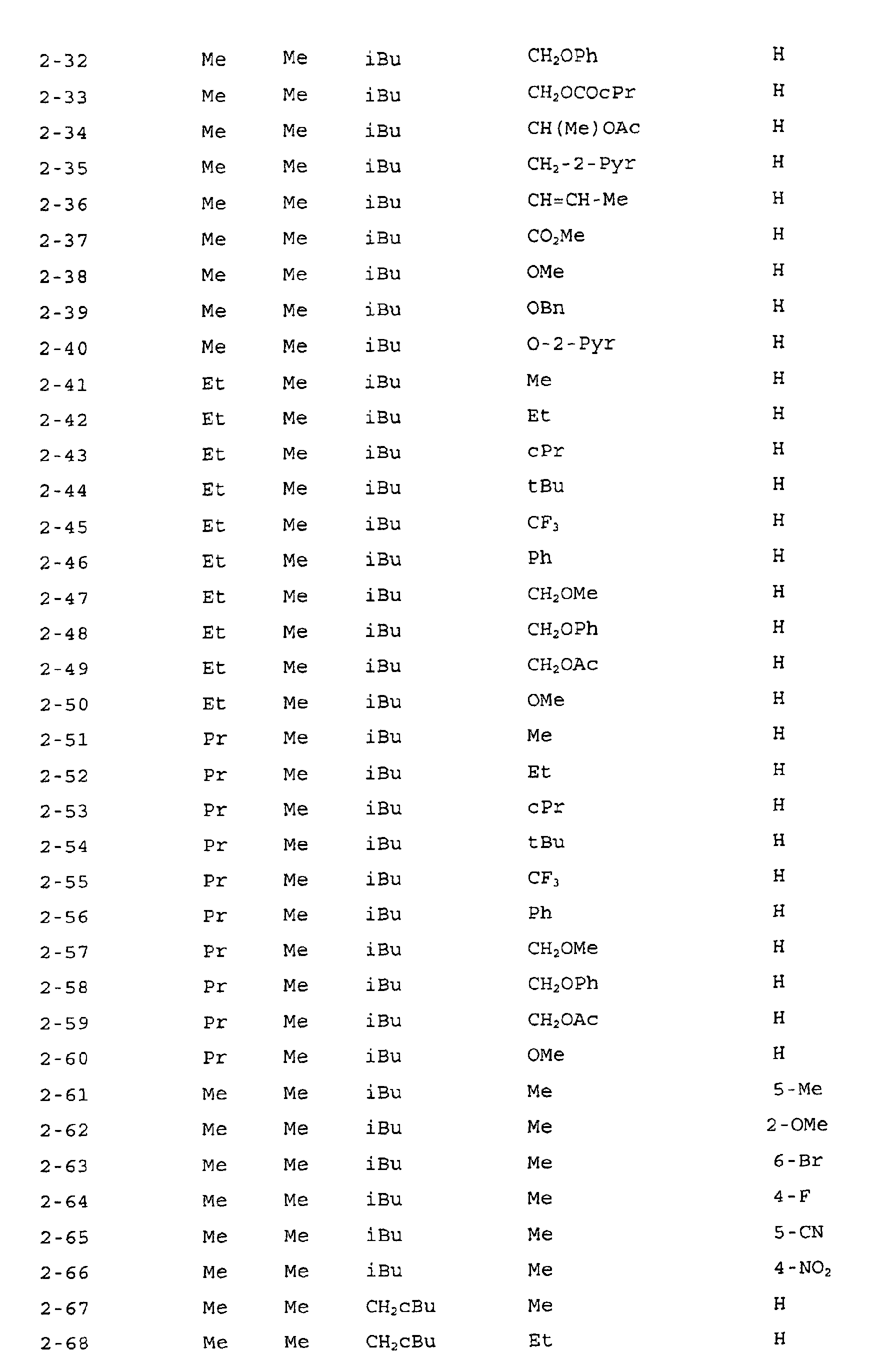

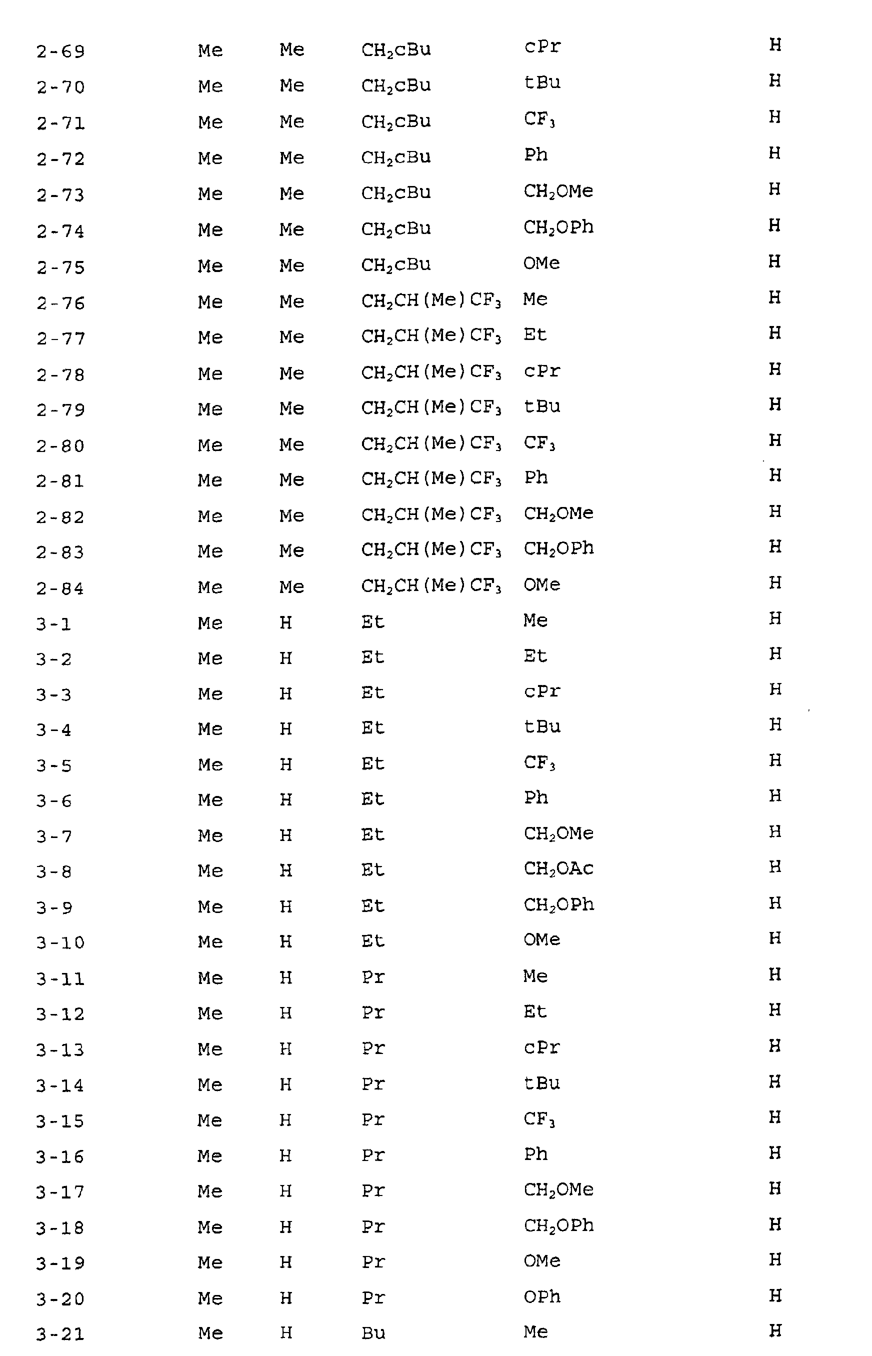

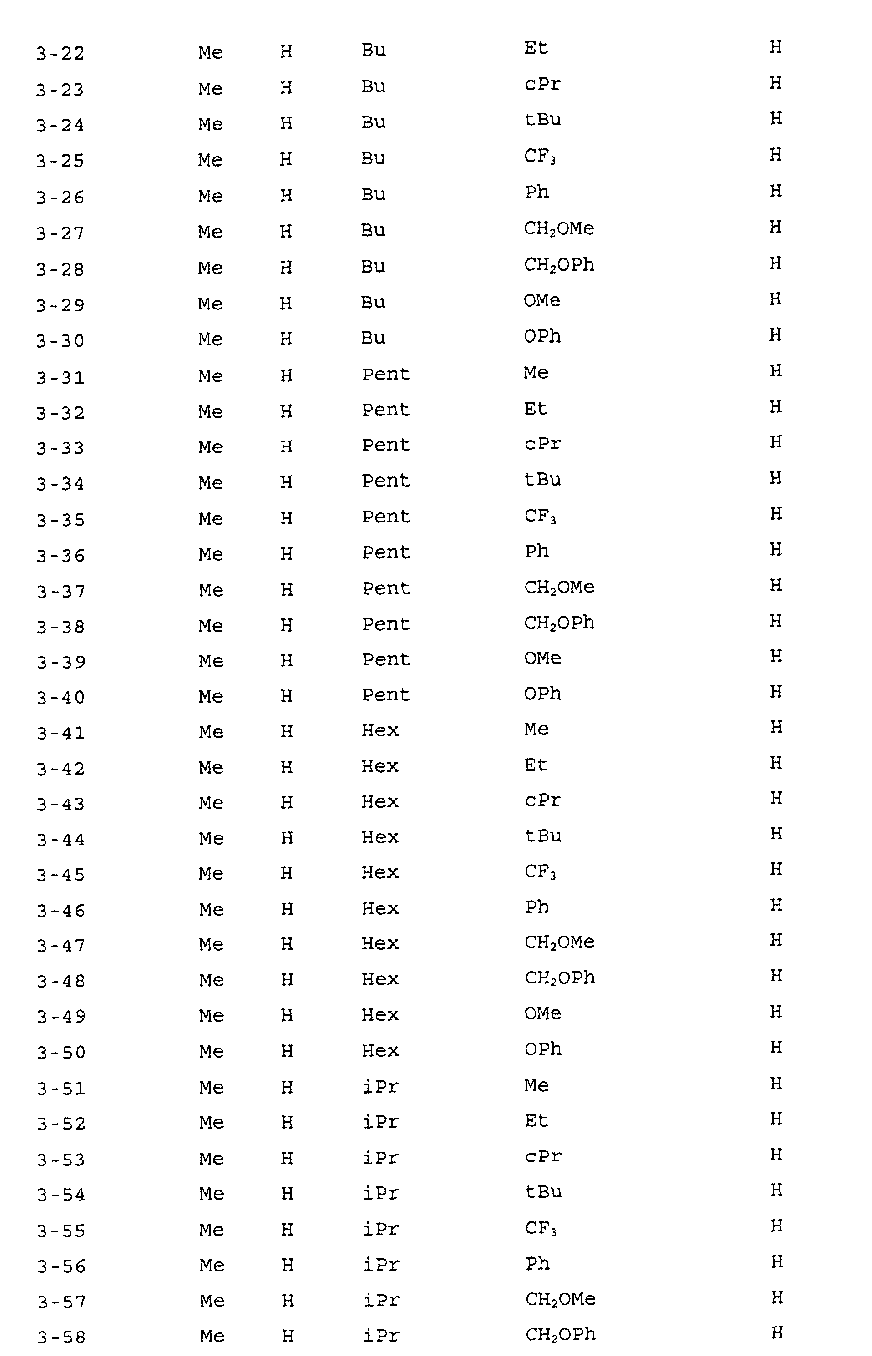

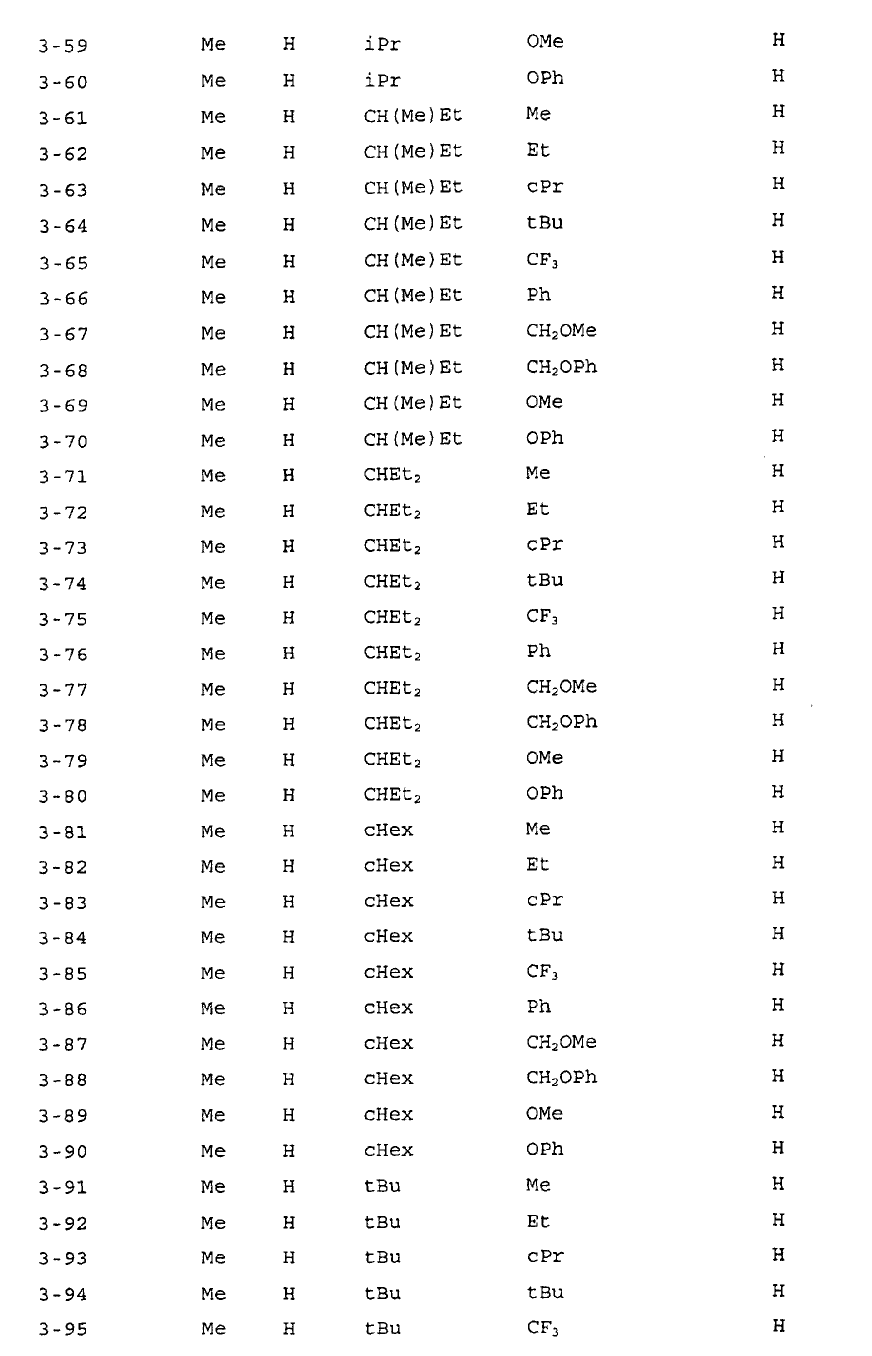

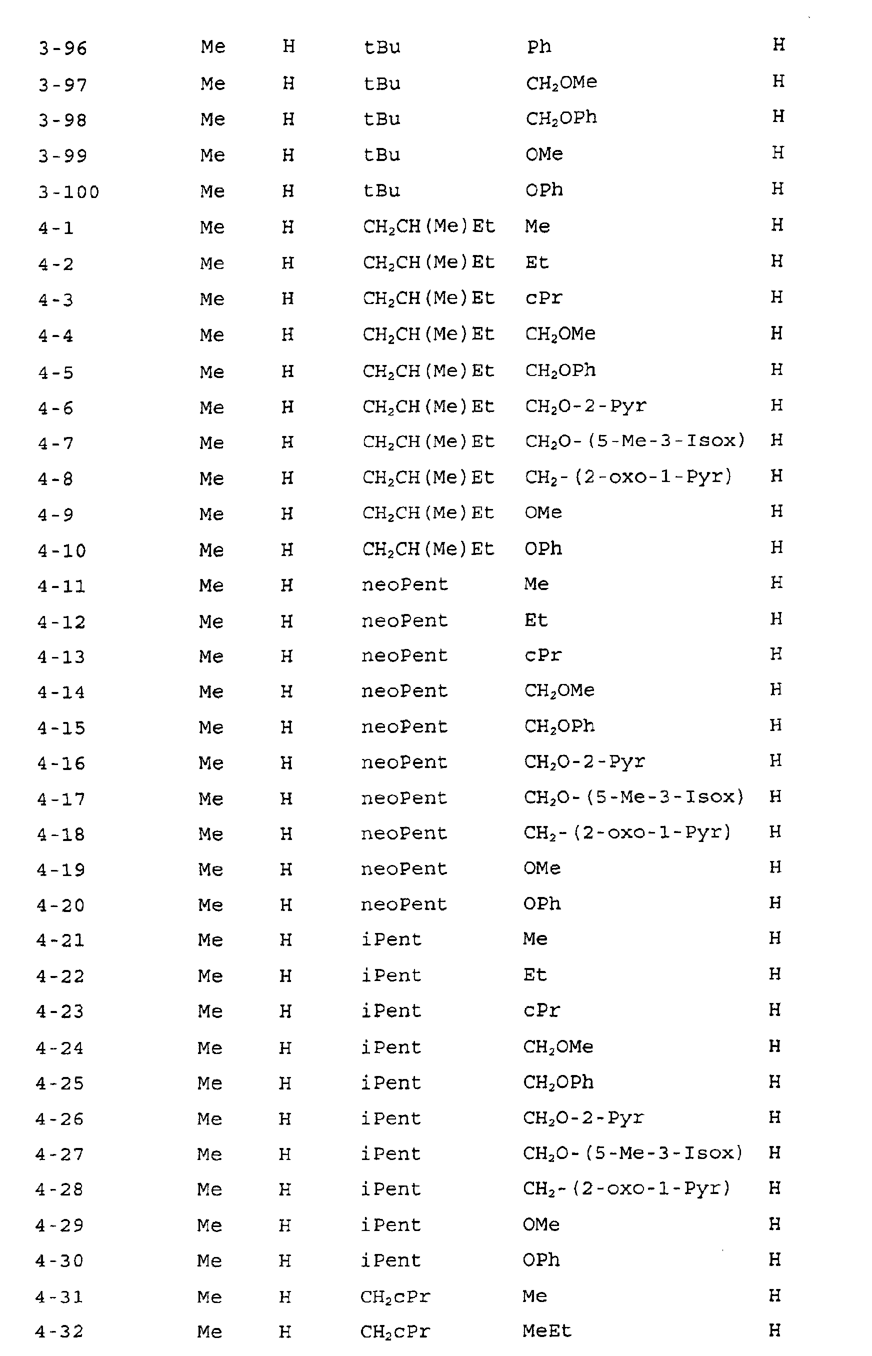

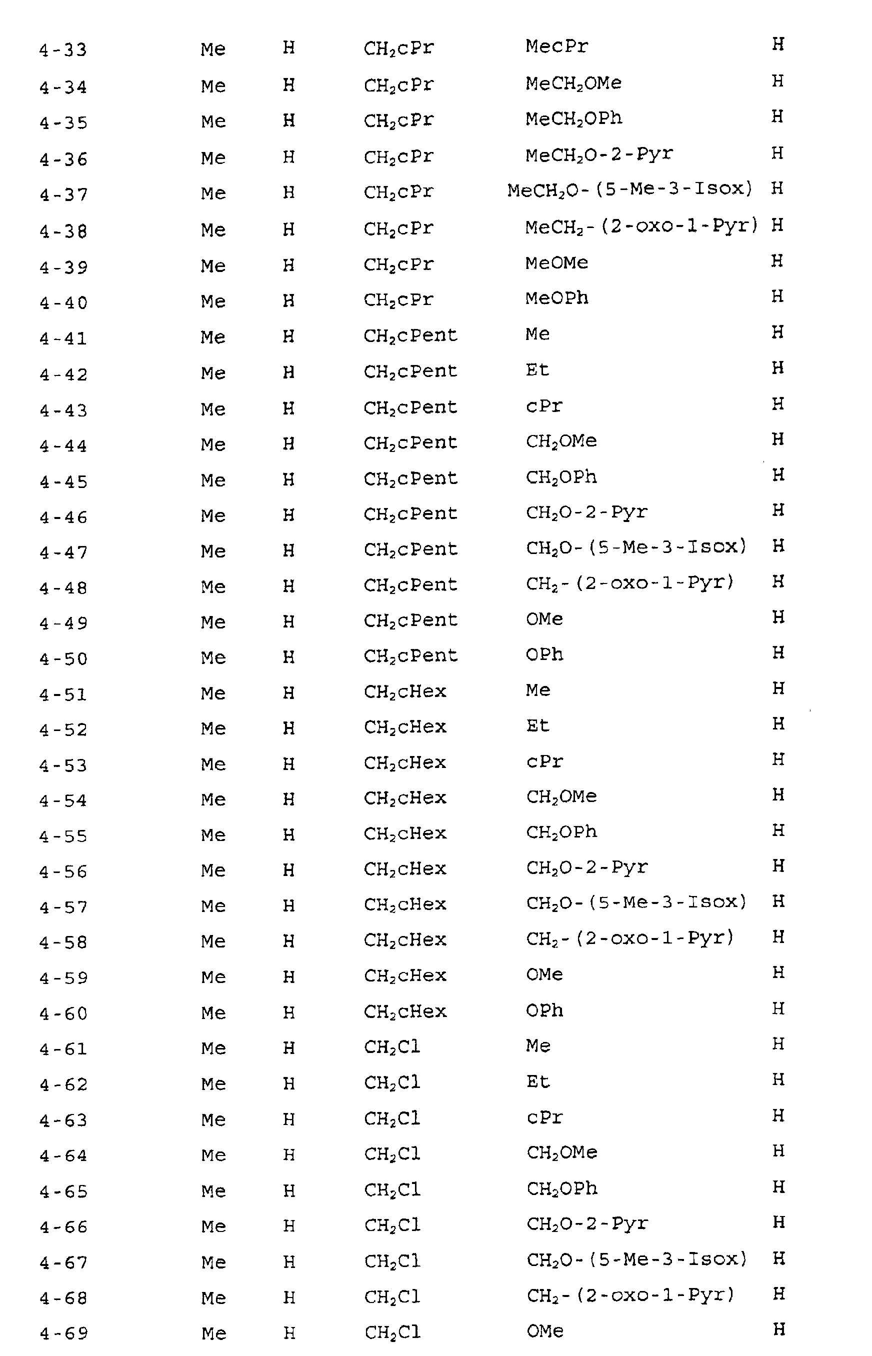

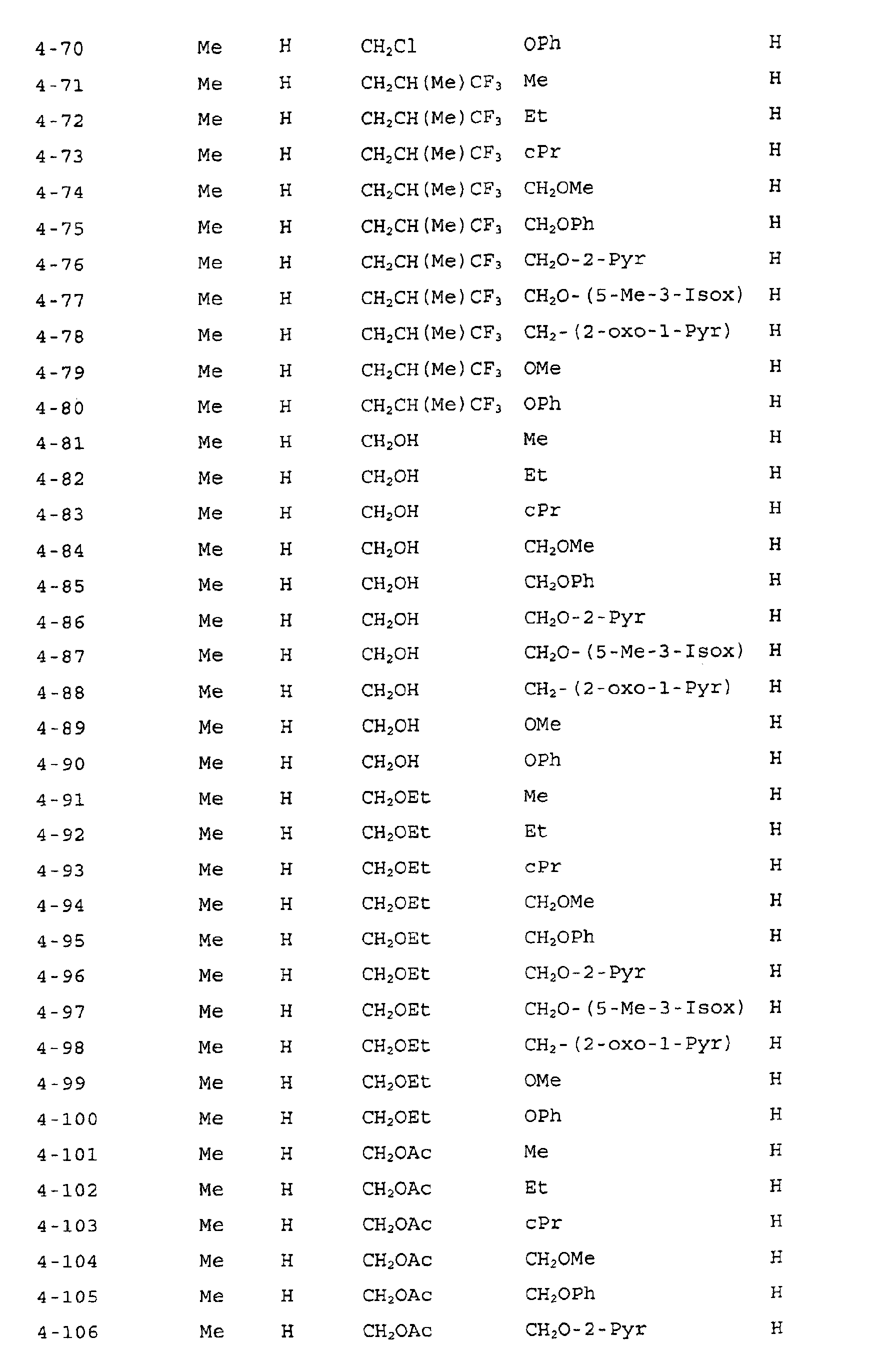

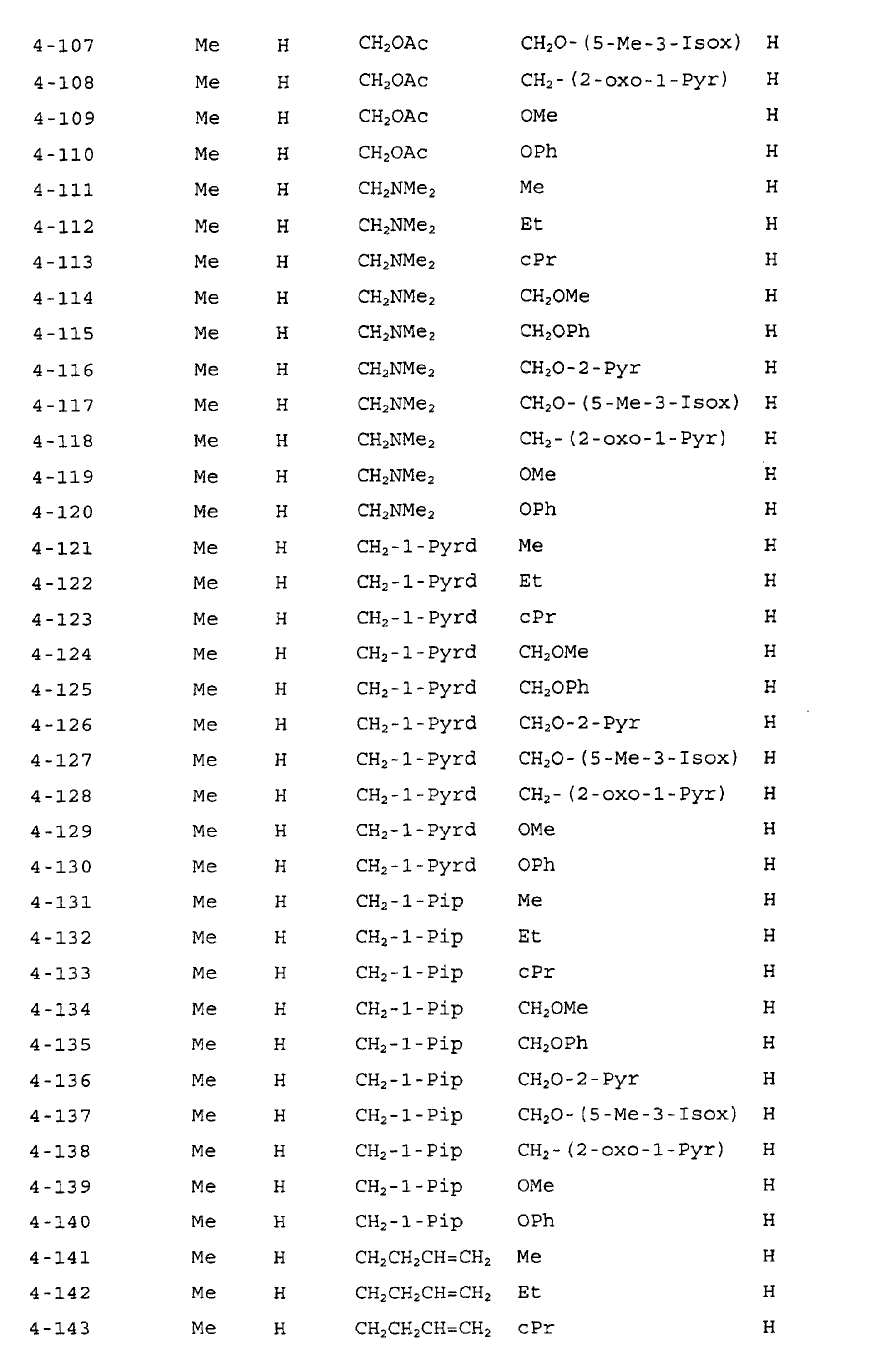

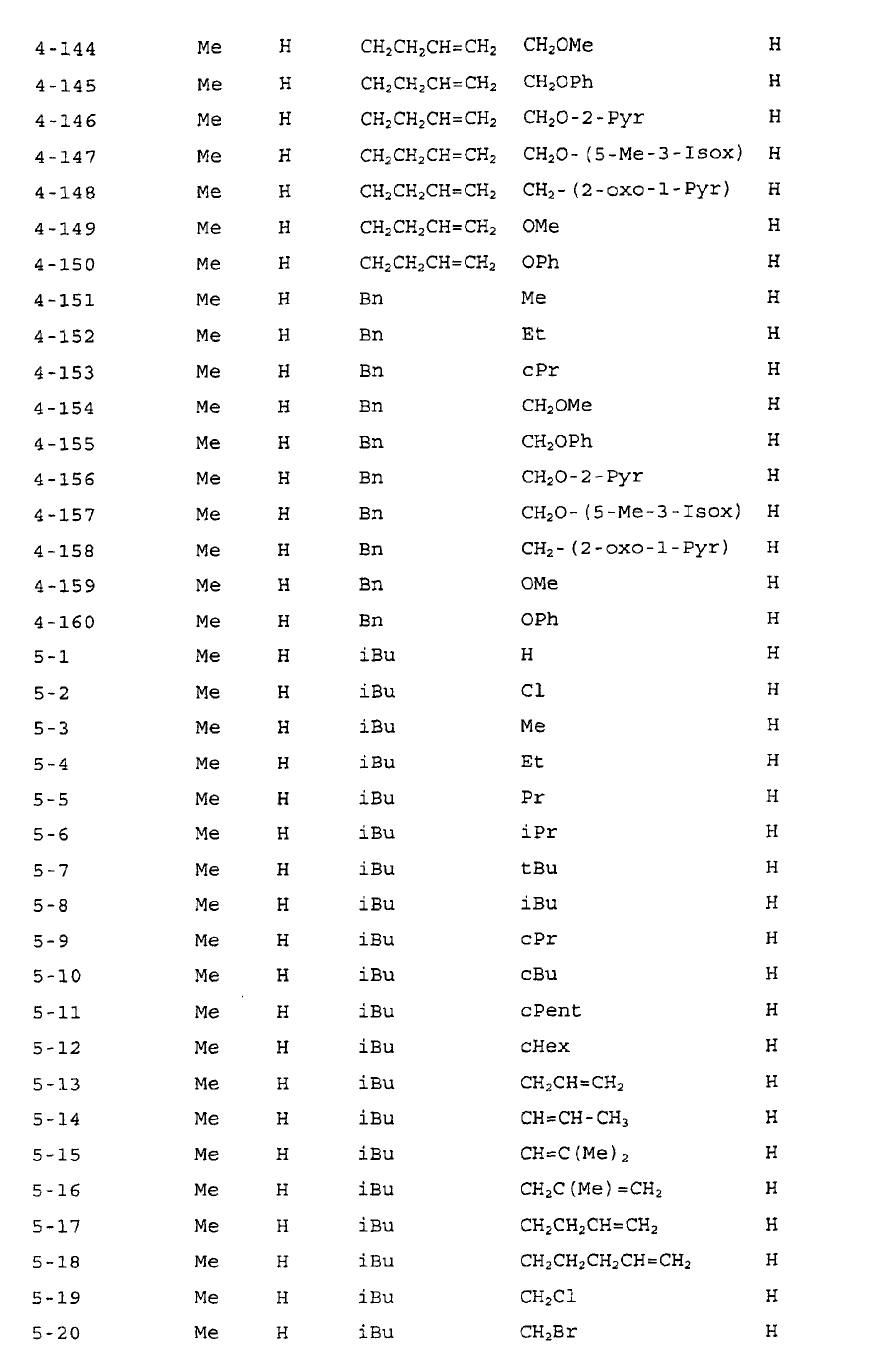

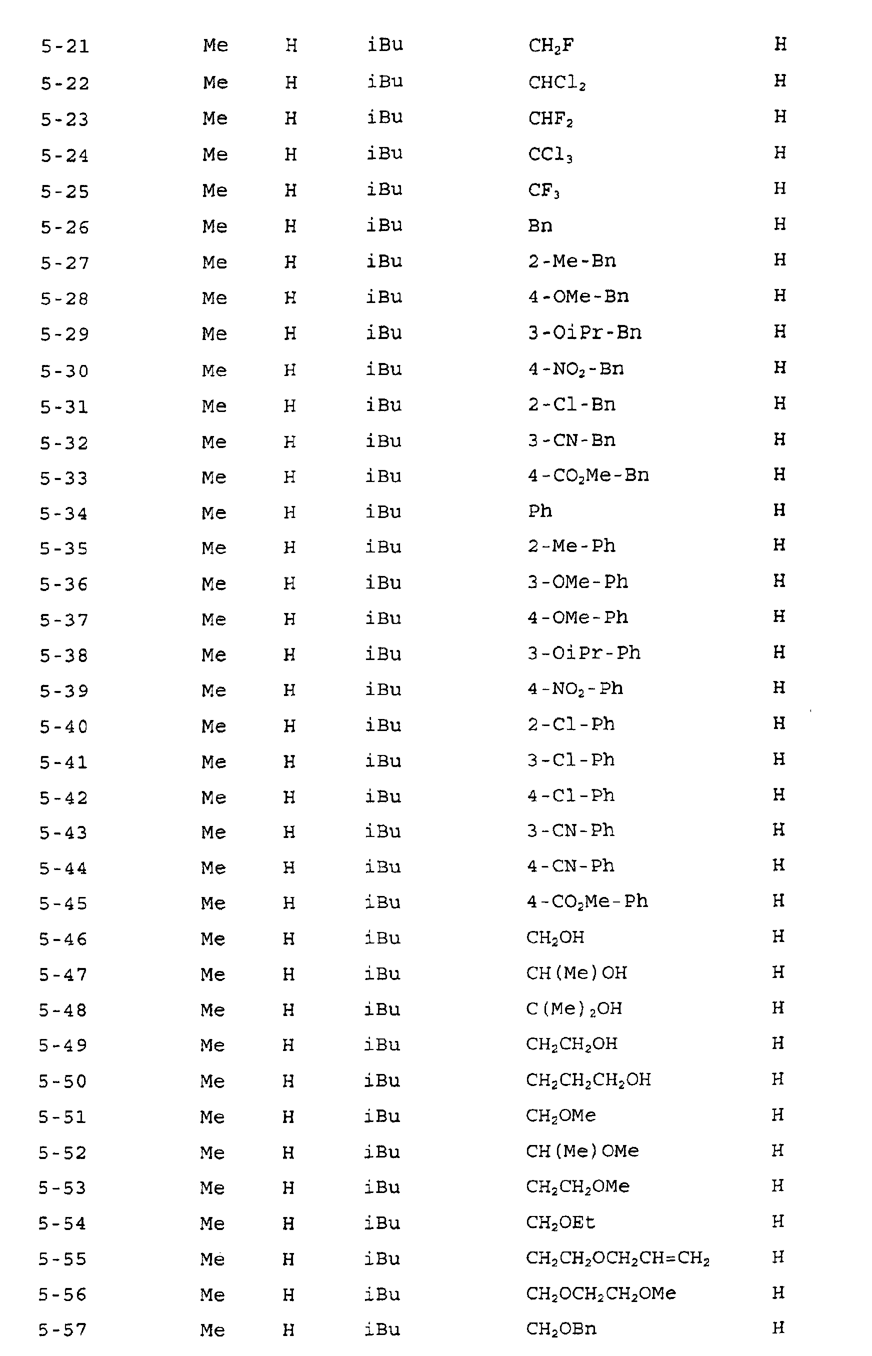

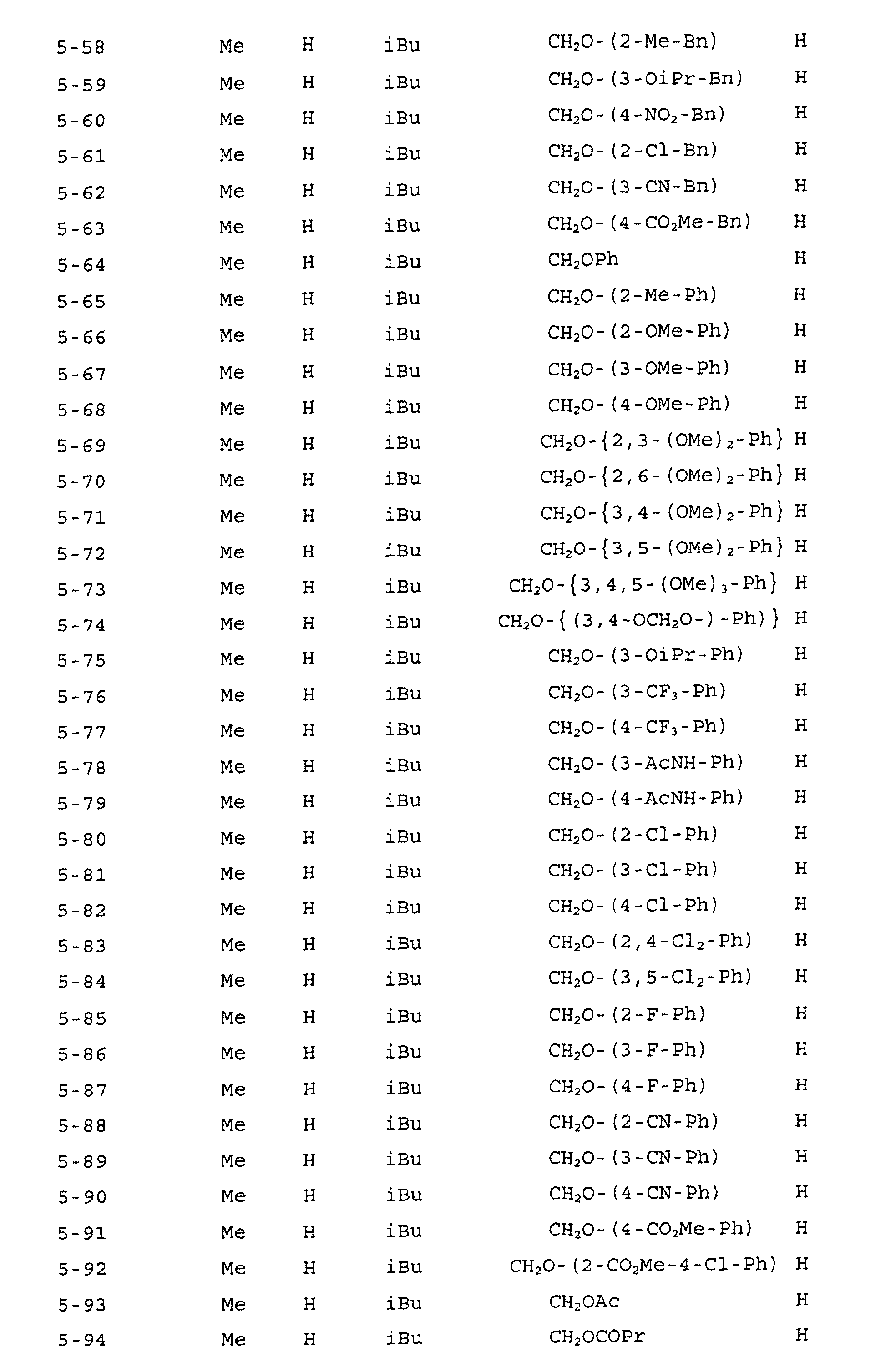

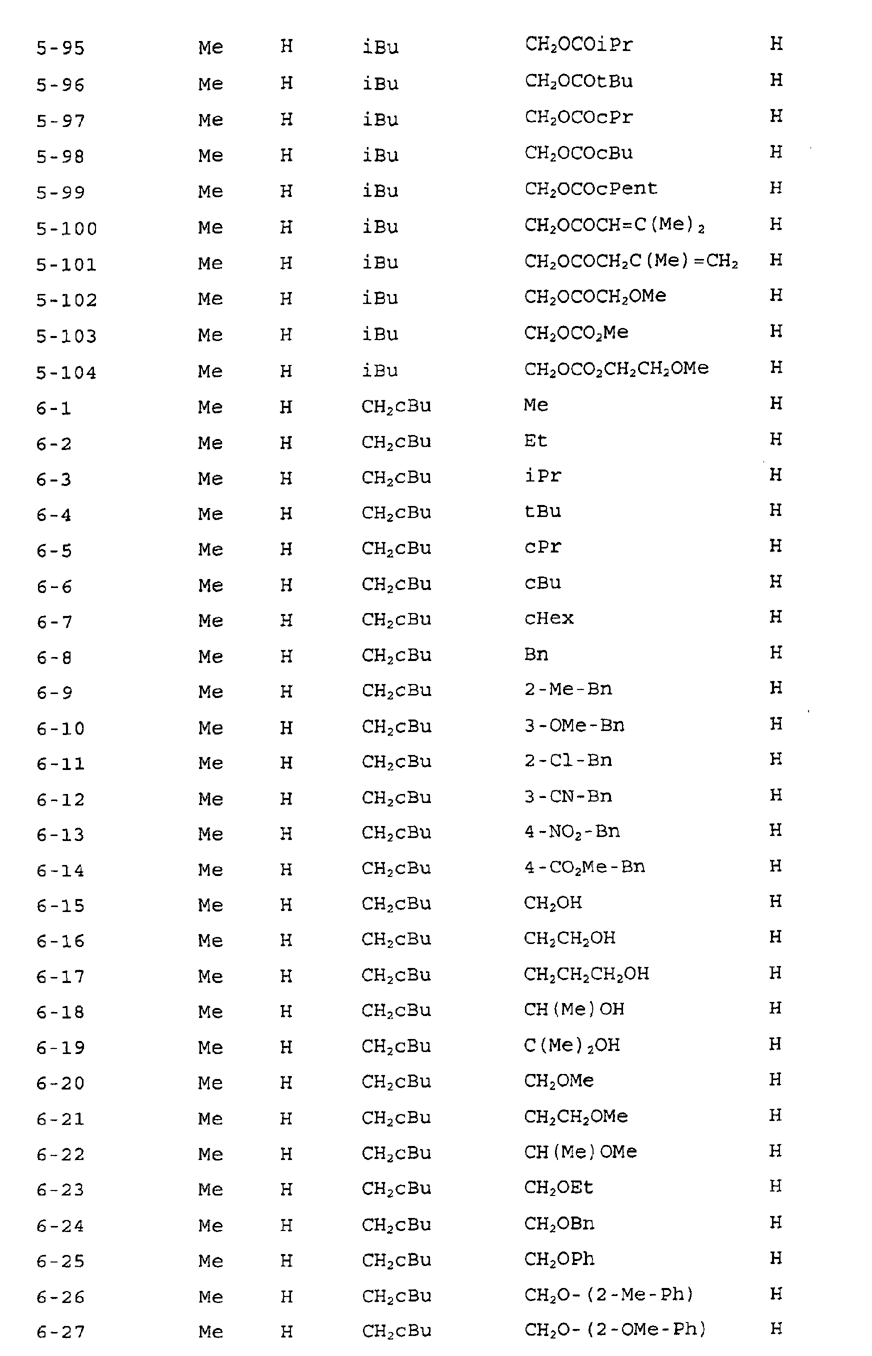

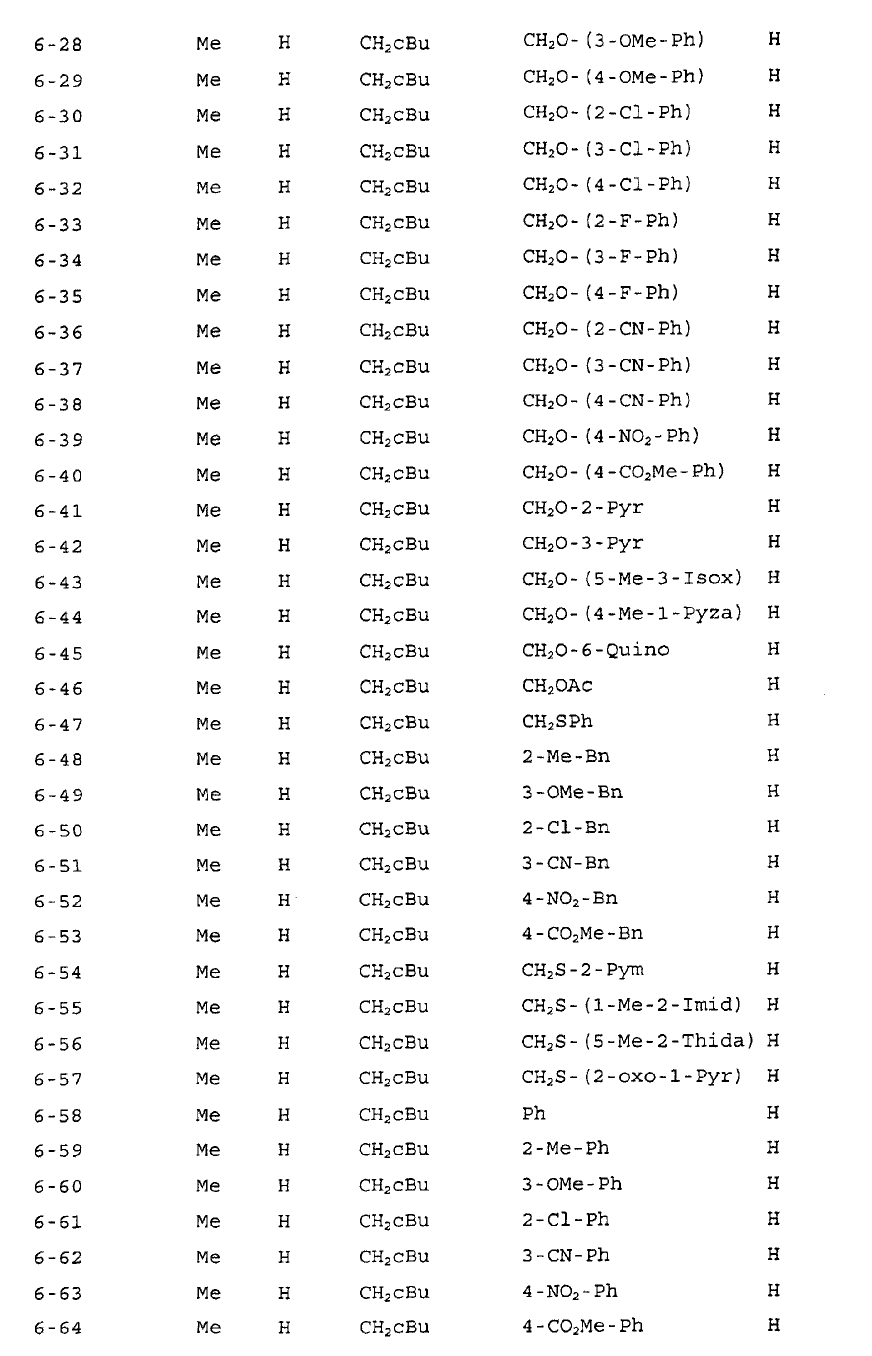

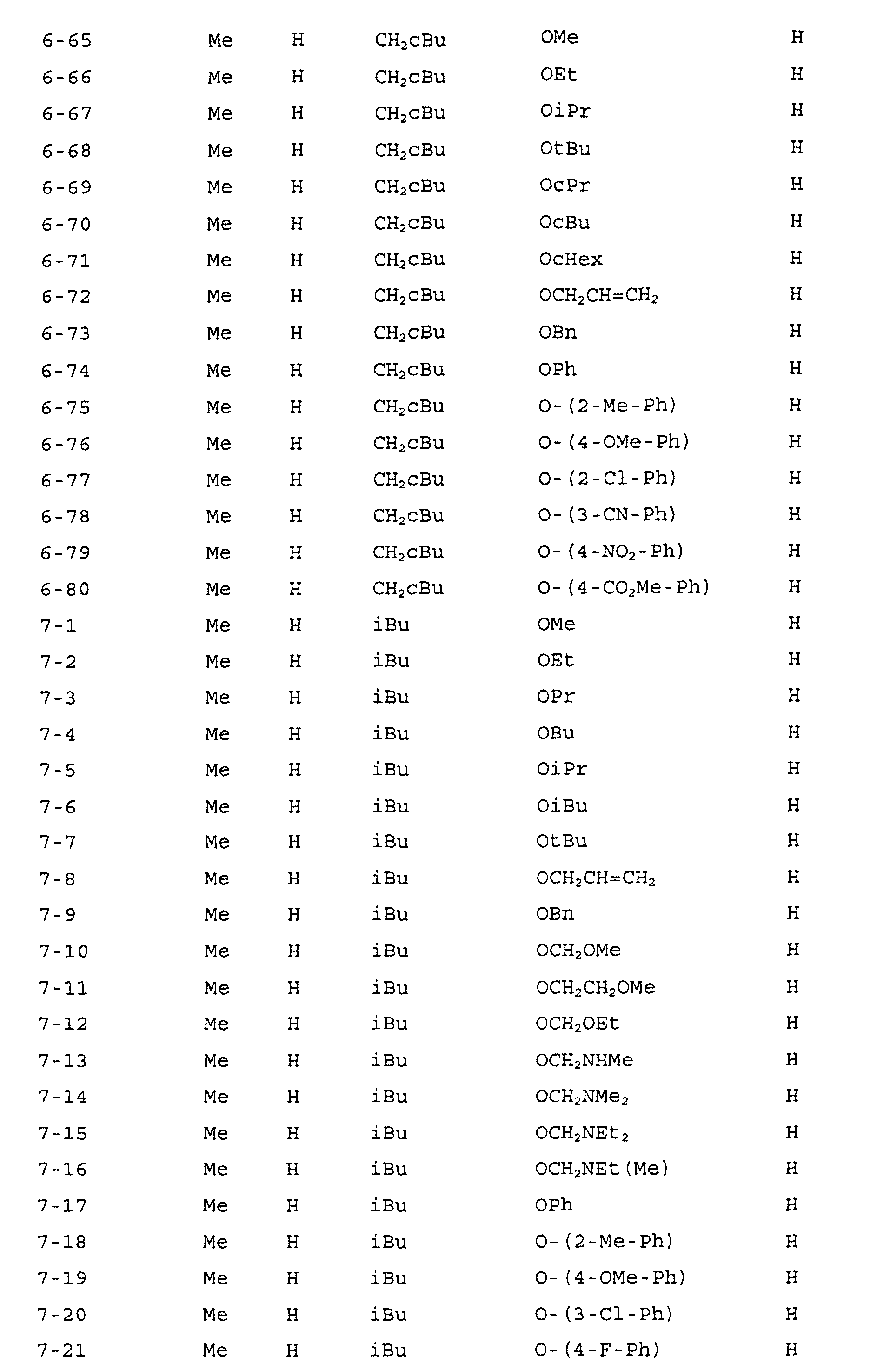

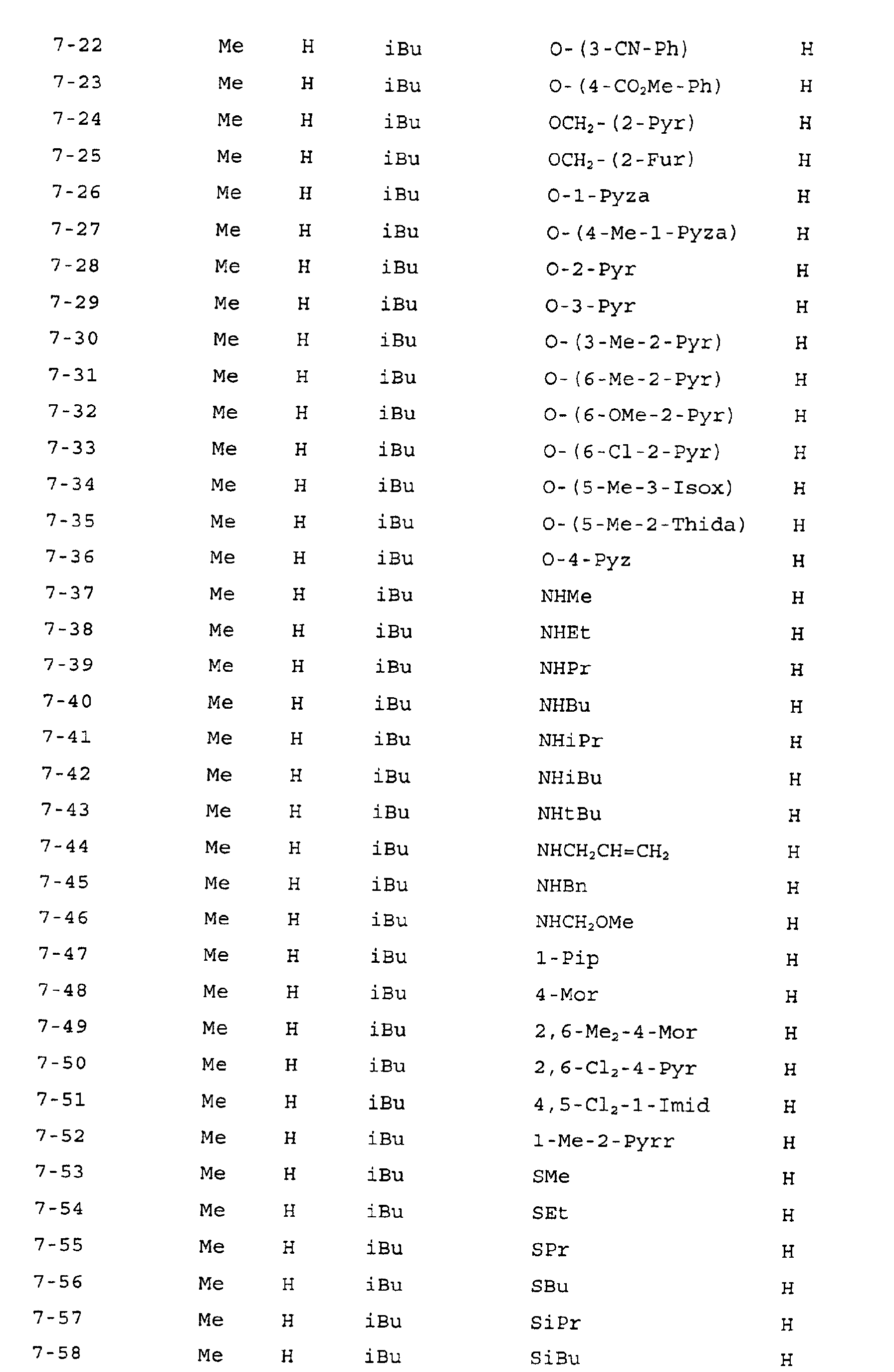

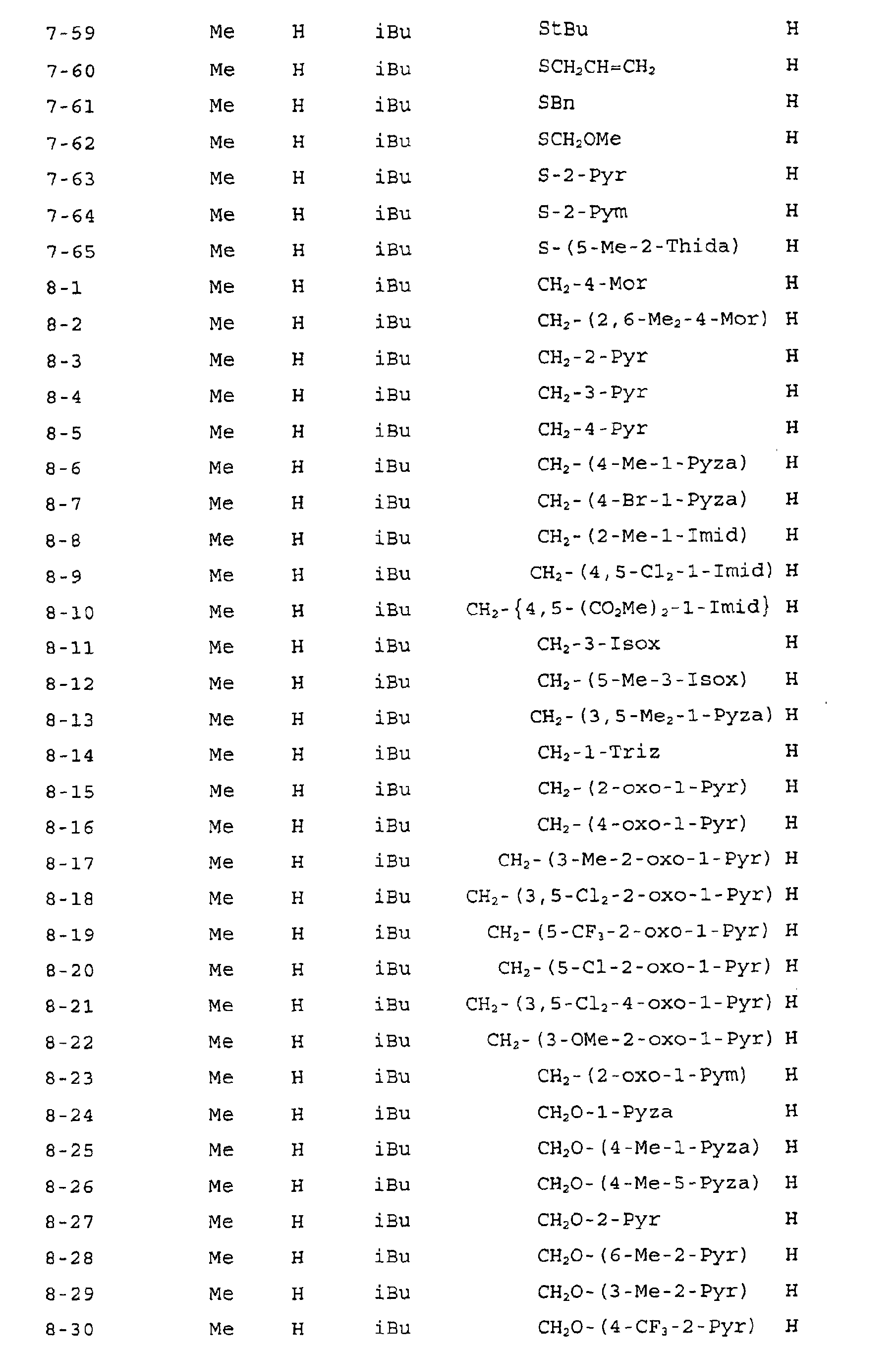

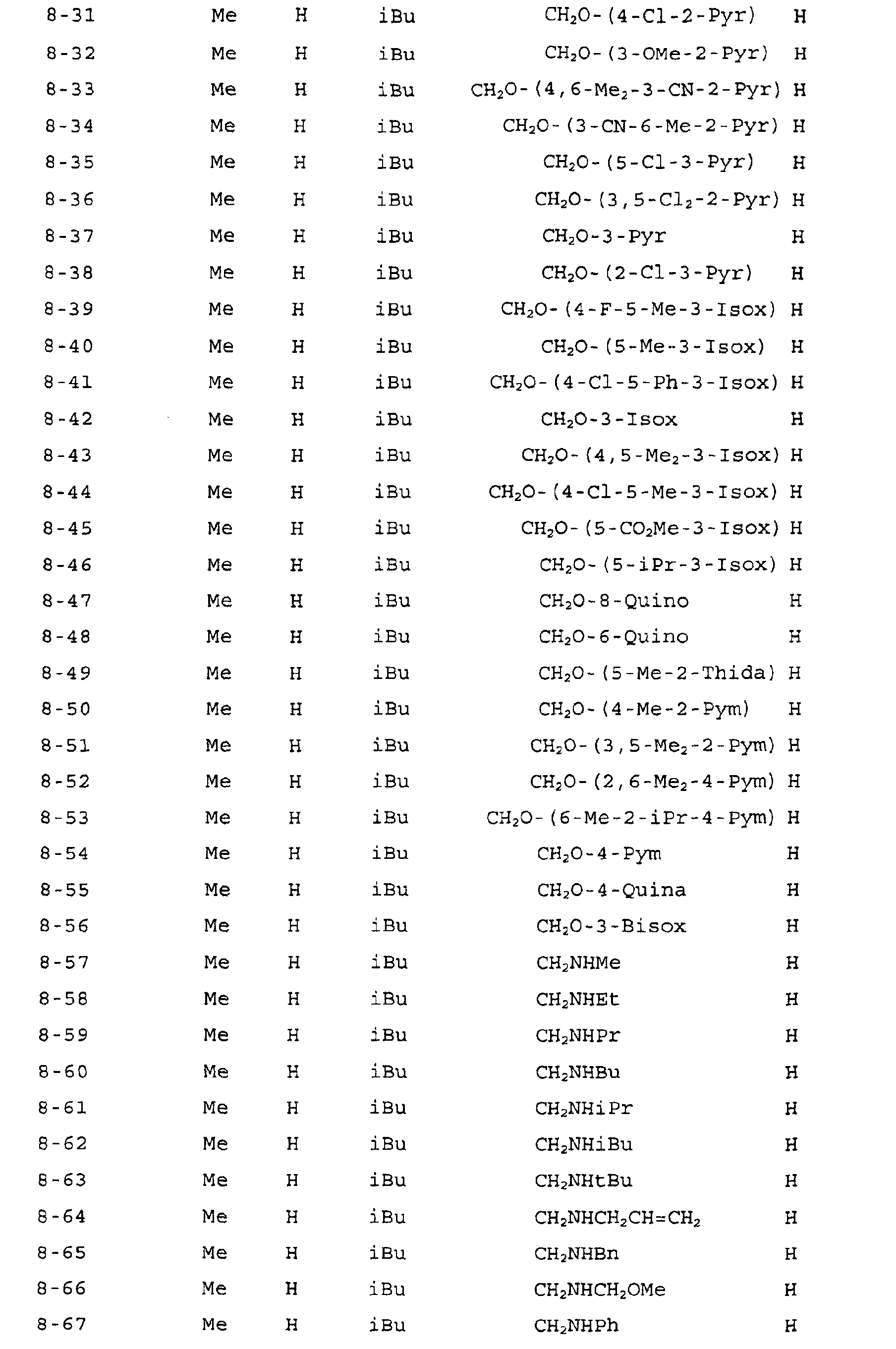

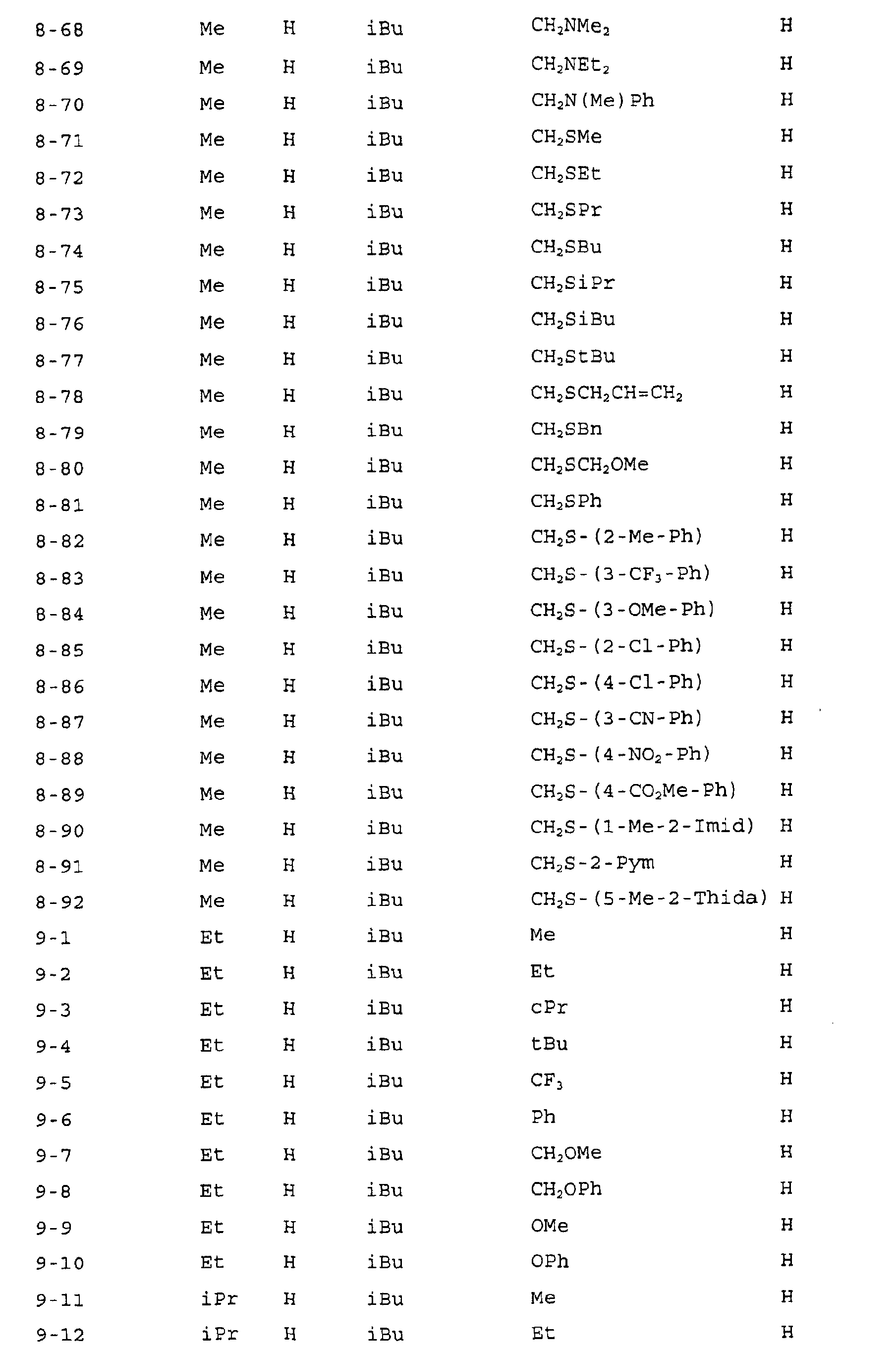

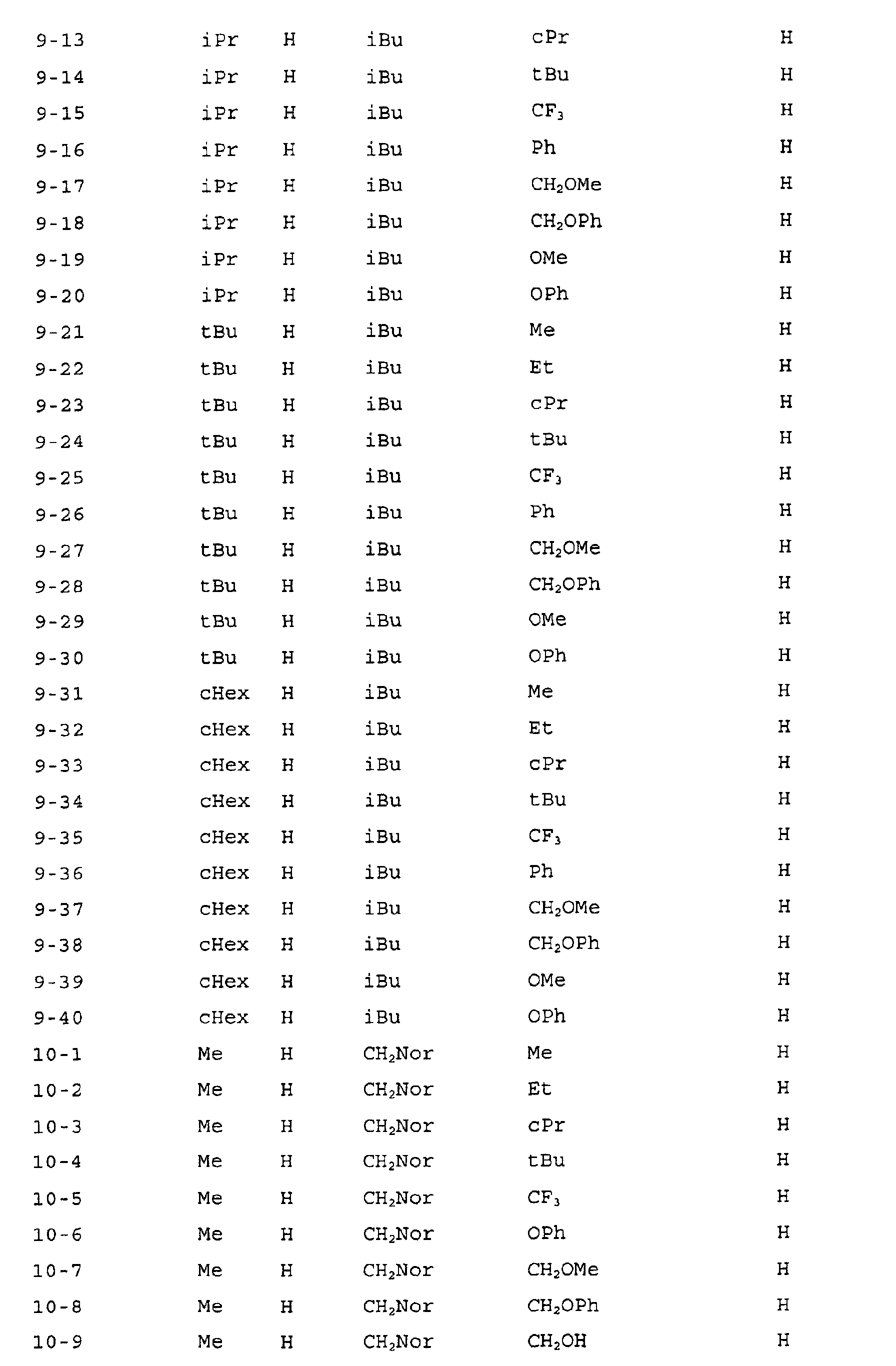

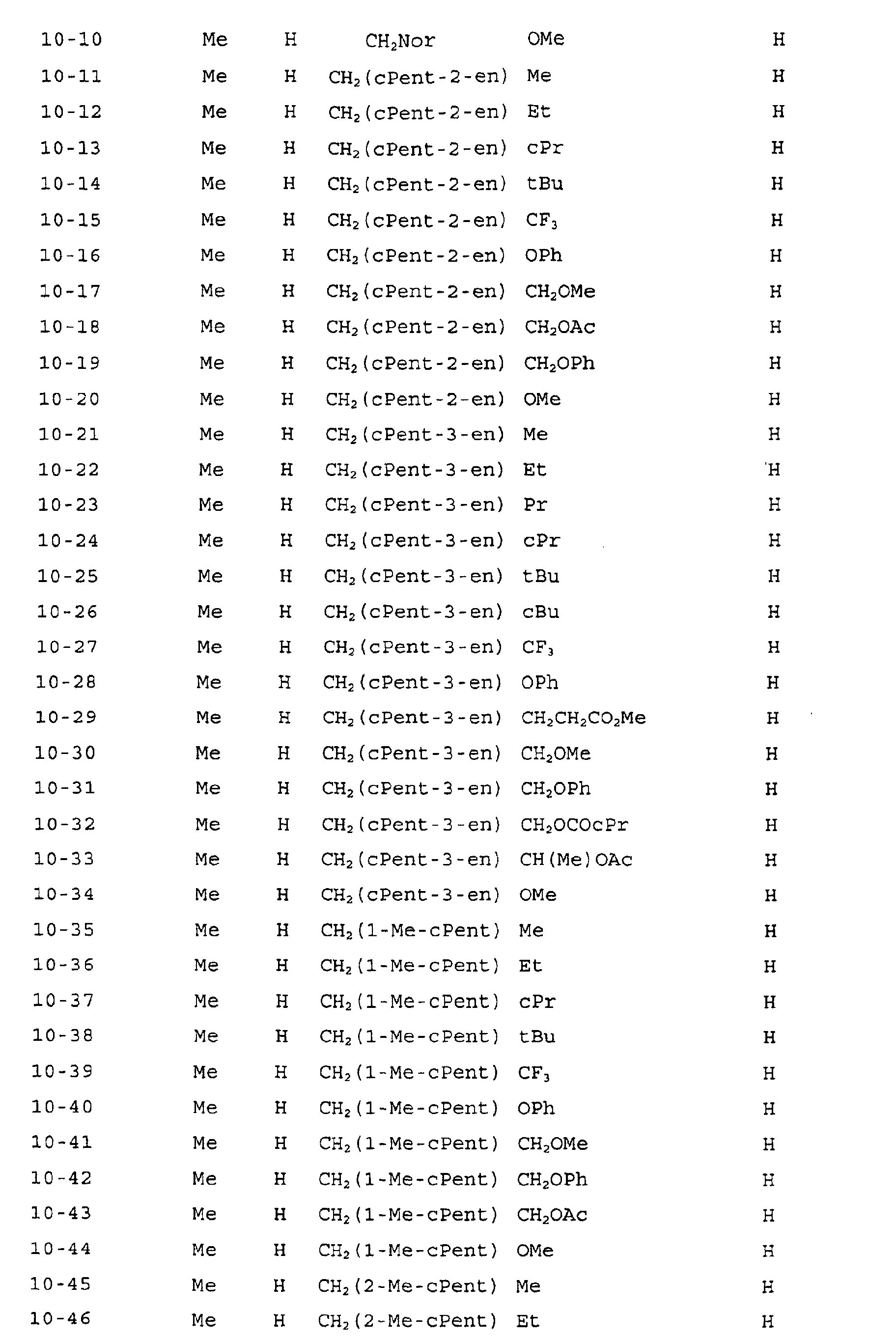

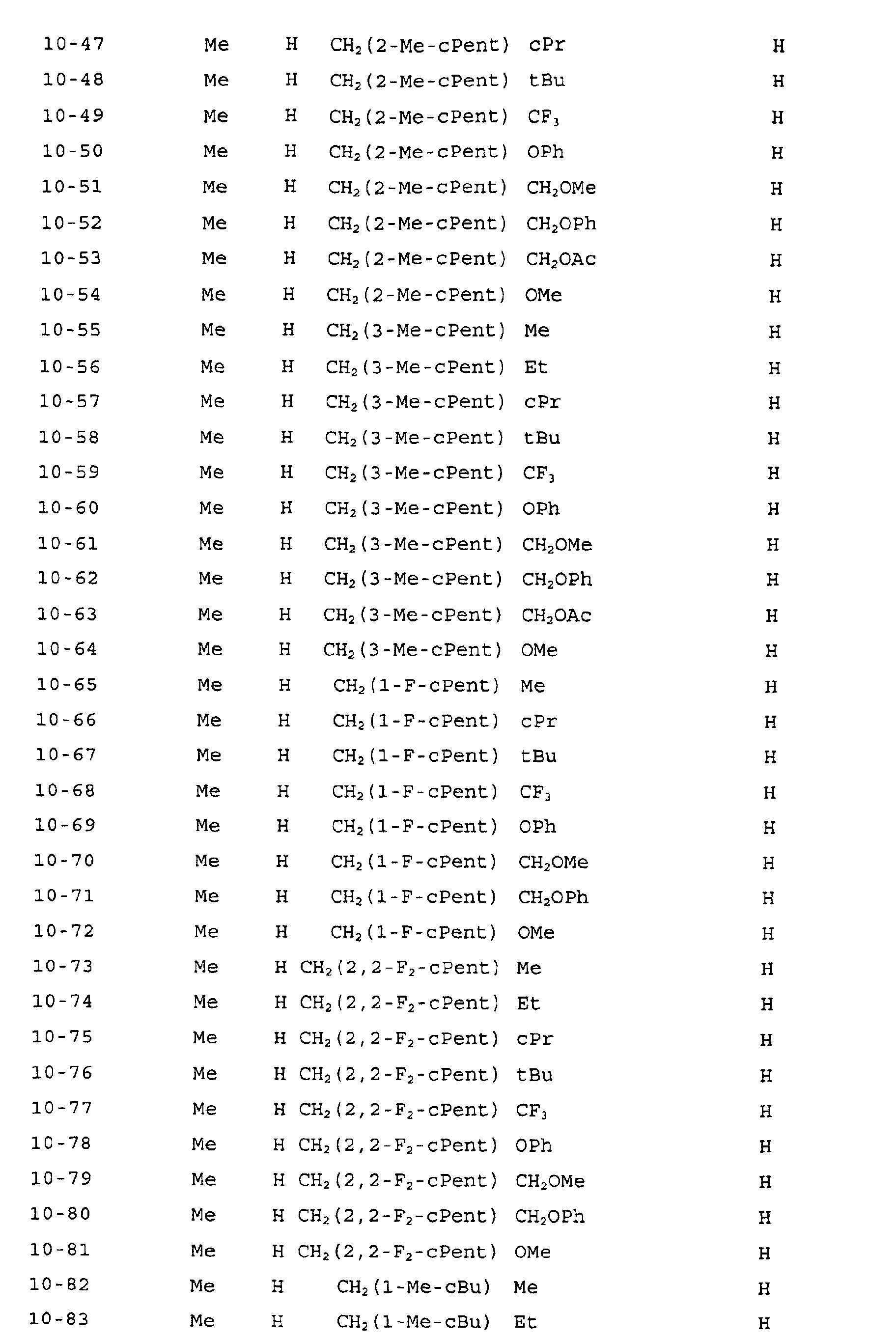

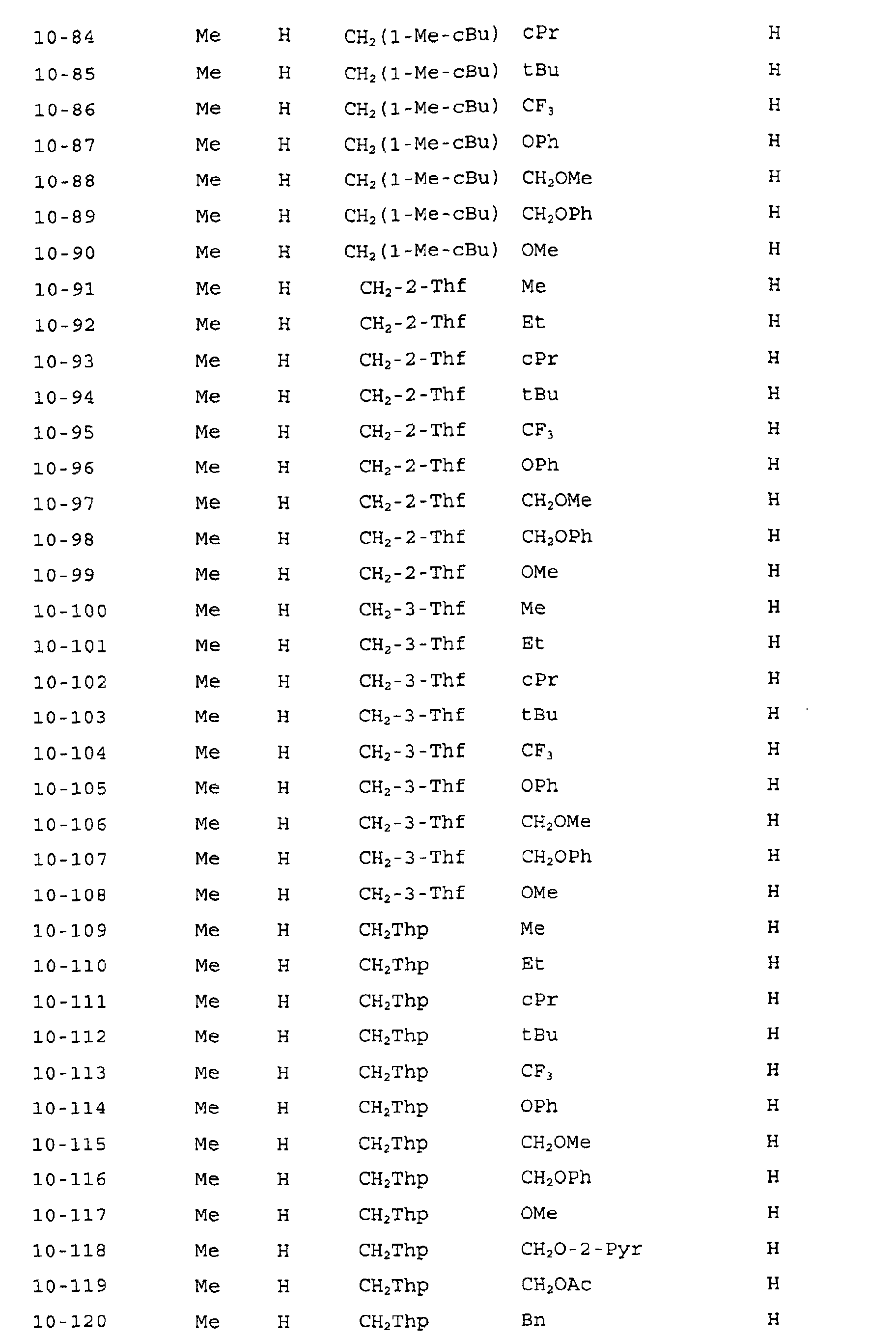

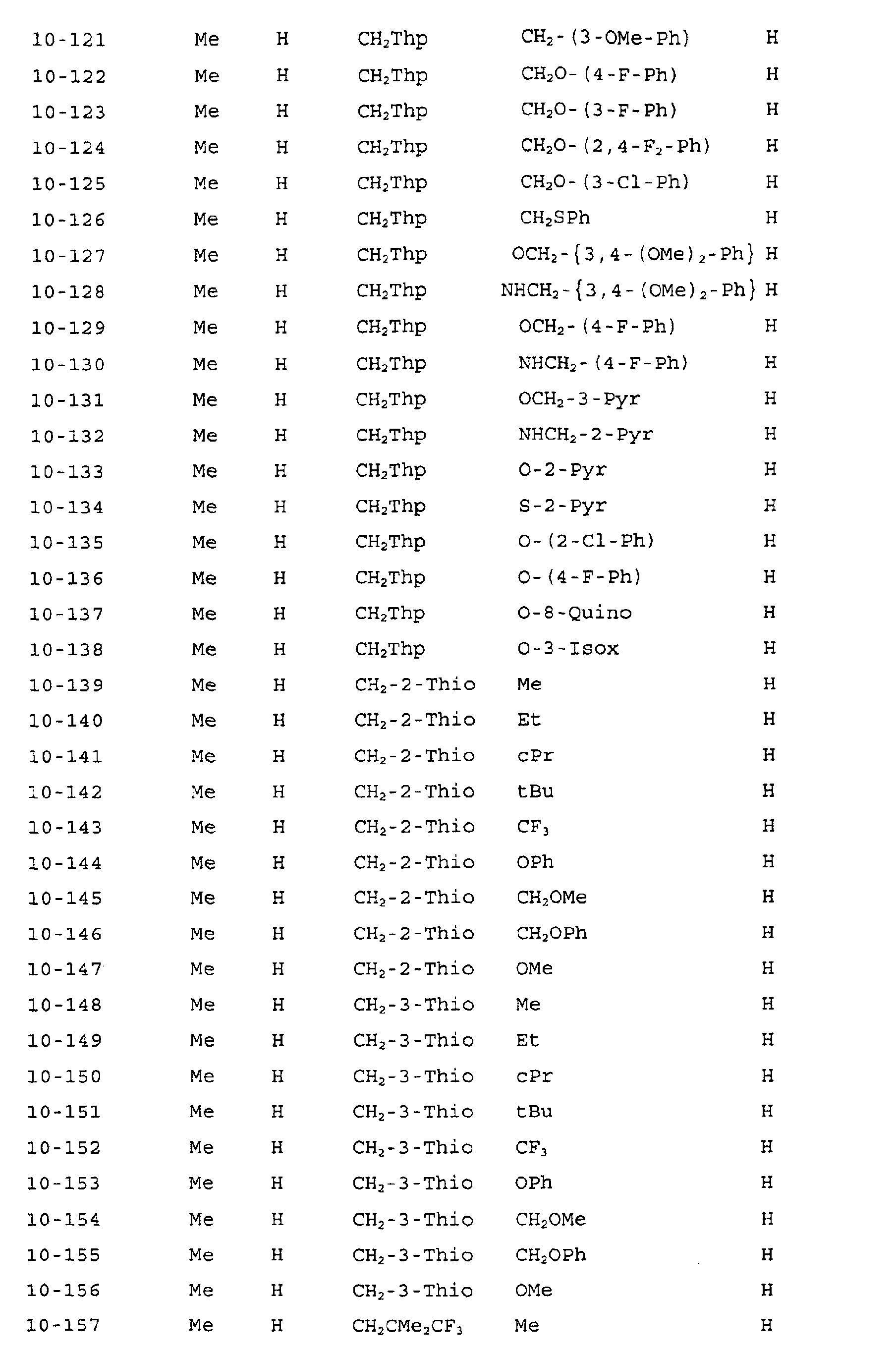

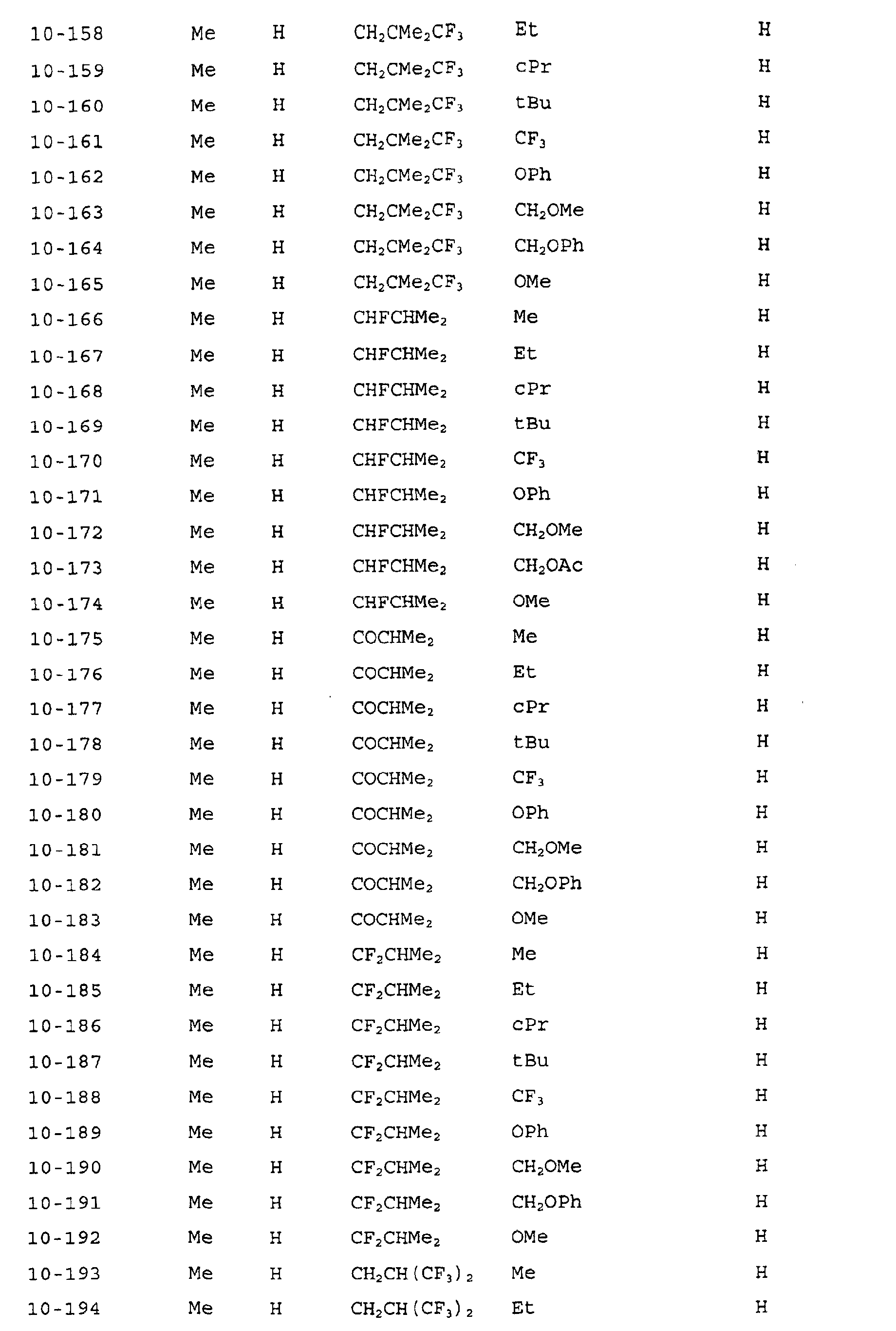

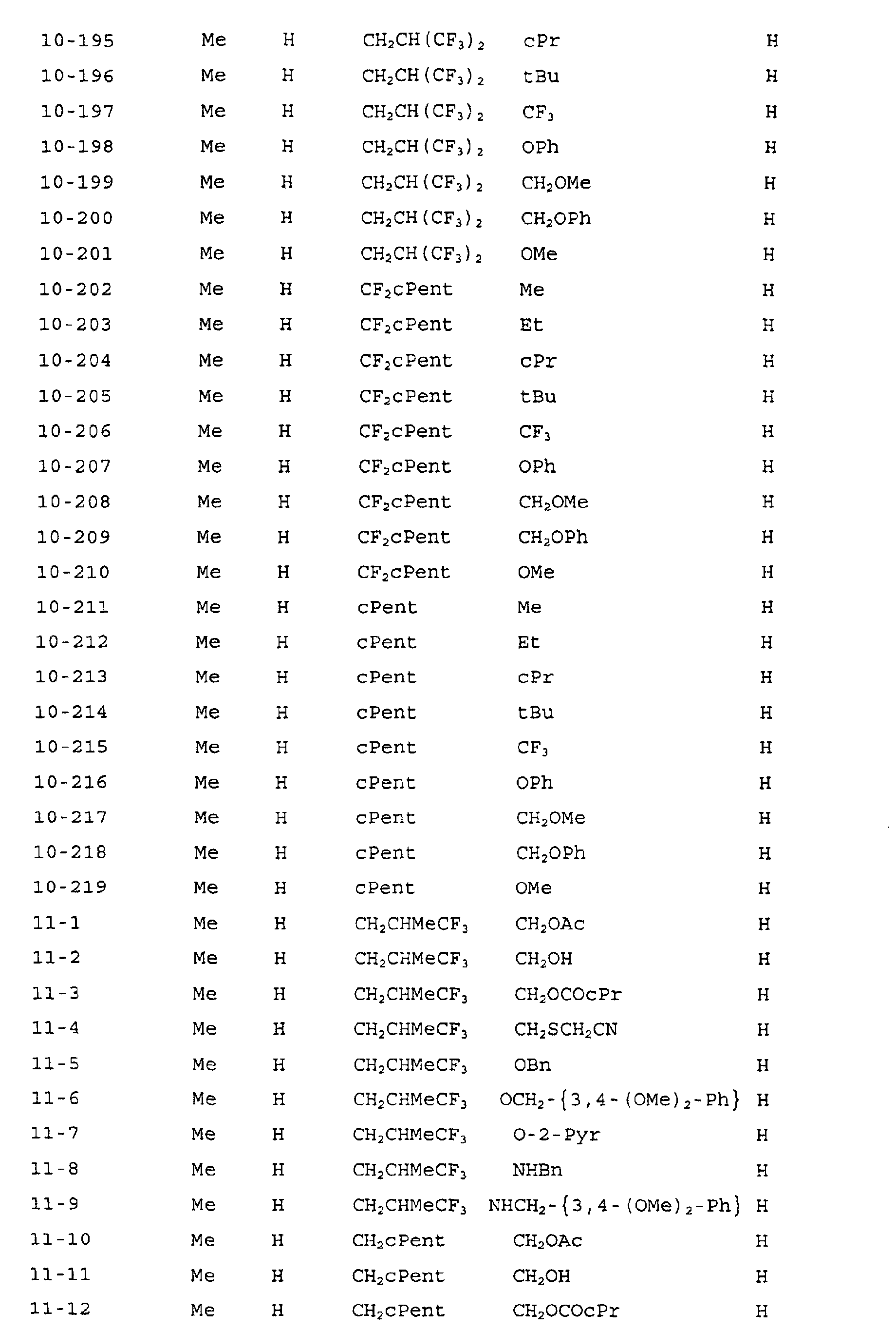

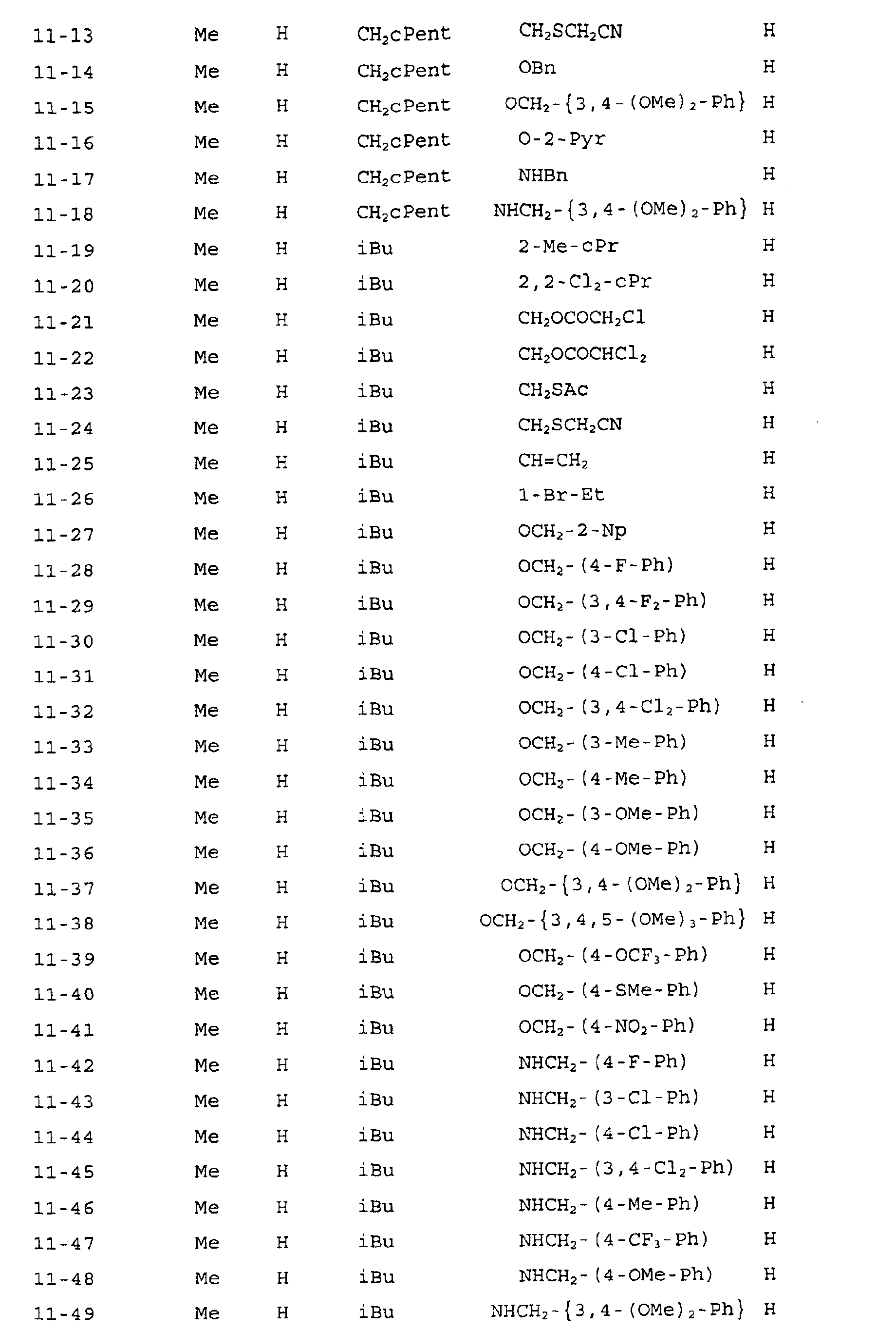

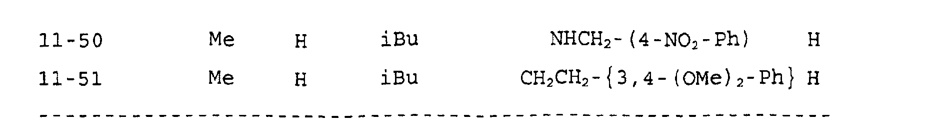

Of the compounds listed above, preferred are Compounds No. 2-38, 2-67, 2-73, 2-75, 3-37, 4-1, 4-2, 4-11, 4-19, 4-24, 4-41, 4-43, 4-44, 4-49, 4-51, 4-54, 4-71, 4-74, 4-76, 4-77, 4-79, 5-3, 5-4, 5-9, 5-10, 5-51, 5-52, 5-53, 5-54, 5-94, 5-95, 5-96, 5-97, 5-100, 5-101, 5-103, 5-104, 6-1, 6-20, 6-55, 6-56, 6-57, 6-65, 6-67, 7-1, 7-24, 7-28, 7-29, 7-30, 7-31, 8-3, 8-4, 8-6, 8-7, 8-12, 8-13, 8-14, 8-15, 8-24, 8-25, 8-27, 8-28, 8-29, 8-32, 8-35, 8-38, 8-40, 8-42, 8-43, 8-48, 8-49, 8-50, 8-54, 8-91, 8-92, 10-1, 10-7, 10-11, 10-17, 10-21, 10-30, 10-51, 10-55, 10-61, 10-65, 10-73, 10-79, 10-81, 10-82, 10-88, 10-100, 10-114, 10-116, 10-118, 10-121, 10-123, 10-165, 10-175, 10-184, 10-201, 10-217 and 11-3;
more preferred are Compounds No. 4-11, 4-24, 4-41, 4-43, 4-44, 4-49, 4-51, 4-54, 4-71, 4-74, 4-76, 4-77, 4-79, 5-3, 5-4, 5-9, 5-10, 5-94, 5-95, 5-96, 5-97, 6-1, 6-20, 6-55, 6-56, 6-57, 6-65, 6-67, 7-1, 7-24, 7-28, 7-29, 7-30, 7-31, 8-3, 8-4, 8-12, 8-24, 8-27, 8-40, 8-42, 8-48, 8-92, 10-11, 10-17, 10-21, 10-30, 10-65, 10-73, 10-79, 10-81, 10-82, 10-88, 10-100, 10-114, 10-116, 10-118, 10-123, 10-165, 10-175, 10-184, 10-201, and 11-3; still more preferred are Compounds No. 4-41, 4-71, 4-79, 6-1, 6-20, 6-65, 7-28, 8-3, 10-114, and 10-165.
The 5-(m-cyanobenzylamino)pyrazole derivatives of the present invention can be prepared in accordance with any one of the below-described Methods A to C.

Method A comprises acylating a 5-aminopyrazole derivative of the formula (II), and benzylating the acylated derivative, thereby preparing the corresponding 5-(m-cyanobenzylamino)pyrazole derivative of the present invention represented by the formula (I).

### (Method A)

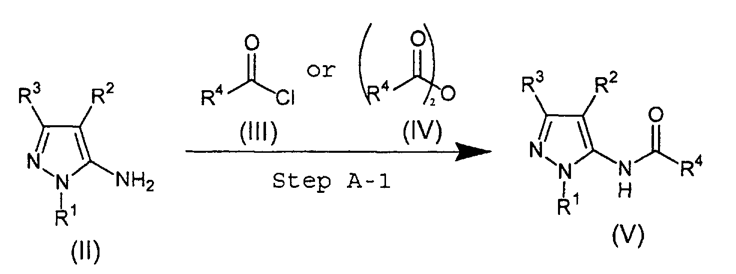

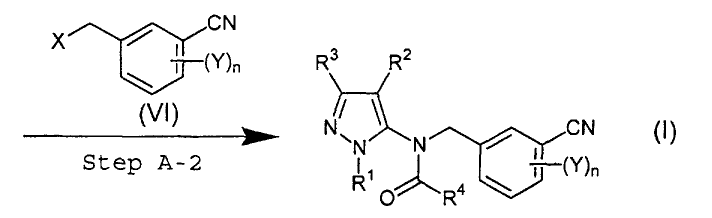

In the above-described reaction scheme, R¹, R², R³, R⁴, Y and n have the same meanings as described above, and X represents a halogen atom, a C₁₋₆ alkylsulfonyl group or a phenylsulfonyl group (said phenylsulfonyl group may be substituted with 1 to 5 substituents which may be the same or different and are selected from the group consisting of halogen atoms and C₁₋₆ alkyl groups) (preferably a chlorine atom, a methylsulfonyl group, a phenylsulfonyl group or a tolylsulfonyl group).

### (Step A-1)

Step A-1 is a step of reacting a 5-aminopyrazole derivative of the formula (II) with an acyl halide derivative of the formula (III) or an acid anhydride derivative of the formula (IV) in an inert solvent in the presence or absence of a base, thereby preparing the corresponding N-acylaminopyrazole derivative of the formula (V).

Compound (III) or (IV) is employed usually in an amount of from 1 to 3 mol, preferably from 1.1 to 1.5 mol, per mol of Compound (II).

When the base is used in this step, there is no particular limitation on it, provided that it may ordinarily be used as a base. Examples include alkali metal carbonates such as sodium carbonate and potassium carbonate, alkali metal bicarbonates such as sodium bicarbonate and potassium bicarbonate, alkali metal hydrides such as sodium hydride, lithium hydride and potassium hydride; alkali metal hydroxides or alkaline earth metal hydroxides such as sodium hydroxide, potassium hydroxide and barium hydroxide; alkali metal alkoxides such as sodium methoxide, sodium ethoxide and potassium t-butoxide; organic bases such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) and 1,8-diazabicyclo[5.4.0]undec-7-ene (DBU); and organic metal bases such as butyl lithium and lithium diisopropylamide. Of these, preferred are the organic bases, with pyridine being more preferred.

The base is employed usually in an amount of from 1 to 30 mol, preferably from 1.1 to 15 mol per mol of Compound (II).

There is no particular limitation on the solvent usable in this step, provided that it has no adverse effects on the reaction. Examples include hydrocarbons such as hexane, cyclohexane, benzene and toluene, halogenated hydrocarbons such as dichloromethane, dichloroethane, chloroform and tetrachloroethane, ethers such as dioxane, diethyl ether, tetrahydrofuran (THF) and ethylene glycol dimethyl ether, amides such as dimethylformamide, dimethylacetamide and hexamethylphosphoric triamide (HMPA), ketones such as acetone, methyl ethyl ketone, methyl isobutyl ketone and cyclohexanone, nitriles such as acetonitrile and isobutyronitrile, and esters such as methyl acetate, ethyl acetate and propyl acetate. Of these, preferred are the halogenated hydrocarbons and ethers, with dichloromethane, dichloroethane and THF being more preferred.

The reaction temperature varies depending on the nature of the starting material, reaction reagent and solvent, but usually ranges from -20 to 150°C, preferably from 0 to 90°C.

The reaction time varies depending on the nature of the starting material, reaction reagent, solvent and reaction temperature, but it usually ranges from 10 minutes to 100 hours, preferably from 30 minutes to 72 hours.

Compound (II) used in this step is a known compound or can be prepared in a known manner (for example, a process as described in Pharmazie, 48(10), 732(1993), or Chemistry and Pharmaceutical Bulletin (Chem. Pharm. Bull.), 35(8), 3235(1987)).

### (Step A-2)

Step A-2 is a step of reacting an N-acylaminopyrazole derivative of the formula (V) with a benzylating reagent of the formula (VI) in an inert solvent in the presence or absence of a base, thereby preparing the compound of the formula (I) of the invention.

Compound (VI) is employed usually in an amount of from 1 to 3 mol, preferably, from 1.1 to 1.5 mol, per mol of Compound (V).

There is no particular limitation on the base usable in this step, provided that it may ordinarily be used as a base. Examples include alkali metal carbonates such as sodium carbonate and potassium carbonate, alkali metal bicarbonates such as sodium bicarbonate and potassium bicarbonate, alkali metal hydrides such as sodium hydride, lithium hydride and potassium hydride; alkali metal hydroxides or alkaline earth metal hydroxides such as sodium hydroxide, potassium hydroxide and barium hydroxide; alkali metal alkoxides such as sodium methoxide, sodium ethoxide and potassium t-butoxide; organic bases such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) and 1,8-diazabicyclo[5.4.0]undec-7-ene (DBU); and organic metal bases such as butyl lithium and lithium diisopropylamide. Of these, preferred are the alkali metal hydrides, with sodium hydride being more preferred.

The base is employed usually in an amount of from 1 to 5 mol, preferably from 1.1 to 2.5 mol per mol of Compound (V).

There is no particular limitation on the solvent usable in the present invention, provided that it has no adverse effects on the reaction. Examples include hydrocarbons such as hexane, cyclohexane, benzene and toluene, halogenated hydrocarbons such as dichloromethane, dichloroethane, chloroform and tetrachloroethane, ethers such as dioxane, diethyl ether, tetrahydrofuran (THF) and ethylene glycol dimethyl ether, amides such as dimethylformamide, dimethylacetamide and hexamethylphosphoric triamide (HMPA), ketones such as acetone, methyl ethyl ketone, methyl isobutyl ketone and cyclohexanone, nitriles such as acetonitrile and isobutyronitrile, and esters such as methyl acetate, ethyl acetate and propyl acetate. Of these, preferred are the amides, with dimethylformamide being more preferred.

The reaction temperature varies depending on the nature of the starting material, reaction reagent and solvent, but usually ranges from -20 to 150°C, preferably from 0 to 90°C.

The reaction time varies depending on the nature of the starting material, reaction reagent, solvent and reaction temperature, but it usually ranges from 10 minutes to 24 hours, preferably from 30 minutes to 10 hours.

Method B comprises benzylating Compound (II) and then acylating the benzylated compound, thereby preparing the corresponding 5-(m-cyanobenzylamino)pyrazole derivative of the present invention reprensented by the formula (I) or (XV).

### (Method B)

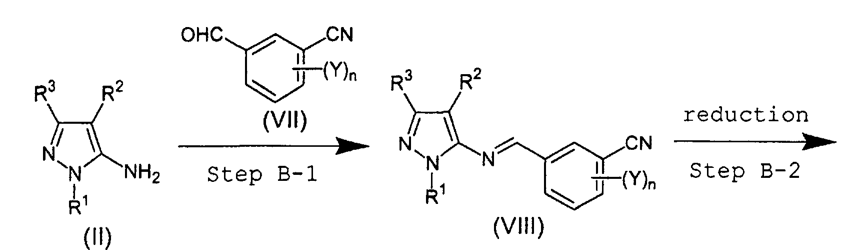

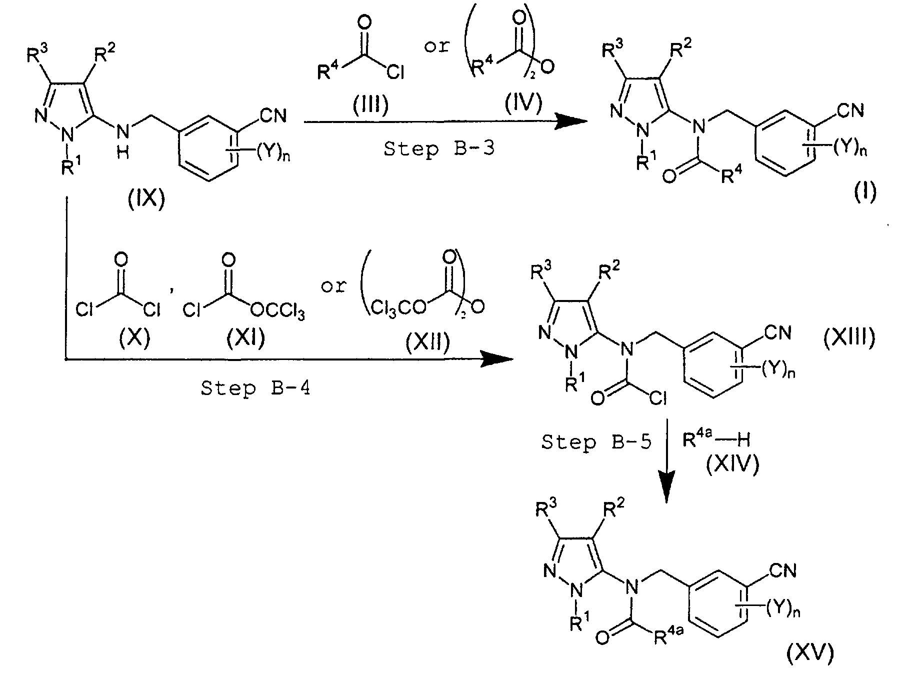

In the above-described reaction scheme, R¹, R², R³, R⁴, Y and n have the same meanings as described above, and
R^{4a} represents a C₁₋₆ alkoxy, (C₁₋₆ alkoxy)-C₁₋₆ alkoxy, (C₁₋ ₆ alkylamino)-C₁₋₆ alkoxy, di(C₁₋₆ alkyl)amino-C₁₋₆ alkoxy, (substituted or unsubstituted heteroaryl)-C₁₋₆ alkoxy (said substituent(s) is (are) 1 to 3 substituents which are selected from the below-described substituent group B and may be the same or different), C₃₋₇ cycloalkoxy, C₃₋₆ alkenyloxy, substituted or unsubstituted phenoxy (said substituent(s) is (are) 1 to 5 substituents which are selected from the below-described substituent group A and may be the same or different), substituted or unsubstituted benzyloxy (said substituent(s) is (are) 1 to 5 substituents which are selected from the below-described substituent group A and may be the same or different), substituted or unsubstituted heteroaryloxy (said substituent(s) is (are) 1 to 3 substituents which are selected from the below-described substituent group B and may be the same or different), substituted or unsubstituted C₁₋₆ alkylamino (said substituent is a phenyl or C₁₋₆ alkoxy group), C₃₋₆ alkenylamino, di(C₁₋₆ alkyl)amino, substituted or unsubstituted 5- or 6-membered nitrogen-containing saturated heterocyclyl (said substituent(s) is (are) 1 or 2 C₁₋₆ alkyl groups which may be the same or different, and said heterocyclyl group may additionally contain one oxygen atom or NH group), substituted or unsubstituted C₁₋₆ alkylthio (said substituent is a phenyl or C₁₋₆ alkoxy group), C₃₋₆ alkenylthio, substituted or unsubstituted phenylthio (said substituent(s) is (are) 1 to 5 substituents which are selected from the below-described substituent group A and may be the same or different), or substituted or unsubstituted heteroarylthio group (said substituent(s) is (are) 1 to 3 substituents which are selected from the below-described substituent group B and may be the same or different).

### (Step B-1)

Step B-1 is a step of condensing a 5-aminopyrazole derivative of the formula (II) with an aldehyde derivative of the formula (VII) in an inert solvent, thereby preparing the corresponding imine derivative of the formula (VIII).

Compound (VII) is added usually in an amount of from 1 to 3 mol, preferably from 1.1 to 1.5 mol, per mol of Compound (II).

There is no particular limitation on the solvent usable in this step, provided that it has no adverse effects on the reaction. Examples include alcohols such as methanol, ethanol and propanol. Of these, ethanol is preferred.

The reaction temperature varies depending on the nature of the starting material, reaction reagent and solvent, but it usually ranges from -50°C to the boiling point of the solvent, preferably from 10 to 30°C.

The reaction time varies depending on the nature of the starting material, reaction reagent, solvent and the reaction temperature, but it usually ranges from 1 to 48 hours, preferably from 4 to 18 hours.

### (Step B-2)

Step B-2 is a step of reacting Compound (VIII) with a reducing agent in an inert solvent, thereby preparing the corresponding secondary amine derivative of the formula (IX).

There is no particular limitation on the reducing agent usable in the present step, provided that it can ordinarily be used as a reducing agent for reducing an imine to the corresponding amine. Examples include lithium aluminum hydride sodium borohydride, sodium cyanoborohydride, alane and diborane. Of these preferred is sodium cyanoborohydride.

The reducing agent is added usually in an amount of 1 to 10 mol, preferably 2 to 5 mol, per mol of Compound (VIII).

There is no particular limitation on the solvent usable in the present step, provided that it has no adverse effects on the reaction. Examples include alcohols such as methanol, ethanol and propanol, ethers such as dioxane, diethyl ether, tetrahydrofuran (THF) and ethylene glycol dimethyl ether, and organic acids such as formic acid, acetic acid and propionic acid. Of these, the organic acids are preferred, with acetic acid being more preferred.

The reaction temperature varies depending on the nature of the starting material, reaction reagent and solvent, but it usually ranges from -50°C to the boiling point of the solvent, preferably from 10 to 30°C.

The reaction time varies depending on the nature of the starting material, reaction reagent, solvent and the reaction temperature, but it usually ranges from 1 to 24 hours, preferably from 4 to 10 hours.

### (Step B-3)

Step B-3 is a step of reacting Compound (IX) with Compound (III) or (IV) in an inert solvent in the presence or absence of a base, thereby preparing the corresponding compound (I) of the invention.

This step can be carried out in accordance with Step A-1.

### (Step B-4)

Step B-4 is a step of reacting Compound (IX) with an acyl chloride forming reagent of the formula (X), (XI) or (XII) in an inert solvent in the presence of a base, thereby preparing the corresponding acyl chloride derivative of the formula (XIII).

Compound (X), (XI) or (XII) is added usually in an amount of from 1 to 5 mol, preferably from 1.1 to 2.5 mol in the case of Compound (X), usually in an amount of from 0.5 to 2.5 mol, preferably from 0.6 to 1 mol in the case of Compound (XI) or usually in an amount of from 0.33 to 1 mol, preferably from 0.35 to 0.5 mol in the case of Comound (XII), each per mol of Compound (IX).

There is no particular limitation on the base usable in this step, provided that it is used as a base in the ordinary reaction. Examples include alkali metal carbonates such as sodium carbonate and potassium carbonate, alkali metal bicarbonates such as sodium bicarbonate and potassium bicarbonate, alkali metal hydrides such as sodium hydride, lithium hydride and potassium hydride; alkali metal hydroxides or alkaline earth metal hydroxides such as sodium hydroxide, potassium hydroxide and barium hydroxide; alkali metal alkoxides such as sodium methoxide, sodium ethoxide and potassium t-butoxide; organic bases such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) and 1,8-diazabicyclo[5.4.0]undec-7-ene (DBU); and organic metal bases such as butyl lithium and lithium diisopropylamide. Of these, preferred are the organic bases, with triethylamine being more preferred.

There is no particular limitation on the solvent usable in the present invention, provided that it has no adverse effects on the reaction. Examples include hydrocarbons such as hexane, cyclohexane, benzene and toluene, halogenated hydrocarbons such as dichloromethane, dichloroethane, chloroform and tetrachloroethane, ethers such as dioxane, diethyl ether, tetrahydrofuran (THF) and ethylene glycol dimethyl ether, amides such as dimethylformamide, dimethylacetamide and hexamethylphosphoric triamide (HMPA), ketones such as acetone, methyl ethyl ketone, methyl isobutyl ketone and cyclohexanone, nitriles such as acetonitrile and isobutyronitrile, and esters such as methyl acetate, ethyl acetate and propyl acetate. Of these, preferred are the halogenated hydrocarbons, with dichloromethane and dichloroethane being more preferred.

The reaction temperature varies depending on the nature of the starting material, reaction reagent and solvent, but usually ranges from -20 to 150°C, preferably from 0 to 40°C.

The reaction time varies depending on the nature of the starting material, reaction reagent, solvent and reaction temperature, but it usually ranges from 10 minutes to 120 hours, preferably from 30 minutes to 72 hours.

### (Step B-5)

Step B-5 is a step of reacting Compound (XIII) with a compound of the formula (XIV) in an inert solvent in the presence or absence of a base, thereby preparing the corresponding compound (XV) of the invention.

Compound (XIV) is usually added in an amount of from 1 to 3 mol, preferably from 1.1 to 1.5 mol, per mol of Compound (XIII).

When the base is employed in this step, there is no particular limitation on the base, provided that it can ordinarily be used as a base. Examples include alkali metal carbonates such as sodium carbonate and potassium carbonate, alkali metal bicarbonates such as sodium bicarbonate and potassium bicarbonate, alkali metal hydrides such as sodium hydride, lithium hydride and potassium hydride; alkali metal hydroxides or alkaline earth metal hydroxides such as sodium hydroxide, potassium hydroxide and barium hydroxide; alkali metal alkoxides such as sodium methoxide, sodium ethoxide and potassium t-butoxide; organic bases such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diazabicyclo[2.2.2]octane (DABCO) and 1,8-diazabicyclo[5.4.0]undec-7-ene (DBU); and organic metal bases such as butyl lithium and lithium diisopropylamide. Of these, preferred are the organic bases with triethylamine being more preferred.

The base is employed usually in an amount of from 1 to 3 mol, preferably from 1.1 to 1.5 mol per mol of Compound (XIII)

There is no particular limitation on the solvent usable in the present invention, provided that it has no adverse effects on the reaction. Examples include hydrocarbons such as hexane, cyclohexane, benzene and toluene, halogenated hydrocarbons such as dichloromethane, dichloroethane, chloroform and tetrachloroethane, ethers such as dioxane, diethyl ether, tetrahydrofuran (THF) and ethylene glycol dimethyl ether, amides such as dimethylformamide, dimethylacetamide and hexamethylphosphoric triamide (HMPA), ketones such as acetone, methyl ethyl ketone, methyl isobutyl ketone and cyclohexanone, nitriles such as acetonitrile and isobutyronitrile, and esters such as methyl acetate, ethyl acetate and propyl acetate. Of these, preferred are the nitriles, with acetonitrile being more preferred.

The reaction temperature varies depending on the nature of the starting material, reaction reagent and solvent, but usually ranges from -20 to 150°C, preferably from 0 to 100°C.

The reaction time varies depending on the nature of the starting material, reaction reagent, solvent or reaction temperature, but it usually ranges from 10 minutes to 120 hours, preferably from 30 minutes to 72 hours. Method C comprises hydrolyzing Compound (I) and acylating the hydrolysate, thereby preparing the corresponding compound (I) of the invetion.

### (Method C)

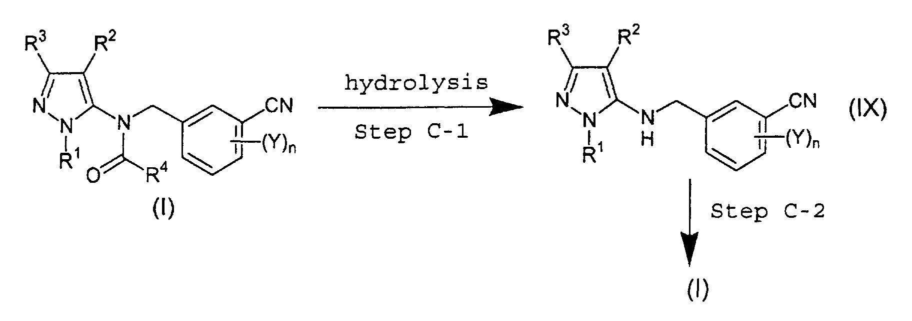

In the above-described reaction scheme, R¹, R², R³, R⁴, Y and n have the same meanings as described above.

### (Step C-1)

Step C-1 is a step of hydrolyzing Compound (I) in the presence of a base in water, thereby preparing the corresponding Compound (IX).

There is no particular limitation on the base usable in this step, provided that it can ordinarily be used as a base. Examples include alkali metal carbonates such as sodium carbonate and potassium carbonate, alkali metal bicarbonates such as sodium bicarbonate and potassium bicarbonate, alkali metal hydrides such as sodium hydride, lithium hydride and potassium hydride; alkali metal hydroxides or alkaline earth metal hydroxides such as sodium hydroxide, potassium hydroxide and barium hydroxide; alkali metal alkoxides such as sodium methoxide, sodium ethoxide and potassium t-butoxide; organic bases such as triethylamine, tributylamine, diisopropylethylamine, N-methylmorpholine, pyridine, 4-(N,N-dimethylamino)pyridine, N,N-dimethylaniline, N,N-diethylaniline, 1,5-diazabicyclo[4.3.0]non-5-ene, 1,4-diagzabicyclo [2.2.2]octone (DABCO) and 1,8-diazabicyclo[2.2.2]octane (DABCO) and 1,8-diazabicyclo[5.4.0]undec-7-ene (DBU); and organic metal bases such as butyl lithium and lithium diisopropylamide. Of these, preferred are the alkali metal hydroxides or alkaline earth metal hydroxides, with sodium hydroxide being more preferred.

The base is added usually in an amount of 1 to 100 mol, preferably 3 to 30 mol per mol of Compound (I).

The reaction temperature varies depending on the nature of the starting material, reaction reagent and solvent, but usually ranges from -20 to 100°C, preferably from 0 to 40°C.

The reaction time varies depending on the nature of the starting material, reaction reagent, solvent and reaction temperature, but it usually ranges from 10 minutes to 24 hours, preferably from 30 minutes to 10 hours.

### (Step C-2)

Step C-2 is a step of acylating Compound (IV), thereby preparing the corresponding compound (I) of the invention.

This step can be carried out in accordance with Step B-3, or Step B-4 and Step B-5.

After completion of each of the above-described reactions, the target compound of each reaction can be collected from the reaction mixture by a known method. For example, it is available by neutralizing the reaction mixture, if necessary, removing therefrom insoluble matter, if any, by filtration, adding to the residue an organic solvent such as ethyl acetate not miscible with water, washing the resulting mixture with water, separating the mixture to obtain the organic layer containing the target compound, drying it over anhydrous magnesium sulfate, and then removing the solvent by distillation.

The target compound thus obtained can be purified further by a known method, for example, recrystallization, reprecipitation or chromatography if necessary. It is needless to say that purification may be terminated at any stage of the purification and a crude product can be provided as an effective ingredient or a starting material to be provided to the subsequent reaction.

The salt of the compound (I) of the invention is prepared by adding an acid to an extracted concentrate of the reaction mixture containing the compound (I) of the present application prepared in each step or a solution of the compound (I) in a suitable solvent.

Examples of the acid used in the above-described reaction include hydrohalic acids such as hydrofluoric acid, hydrochloric acid, hydrobromic acid and hydroiodic acid, inorganic acids such as nitric acid, perchloric acid, sulfuric acid and phosphoric acid, lower alkylsulfonic acids such as methanesulfonic acid, trifluoromethanesulfonic acid and ethanesulfonic acid, arylsulfonic acids such as benzenesulfonic acid and p-toluenesulfonic acid, organic acids metal such as succinic acid and oxalic acid, and organic acid amide compounds such as saccharin.

The acid is added usually in an amount of 1 to 10 equivalents, preferably 1 to 5 equivalents.

There is no particular limitation on the solvent usable in the reaction, provided that it has no adverse effects on the reaction. Preferred examples include ethers such as ether, diisopropyl ether, tetrahydrofuran (THF), and dioxane, and alcohols such as methanol and ethanol.

The reaction temperature ranges from -20 to 50°C, preferably -10°C to 30°C.

The reaction time varies depending on the temperature and nature of the solvent, but it usually ranges from 10 minutes to 1 hour.

The salt thus formed is isolated by a known method. It is isolated by filtration when precipitation as crystals is desired. When it is a water soluble salt, it is isolated as an aqueous solution by separation into an organic solvent and water.

The compounds of the invention can be used as an effective ingredient of a pesticide against harmful organisms. For example, as an agricultural or horticultural fungicide, they exhibit excellent control effects against diseases caused by various plant pathogens. They exhibit particularly excellent control effects against downy mildew of cucumbers or grape vines, late blight of tomatoes or potatoes, or various diseases caused by *Oomycetes* including *Pythium* and *Aphanomyces.* The invention compounds exhibit not only excellent preventive effects with a long aftereffect, but also excellent treatment effects, so that even afer infection, diseases can be controlled by the treatment with them. Soil treatment with the invention compounds is also effective for the control of diseases. Moreover, they have beneficial effects against various drug-resistant fungi (downy mildew fungus resistant to metalaxyl, down mildew fungus resistant to strobilurin fungicide, etc.) as well as sensitive fungi.

Upon use of the invention compounds, they can be formulated, similar to the conventional agricultural chemicals, as preparations in a variety of forms such as emulsifiable concentrates, dust formulations, wettable powders, liquid formulations, granules and suspensions. Upon practical use of these preparations, they can be used as are or diluted with a diluent such as water to a predetermined concentration.

Examples of an adjuvant include carrier, emulsifier, suspending agent, dispersant, extender, penetrant, humectant, thickener and stabilizer. They can be added according to need.

The carriers usable here can be classified into a solid carrier and a liquid carrier. Examples of the solid carrier include animal or plant powder such as starch, sugar, cellulose powder, cyclodextrin, activated charcoal, soybean meal, wheat flour, bran powder, wood meal, fish meal, and powdery milk; and mineral powders such as talc, kaolin, bentonite, organic bentonite, calcium carbonate, calcium sulfate, sodium bicarbonate, zeolite, diatomaceous earth, white carbon, clay, alumina, silica, and sulfur powder. Examples of the liquid carrier include water, animal or plant oils such as soybean oil, cotton seed oil and corn oil, alcohols such as ethyl alcohol and ethylene glycol, ketones such as acetone and methyl ethyl ketone, ethers such as dioxane and tetrahydrofuran, aliphatic hydrocarbons such as kerosene, coal oil and liquid paraffin, aromatic hydrocarbons such as xylene, trimethylbenzene, tetramethylbenzene, cyclohexane and sorbent naphtha, halogenated hydrocarbons such as chloroform and chlrobenzene, amides such as dimethylformamide, esters such as ethyl acetate and glycerin ester of a fatty acid, nitriles such as acetonitrile, sulfur-containing compounds such as dimethylsulfoxide, and N-methylpyrrolidone.

The mass ratio of the compound of the invention to the adjuvant usually ranges from 0.05:99.95 to 90:10, preferably from 0.2:99.8 to 80:20.

The concentration or amount of the compound of the invention varies, depending on the crop to be applied, using method, form of preparation or application rate. For foliar treatment, 0.1 to 10000 ppm, preferably 1 to 1000 ppm in terms of an effective ingredient is usually applied, for soil treatment, 10 to 100000 g/ha, preferably 200 to 20000 g/ha is usually applied.

The compound of the invention can be used as a mixture or in combination with another agricultural chemical if necessary, for example, an insecticide, miticide, attractant, nematicide, fungicide, antiviral agent, herbicide, or plant growth regulator. Combined use with an insecticide, miticide, nematicide or fungicide is preferred.

Examples of the insecticide, miticide or nematicide include:
organic phosphate compounds such as O-(4-bromo-2-chlorophenyl)-O-ethyl S-propylphosphorothioate (common name: profenofos), 0-(2,2-dichlorovinyl) O,O-dimethylphosphate (common name: dichlorvos), O-ethyl O-[3-methyl-4-(methylthio)phenyl] N-isopropylphosphoramidate (common name: fenamiphos), O,O-dimethyl O-(4-nitro-m-tolyl)phosphorothioate (common name: fenitrothion), O-ethyl O-(4-nitrophenyl)phenylphosphonothioate (common name: EPN), O,O-diethyl O-(2-isopropyl-6-methylpyrimidin-4-yl)phosphorothioate (common name: diazinon), O,O-dimethyl O-(3,5,6-trichloro-2-pyridyl)phosphorothioate (common name: chlorpyrifos-methyl), O,S-dimethyl N-acetylphosphoramide thioate (common name: acephate) and 0-(2,4-dichlorophenyl) O-ethyl S-propylphosphorodithioate (common name: prothiofos) ; carbamate compounds such as 1-naphthyl N-methylcarbamate (common name: carbaryl), 2-isopropoxyphenyl N-methylcarbamate (common name: propoxur), 2-methyl-2-(methylthio)propionaldehyde O-methylcarbamoyloxime (common name: aldicarb), 2,3-dihydro-2,2-dimethylbenzofuran-7-yl N-methylcarbamate (common name: carbofuran), dimethyl N,N'-[thiobis{(methylimino)carbonyloxy}]bisethaneimidothioate (common name: thiodicarb), S-methyl N-(methylcarbamoyloxy)thioacetoimidate (common name: methomyl), N,N-dimethyl-2-methylcarbamoyloxyimino-2-(methylthio)acetamide (common name: oxamyl), 2-(ethylthiomethyl)phenyl N-methylcarbamate (common name: ethiofencarb), 2-dimethylamino-5,6-dimethylpyrimidin-4-yl N,N-dimethylcarbamate (common name: pirimicarb) and 2-sec-butylphenyl N-methylcarbamate (common name: fenobucarb);
nereis toxin derivatives such as S,S'-2-dimethylaminotrimethylenebis (thiocarbamate) (common name: cartap), and N,N-dimethyl-1,2,3-trithian-5-ylamine (common name: thiocyclam); organic chlorine compounds such as 2,2,2-trichloro-1,1-bis(4-chlorophenyl)ethanol (common name: dicofol) and 4-chlorophenyl-2,4,5-trichlorophenylsulfone (common name: tetradifon); organic metal compounds such as bis[tris(2-methyl-2-phenylpropyl)tin] oxide (common name: fenbutatin oxide);
pyrethroid compounds such as (RS)-α-cyano-3-phenoxybenzyl (RS)-2-(4-chlorophenyl)-3-methylbutyrate (common name: fenvalerate), 3-phenoxybenzyl (1RS)-cis,trans-3-(2,2-dichlorovinyl)-2,2-dimethylcyclopropanecarboxylate (common name: permethrin), (RS)-α-cyano-3-phenoxybenzyl (1RS)-cis,trans-3-(2,2-dichlorovinyl)-2,2-dimethylcyclopropanecarboxylate (common name: cypermethrin), (S)-α-cyano-3-phenoxybenzyl (1R)-cis-3-(2,2-dibromovinyl)-2,2-dimethylcyclopropanecarboxylate (common name: deltamethrin), (RS)-α-cyano-3-phenoxybenzyl (1RS)-cis,trans-3-(2-chloro-3,3,3-trifluoropropenyl)-2,2-dimethylcyclopropanecarboxylate (common name: cyhalothrin), 4-methyl-2,3,5,6-tetrafluorobenzyl-3-(2-chloro-3,3,3-trifluoro-1-propenyl)-2,2-dimethylcyclopropanecarboxylate (common name: tefluthrin), and 2-(4-ethoxyphenyl)-2-methylpropyl 3-phenoxybenzyl ether (common name: etofenprox);
benzoylurea compounds such as 1-(4-chlorophenyl)-3-(2,6-difluorobenzoyl)urea (common name: diflubenzuron), 1-[3,5-dichloro-4-(3-chloro-5-trifluoromethyl-2-pyridyloxy)phenyl]-3-(2,6-difluorobenzoyl)urea (common name: chlorfluazuron), and 1-(3,5-dichloro-2,4-difluorophenyl)-3-(2,6-difluorobenzoyl)urea (common name: teflubenzuron), juvenile hormone analogue compounds such as isopropyl (2E,4E)-11-methoxy-3,7,11-trimethyl-2,4-dodecadienoate (common name: methoprene);
pyridazinone compounds such as 2-t-butyl-5-(4-t-butylbenzylthio)-4-chloro-3(2H)-pyridazinone (common name: pyridaben); pyrazole compounds such as t-butyl 4-[(1,3-dimethyl-5-phenoxypyrazol-4-yl)methyleneaminoxymethyl] benzoate (common name: fenpyroximate);
nitro compounds such as 1-(6-chloro-3-pyridylmethyl)-N-nitro-imidazolidin-2-ylideneamine (common name: imidacloprid); and, as a dinitro compound, organic sulfur compound, urea compound, triazine compound, hydrazine compound, or the other compound, 2-tert-butylimino-3-isopropyl-5-phenyl-3,4,5,6-tetrahydro-2H-1,3,5-thiadiazin-4-one (common name: buprofezin), trans-(4-chlorophenyl)-N-cyclohexyl-4-methyl-2-oxothiazolidinone-3-carboxamide (common name: hexythiazox), N-methylbis(2,4-xylyliminomethyl)amine (common name: amitraz), N'-(4-chloro-o-tolyl)-N,N-dimethylformamidine (common name: chlorodimeform) and (4-ethoxyphenyl)-[3-(4-fluoro-3-phenoxyphenyl)propyl](dimethyl)silane (common name:
silafluofen). Moreover, the compounds of the invention can be used as a mixture or in combination with a microbial pesticide such as BT preparation or antiviral against insect-borne virus, or an antibiotic such as avermectin or milbemycin.
Examples of the fungicide usable here include:
pyrimidinamine compounds such as 2-anilino-4-methyl-6-(1-propynyl)-pyrimidine (common name: mepanipyrim) and 4,6-dimethyl-N-phenyl-2-pyrimidinamine (common name: pyrimethanil);
azole compounds such as 1-(4-chlorophenoxy)-3,3-dimethyl-1-(1H-1,2,4-triazol-1-yl)-butanone (common name: triadimefon), 1-(biphenyl-4-yloxy)-3,3-dimethyl-1-(1H-1,2,4-triazol-1-yl)butan-2-ol (common name: bitertanol), 1-[N-(4-chloro-2-trifluoromethylphenyl)-2-propoxyacetoimidoyl]imidazole (common name: triflumizole), 1-[2-(2,4-dichlorophenyl)-4-ethyl-1,3-dioxolan-2-ylmethyl]-1H-1,2,4-triazole (common name: etaconazole), 1-[2-(2,4-dichlorophenyl)-4-propyl-1,3-dioxolan-2-ylmethyl]-1H-1,2,4-triazole (common name: propiconazole), 1-[2-(2,4-dichlorophenyl)pentyl]-1H-1,2,4-triazole (common name: penconazole), bis(4-fluorophenyl)(methyl) (1H-1,2,4-triazol-1-ylmethyl]silane (common name: flusilazole), 2-(4-chlorophenyl)-2-(1H-1,2,4-triazol-1-ylmethyl)hexanenitrile (common name: myclobutanil), (2RS,3RS)-2-(4-chlorophenyl)-3-cyclopropyl-1-(1H-1,2,4-triazol-1-yl)butan-2-ol (common name: cyproconazole), RS-1-(4-chlorophenyl)-4,4-dimethyl-3-(1H-1,2,4-triazol-1-ylmethyl)pentan-3-ol (common name: tebuconazole), (RS)-2-(2,4-dichlorophenyl)-1-(1H-1,2,4-triazol-1-yl)hexan-2-ol (common name: hexaconazole), (2RS,5RS)-5-(2,4-dichlorophenyl)tetrahydro-5-(1H-1,2,4-triazol-1-ylmethyl)-2-furyl 2,2,2-trifluoroethyl ether (common name: furconazole-cis), N-propyl-N-[2-(2,4,6-trichlorophenoxy)ethyl]imidazole-1-carboxamide (common name: prochloraz), 2-(4-fluorophenyl)-1-(1H-1,2,4-triazol-1-yl)-3-trimethylsilylpropan-2-ol (common name: simeconazole);
quinoxaline series compounds such as 6-methyl-1,3-dithiolo[4,5-b]quinoxalin-2-one (common name: quinomethionate); dithiocarbamate compounds such as polymeric manganese ethylenebis(dithiocarbamate) (common name: maneb), polymeric zinc ethylenebis(dithiocarbamate) (common name: zineb), manganese ethylenebis(dithiocarbamate) (maneb) complex with zinc (common name: mancozeb), dizincbis(dimethyldithiocarbamate)ethylenebis (dithiocarbamate) (common name: polycarbamate), and polymeric zinc propylenebis(dithiocarbamate) (common name: propineb);
organochlorine compounds such as 4,5,6,7-tetrachlorophthalide (common name: phthalide), tetrachloroisophthalonitrile (common name: chlorothalonil) and pentachloronitrobenzene (common name: quintozene), benzimidazole compounds such as methyl 1-(butylcarbamoyl)benzimidazol-2-ylcarbamate (common name: benomyl), dimethyl 4,4'-(o-phenylene)bis(3-thioallophanate) (common name: thiophanate-methyl) and methyl benzimidazol-2-ylcarbamate (common name: carbendazim);
pyridinamine compounds such as 3-chloro-N-(3-chloro-2,6-dinitro-4-α,α,α-trifluorotolyl)-5-trifluoromethyl-2-pyridinamine (common name: fluazinam); cyanoacetamide compounds such as 1-(2-cyano-2-methoxyiminoacetyl)-3-ethylurea (common name: cymoxanil),
phenylamide compounds such as methyl N-(2-methoxyacetyl)-N-(2,6-xylyl)-DL-alaninate (common name: metalaxyl), 2-methoxy-N-(2-oxo-1,3-oxazolidin-3-yl)aceto-2',6'-xylidide (common name: oxadixyl), (±)-α-2-chloro-N-(2,6-xylylacetamido)-γ-butyrolactone (common name: ofurace), methyl N-phenylacetyl-N-(2,6-xylyl)-DL-alaninate (common name: benalaxyl), methyl N-(2-furoyl)-N-(2,6-xylyl)-DL-alaninate (common name: furalaxyl) and (±)-α-[N-(3-chlorophenyl)cyclopropanecarboxamido]-γ-butyrolactone (common name: cyprofuran);
sulfenic acid compounds such as N-dichlorofluoromethylthio-N',N'-dimethyl-N-phenylsulfamide (common name: dichlofluanid); copper compounds such as copper hydroxide (common name: copper (II) hydroxide), and copper 8-quinolinolate (common name: oxine-copper),
isoxazole compounds such as 5-methylisoxazol-3-ol (common name: hydroxyisoxazole); organophosphorus compounds such as aluminum tris(ethyl phosphonate) (common name: fosetyl-aluminium), O-2,6-dichloro-p-tolyl-O,O-dimethyl phosphorothioate (common name: tolclofos-methyl), S-benzyl O,O-diisopropylphosphorothioate, O-ethyl S,S-diphenylphosphorodithioate and aluminum ethylhydrogenphosphonate,
N-halogenothioalkyl compounds such as N-(trichloromethylthio) cyclohex-4-ene-1,2-dicarboximide (common name: captan), N-(1,1,2,2-tetrachloroethylthio)cyclohex-4-ene-1,2-dicarboximide (common name: captafol), and N-(trichloromethylthio)phthalimide (common name: folpet);
dicarboxyimide compounds such as N-(3,5-dichlorophenyl)-1,2-dimethylcyclopropane-1,2-dicarboximide (common name: procymidone), 3-(3,5-dichlorophenyl)-N-isopropyl-2,4-dioxoimidazolidine-1-carboxamide (common name: iprodione) and (RS)-3-(3,5-dichlorophenyl)-5-methyl-5-vinyl-1,3-oxazolidine-2,4-dione (common name: vinclozolin);
benzanilide compounds such as α,α,α-trifluoro-3'-isopropoxy-o-toluanilide (common name: flutolanil) and 3'-isopropoxy-o-toluanilide (common name: mepronil);
piperazine compounds such as N,N'-[piperazine-1,4-diylbis[(trichloromethyl)methylene]]diformamide (common name: triforine); pyridine compounds such as 2',4'-dichloro-2-(3-pyridyl)acetophenone O-methyloxime (common name: pyrifenox);
carbinol compounds such as (±)-2,4'-dichloro-α-(pyrimidin-5-yl)benzhydryl alcohol (common name: fenarimol), and (±)-2,4'-difluoro-α-(1H-1,2,4-triazol-1-ylmethyl)benzhydryl alcohol (common name: flutriafol);
piperidine compounds such as (RS)-1-[3-(4-tertiary-butylphenyl)-2-methylpropyl]piperidine (common name: fenpropidin);
morpholine compounds such as (+)-cis-4-[3-(4-tertiary-butylphenyl)-2-methylpropyl]-2,6-dimethylmorpholine (common name: fenpropimorph); organic tin compounds such as triphenyltin hydroxide (common name: fentin hydroxide), and triphenyltin acetate (common name: fentin acetate),
urea compounds such as 1-(4-chlorobenzyl)-1-cyclopentyl-3-phenylurea (common name: pencycuron);
cinnamic acid compounds such as (E,Z)-4-[3-(4-chlorophenyl)-3-(3,4-dimethoxyphenyl)acryloyl]morpholine (common name: dimethomorph),
phenylcarbamate compounds such as isopropyl 3,4-diethoxycarbanilate (diethofencarb), and
cyanopyrrole compounds such as 3-cyano-4-(2,2-difluoro-1,3-benzodioxol-4-yl)pyrrole (common name: fludioxonil) and 3-(2',3'-dichlorophenyl)-4-cyano-pyrrole (common name: fenpiclonil).

The compounds of the invention will hereinafter be described more specifically using Examples, Formulation Examples, and Tests. It should however be borne in mind that the present invention is not limited to or by them. Mass spectrometry was carried out by EI unless otherwise specifically indicated, but it is indicated as (APCI) when atmospheric pressure chemical ionization is employed.

### [BEST MODE FOR CARRYING OUT THE INVENTION]

### (Example 1)

### N-(3-Cyanobenzyl)-N-[3-(cyclobutylmethyl)-1-methyl-1H-pyrazol-5-yl]-2-methoxyacetamide (Compound of Compound No. 6-20)

### (1) N-[3-(Cyclobutylmethyl)-1-methyl-1H-pyrazol-5-yl]-2-methoxyacetamide (Step A-1)

In dichloroethane (60 ml) was dissolved 5-amino-3-(cyclobutylmethyl)-1-methyl-lH-pyrazole (3.0 g, 18.2 mmol). Methoxyacetyl chloride (2.1 ml, 22.7 mmol) was added to the resulting solution while stirring, followed by the dropwise addition of pyridine (1.8 ml, 22.7 mmol). The resulting mixture was stirred at room temperature for 18 hours. After completion of the reaction, water was added to the reaction mixture, which was then extracted with ethyl acetate. The organic layer was separated, washed with water, washed with a small amount of a 10% aqueous sodium bicarbonate solution, and dried over magnesium sulfate (MgSO₄). After filtration, the solvent was distilled off under reduced pressure, whereby 4.5 g of the title compound was obtained as an oil.

Nuclear magnetic resonance spectrum (¹H-NMR) (200 MHz, CDCl₃) δ ppm: 1.63-2.00 (6H,m), 2.48-2.70 (3H,m), 3.50 (3H,s), 3.68 (3H,s), 4.05 (2H,s), 6.08 (1H,s), 8.09 (1H,s).

### (2) N-(3-Cyanobenzyl)-N-[3-(cyclobutylmethyl)-1-methyl-1H-pyrazol-5-yl]-2-methoxyacetamide (Compound of Compound No. 6-20, Step A-2)

In N,N-dimethylformamide (40 ml) was dissolved the N-[3-(cyclobutylmethyl)-1-methyl-1H-pyrazol-5-yl]-2-methoxyacetamide (4.3 g, 18.0 mmol) obtained in the above-described step (1). In an ice bath, sodium hydride (0.9 g, 21.5 mmol) was added to the resulting solution. Evolution of hydrogen was then observed. After 30 minutes, when hydrogen evolution had ceased, to the reaction mixture was added 3-cyanobenzyl bromide (4.2 g, 21.5 mmol) and the mixture was stirred at room temperature for 1 hour. After completion of the reaction, the reaction mixture was poured into ice and then extracted with ethyl acetate. The organic layer was separated, washed sufficiently with water and dried over magnesium sulfate (MgSO₄). After filtration, the solvent was distilled off under reduced pressure. The residue (7.8 g) was then purified by chromatography on a silica gel column (eluting solvent: hexane/ethyl acetate = 1/1), whereby 4.6 g (yield: 69%) of the title compound of the present invention was obtained as colorless crystals (melting point: 64 to 67°C).

¹H-NMR (200 MHz,CDCl₃) δ ppm: 1.50-2.10 (6H,m), 2.48-2.64 (3H,m), 3.36 (3H,s), 3.45 (3H,s), 3.64 (1H,d,J=12.5Hz), 3.85 (1H,d,J=12.5Hz), 4.46 (1H,d,J=13.2Hz), 5.06 (1H,d,J=13.2Hz), 5.60 (1H,s), 7.39-7.63 (4H,m).

Mass spectrum (MS) m/z: 352 (M⁺), 324, 311, 298, 279.

### (Example 2)

### Methyl 3-cyanobenzyl[1-methyl-3-(3,3,3-trifluoro-2-methylpropyl)-1H-pyrazol-5-yl]carbamate (Compound of Compound No. 4-79)

### (1) Methyl 1-methyl-3-(3,3,3-trifluoro-2-methylpropyl)-1H-pyrazol-5-ylcarbamate (Step A-1)

In tetrahydrofuran (120 ml) was dissolved 5-amino-1-methyl-3-(3,3,3-trifluoro-2-methylpropyl)-1H-pyrazole (5.8 g, 27.9 mmol). Potassium carbonate (7.7 g, 55.7 mmol) was added to the resulting solution while stirring, followed by the dropwise addition of methyl chloroformate (3.2 ml, 41.8 mmol). The resulting mixture was stirred further at room temperature for 72 hours. After completion of the reaction, the reaction mixture was added to water and extracted with ethyl acetate. The organic layer was separated, washed with water, washed with a small amount of a 10% aqueous sodium bicarbonate solution, and dried over magnesium sulfate (MgSO₄). After filtration, the solvent was distilled off under reduced pressure, whereby 7.4 g of the title compound was obtained as an oil.

¹H-NMR (200 MHz,CDCl₃) δ ppm: 1.10 (3H,d,J=6.2Hz), 2.40-2.57 (2H,m), 2.88-3.01 (1H,m), 3.70 (3H,s), 3.80 (3H,s), 6.00 (1H,s), 6.32 (1H,s).

### (2) Methyl 3-cyanobenzyl [1-methyl-3-(3,3,3-trifluoro-2-methylpropyl)-1H-pyrazol-5-yl]carbamate (Compound of Compound No. 4-79, Step A-2)

In N,N-dimethylformamide (70 ml) was dissolved the methyl 1-methyl-3-(3,3,3-trifluoro-2-methylpropyl)-1H-pyrazol-5-ylcarbamate (7.4g, 27.9 mmol) obtained in the above-described step (1). In an ice bath, sodium hydride (1.3 g, 33.4 mmol) was added to the resulting solution. Evolution of hydrogen was then observed. After 30 minutes, when hydrogen evolution had ceased, to the reaction mixture was added 3-cyanobenzyl bromide (6.6 g, 33.4 mmol) and the mixture was stirred at room temperature for 1 hour. After completion of the reaction, the reaction mixture was poured into ice and then extracted with ethyl acetate. The organic layer was separated, washed sufficiently with water and dried over magnesium sulfate (MgSO₄). After filtration, the solvent was distilled off under reduced pressure. The residue (11.6 g) was purified by chromatography on a silica gel column (eluting solvent: hexane/ethyl acetate = 1/1), whereby 7.4 g (yield: 87%) of the title compound of the present invention was obtained as colorless crystals (melting point: 44 to 47°C).

¹H-NMR (200 MHz,CDCl₃) δ ppm: 1.08 (3H,d,J=6.2Hz), 2.41-2.58 (2H,m), 2.89-2.97 (1H,m), 3.42 (3H,s), 3.80 (3H,s), 4.73 (2H,s), 5.69 (1H,s) 7.44-7.47 (2H,m), 7.57-7.65 (2H,m).
MS m/z: 380 (M⁺), 321, 284, 264, 220.

### (Example 3)

### 2-Pyridyl 3-cyanobenzyl(3-isobutyl-1-methyl-1H-pyrazol-5-yl]carbamate (Compound of Compound No. 7-28)

### (1) 2-[(3-Isobutyl-1-methyl-1H-pyrazol-5-yl)amino]-2-oxoethyl acetate (Step A-1)

In dichloroethane (25 ml) was dissolved 5-amino-3-isobutyl-1-methyl-1H-pyrazole (1.0 g, 6.5 mmol). Acetoxyacetyl chloride (0.8 ml, 7.8 mmol) was added to the resulting solution while stirring, followed by the dropwise addition of pyridine (0.6 ml, 7.8 mmol). The resulting mixture was stirred further at room temperature for 3 hours. After completion of the reaction, water was added to the reaction mixture which was then extracted with ethyl acetate. The organic layer was separated, washed with water, washed with a small amount of a 10% aqueous sodium bicarbonate solution, and dried over magnesium sulfate (MgSO₄). After filtration, the solvent was distilled off under reduced pressure, whereby 1.8 g of the title compound was obtained as an oil.

¹H-NMR (200 MHz,CDCl₃) δ ppm: 0.93 (6H,d,J=6.6Hz), 1.79-1.99 (1H,m), 2.21 (3H,s), 2.43 (2H,d,J=7.3Hz), 3.68 (3H,s), 4.71 (2H,s), 6.04 (1H,s), 7.78 (1H,s).

### (2) 2-[(3-Cyanobenzyl)(3-isobutyl-1-methyl-1H-pyrazol-5-yl)amino]-2-oxoethyl acetate (Compound of Compound No. 5-93, Step A-2)

In N,N-dimethylformamide (20 ml) was dissolved the 2-[(3-isobutyl-1-methyl-1H-pyrazol-5-yl)amino]-2-oxoethyl acetate (1.8 g, 65 mmol) obtained in the above-described Step (1). In an ice bath, sodium hydride (0.3 g, 7.2 mmol) was added to the resulting solution. Then, evolution of hydrogen was observed. After 30 minutes, when hydrogen evolution had ceased, to the reaction mixture was added 3-cyanobenzyl bromide (1.4 g, 7.2 mmol) and the mixture was stirred at room temperature for 1 hour. After completion of the reaction, the reaction mixture was poured into ice and then extracted with ethyl acetate. The organic layer was separated, washed sufficiently with water and dried over magnesium sulfate (MgSO₄). After filtration, the solvent was distilled off under reduced pressure. The residue (7.8 g) was purified by chromatography on a silica gel column (eluting solvent: hexane/ethyl acetate = 1/1), 1.6 g (yield: 96%) of the title compound of the present invention was obtained as an oil.

¹H-NMR (200 MHz,CDCl₃) δ ppm: 0.90 (6H,d,J=6.6Hz), 1.83 (1H,m), 2.16 (3H,s), 2.41 (2H,d,J=7.0Hz), 3.55 (3H,s), 4.30 (2H,m), 4.44 (1H,d,J=14.0Hz), 5.08 (1H,d,J=14.0Hz), 5.69 (1H,s), 7.40-7.63 (4H,m).
MS m/z: 368 (M⁺), 353, 326, 267, 116.

### (3) 3-{[(3-Isobutyl-1-methyl-1H-pyrazol-5-yl)amino]methyl}benzonitrile (Step C-1)

In methanol (150 ml) was dissolved the 2-[(3-cyanobenzyl)(3-isobutyl-1-methyl-1H-pyrazol-5-yl)amino]-2-oxoethyl acetate (4.4 g, 11.9 mmol) obtained by the above-described step (2). To the resulting solution was added a 2 mol/L aqueous sodium hydroxide solution (178 ml, 360 mmol), followed by stirring at room temperature for 4 hours. After completion of the reaction, methanol was distilled off. The residue was added to water and extracted with ethyl acetate. The organic layer was separated, washed with water and then dried over magnesium sulfate (MgSO₄). After filtration, the solvent was distilled off under reduced pressure. The residue (3.8 g) was purified by chromatography on a silica gel column (eluting solvent: hexane/ethyl acetate = 1/2), whereby 3.0 g (yield: 95%) of the title compound as an oil.

¹H-NMR (200 MHz, CDCl₃) δ ppm: 0.91 (6H,d,J=6.6Hz), 1.85 (1H,m), 2.35 (2H,d,J=7.0Hz), 3.62 (3H,s), 4.30 (2H,d,J=5.9Hz), 5.23 (1H,s), 7.43-7.68 (4H,m).

### (4) 3-Cyanobenzyl(3-isobutyl-1-methyl-1H-pyrazol-5-yl)carbamoyl chloride (Step B-4)

In methylene chloride (5 ml) was dissolved 3-{[(3-isobutyl-1-methyl-1H-pyrazol-5-yl)amino]methyl}benzonitrile (0.6 g, 2.2 mmol) obtained by the above-described step (3). In an ice bath, triethylamine (0.3 ml, 2.2 mmol) and triphosgene (130 mg, 0.7 mmol) were added successively to the resulting solution, followed by stirring at room temperature for 1 hour. After completion of the reaction, the reaction mixture was poured into ice and extracted with ethyl acetate. The organic layer was separated, washed sufficiently with water and then dried over magnesium sulfate (MgSO₄). After filtration, the solvent was distilled off under reduced pressure, whereby 0.7 g (yield: 97%) of the title compound was obtained as an oil.

¹H-NMR (200 MHz, CDCl₃) δ ppm: 0.90 (6H,d,J=6.6Hz), 1.86 (1H,m), 2.42 (2H,d,J=7.3Hz), 3.45 (3H,s), 4.70 (1H,d,J=14.5Hz), 4.90 (1H,d,J=14.5Hz), 5.74 (1H,s), 7.48-7.70 (4H,s).

### (5) 2-Pyridyl 3-cyanobenzyl(3-isobutyl-1-methyl-1H-pyrazol-5-yl)carbamate (Compound of Compound No. 7-28, Step B-5)

In acetonitrile (3 ml) was dissolved the 3-cyanobenzyl(3-isobutyl-1-methyl-1H-pyrazol-5-yl)carbamoyl chloride (120 mg, 0.4 mmol) obtained by the above-described step (4), followed by the addition of 2-hydroxypyridine (34.5 mg, 0.4 mmol). The resulting mixture was heated under reflux for 8 hours. After completion of the reaction, the reaction mixture was poured into ice and extracted with ethyl acetate. The organic layer was separated, washed sufficiently with water and then dried over magnesium sulfate (MgSO₄). After filtration, the solvent was distilled off under reduced pressure. The residue was purified by chromatography on a silica gel column (eluting solvent: hexane/ethyl acetate = 1/1), whereby 99.1 mg (yield: 70%) of the title compound of the invention was obtained as an oil.

¹H-NMR (200 MHz, CDCl₃) δ ppm: 0.91 (6H,d,J=6.6Hz), 1.86 (1H,m), 2.41 (2H,d,J=7.0Hz), 3.59 (3H,s), 4.83 (2H,s), 5.78 (1H,s), 7.01 (1H,d,J=8.1Hz), 7.24 (1H,t,J=7.0Hz), 7.43-7.67 (4H,m), 7.78 (1H,t,J=8.1Hz), 8.39 (1H,d,J=3.3Hz).
MS m/z: 389 (M⁺), 374, 347, 295, 267, 252, 226, 211, 178, 136, 116.

### (Example 4)

### N-(3-Cyanobenzyl)-N-(1,4-dimethyl-3-phenyl-1H-pyrazol-5-yl)cyclopropanecarboxamide (Compound of Compound No. 1-14)

### (1) 3-{[(1,4-Dimethyl-3-phenyl-1H-pyrazol-5-yl)imino]methyl}benzonitrile (Step B-1)

In ethanol (120 ml) was dissolved 5-amino-1,4-dimethyl-3-phenyl-1H-pyrazole (8.5 g, 45.4 mmol), followed by the addition of 3-cyanobenzaldehyde (6.3 g, 47.7 mmol) under stirring. The resulting mixture was stirred further at room temperature for 18 hours. After completion of the reaction, the solvent was distilled off under reduced pressure, whereby 13.5 g of the title compound was obtained as an oil.

¹H-NMR (200 MHz, CDCl₃) δ ppm: 2.34 (3H,s), 3.98 (3H,s), 7.20-7.80 (5H,m), 7.90-8.20 (4H,m), 8.66 (1H,s).

### (2) 3-{[(1,4-Dimethyl-3-phenyl-1H-pyrazol-5-yl)amino]methyl}benzonitrile (Step B-2)

In acetic acid (45 ml) was dissolved the 3-{[(1,4-dimethyl-3-phenyl-lH-pyrazol-5-yl)imino]methyl}benzonitrile (13.5 g, 44.9 mmol) obtained by the above-described step (1), followed by the addition of sodium cyanoborohydride (5.6 g, 90.0 mmol) in an ice bath. The resulting mixture was stirred further at room temperature for 1 hour. After completion of the reaction, the reaction mixture was poured into ice and extracted with ethyl acetate. The organic layer was separated, washed sufficiently with water and then dried over magnesium sulfate (MgSO₄). After filtration, the solvent was distilled off under reduced pressure, whereby 12.9 g (yield: 95%) of the title compound of the invention was obtained as an oil.

¹H-NMR (200 MHz, CDCl₃) δ ppm: 2.01 (3H,s), 3.26 (1H,t,J=6.8Hz), 3.72 (3H,s), 4.19 (2H,d,J=6.8Hz), 7.26-7.68 (9H,m).

### (3) N-(3-Cyanobenzyl)-N-(1,4-dimethyl-3-phenyl-1H-pyrazol-5-yl)cyclopropanecarboxamide (Compound of Compound No. 1-14, Step B-3)

In methylene chloride (3.0 ml) was dissolved the 3-{[(1,4-dimethyl-3-phenyl-lH-pyrazol-5-yl)amino]methyl}benzonitrile (50 m g, 0.17 mmol) obtained by the above-described step (2). Cyclopropanecarbonyl chloride (0.03 ml, 0.33 mmol) was added to the resulting solution while stirring, followed by the dropwise addition of pyridine (0.03 ml, 0.33 mmol). The resulting mixture was stirred further at room temperature for 16 hours. After completion of the reaction, the reaction mixture was added to water and then extracted with ethyl acetate. The organic layer was separated, washed with water, washed with a small amount of a 10% aqueous sodium bicarbonate solution, and dried over magnesium sulfate (MgSO₄). After filtration, the solvent was distilled off under reduced pressure. The residue was purified by chromatography on a silica gel column (eluting solvent: hexane/ethyl acetate = 1/1), whereby 50.0 mg (yield: 82%) of the title compound of the present invention was obtained as an oil.

¹H-NMR (200 MHz, CDCl₃) δ ppm: 0.90-1.30 (4H,m), 1.62-1.78 (1H,m), 1.86 (3H,s), 3.50 (3H,s), 4.77 (1H,d,J=14.0Hz), 4.88 (1H,d,J=14.0Hz), 7.37-7.68 (9H,m).

In a similar manner to that employed in Examples 1 to 4, the below-described compounds were prepared.

### (Example 5)

### Compound of Compound No. 1-1

MS (APCI) m/z: 331((M+H)⁺).

### (Example 6)

### Compound of Compound No. 1-7

¹H-NMR(200MHz, CDCl₃) δppm: 3.37 (3H, s), 3.55 (3H, s), 3.72 (1H, d, J=14.3Hz), 3.95 (1H, d, J=14.0Hz), 4.61 (1H, d, J=14.0Hz), 5.06 (1H, d, J=14.0Hz), 6.22 (1H, s), 7.30-7.74 (9H, m).

### (Example 7)

### Compound of Compound No. 1-8

¹H-NMR(200MHz, CDCl₃) δppm: 2.17 (3H, s), 3.64 (3H, s), 4.37 (2H, m), 4.58 (1H, d, J=14.0Hz), 5.07 (1H, d, J=14.0Hz), 6.28 (1H, s), 7.31-7.73 (9H, m).
MS m/z: 388(M⁺), 315, 288, 186, 172, 116.

### (Example 8)

### Compound of Compound No. 1-9

¹H-NMR(200MHz, CDCl₃) δppm: 3.08 (1H, br-t), 3.54 (3H, s), 3.70-3.82 (1H, br-s), 3.95-4.07 (1H, br-s), 4.65-4.77 (1H, br-s), 5.03-5.15 (1H, br-s), 6.23 (1H, s), 7.33-7.73 (9H, m).
MS m/z: 346(M⁺), 315, 288, 200, 186, 172, 116.

### (Example 9)

### Compound of Compound No. 1-10

¹H-NMR(200MHz, CDCl₃) δppm: 3.51 (3H, s), 3.80 (3H, br-s), 4.79 (2H, s), 6.20 (1H, s), 7.29-7.77 (9H, m).

### (Example 10)

### Compound of Compound No. 1-11

¹H-NMR(200MHz, CDCl₃) δppm: 1.79 (3H, s), 1.88 (3H, s), 3.48 (3H, s), 4.68 (1H, d, J=14.0Hz), 4.91 (1H, d, J=14.0Hz), 7.32-7.66 (9H, m).
MS m/z: 344(M⁺), 301, 228, 186, 116.

### (Example 11)

### Compound of Compound No. 1-12

¹H-NMR(200MHz, CDCl₃) δppm: 1.12 (3H, d, J=7.3Hz), 1.79 (3H, s), 1.95-2.10 (2H, m), 3.47 (3H, s), 4.70 (1H, d, J=14.0Hz), 4.91 (1H, d, J=14.0Hz), 7.32-7.68 (9H, m).
MS m/z: 358(M⁺), 302, 242, 186, 116.

### (Example 12)

### Compound of Compound No. 1-13

¹H-NMR(200MHz, CDCl₃) δppm: 0.89 (3H, t, J=7.5Hz), 1.66 (2H, m), 1.79 (3H, s), 1.99 (2H, m), 3.46 (3H, s), 4.70 (1H, d, J=14.0Hz), 4.97 (1H, d, J=14.0Hz), 7.32-7.68 (9H, m).

### (Example 13)

### Compound of Compound No. 1-15

¹H-NMR(200MHz, CDCl₃) δppm: 1.75 (3H, s), 1.77-1.93 (4H, m), 2.22-2.46 (2H, m), 2.75-2.92 (1H, m), 3.43 (3H, s), 4.66 (1H, d, J=14.0Hz), 4.90 (1H, d, J=14.0 Hz), 7.34-7.69 (9H, m).
MS m/z: 384(M⁺), 302, 186, 116.

### (Example 14)

### Compound of Compound No. 1-16

MS (APCI) m/z: 387((M+H)⁺).

### (Example 15)

### Compound of Compound No. 1-17

¹H-NMR(200MHz, CDCl₃) δppm: 1.40-1.92 (8H, m), 1.80 (3H, s), 2.40 (1H, m), 3.46 (3H, s), 4.70 (1H, d, J=14.0 Hz), 4.90 (H, d, J=14.0Hz), 7.34-7.70 (9H, m).
MS m/z: 398(M⁺), 302, 186, 116.

### (Example 16)

### Compound of Compound No. 1-18

MS m/z: 380(M⁺+2), 378(M⁺), 329, 301, 262, 213, 186, 116.

### (Example 17)

### Compound of Compound No. 1-19

¹H-NMR(200MHz, CDCl₃) δppm: 1.74 (3H, s), 3.52 (3H, s), 4.67 (1H, d, J=14.0Hz), 5.04 (1H, d, J=14.0Hz), 5.76 (1H, m), 7.38-7.68 (9H, m).
MS m/z: 380(M⁺), 302, 186, 116.

### (Example 18)

### Compound of Compound No. 1-20

MS (APCI) m/z: 399((M+H)⁺).

### (Example 19)

### Compound of Compound No. 1-21

MS m/z: 416(M⁺), 385, 302, 186, 116.

### (Example 20)

### Compound of Compound No. 1-22

¹H-NMR(200MHz, CDCl₃) δppm: 1.72 (3H, s), 3.58 (3H, s), 3.61 (3H, s), 4.64 (1H, d, J=14.0Hz), 5.03 (1H, s, J=14.0Hz), 7.35-7.67 (9H, m).
MS m/z: 388(M⁺), 302, 280, 116.

### (Example 21)

### Compound of Compound No. 1-23

¹H-NMR(200MHz, CDCl₃) δppm: 1.76 (3H, s), 3.21 (2H, dd, J=20.0, 16.0Hz), 3.56 (3H, s), 4.62 (1H, d, J=14.0Hz), 5.04 (1H, d, J=14.0Hz), 7.38-7.67 (9H, m).
MS m/z: 369(M⁺), 302, 253, 213, 186, 116.

### (Example 22)

### Compound of Compound No. 1-24

MS (APCI) m/z: 375((M+H)⁺).

### (Example 23)

### Compound of Compound No. 1-25

¹H-NMR(200MHz, CDCl₃) δppm: 1.80 (3H, s), 2.16 (3H, s), 3.58 (3H, s), 4.22 (1H, d, J=15.2Hz), 4.32 (1H, d, J=15.2 Hz), 4.64 (1H, d, J=14.1Hz), 4.99 (1H, d, J=14.1Hz), 7.34-7.67 (9H, m). MS m/z: 402(M⁺), 301, 286, 214, 186, 116.

### (Example 24)

### Compound of Compound No. 1-26

¹H-NMR(200MHz, CDCl₃) δppm: 1.78 (3H, s), 3.53 (3H, s), 4.31 (1H, d, J= 14.7Hz), 4.44 (1H, d, J=14.7Hz), 4.65 (1H, d, J= 13.9Hz), 5.04 (1H, d, J=13.9Hz), 6.79 (2H, d J=7.5Hz), 6.97 (1H, d J=7.5Hz), 7.34-7.67 (9H, m).
MS m/z: 436(M⁺), 320, 249, 213, 199, 116.

### (Example 25)

### Compound of Compound No. 1-27

¹H-NMR(200MHz, CDCl₃) δppm: 1.44 (2H, d. J=6.2Hz), 1.55, 1.76 (3H, s), 3.29, 3.49 (3H, s), 4.58-4.69 (2H, m), 4.98-5.12 (1H, m), 6.60-7.64 (14H, m) (mixture of 2 diastereomers).
MS m/z: 450(M⁺), 357, 302, 214, 121.

### (Example 26)

### Compound of Compound No. 1-28

¹H-NMR(200MHz, CDCl₃) δppm: 1.69 (3H, s), 3.29 (3H, s), 3.41 (2H, s), 4.62 (1H, d, J=14.0 Hz), 4.96 (1H, d, J=14.0Hz), 7.23-7.67 (11H, m), 8.52 (1H, s), 8.51 (1H, d, J=3.7Hz).

### (Example 27)

### Compound of Compound No. 1-29

¹H-NMR(200MHz, CDCl₃) δppm: 1.80 (3H, dd, J=7.0, 1.5Hz), 1.80 (3H, s), 3.42 (3H, s), 4.85 (2H, dd, J=9.5, 9.0 Hz), 5.59 (1H, dd, J=16, 1.8Hz), 7.05-7.12 (1H, m), 7.34-7.71 (9H, m).
MS m/z: 370(M⁺), 302, 254, 186, 116.

### (Example 28)

### Compound of Compound No. 1-30

MS (APCI) m/z: 407((M+H)⁺).

### (Example 29)

### Compound of Compound No. 1-31

¹H-NMR(200MHz, CDCl₃) δppm: 1.79 (3H, s), 3.38 (3H, s), 4.90 (1H, d, J=14.0Hz), 5.02 (1H, d, J=14.0Hz), 5.86 (1H, d, J=3.7Hz), 6.31 (1H, dd, J=3.7, 1.8Hz), 7.35-7.74 (10H, m).
MS m/z: 396(M⁺), 302, 280, 209, 186, 116.

### (Example 30)

### Compound of Compound No. 1-32

¹H-NMR(200MHz, CDCl₃) δppm: 1.85 (3H, s), 3.35 (3H, s), 4.91 (1H, d, J=14.0Hz), 5.02 (1H, d, J=14.0Hz), 6.94 (1H, dd, J=8.7, 4.8Hz), 7.15 (1H, dd, J=3.7, 1.1Hz), 7.35-7.75 (10H, m).
MS m/z: 412(M⁺), 384, 302, 186, 116.

### (Example 31)

### Compound of Compound No. 1-33

¹H-NMR(200MHz, CDCl₃) δppm: 1.77 (3H, s), 3.05 (3H, s), 3.35 (3H, s), 4.98 (2H, d, J=1.8Hz), 7.40-7.74 (9H, s).
MS m/z: 428(M⁺), 400, 284, 116.

### (Example 32)

### Compound of Compound No. 1-34

¹H-NMR(200MHz, CDCl₃) δppm: 1.87 (3H, s), 3.41 (3H, s), 3.75 (3H, s), 4.66 (1H, d, J=14.3Hz), 4.82 (1H, d, J=14.3Hz), 7.32-7.66 (9H, m).
MS m/z: 360(M⁺), 301, 244, 212, 116.

### (Example 33)

### Compound of Compound No. 1-35

¹H-NMR(200MHz, CDCl₃) δppm: 1.24 (3H, t, J=6.2Hz), 1.87 (3H, s), 3.42 (3H, s), 4.26 (2H, q, J=6.2Hz), 4.65 (1H, d, J=14.4Hz), 4.82 (1H, d, J=14.4Hz), 7.31-7.68 (9H, m).
MS m/z: 374(M⁺), 301, 258, 214, 186, 116.

### (Example 34)

### Compound of Compound No. 1-36

¹H-NMR(200MHz, CDCl₃) δppm: 0.92 (3H, t, J=7.5Hz), 1.91 (3H, s), 2.35 (2H, m), 3.38 (3H, s), 4.68 (1H, d, J=13.9 Hz), 4.98 (1H, d, J=13.9Hz), 7.34-7.66 (9H, m).
MS m/z: 358(M⁺), 301, 242, 200, 116.

### (Example 35)

### Compound of Compound No. 1-45

¹H-NMR(200MHz, CDCl₃) δppm: 0.95 (3H, t, J=7.5Hz), 2.34 (2H, m), 3.35 (3H, s), 3.76 (3H, s), 4.56 (1H, d, J=14.3 Hz), 4.94 (1H, d, J=14.3 Hz), 7.28-7.68 (9H, m).
MS m/z: 374(M⁺), 359, 258, 185, 129, 116.

### (Example 36)

### Compound of Compound No. 1-57

¹H-NMR(200MHz, CDCl₃) δppm: 1.78 (3H, s), 1.88 (3H, s), 2.38 (3H, s), 3.48 (3H, s), 4.68 (1H, d, J=13.9Hz), 4.91 (1H, d, J=13.9Hz), 7.23 (2H, d, J=7.7Hz), 7.37-7.66 (6H, m).
MS m/z: 358(M⁺), 315, 242, 200, 116.

### (Example 37)

### Compound of Compound No. 1-62

¹H-NMR(200MHz, CDCl₃) δppm: 1.80 (3H, s), 1.88 (3H, s), 3.48 (3H, s), 4.70 (1H, d, J=14.0Hz), 4.90 (1H, d, J=14.0Hz), 7.04 (1H, m), 7.33-7.65 (7H, m).
MS m/z: 362(M⁺), 320, 246, 204, 116.

### (Example 38)

### Compound of Compound No. 1-63

¹H-NMR(200MHz, CDCl₃) δppm: 1.78 (3H, s), 1.88 (3H, s), 3.48 (3H, s), 4.69 (1H, d, J=14.1Hz), 4.91 (1H, d, J=14.1Hz), 7.11 (2H, t, J=8.8Hz), 7.43-7.61 (2H, m), 7.62-7.66 (4H, m).
MS m/z: 362(M⁺), 319, 246, 204, 133, 116.

### (Example 39)

### Compound of Compound No. 1-67

¹H-NMR(200MHz, CDCl₃) δppm: 1.85 (3H, s), 2.37 (3H, s), 3.41 (3H, s), 3.75 (3H, s), 4.65 (1H, d, J=14.3Hz), 4.82 (1H, d, J=14.3Hz), 7.21 (2H, d, J=8.1Hz), 7.26-7.67 (6H, m).
MS m/z: 374(M⁺), 315, 258, 149, 116.

### (Example 40)

### Compound of Compound No. 1-72

¹H-NMR(200MHz, CDCl₃) δppm: 1.87 (3H, s), 3.41 (3H, s), 3.76 (3H, s), 4.64 (1H, d, J=14.0 Hz), 4.83 (1H, d, J=14.0Hz), 7.01 (1H, m), 7.31-7.66 (7H, m).

### (Example 41)

### Compound of Compound No. 1-73

¹H-NMR(200MHz, CDCl₃) δppm: 1.85 (3H, s), 3.41 (3H, s), 3.76 (3H, s), 4.65 (1H, d, J=14.5Hz), 4.83 (1H, d, J=14.5Hz), 7.10 (2H, t, J=8.8Hz), 7.27-7.47 (2H, m), 7.58-7.65 (4H, m).
MS m/z: 378(M⁺), 319, 262, 134, 116.

### (Example 42)

### Compound of Compound No. 1-76

MS (APCI) m/z: 359((M+H)⁺).

### (Example 43)

### Compound of Compound No. 1-78

¹H-NMR(200MHz, CDCl₃) δppm: 0.87 (3H, t, J=7.5Hz), 1.33 (3H, t, J=7.2Hz), 1.63 (2H, m), 2.02 (2H, m), 3.77 (2H, m), 4.84 (1H, d, J=14.0Hz), 5.31 (1H, d, J=14.0Hz), 7.32-7.72 (9H, m).

### (Example 44)

### Compound of Compound No. 1-81

MS (APCI) m/z: 421((M+H)⁺).

### (Example 45)

### Compound of Compound No. 1-82

MS (APCI) m/z: 389((M+H)⁺).

### (Example 46)

### Compound of Compound No. 1-85

¹H-NMR(200MHz, CDCl₃) δppm: 1.26 (3H, t, J=7.3Hz), 1.81 (3H, s), 3.69 (2H, m), 3.75 (3H, s), 4.74 (2H, s), 7.29-7.67 (9H, m).

### (Example 47)

### Compound of Compound No. 1-86

¹H-NMR(200MHz, CDCl₃) δppm: 1.81 (3H, m), 1.88 (3H, s), 2.35 (3H, s), 3.49 (3H, s), 4.67 (1H, d, J=14.0Hz), 4.87 (1H, d, J=14.0Hz), 7.30-7.67 (8H, m).
MS m/z: 358(M⁺), 315, 228, 186, 130.

### (Example 48)

### Compound of Compound No. 1-88

¹H-NMR(200MHz, CDCl₃) δppm: 1.80 (3H, m), 1.88 (3H, s), 3.54 (3H, s), 4.58 (1H, d, J=14.0Hz), 4.94 (1H, d, J=14.0Hz), 7.16 (1H, t, J=8.4Hz), 7.34-7.67 (7H, m).
MS m/z: 362(M⁺), 319, 228, 186, 134, 116.

### (Example 49)

### Compound of Compound No. 1-89

¹H-NMR(200MHz, CDCl₃) δppm: 1.77 (3H, m), 1.93 (3H, s), 3.59 (3H, s), 4.87 (1H, d, J=14.0Hz), 5.20 (1H, d, J=14.0Hz), 7.33-7.65 (7H, m), 7.91 (1H, d, J=1.8Hz).
MS (APCI) m/z: 424((M+H)⁺).

### (Example 50)

### Compound of Compound No. 2-10

¹H-NMR(200MHz, CDCl₃) δppm: 1.62 (3H, s), 2.14 (3H, s), 3.31 (3H, s), 3.73 (3H, s), 4.62 (1H, d, J=14.3Hz), 4.75 (1H, d, J=14.3Hz), 7.31-7.63 (4H, m).
MS m/z: 298(M⁺), 239, 182, 150, 116.

### (Example 51)

### Compound of Compound No. 2-11

¹H-NMR(200MHz, CDCl₃) δppm: 1.58 (3H, s), 2.04 (3H, s), 2.23 (3H, s), 3.91 (1H, d, J=14.1Hz), 5.07 (1H, d, J=14.1Hz), 7.30-7.52 (9H, m).
MS m/z: 344(M⁺), 331, 301, 258, 198.

### (Example 52)

### Compound of Compound No. 2-17

¹H-NMR(200MHz, CDCl₃) δppm: 1.48 (3H, s), 2.23 (3H, s), 3.39 (3H, s), 3.90 (2H, s), 3.92 (1H, d, J=13.9Hz), 5.14 (1H, d, J=13.9Hz), 7.28-7.54 (9H, m).
MS m/z: 374(M⁺), 302, 228, 186, 116.

### (Example 53)

### Compound of Compound No. 2-21

¹H-NMR(200MHz, CDCl₃) δppm: 0.90 (6H, d, J=6.6Hz), 1.48 (3H, s), 1.70-1.94 (1H, m), 1.82 (3H, s), 2.37 (2H, d, J=7.3Hz), 3.41 (3H, s), 4.53 (1H, d, J=13.7Hz), 4.96 (1H, d, J=13.7Hz), 7.36-7.62 (4H, m).
MS m/z: 324(M⁺), 282, 240, 166, 116.

### (Example 54)

### Compound of Compound No. 2-22

¹H-NMR(200MHz, CDCl₃) δppm: 0.87 (3H, t, J=7.3Hz), 0.90 (6H, d, J=6.6Hz), 1.41 (3H, s), 1.62 (2H, m), 1.84-2.04 (3H, m), 2.37 (2H, d, J=7.0Hz), 3.39 (3H, s), 4.54 (1H, d, J=14.0Hz), 4.97 (1H, d, J=14.0Hz), 7.37-7.61 (4H, m).
MS m/z: 338(M⁺), 296, 282, 240, 116.

### (Example 55)

### Compound of Compound No. 2-23

¹H-NMR(200MHz, CDCl₃) δppm: 0.90 (6H, d, J=6.6Hz), 1.09 (3H, t, J=7.3Hz), 1.47 (3H, s), 1.62 (2H, m), 1.82-1.96 (3H, m), 2.38 (2H, d, J=7.3Hz), 3.39 (3H, s), 4.54 (1H, d, J=14.0Hz), 4.97 (1H, d, J=14.0Hz), 7.35-7.61 (4H, m).
MS m/z: 352(M⁺), 310, 282, 240, 116.

### (Example 56)

### Compound of Compound No. 2-24

¹H-NMR(200MHz, CDCl₃) δppm: 0.71 (2H, m), 0.91 (6H, d, J=6.6Hz), 1.05 (2H, m), 1.17 (1H, m), 1.55 (3H, s), 1.91 (1H, m), 2.38 (2H, d, J=7.3Hz), 3.42 (3H, s), 4.62 (1H, d, J=14.0Hz), 4.91 (1H, d, J=14.0Hz), 7.36-7.61 (4H, m).

### (Example 57)

### Compound of Compound No. 2-26

¹H-NMR(200MHz, CDCl₃) δppm: 0.91 (6H, d, J=6.6Hz), 1.43 (3H, s), 1.70-2.00 (5H, m), 2.18-2.40 (2H, m), 2.37 (2H, d, J=7.3Hz), 2.78 (1H, m), 3.36 (3H, s), 4.50 (1H, d, J=14.0Hz), 4.95 (1H, d, J=14.0Hz), 7.35-7.61 (4H, m).
MS m/z: 364(M⁺), 322, 282, 240, 116.

### (Example 58)

### Compound of Compound No. 2-29

¹H-NMR(200MHz, CDCl₃) δppm: 0.92 (6H, dd, J=6.6, 1.8Hz), 1.47 (3H, s), 1.82-2.02 (1H, m), 2.40 (2H, d, J=7.0Hz), 3.32 (3H, s), 3.58 (3H, s), 4.84 (1H, d, J=14.0Hz), 4.97 (1H, d, J=14.0Hz), 5.76 (1H, d, J=3.7Hz), 6.30 (1H, dd, J=3.7, 1.8Hz), 7.38-7.64 (4H, m).
MS m/z: 376(M⁺), 334, 282, 116.

### (Example 59)

### Compound of Compound No. 2-30

¹H-NMR(200MHz, CDCl₃) δppm: 0.90 (6H, d, J=6.6Hz), 1.49 (3H, s), 1.90 (1H, m), 2.19 (2H, t, J=6.2Hz), 2.37 (2H, d, J=7.0Hz), 2.49-2.83 (2H, m), 3.49 (3H, s), 3.70 (3H, s), 4.52 (1H, d, J=14.0Hz), 5.01 (1H, d, J=14.0Hz), 7.36-7.62 (4H, m).
MS m/z: 396(M⁺), 365, 354, 282, 240, 116.

### (Example 60)

### Compound of Compound No. 2-31

¹H-NMR(200MHz, CDCl₃) δppm: 0.90 (6H, d, J=6.6Hz), 1.44 (3H, s), 1.83-1.91 (1H, m), 2.37 (2H, d, J=7.3Hz), 3.36 (3H, s), 3.42 (3H, s), 3.59 (1H, d, J=15.0Hz), 3.71 (1H, d, J=15.0Hz), 4.49 (1H, d, J=13.7Hz), 5.03 (1H, d, J=13.7Hz), 7.37-7.63 (4H, m).
MS m/z: 354(M⁺), 339, 312, 281, 238.

### (Example 61)

### Compound of Compound No. 2-32

¹H-NMR(200MHz, CDCl₃) δppm: 0.90 (6H, d, J=6.6Hz), 1.47 (3H, s), 1.82-1.96 (1H, m), 2.37 (2H, d, J=7.3Hz), 3.46 (3H, s), 4.23 (1H, d, J=14.7Hz), 4.36 (1H, d, J=14.7Hz), 4.48 (1H, d, J=13.9Hz), 5.11 (1H, d, J=13.9Hz), 6.77 (2H, d, J=7.7Hz), 6.97 (1H, t, J=7.3Hz), 7.22-7.30 (2H, m), 7.38-7.64 (4H, m).
MS m/z: 416(M⁺), 374, 323, 300, 281.

### (Example 62)

### Compound of Compound No. 2-33

¹H-NMR(200MHz, CDCl₃) δppm: 0.90 (6H, d, J=6.6Hz), 0.90-1.26 (4H, m), 1.48 (3H, s), 1.60-2.04 (2H, m), 2.36 (2H, d, J=7.3Hz), 3.51 (3H, s), 4.21 (2H, m), 4.47 (1H, d, J=13.9Hz), 5.06 (1H, d, J=13.9Hz), 7.38-7.64 (4H, m).
MS m/z: 408(M⁺), 366, 281, 127, 116.

### (Example 63)

### Compound of Compound No. 2-34

¹H-NMR(200MHz, CDCl₃) δppm: 0.86-0.89 (6H, m), 1.26 (3H, t, J=7.0Hz), 1.50, 1.58 (3H, s), 1.82-2.00 (1H, m), 2.10, 2.12 (3H, s), 2.34, 2.37 (2H, d, J=7.3Hz), 3.49, 3.54 (3H, s), 4.20, 5.31 (1H, d, J=14.0Hz), 4.60, 4.83 (1H, q, J=6.6Hz), 4.75 (1H, s), 7.36-7.63 (4H, s) (mixture of 2 diastereomers).
MS m/z: 396(M⁺), 354, 282, 116.

### (Example 64)

### Compound of Compound No. 2-35

¹H-NMR(200MHz, CDCl₃) δppm: 0.89 (6H, d, J=6.6Hz), 1.73 (3H, s), 1.82-1.89 (1H, m), 2.35 (2H, d, J=7.3Hz), 3.64 (3H, s), 4.15 (2H, s), 4.91 (2H, s), 6.85-6.93 (3H, m), 7.42-7.66 (4H, m), 8.09-8.13 (1H, m).
MS m/z: 417(M⁺), 366, 282, 240, 136.

### (Example 65)

### Compound of Compound No. 2-36

¹H-NMR(200MHz, CDCl₃) δppm: 0.91 (6H, d, J=6.6Hz), 1.48 (3H, s), 1.79 (3H, dd, J=7.0, 1.5Hz), 1.92 (1H, m), 2.39 (2H, d, J=7.3Hz), 3.36 (3H, s), 4.63 (1H, d, J=14.0Hz), 4.97 (1H, d, J=14.0Hz), 5.51 (1H, dd, J=15, 1.5Hz), 7.09 (1H, m), 7.36-7.61 (4H, m).
MS m/z: 350(M⁺), 308, 282, 240, 116.

### (Example 66)

### Compound of Compound No. 2-37

¹H-NMR(200MHz, CDCl₃) δppm: 0.86 (6H, d, J=6.6Hz), 1.41 (3H, s), 1.73-1.92 (1H, m), 2.33 (2H, d, J=7.3Hz), 3.51 (3H, s), 3.58 (3H, s), 4.48 (1H, d, J=14.0Hz), 5.09 (1H, d, J=14.0Hz), 7.40-7.67 (4H, m).
MS m/z: 368(M⁺), 326, 282, 150, 116.

### (Example 67)

### Compound of Compound No. 2-38

¹H-NMR(200MHz, CDCl₃) δppm: 0.90 (6H, d, J=6.6Hz), 1.57 (3H, s), 1.83-1.97 (1H, m), 2.36 (2H, d, J=7.7Hz), 3.33 (3H, s), 3.72 (3H, bs), 4.68 (2H, s), 7.36-7.42 (2H, m) 7.55-7.63 (2H, m).
MS m/z: 340(M+), 325, 298, 281, 239.

### (Example 68)

### Compound of Compound No. 2-39

¹H-NMR(200MHz, CDCl₃) δppm: 0.89 (6H, d, J=6.6Hz), 1.59 (3H, s), 1.74-1.95 (1H, m), 2.36 (2H, d, J=7.3Hz), 3.23 (3H, s), 4.68 (2H, s), 5.16 (2H, s), 7.12-7.61 (4H, m).

MS m/z: 402(M⁺), 360, 316, 267, 116.

### (Example 69)

### Compound of Compound No. 2-40

¹H-NMR(200MHz, CDCl₃) δppm: 0.91 (6H, d, J=6.6Hz), 1.70 (3H, s), 1.75-1.98 (1H, m), 2.38 (2H, d, J=7.0Hz), 3.52 (3H, s), 4.79 (2H, s), 6.97 (1H, d, J=8.1Hz), 7.18-7.81 (6H, m), 8.38 (1H, m).
MS m/z: 403(M⁺), 361, 281, 266, 238, 192, 150, 116.

### (Example 70)

### Compound of Compound No. 2-41

¹H-NMR(200MHz, CDCl₃) δppm: 0.90 (6H, d, J=7.0Hz), 1.29 (3H, t, J=7.2Hz), 1.44 (3H, s), 1.84 (3H, s), 1.87-2.04 (1H, m), 2.38 (2H, d, J=7.3Hz), 3.74 (2H, q, J=7.2Hz), 4.42 (1H, d, J=13.9Hz), 5.11 (1H, d, J=13.9Hz), 7.36-7.64 (4H, m).
MS m/z: 338(M⁺), 296, 266, 254, 180, 116.

### (Example 71)

### Compound of Compound No. 2-43

¹H-NMR(200MHz, CDCl₃) δppm: 0.67-0.79 (2H, m), 0.90 (6H, d, J=6.6Hz), 0.96-1.09 (2H, m), 1.16-1.26 (1H, m), 1.28 (3H, t, J=7.3Hz), 1.48 (3H, s), 1.84-1.98 (1H, m), 2.39 (2H, d, J=7.0Hz), 3.76 (2H, q, J=7.3Hz), 4.47 (1H, d, J=13.7Hz), 5.08 (1H, d, J=13.7Hz), 7.36-7.62 (4H, m).
MS m/z: 364(M⁺), 322, 295, 254, 183, 116.

### (Example 72)

### Compound of Compound No. 2-47

¹H-NMR(200MHz, CDCl₃) δppm: 0.90 (6H, d, J=6.6Hz), 1.32 (3H, t, J=7.1Hz), 1.41 (3H, s), 1.84-1.97 (1H, m), 2.38 (2H, d, J=7.3Hz), 3.37 (3H, s), 3.59-3.81 (2H, m), 3.68 (2H, q, J=7.1Hz), 4.41 (1H, d, J=13.8Hz), 5.17 (1H, d, J=13.8Hz), 7.40-7.66 (4H, m).
MS m/z: 368(M⁺), 326, 295, 252, 116.

### (Example 73)

### Compound of Compound No. 2-48

¹H-NMR(200MHz, CDCl₃) δppm: 0.89 (6H, d, J=6.6Hz), 1.32 (3H, t, J=7.1Hz), 1.44 (3H, s), 1.83-1.97 (1H, m), 2.39 (2H, d, J=7.0Hz), 3.67-3.83 (2H, m), 4.29 (2H, q, J=7.1Hz), 4.41 (1H, d, J=13.8Hz), 5.19 (1H, d, J=13.8Hz), 6.75-7.03 (4H, m), 7.21-7.62 (4H, m).
MS m/z: 430(M⁺), 388, 295, 165, 116.

### (Example 74)

### Compound of Compound No. 2-50

¹H-NMR(200MHz, CDCl₃) δppm: 0.90 (6H, d, J=6.6Hz), 1.20 (3H, t, J=7.1Hz), 1.52 (3H, s), 1.83-1.97 (1H, m), 2.37 (2H, d, J=7.0Hz), 3.64 (2H, q, J=7.1Hz), 3.72 (3H, s), 4.60 (1H, d, J=14.1Hz), 4.78 (1H, d, J=14.1Hz), 7.33-7.64 (4H, m).
MS m/z: 354(M⁺), 339, 312, 295, 116.

### (Example 75)

### Compound of Compound No. 2-61

¹H-NMR(200MHz, CDCl₃) δppm: 0.90 (6H, d, J=6.6Hz), 1.49 (3H, s), 1.82 (3H, s), 1.82-1.92 (1H, m), 2.34 (3H, s), 2.37 (2H, d, J=7.3Hz), 3.41 (3H, s), 4.49 (1H, d, J=14.0Hz), 4.93 (1H, d, J=14.0Hz), 7.26 (1H, s), 7.32 (1H, s), 7.40 (1H, s).
MS m/z: 338(M⁺), 296, 254, 130.

### (Example 76)

### Compound of Compound No. 2-63

¹H-NMR(200MHz, CDCl₃) δppm: 0.89 (6H, d, J=6.6Hz), 1.50 (3H, s), 1.85 (3H, s), 1.80-1.98 (1H, m), 2.35 (2H, d, J=7.0Hz), 3.48 (3H, s), 4.80 (1H, d, J=14.0Hz), 5.18 (1H, d, J=14.0Hz), 7.42 (1H, dd, J=8.4, 2.2Hz), 7.62 (1H, d, J=8.4Hz), 7.80 (1H, d, J=2.2Hz).
MS (APCI) m/z: 404((M+H)⁺).

### (Example 77)

### Compound of Compound No. 2-64

¹H-NMR(200MHz, CDCl₃) δppm: 0.91 (6H, d, J=6.6Hz), 1.50 (3H, s), 1.82 (3H, s), 1.85-1.98 (1H, m), 2.38 (2H, d, J=7.3Hz), 3.46 (3H, s), 4.43 (1H, d, J=14.0Hz), 5.00 (1H, d, J=14.0Hz), 7.15 (1H, t, J=8.4Hz), 7.46-7.53 (2H, m).
MS m/z: 342(M⁺), 300, 258, 134.

### (Example 78)

### Compound of Compound No. 2-67

¹H-NMR(200MHz, CDCl₃) δppm: 1.49 (3H, s), 1.58-2.10 (6H, m), 1.81 (3H, s), 2.58 (2H, s), 2.50-2.65 (1H, m), 3.40 (3H, s), 4.52 (1H, d, J=13.9Hz), 4.95 (1H, d, J=13.9Hz), 7.40-7.63 (4H, m).
MS m/z: 336(M⁺), 321, 308, 293, 282.

### (Example 79)

### Compound of Compound No. 2-73

¹H-NMR(200MHz, CDCl₃) δppm: 1.45 (3H, s), 1.55-2.10 (6H, m), 1.83-1.91 (1H, m), 2.58 (2H, s) 2.50-2.65 (1H, m), 3.35 (3H, s), 3.42 (3H, s), 3.58 (1H, d, J=15.2Hz), 3.71 (1H, d, J=15.2Hz), 4.48 (1H, d, J=13.7Hz), 5.03 (1H, d, J=13.7Hz), 7.37-7.63 (4H, m).
MS m/z: 366(M⁺), 351, 325, 312, 293.

### (Example 80)

### Compound of Compound No. 2-74

¹H-NMR(200MHz, CDCl₃) δppm: 1.47 (3H, s), 1.55-2.10 (6H, m), 1.83-1.91 (1H, m), 2.57 (2H, s), 2.45-2.65 (1H, m), 3.45 (3H, s), 4.21 (1H, d, J=14.8Hz), 4.34 (1H, d, J=14.8Hz), 4.47 (1H, d, J=13.9Hz), 5.09 (1H, d, J=13.9Hz), 6.76 (2H, d, J=8.2Hz), 6.97 (1H, t, J=7.7Hz), 7.22-7.30 (2H, m), 7.35-7.70 (4H, m). MS m/z: 428(M'), 400, 387, 374, 293.

### (Example 81)

### Compound of Compound No. 2-75

¹H-NMR(200MHz, CDCl₃) δppm: 1.58 (3H, s), 1.55-2.10 (6H, m), 1.83-1.91 (1H, m), 2.58 (2H, s), 2.45-2.65 (1H, m), 3.32 (3H, s), 3.72 (3H, bs), 4.67 (2H, s), 7.36-7.42 (2H, m) 7.55-7.63 (2H, m).
MS m/z: 352(M⁺), 324, 311, 298, 116.

### (Example 82)

### Compound of Compound No. 2-76

¹H-NMR(200MHz, CDCl₃) δppm: 1.08 (3H, d, J=6.6Hz), 1.51 (3H, d, J=4.0Hz), 1.82 (3H, s), 2.37-2.92 (3H, m), 3.41 (3H, d, J=3.7Hz), 4.57 (1H, dd, J=8.4, 13.9Hz), 4.90 (1H, dd, J=9.5, 13.9Hz), 7.37-7.64 (4H, m).
MS m/z: 378(M⁺), 335, 282, 220, 157, 116.

### (Example 83)

### Compound of Compound No. 2-78

¹H-NMR(200MHz, CDCl₃) δppm: 0.69-1.26 (4H, m), 1.09 (3H, d, J=6.6Hz), 1.58 (3H, d, J=4.8Hz), 2.41-2.94 (3H, m), 3.43 (3H, d, J=4.4Hz), 4.66 (1H, dd, J=9.5, 13.9Hz), 4.90 (1H, dd, J=10.6, 13.9Hz), 7.37-7.63 (4H, m).
MS m/z: 404(M⁺), 336, 183, 116.

### (Example 84)

### Compound of Compound No. 2-82

¹H-NMR(200MHz, CDCl₃) δppm: 1.09 (3H, d, J=6.6Hz), 1.47 (3H, d, J=4.0Hz), 2.41-2.91 (3H, m), 3.39 (3H, d, J=1.1Hz), 3.43 (3H, d, J=3.7Hz), 3.58 (1H, d, J=15.0Hz), 3.70 (1H, d, J=15.0Hz), 4.52 (1H, dd, J=8.4, 13.9Hz), 5.02 (1H, dd, J=9.5, 13.9Hz), 7.39-7.65 (4H, m).
MS m/z: 408(M⁺), 335, 312, 292, 116.

### (Example 85)

### Compound of Compound No. 2-83

¹H-NMR(200MHz, CDCl₃) δppm: 1.00 (3H, dd, J=4.0, 6.6Hz), 1.46 (3H, d, J=2.9Hz), 2.36-2.88 (3H, m), 3.44 (2H, d, J=1.8Hz), 4.24-4.37 (2H, m), 4.47 (2H, dd, J=10.1, 13.9Hz), 5.07 (1H, dd, J=7.3, 13.9Hz), 6.72 (2H, d, J=8.1Hz), 6.94 (1H, t, J=7.3Hz), 7.22 (2H, t, J=8.1Hz), 7.36-7.62 (4H, m).
MS m/z: 470(M⁺), 374, 354, 116.

### (Example 86)

### Compound of Compound No. 2-84

¹H-NMR(200MHz, CDCl₃) δppm: 1.08 (3H, dd, J=2.6, 6.6Hz), 1.60 (3H, d, J=4.0Hz), 2.37-2.94 (3H, m), 3.34 (3H, d, J=4.0Hz), 3.74 (3H, s), 4.70 (1H, dd, J=4.7, 14.3Hz), 4.70 (1H, s), 7.42-7.65 (4H, m).
MS m/z: 394(M⁺), 335, 298, 278, 116.

### (Example 87)

### Compound of Compound No. 3-1

¹H-NMR(200MHz, CDCl₃) δppm: 1.21 (3H, t, J=7.7Hz), 1.90 (3H, s), 2.59 (2H, q, J=7.7Hz), 3.44 (3H, s), 4.60 (1H, bs), 4.94 (1H, bs), 5.92 (1H, s),7.39-7.64 (4H, m).
MS m/z: 282(M⁺), 240, 138, 124, 116.

### (Example 88)

### Compound of Compound No. 3-3

¹H-NMR(200MHz, CDCl₃) δppm: 0.67-0.90 (2H, m), 0.96-1.38 (3H, m), 1.21 (3H, t, J=7.7Hz), 2.59 (2H, q, J=7.7Hz), 3.46 (3H, s), 4.64 (1H, d, J=14.3Hz), 4.95 (1H, d, J=14.3 Hz), 5.80 (1H, s), 7.38-7.51 (2H, m), 7.55-7.62 (2H, m).
MS m/z: 308(M⁺), 240, 124, 116, 89.

### (Example 89)

### Compound of Compound No. 3-7

¹H-NMR(200MHz, CDCl₃) δppm: 1.21 (3H, t, J=7.7Hz), 2.58 (2H, q, J=7.7Hz), 3.37 (3H, s), 3.44 (3H, s), 3.65 (1H, d, J=14.7Hz), 3.88 (1H, d, J=14.7Hz), 4.52 (1H, d, J=13.6Hz), 5.02 (1H, d, J=13.6Hz), 5.69 (1H, s), 7.39-7.64 (4H, m).
MS m/z: 312(M⁺), 284, 239, 168, 116.

### (Example 90)

### Compound of Compound No. 3-9

¹H-NMR(200MHz, CDCl₃) δppm: 1.19 (3H, t, J=7.6Hz), 2.58 (2H, q, J=7.6Hz), 3.48 (3H, s), 4.32 (1H, d, J=14.9Hz), 4.51 (2H, d, J=14.9Hz), 5.09 (1H, d, J=14.9Hz), 5.73 (1H, s), 6.79 (2H, d, J=6.2Hz), 6.98 (1H, t, J=7.3Hz), 7.22-7.30 (2H, m), 7.40-7.64 (4H, m).
MS m/z: 374(M⁺), 281, 253, 239, 116.

### (Example 91)

### Compound of Compound No. 3-11

¹H-NMR(200MHz, CDCl₃) δppm: 0.93 (3H, t, J=7.3Hz), 1.52-1.71 (2H, m), 1.89 (3H, s), 2.52 (2H, t, J=7.5Hz), 3.44 (3H, s), 4.57 (1H, br), 4.96 (1H, br), 7.38-7.63 (4H, m).
MS m/z: 296(M⁺), 268, 254, 226, 152.

### (Example 92)

### Compound of Compound No. 3-13

¹H-NMR(200MHz, CDCl₃) δppm: 0.72-0.80 (2H, m), 0.94 (3H, t, J=7.5Hz), 0.94-1.16 (2H, m), 1.16-1.34 (1H, m), 1.54-1.74 (2H, m), 2.53 (2H, t, J=7.7Hz), 3.46 (3H, s), 4.62 (1H, d, J=14.3Hz), 4.97 (1H, d, J=14.3 Hz), 5.77 (1H, s), 7.38-7.50 (2H, m), 7.54-7.62 (2H, m).
MS m/z: 322(M⁺), 294, 254, 226, 116.

### (Example 93)

### Compound of Compound No. 3-17

¹H-NMR(200MHz, CDCl₃) δppm: 0.93 (3H, t, J=7.3Hz), 1.52-1.71 (2H, m), 2.52 (2H, t, J=7.5Hz), 3.37 (3H, s), 3.45 (3H, s), 3.65 (1H, d, J=14.5 Hz), 3.88 (1H, d, J=14.5Hz), 4.50 (1H, d, J=13.6Hz), 5.06 (1H, d, J=13.6Hz), 5.67 (1H, s), 7.38-7.64 (4H, m).
MS m/z: 326(M⁺), 311, 298, 270, 253.

### (Example 94)

### Compound of Compound No. 3-18

¹H-NMR(200MHz, CDCl₃) δppm: 0.91 (3H, t, J=7.3Hz), 1.50-1.69 (2H, m), 2.52 (2H, t, J=7.5Hz), 3.50 (3H, s), 4.32 (1H, d, J=13.2 Hz), 4.40-4.55 (2H, m), 5.13 (1H, d, J=13.2Hz), 5.70 (1H, s), 6.78 (2H, d, J=7.7Hz), 6.98 (1H, t, J=7.3Hz), 7.22-7.30 (2H, m), 7.39-7.64 (4H, m).
MS m/z: 388(M⁺), 360, 295, 267, 253.

### (Example 95)

### Compound of Compound No. 3-19

¹H-NMR(200MHz, CDCl₃) δppm: 0.93 (3H, t, J=7.3Hz), 1.55-1.67 (2H, m), 2.50 (2H, t, J=7.7Hz), 3.41 (3H, s), 3.77 (3H, s), 4.72 (2H, s), 5.65 (1H, s), 7.43-7.50 (2H, m) 7.58-7.63 (2H, m).
MS m/z: 312(M'), 297, 284, 196, 116.

### (Example 96)

### Compound of Compound No. 3-20

¹H-NMR(200MHz, CDCl₃) δppm: 0.95 (3H, t, J=7.3Hz), 1.58-1.73 (2H, m), 2.68 (2H, t, J=7.5Hz), 3.53 (3H, s), 4.81 (2H, s), 5.79 (1H, s), 7.06-7.67 (9H, m).
MS m/z: 374(M⁺), 346, 281, 137, 116.

### (Example 97)

### Compound of Compound No. 3-21

¹H-NMR(200MHz, CDCl₃) δppm: 0.92 (3H, t, J=7.1Hz), 1.22-1.43 (2H, m), 1.50-1.68 (2H, m), 1.89 (3H, s), 2.54 (2H, t, J=7.7Hz), 3.44 (3H, s), 4.57 (1H, br), 4.95 (1H, br), 5.67 (1H, s), 7.42-7.50 (2H, m), 7.55-7.62 (2H, m).
MS m/z: 310(M⁺), 268, 226, 166, 116.

### (Example 98)

### Compound of Compound No. 3-27

¹H-NMR(200MHz, CDCl₃) δppm: 0.93 (3H, t, J=7.1Hz), 1.26-1.43 (2H, m), 1.50-1.67 (2H, m), 2.55 (2H, t, J=7.7Hz), 3.37 (3H, s), 3.45 (3H, s), 3.62 (1H, d, J=13.9Hz), 3.88 (1H, d, J=13.9Hz), 4.50 (1H, d, J=13.6Hz), 5.05 (1H, d, J=13.6Hz), 5.67 (1H, s), 7.39-7.64 (4H, m).
MS m/z: 340(M⁺), 311, 298, 270, 116.

### (Example 99)

### Compound of Compound No. 3-28

¹H-NMR(200MHz, CDCl₃) δppm: 0.91 (3H, t, J=7.1Hz), 1.26-1.37 (2H, m), 1.48-1.62 (2H, m), 2.54 (2H, t, J=7.5Hz), 3.50 (3H, s), 4.30 (1H, d, J=13.6Hz), 4.42-4.57 (2H, m), 5.12 (1H, d, J=13.6Hz), 5.70 (1H, s), 6.78 (2H, d, J=8.4 Hz), 6.98 (1H, t, J=7.3Hz), 7.22-7.69 (6H, m).
MS m/z: 402(M⁺), 360, 281, 137, 116.

### (Example 100)

### Compound of Compound No. 3-29

¹H-NMR(200MHz, CDCl₃) δppm: 0.92 (3H, t, J=7.3Hz), 1.26-1.45 (2H, m), 1.50-1.66 (2H, m), 2.53 (2H, t, J=7.7Hz), 3.41 (3H, s), 3.77 (3H, s), 4.72 (2H, s), 5.65 (1H, s), 7.43-7.47 (2H, m) 7.59-7.63 (2H, m).
MS m/z: 326(M⁺), 311, 297, 284, 168.

### (Example 101)

### Compound of Compound No. 3-30

¹H-NMR(200MHz, CDCl₃) δppm: 0.93 (3H, t, J=7.1Hz), 1.27-1.45 (2H, m), 1.52-1.67 (2H, m), 2.56 (2H, t, J=7.7Hz), 3.53 (3H, s), 4.81 (2H, bs), 5.79 (1H, s), 7.08 (2H, d, J=7.7Hz),7.19-7.67 (7H, m).
MS m/z: 388(M⁺), 346, 295, 137, 116.

### (Example 102)

### Compound of Compound No. 3-31

¹H-NMR(200MHz, CDCl₃) δppm: 0.89 (3H, t, J=6.8Hz), 1.20-1.42 (4H, m), 1.50-1.70 (2H, m), 1.89 (3H, s), 2.53 (2H, t, J=7.7Hz), 3.44 (3H, s), 4.55 (1H, br), 4.95 (1H, br), 5.68 (1H, s), 7.39-7.50 (2H, m), 7.56-7.62 (2H, m).
MS m/z: 324(M⁺), 295, 281, 268, 226.

### (Example 103)

### Compound of Compound No. 3-37

¹H-NMR(200MHz, CDCl₃) δppm: 0.90 (3H, t, J=6.8Hz), 1.22-1.42 (4H, m), 1.50-1.67 (2H, m), 2.53 (2H, t, J=7.7Hz), 3.37 (3H, s), 3.45 (3H, s), 3.65 (1H, d, J=12.5Hz), 3.89 (1H, d, J=12.5Hz), 4.50 (1H, d, J=14.1Hz), 5.05 (1H, d, J=14.1Hz), 5.67 (1H, s), 7.39-7.64 (4H, m).
MS m/z: 354(M⁺), 325, 312, 298, 270.

### (Example 104)

### Compound of Compound No. 3-38

¹H-NMR(200MHz, CDCl₃) δppm: 0.88 (3H, t, J=6.8Hz), 1.21-1.41 (4H, m), 1.49-1.64 (2H, m), 2.53 (2H, t, J=7.7Hz), 3.49 (3H, s), 4.31 (1H, d, J=15.0Hz), 4.42-4.56 (2H, m), 5.12 (1H, d, J=13.9Hz), 5.70 (1H, s), 6.78 (2H, d, J=8.4Hz), 6.98 (1H, t, J=7.3Hz), 7.20-7.69 (6H, m).
MS m/z: 416(M⁺), 387, 374, 360, 295.

### (Example 105)

### Compound of Compound No. 3-39

¹H-NMR(200MHz, CDCl₃) δppm: 0.89 (3H, t, J=6.6Hz), 1.24-1.41 (4H, m), 1.52-1.69 (2H, m), 2.52 (2H, t, J=7.9Hz), 3.41 (3H, s), 3.77 (3H, s), 4.72 (2H, s), 5.66 (1H, s) 7.43-7.70 (4H, m). MS m/z: 340(M⁺), 311, 297, 284, 116.

### (Example 106)

### Compound of Compound No. 3-40

¹H-NMR(200MHz, CDCl₃) δppm: 0.90 (3H, t, J=6.6Hz), 1.25-1.45 (4H, m), 1.52-1.70 (2H, m), 2.55 (2H, t, J=7.7Hz), 3.54 (3H, s), 4.82 (2H, bs), 5.79 (1H, s), 7.08 (2H, d, J=8.1Hz), 7.19-7.68 (7H, m).
MS m/z: 402(M⁺), 373, 360, 346, 284.

### (Example 107)

### Compound of Compound No. 3-41

¹H-NMR(200MHz, CDCl₃) δppm: 0.88 (3H, t, J=6.3Hz), 1.20-1.40 (6H, m), 1.52-1.66 (2H, m), 1.90 (3H, s) 2.54 (2H, t, J=7.7Hz), 3.44 (3H, s), 4.58 (1H, br), 4.95 (1H, br), 5.69 (1H, s), 7.42-7.63 (4H, m).
MS m/z: 338(M⁺), 295, 281, 268, 226.

### (Example 108)

### Compound of Compound No. 3-47

¹H-NMR(200MHz, CDCl₃) δppm: 0.88 (3H, t, J=6.6Hz), 1.22-1.40 (6H, m), 1.50-1.67 (2H, m), 2.53 (2H, t, J=7.7Hz), 3.36 (3H, s), 3.44 (3H, s), 3.65 (1H, d, J=12.5Hz), 3.86 (1H, d, J=12.5Hz), 4.49 (1H, d, J=12.5Hz), 5.05 (1H, d, J=12.5Hz), 5.67 (1H, s), 7.37-7.64 (4H, m).
MS m/z: 368(M⁺), 339, 326, 311, 298.

### (Example 109)

### Compound of Compound No. 3-48

¹H-NMR(200MHz, CDCl₃) δppm: 0.88 (3H, t, J=6.4Hz), 1.21-1.40 (6H, m), 1.47-1.65 (2H, m), 2.53 (2H, t, J=7.7Hz), 3.49 (3H, s), 4.31 (1H, d, J=14.7Hz), 4.42-4.56 (2H, m), 5.11 (1H, d, J=13.9Hz), 5.71 (1H, s), 6.78 (2H, d, J=8.1Hz), 6.98 (1H, t, J=7.3Hz), 7.22-7.28 (2H, m), 7.39-7.64 (4H, m).
MS m/z: 430(M⁺), 388, 373, 360, 309.

### (Example 110)

### Compound of Compound No. 3-49

¹H-NMR(200MHz, CDCl₃) δppm: 0.88 (3H, t, J=6.4Hz), 1.22-1.40 (6H, m), 1.48-170 (2H, m), 2.52 (2H, t, J=7.7Hz), 3.41 (3H, s), 3.77 (3H, s), 4.72 (2H, s), 5.66 (1H, s), 7.40-7.64 (4H, m).
MS m/z: 354(M⁺), 325, 312, 297, 284.

### (Example 111)

### Compound of Compound No. 3-50

¹H-NMR(200MHz, CDCl₃) δppm: 0.88 (3H, t, J=6.4Hz), 1.22-1.40 (6H, m), 1.52-1.71 (2H, m), 2.55 (2H, t, J=7.7Hz), 3.53 (3H, s), 4.82 (2H, bs), 5.79 (1H, s), 7.08 (2H, d, J=7.3Hz), 7.19-7.67 (7H, m).
MS m/z: 416(M⁺), 374, 359, 346, 323.

### (Example 112)

### Compound of Compound No. 3-51

¹H-NMR(200MHz, CDCl₃) δppm: 1.22 (6H, d, J=7.0Hz), 1.87 (3H, s), 2.79-2.98 (1H, m), 3.44 (3H, s), 4.58 (1H, br), 4.94 (1H, br), 5.70 (1H, s), 7.38-7.65 (4H, m).
MS m/z: 296(M⁺), 281, 254, 239, 226.

### (Example 113)

### Compound of Compound No. 3-53

¹H-NMR(200MHz, CDCl₃) δppm: 0.72-0.85 (2H, m), 1.05-1.33 (3H, m), 1.22 (6H, d, J=6.2Hz), 2.82-2.96 (1H, m), 3.46 (3H, s), 4.63 (1H, d, J=14.3Hz), 4.95 (1H, d, J=14.3 Hz), 5.79 (1H, s), 7.38-7.62 (4H, m).
MS m/z: 322(M⁺), 254, 138, 116, 89.

### (Example 114)

### Compound of Compound No. 3-57

¹H-NMR(200MHz, CDCl₃) δppm: 1.21 (6H, d, J=7.0Hz), 2.81-2.95 (1H, m), 3.37 (3H, s), 3.45 (3H, s), 3.64 (1H, d, J=14.8Hz), 3.88 (1H, d, J=14.8Hz), 4.51 (1H, d, J=14.8Hz), 5.03 (1H, d, J=14.8 Hz), 5.68 (1H, s), 7.39-7.64 (4H, m).
MS m/z: 326(M⁺), 311, 297, 253, 182.

### (Example 115)

### Compound of Compound No. 3-58

¹H-NMR(200MHz, CDCl₃) δppm: 1.19 (6H, d, J=7.0Hz), 2.80-2.94 (1H, m), 3.50 (3H, s), 4.32 (1H, d, J=13.2Hz), 4.42-4.56 (2H, m), 5.10 (1H, d, J=13.2Hz), 5.71 (1H, s), 6.77 (2H, d, J=8.4Hz), 6.98 (1H, t, J=7.3Hz), 7.22-7.30 (2H, m), 7.39-7.69 (4H, m).
MS m/z: 388(M⁺), 295, 267, 253, 225.

### (Example 116)

### Compound of Compound No. 3-61

¹H-NMR(200MHz, CDCl₃) δppm: 0.84 (3H, t, J=7.3Hz), 1.19 (3H, d, J=7.0Hz), 1.48-1.65 (2H, m), 2.56-2.76 (1H, m), 3.46 (3H, s), 4.62 (1H, d, J=14.5Hz), 4.96 (1H, d, J=14.5Hz), 5.75 (1H, s), 7.37-7.62 (4H, m).
MS m/z: 310(M⁺), 295, 282, 268, 240.

### (Example 117)

### Compound of Compound No. 3-63

¹H-NMR(200MHz, CDCl₃) δppm: 0.72-0.92 (2H, m), 0.85 (3H, t, J=7.3Hz), 1.02-1.32 (3H, m), 1.20 (3H, d, J=7.0Hz), 1.48-1.64 (2H, m), 2.56-2.75 (1H, m), 3.46 (3H, s), 4.62 (1H, d, J=14.5Hz), 4.96 (1H, d, J=14.5 Hz), 5.75 (1H, s), 7.37-7.62 (4H, m).
MS m/z: 336(M⁺), 308, 268, 240, 116.

### (Example 118)

### Compound of Compound No. 3-67

¹H-NMR(200MHz, CDCl₃) δppm: 0.84 (3H, t, J=7.8Hz), 1.19 (3H, d, J=7.0Hz), 1.43-1.64 (2H, m), 2.56-2.74 (1H, m), 3.36 (3H, s), 3.46 (3H, s), 3.65 (1H, d, J=14.7Hz), 3.88 (1H, d, J=14.7Hz), 4.49 (1H, d, J=14.1Hz), 5.06 (1H, d, J=14.1Hz), 5.64 (1H, s), 7.39-7.64 (4H, m) .
MS m/z: 340(M⁺), 325, 312, 284, 267.

### (Example 119)

### Compound of Compound No. 3-68

¹H-NMR(200MHz, CDCl₃) δppm: 0.84 (3H, t, J=7.8Hz), 1.16 (3H, d, J=7.0Hz), 1.45-1.63 (2H, m), 2.55-2.72 (1H, m), 3.50 (3H, s), 4.32 (1H, d, J=14.7Hz), 4.37-4.55 (2H, m), 5.13 (1H, d, J=12.5Hz), 5.67 (1H, s), 6.77 (2H, d, J=8.1Hz), 6.97 (1H, t, J=7.3Hz), 7.22-7.30 (2H, m), 7.39-7.67 (4H, m).
MS m/z: 402(M⁺), 387, 374, 281, 267.

### (Example 120)

### Compound of Compound No. 3-71

¹H-NMR(200MHz, CDCl₃) δppm: 0.79 (6H, t, J=7.5Hz), 1.36-1.68 (4H, m), 1.89 (3H, s), 2.34-2.46 (1H, m), 3.45 (3H, s), 4.52 (1H, d, J=12.1Hz), 5.00 (1H, d, J=12.1Hz), 5.61 (1H, s), 7.37-7.63 (4H, m).
MS m/z: 324(M⁺), 296, 281, 254, 239.

### (Example 121)

### Compound of Compound No. 3-73

¹H-NMR(200MHz, CDCl₃) δppm: 0.72-0.92 (2H, m), 0.80 (6H, t, J=7.1Hz), 1.00-1.28 (3H, m), 1.37-1.72 (4H, m), 2.34-2.49 (1H, m), 3.46 (3H, s), 4.63 (1H, d, J=13.9Hz), 4.97 (1H, d, J=13.9Hz), 5.70 (1H, s), 7.36-7.62 (4H, m).
MS m/z: 350(M⁺), 322, 282, 254, 116.

### (Example 122)

### Compound of Compound No. 3-77

¹H-NMR(200MHz, CDCl₃) δppm: 0.79 (6H, t, J=7.3Hz), 1.40-1.69 (4H, m), 2.34-2.48 (1H, m), 3.36 (3H, s), 3.47 (3H, s), 3.65 (1H, d, J=14.8Hz), 3.87 (1H, d, J=14.8Hz), 4.47 (1H, d, J=14.1Hz), 5.09 (1H, d, J=14.1Hz), 5.59 (1H, s), 7.38-7.63 (4H, m).
MS m/z: 354(M⁺), 326, 311, 298, 281.

### (Example 123)

### Compound of Compound No. 3-78

¹H-NMR(200MHz, CDCl₃) δppm: 0.75-0.88 (6H, m), 1.38-1.83 (4H, m), 2.33-2.47 (1H, m), 3.52 (3H, s), 4.32 (1H, d, J=13.9Hz), 4.42-4.52 (2H, m), 5.18 (1H, d, J=13.9Hz), 5.63 (1H, s), 6.77 (2H, d, J=8.4Hz), 6.97 (1H, t, J=7.3Hz), 7.20-7.30 (2H, m), 7.39-7.63 (4H, m).
MS m/z: 416(M⁺), 401, 388, 373, 295.

### (Example 124)

### Compound of Compound No. 3-81

¹H-NMR(200MHz, CDCl₃) δppm: 1.12-1.47 (5H, m), 1.65-1.96 (5H, m), 1.89 (3H, s), 2.48-2.63 (1H, m), 3.44 (3H, s), 4.54 (1H, br), 4.98 (1H, br), 5.67 (1H, s), 7.39-7.63 (4H, m).
MS m/z: 336(M⁺), 293, 281, 268, 239.

### (Example 125)

### Compound of Compound No. 3-83

¹H-NMR(200MHz, CDCl₃) δppm: 0.71-0.85 (2H, m), 1.00-1.48 (8H, m), 1.66-1.98 (5H, m), 2.48-2.62 (1H, m), 3.47 (3H, s), 4.61 (1H, d, J=14.3Hz), 4.96 (1H, d, J=14.3Hz), 5.76 (1H, s), 7.38-7.68 (4H, m).
MS m/z: 362(M⁺), 321, 307, 294, 239.

### (Example 126)

### Compound of Compound No. 3-87

¹H-NMR(200MHz, CDCl₃) δppm: 1.16-1.42 (5H, m), 1.65-1.96 (5H, m), 2.45-2.60 (1H, m), 3.36 (3H, s), 3.45 (3H, s), 3.66 (1H, br), 3.87 (1H, br), 4.48 (1H, br), 5.02 (1H, br), 5.66 (1H, s), 7.36-7.63 (4H, m).
MS m/z: 366(M+), 351, 325, 311, 298.

### (Example 127)

### Compound of Compound No. 3-88

¹H-NMR(200MHz, CDCl₃) δppm: 1.16-1.47 (5H, m), 1.65-1.95 (5H, m), 2.46-2.60 (1H, m), 3.50 (3H, s), 4.32 (1H, d, J=10.3Hz), 4.42-4.54 (2H, m), 5.10 (1H, d, J=11.7 Hz), 5.69 (1H, s), 6.77 (2H, d, J=8.1Hz), 6.98 (1H, t, J=7.3Hz), 7.22-7.30 (2H, m), 7.39-7.64 (4H, m).
MS m/z: 428(M⁺), 387, 373, 360, 293.

### (Example 128)

### Compound of Compound No. 3-91

¹H-NMR(200MHz, CDCl₃) δppm: 1.25 (9H, s), 1.89 (3H, s), 3.45 (3H, s), 4.54 (1H, br), 4.96 (1H, br), 5.71 (1H, s), 7.38-7.69 (4H, m).
MS m/z: 310(M⁺), 295, 268, 253, 226.

### (Example 129)

### Compound of Compound No. 3-93

¹H-NMR(200MHz, CDCl₃) δppm: 0.71-0.85 (2H, m), 1.00-1.13 (2H, m) 1.26 (9H, s), 1.20-1.34 (1H, m), 3.47 (3H, s), 4.61 (1H, d, J=14.1Hz), 4.96 (1H, d, J=14.1Hz), 5.80 (1H, s), 7.38-7.62 (4H, m).
MS m/z: 336(M⁺), 321, 268, 253, 226.

### (Example 130)

### Compound of Compound No. 3-97

¹H-NMR(200MHz, CDCl₃) δppm: 1.25 (9H, s), 3.37 (3H, s), 3.46 (3H, s), 3.66 (1H, d, J=14.7Hz), 3.88 (1H, d, J=14.7Hz), 4.49 (1H, d, J=13.6Hz), 5.04 (1H, d, J=13.6Hz), 7.39-7.64 (4H, m). MS m/z: 340(M⁺), 325, 297, 267, 253.

### (Example 131)

### Compound of Compound No. 3-98

¹H-NMR(200MHz, CDCl₃) δppm: 1.23 (9H, s), 3.51 (3H, s), 4.32 (1H, d, J=14.7Hz), 4.43-4.55 (2H, m), 5.11 (1H, d, J=13.9Hz), 5.73 (1H, s), 6.77 (2H, d, J=8.4Hz), 6.97 (1H, t, J=7.3Hz), 7.22-7.30 (2H, m), 7.39-7.64 (4H, m).
MS m/z: 402(M⁺), 387, 309, 281, 267.

### (Example 132)

### Compound of Compound No. 3-99

¹H-NMR(200MHz, CDCl₃) δppm: 1.58 (3H, s), 1.55-2.10 (6H, m), 1.83-1.91 (1H, m), 2.58 (2H, s), 2.45-2.65 (1H, m), 3.32 (3H, s), 3.72 (3H, bs), 4.67 (2H, s), 7.36-7.42 (2H, m) 7.55-7.63 (2H, m).
MS m/z: 352(M⁺), 324, 311, 298, 116.

### (Example 133)

### Compound of Compound No. 4-1

¹H-NMR(200MHz, CDCl₃) δppm: 0.83-0.93 (6H, m), 1.06-1.47 (2H, m), 1.55-1.79 (1H, m), 1.89 (3H, s), 2.32 (1H, dd, J=8.1, 14.1Hz), 2.55 (1H, dd, J=6.2, 14.1Hz), 3.45 (3H, s), 4.52 (1H, br), 5.01 (1H, br), 5.64 (1H, s), 7.38-7.62 (4H, m).
MS m/z: 324(M⁺), 309, 295, 281, 268.

### (Example 134)

### Compound of Compound No. 4-4

¹H-NMR(200MHz, CDCl₃) δppm: 0.83-0.93 (6H, m), 1.07-1.46 (2H, m), 1.55-1.72 (1H, m), 2.32 (1H, dd, J=8.1, 14.0Hz), 2.54 (1H, dd, J=6.0, 14.0Hz), 3.37 (3H, s), 3.46 (3H, s), 3.65 (1H, d, J=14.7Hz), 3.88 (1H, d, J=14.7Hz), 4.46 (1H, d, J=13.9Hz), 5.09 (1H, d, J=13.9Hz), 5.63 (1H, s), 7.39-7.64 (4H, m).
MS m/z: 354(M⁺), 339, 325, 298, 116.

### (Example 135)

### Compound of Compound No. 4-5

¹H-NMR(200MHz, CDCl₃) δppm: 0.81-0.90 (6H, m), 1.07-1.41 (2H, m), 1.56-1.70 (1H, m), 2.32 (1H, dd, J=8.1, 14.3Hz), 2.54 (1H, dd, J=6.4, 14.3Hz), 3.51 (3H, s), 4.32 (1H, d, J=14.5 Hz), 4.41-4.52 (2H, m), 5.17 (1H, d, J=14.0Hz), 5.67 (1H, s), 6.78 (2H, d, J=7.6Hz), 6.98 (1H, t, J=7.6Hz), 7.23-7.29 (2H, m), 7.40-7.68 (4H, m).
MS m/z: 416(M⁺), 401, 360, 295, 116.

### (Example 136)

### Compound of Compound No. 4-9

¹H-NMR(200MHz, CDCl₃) δppm: 0.84-0.93 (6H, m), 1.07-1.73 (3H, m), 2.31 (1H, dd, J=8.2, 14.2Hz), 2.54 (1H, dd, J=5.9, 14.2Hz), 3.42 (3H, s), 3.78 (3H, s), 4.73 (2H, s), 5.63 (1H, s), 7.44-7.70 (4H, m).
MS m/z: 340(M⁺), 325, 311, 284, 116.

### (Example 137)

### Compound of Compound No. 4-10

¹H-NMR(200MHz, CDCl₃) δppm: 0.85-0.94 (6H, m), 1.08-1.50 (2H, m), 1.57-1.73 (1H, m), 2.34 (1H, dd, J=8.1, 14.1Hz), 2.57 (1H, dd, J=6.2, 14.1Hz), 3.54 (3H, s), 4.82 (2H, s), 5.76 (1H, s), 7.08 (2H, d, J=8.1Hz), 7.20-7.66 (7H, m).
MS m/z: 402(M⁺), 387, 373, 346, 309.

### (Example 138)

### Compound of Compound No. 4-11

¹H-NMR(200MHz, CDCl₃) δppm: 0.89 (9H, s), 1.89 (3H, s), 2.41 (2H, s), 3.47 (3H, s), 4.50 (1H, br), 5.07 (1H, br), 5.63 (1H, s), 7.38-7.63 (4H, m).
MS m/z: 324(M⁺), 309, 284, 268, 226.

### (Example 139)

### Compound of Compound No. 4-14

¹H-NMR(200MHz, CDCl₃) δppm: 0.89 (9H, s), 2.41 (2H, s), 3.36 (3H, s), 3.48 (3H, s), 3.65 (1H, d, J=14.5Hz), 3.88 (1H, d, J=14.5Hz), 4.43 (1H, d, J=13.6Hz), 5.13 (1H, d, J=13.6Hz), 5.61 (1H, s), 7.39-7.62 (4H, m).
MS m/z: 354(M⁺), 339, 298, 182, 116.

### (Example 140)

### Compound of Compound No. 4-15

¹H-NMR(200MHz, CDCl₃) δppm: 0.87 (9H, s), 2.41 (2H, s), 3.53 (3H, s), 4.29-4.53 (3H, m), 5.21 (1H, d, J=13.6Hz), 5.67 (1H, s), 6.78 (2H, d, J=8.4Hz), 6.98 (1H, t, J=7.3Hz), 7.22-7.65 (6H, m).
MS m/z: 416(M⁺), 401, 360, 244, 116.

### (Example 141)

### Compound of Compound No. 4-19

¹H-NMR(200MHz, CDCl₃) δppm: 0.90 (9H, s), 2.40 (2H, s), 3.42 (3H, s), 3.77 (3H, s), 4.73 (2H, s), 5.61 (1H, s), 7.43-7.64 (4H, m).
MS m/z: 340(M⁺), 325, 284, 168, 116.

### (Example 142)

### Compound of Compound No. 4-20

¹H-NMR(200MHz, CDCl₃) δppm: 0.92 (9H, s), 2.43 (2H, s), 3.55 (3H, s), 4.82 (2H, bs), 5.75 (1H, s), 7.09 (2H, d, J=8.1Hz), 7.19-7.66 (7H, m).
MS m/z: 402(M⁺), 387, 346, 136, 116.

### (Example 143)

### Compound of Compound No. 4-21

¹H-NMR(200MHz, CDCl₃) δppm: 0.92 (6H, d, J=6.6Hz), 1.42-1.64 (3H, m), 1.89 (3H, s), 2.54 (2H, d, J=7.9Hz), 3.44 (3H, s), 4.57 (1H, m), 4.90 (1H, m), 5.69 (1H, s), 7.39-7.63 (4H, m). MS m/z: 324(M⁺), 309, 281, 268, 226, 116.

### (Example 144)

### Compound of Compound No. 4-23

¹H-NMR(200MHz, CDCl₃) δppm: 0.69-1.25 (5H, m), 0.88 (6H, d, J=6.2Hz), 1.43-1.50 (3H, m), 2.51 (2H, d, J=7.9Hz), 3.42 (3H, s), 4.58 (1H, d, J=14.5Hz), 4.92 (1H, d, J=14.5Hz), 5.74 (1H, s), 7.38-7.57 (4H, m).
MS m/z: 350(M⁺), 335, 294, 226, 116.

### (Example 145)

### Compound of Compound No. 4-24

¹H-NMR(200MHz, CDCl₃) δppm: 0.92 (6H, d, J=6.2Hz), 1.42-1.60 (3H, m), 2.55 (2H, d, J=7.9Hz), 3.37 (3H, s), 3.45 (3H, s), 3.65 (1H, d, J=13.8Hz), 3.89 (1H, d, J=13.8Hz), 4.51 (1H, d, J=14.3Hz), 5.05 (1H, d, J=14.3Hz), 5.68 (1H, s), 7.39-7.65 (4H, m).
MS m/z: 354(M⁺), 339, 311, 298, 226, 116.

### (Example 146)

### Compound of Compound No. 4-25

¹H-NMR(200MHz, CDCl₃) δppm: 0.90 (6H, d, J=6.2Hz), 1.39-1.57 (3H, m), 2.54 (2H, t, J=7.9Hz), 3.49 (3H, s), 4.30 (1H, d, J=14.3Hz), 4.46-4.52 (3H, m), 5.12 (1H, d, J=14.3Hz), 5.72 (1H, s), 6.78 (2H, d, J=8.4Hz), 6.97 (1H, t, J=7.3Hz), 7.20-7.64 (6H, m).
MS m/z: 416(M⁺), 360, 295, 116.

### (Example 147)

### Compound of Compound No. 4-29

¹H-NMR(200MHz, CDCl₃) δppm: 0.92 (6H, d, J=6.2Hz), 1.43-1.58 (3H, m), 2.53 (2H, t, J=7.9Hz), 3.41 (3H, s), 3.77 (3H, s), 4.72 (2H, s), 5.66 (1H, s), 7.44-7.64 (4H, m).
MS m/z: 340(M⁺), 294, 168, 116.

### (Example 148)

### Compound of Compound No. 4-31

¹H-NMR(200MHz, CDCl₃) δppm: 0.12-0.19 (2H, m), 0.46-0.56 (2H, m), 0.86-1.06 (1H, m), 1.90 (3H, s), 2.47 (2H, d, J=7.0Hz), 3.45 (3H, s), 4.57 (1H, br), 4.96 (1H, br), 5.78 (1H, s), 7.39-7.63 (4H, m).
MS m/z: 308(M⁺), 265, 238, 225, 164.

### (Example 149)

### Compound of Compound No. 4-33

¹H-NMR(200MHz, CDCl₃) δppm: 0.13-0.20 (2H, m), 0.47-0.56 (2H, m), 0.72-1.34 (6H, m), 2.48 (2H, d, J=7.0Hz), 3.47 (3H, s), 4.63 (1H, d, J=14.1Hz), 4.97 (1H, d, J=14.1Hz ), 5.87 (1H, s), 7.39-7.62 (4H, m).
MS m/z: 334(M⁺), 266, 183, 164, 116.

### (Example 150)

### Compound of Compound No. 4-34

¹H-NMR(200MHz, CDCl₃) δppm: 0.12-0.19 (2H, m), 0.47-0.56 (2H, m), 0.87-1.03 (1H, m), 2.47 (2H, d, J=7.0Hz), 3.37 (3H, s), 3.46 (3H, s), 3.66 (1H, d, J=13.9Hz), 3.90 (1H, d, J=13.9Hz), 4.51 (1H, d, J=13.7Hz), 5.06 (1H, d, J=13.7Hz), 5.77 (1H, s), 7.40-7.64 (4H, m).
MS m/z: 338(M⁺), 323, 309, 297, 265.

### (Example 151)

### Compound of Compound No. 4-35

¹H-NMR(200MHz, CDCl₃) δppm: 0.12-0.18 (2H, m), 0.45-0.54 (2H, m), 0.84-1.03 (1H, m), 2.47 (2H, d, J=7.0Hz), 3.50 (3H, s), 4.33 (1H, d, J=14.3Hz), 4.47-4.56 (2H, m), 5.13 (1H, d, J=14.3Hz), 5.80 (1H, s), 6.79 (2H, d, J=7.7Hz), 6.98 (1H, t, J=7.3Hz), 7.21-7.65 (6H, m).
MS m/z: 400(M⁺), 359, 307, 279, 265.

### (Example 152)

### Compound of Compound No. 4-40

¹H-NMR(200MHz, CDCl₃) δppm: 0.14-0.21 (2H, m), 0.47-0.57 (2H, m), 0.88-1.04 (1H, m), 2.49 (2H, d, J=7.0Hz), 3.54 (3H, s), 4.82 (2H, bs), 5.90 (1H, s), 7.09 (2H, d, J=7.7Hz), 7.19-7.67 (7H, m).
MS m/z: 386(M⁺), 293, 265, 176, 136.

### (Example 153)

### Compound of Compound No. 4-41

¹H-NMR(200MHz, CDCl₃) δppm: 1.15-1.24 (2H, m), 1.49-1.74 (6H, m), 1.89 (3H, s), 1.99-2.11 (1H, m), 2.53 (2H, d, J=7.3Hz), 3.45 (3H, s), 4.55 (1H, m), 4.99 (1H, m), 5.67 (1H, s), 7.39-7.63 (4H, m).
MS m/z: 336(M⁺), 293, 268, 226, 116.

### (Example 154)

### Compound of Compound No. 4-43

¹H-NMR(200MHz, CDCl₃) δppm: 0.72-0.81 (2H, m), 0.99-1.28 (5H, m), 1.49-1.75 (6H, m), 2.00-2.12 (1H, m), 2.54 (2H, d, J=7.3Hz), 3.47 (3H, s), 4.60 (1H, d, J=14.3Hz), 5.00 (1H, d, J=14.3Hz), 5.76 (1H, s), 7.38-7.63 (4H, m).
MS m/z: 362(M⁺), 294, 226, 116.

### (Example 155)

### Compound of Compound No. 4-44

¹H-NMR(200MHz, CDCl₃) δppm: 1.11-1.23 (2H, m), 1.49-1.76 (6H, m), 1.99-2.14 (1H, m), 2.53 (2H, d, J=7.3Hz), 3.36 (3H, s), 3.46 (3H, s), 3.65 (1H, d, J=15.0Hz), 3.88 (1H, d, J=15.0Hz), 4.46 (1H, d, J=14.1Hz), 5.09 (1H, d, J=14.1Hz), 5.65 (1H, s), 7.39-7.64 (4H, m).
MS m/z: 366(M⁺), 298, 270, 116.

### (Example 156)

### Compound of Compound No. 4-45

¹H-NMR(200MHz, CDCl₃) δppm: 1.09-1.22 (2H, m), 1.45-1.70 (6H, m), 1.96-2.07 (1H, m), 2.53 (2H, t, J=7.7Hz), 3.51 (3H, s), 4.28-4.53 (3H, m), 5.17 (1H, d, J=13.9Hz), 5.69 (1H, s), 6.77 (2H, d, J=8.4Hz), 6.97 (1H, t, J=7.3Hz), 7.20-7.64 (6H, m).
MS m/z: 428(M⁺), 360, 294, 116.

### (Example 157)

### Compound of Compound No. 4-49

¹H-NMR(200MHz, CDCl₃) δppm: 1.12-1.25 (2H, m), 1.48-1.78 (6H, m), 1.95-2.17 (1H, m), 2.52 (2H, t, J=7.3Hz), 3.41 (3H, s), 3.77 (3H, s), 4.73 (2H, s), 5.64 (1H, s), 7.40-7.69 (4H, m).
MS m/z: 352(M⁺), 311, 284, 116.

### (Example 158)

### Compound of Compound No. 4-51

¹H-NMR(200MHz, CDCl₃) δppm: 0.82-1.78 (11H, m), 1.90 (3H, s), 2.40 (2H, d, J=7.0Hz), 3.45 (3H, s), 4.47 (1H, br), 5.03 (1H, br), 5.62 (1H, s), 7.28-7.62 (4H, m).
MS m/z: 350(M⁺), 307, 268, 226, 116.

### (Example 159)

### Compound of Compound No. 4-54

¹H-NMR(200MHz, CDCl₃) δppm: 0.82-1.75 (11H, m), 2.40 (2H, d, J=7.0Hz), 3.37 (3H, s), 3.47 (3H, s), 3.66 (1H, d, J=14.7Hz), 3.88 (1H, d, J=14.7Hz), 4.44 (1H, d, J=14.7Hz), 5.11 (1H, d, J=14.7Hz), 5.60 (1H, s), 7.38-7.64 (4H, m).
MS m/z: 380(M⁺), 298, 226, 137, 116.

### (Example 160)

### Compound of Compound No. 4-55

¹H-NMR(200MHz, CDCl₃) δppm: 0.80-1.74 (11H, m), 2.40 (2H, d, J=7.0Hz), 3.53 (3H, s), 4.28-4.53 (3H, m), 5.20 (1H, d, J=13.6Hz), 5.65 (1H, s), 6.78 (2H, d, J=7.7Hz), 6.98 (1H, t, J=7.3Hz), 7.22-7.69 (6H, m).
MS m/z: 442(M⁺), 360, 224, 137, 116.

### (Example 161)

### Compound of Compound No. 4-59

¹H-NMR(200MHz, CDCl₃) δppm: 0.81-1.78 (11H, m), 2.39 (2H, d, J=7.0Hz), 3.42 (3H, s), 3.77 (3H, s), 4.72 (2H, s), 5.60 (1H, s), 7.46-7.69 (4H, m).
MS m/z: 366(M⁺), 284, 137, 116,80.

### (Example 162)

### Compound of Compound No. 4-60

¹H-NMR(200MHz, CDCl₃) δppm: 0.81-1.80 (11H, m), 2.42 (2H, d, J=7.0Hz), 3.54 (3H, s), 4.82 (2H, bs), 5.74 (1H, s), 7.08 (2H, d, J=7.7Hz), 7.19-7.66 (7H, m).
MS m/z: 428(M⁺), 346, 149, 136,116.

### (Example 163)

### Compound of Compound No. 4-61

¹H-NMR(200MHz, CDCl₃) δppm: 1.91 (3H, s), 3.48 (3H, s), 4.50 (2H, s), 4.56 (1H, br), 4.96 (1H, br), 5.98 (1H, s), 7.40-7.50 (2H, m), 7.57-7.64 (2H, m).
MS m/z: 302(M⁺), 267, 260, 144, 116.

### (Example 164)

### Compound of Compound No. 4-71

¹H-NMR(200MHz, CDCl₃) δppm: 1.08 (3H, d, J=6.2Hz), 1.89 (3H, s), 2.45-2.60 (2H, m), 2.87-3.01 (1H, m), 3.45 (3H, s), 4.47 (1H, br), 5.00 (1H, br), 5.71 (1H, s), 7.39-7.62 (4H, m).
MS m/z: 364(M⁺), 322, 268, 226, 206.

### (Example 165)

### Compound of Compound No. 4-74

¹H-NMR(200MHz, CDCl₃) δppm: 1.09 (3H, d, J=6.6Hz), 2.46-2.59 (2H, m), 2.87-3.00 (1H, m), 3.36 (3H, s), 3.46 (3H, s), 3.65 (1H, d, J=13.6Hz), 3.85 (1H, d, J=13.6 Hz), 4.50 (1H, d, J=11.7Hz), 5.07 (1H, d, J=11.7Hz), 5.69 (1H, s), 7.40-7.65 (4H, m).
MS m/z: 394(M⁺), 379, 366, 335, 321.

### (Example 166)

### Compound of Compound No. 4-75

¹H-NMR(200MHz, CDCl₃) δppm: 1.01 (3H, d, J=6.6Hz), 2.42-2.60 (2H, m), 2.85-2.99 (1H, m), 3.51 (3H, s), 4.30-4.50 (3H, m), 5.15 (1H, m), 5.72 (1H, s), 6.76 (2H, d, J=8.1 Hz), 6.98 (1H, t, J=7.3Hz), 7.21-7.30 (2H, m), 7.40-7.68 (4H, m).
MS m/z: 456(M⁺), 363, 335, 321, 116.

### (Example 167)

### Compound of Compound No. 4-76

¹H-NMR(200MHz, CDCl₃) δppm: 1.06 (3H, d, J=6.2Hz), 2.45-2.58 (2H, m), 2.87-3.02 (1H, m), 3.68 (3H, s), 4.42-4.52 (1H, m), 4.60 (2H, s), 5.07-5.18 (1H, m), 5.80 (1H, s), 6.85-6.96 (2H, m), 7.39-7.66 (5H, m), 8.05-8.08 (1H, m).
FAB MS m/z 458((M+H)⁺).

### (Example 168)

### Compound of Compound No. 4-77

¹H-NMR(200MHz, CDCl₃) δppm: 1.08 (3H, d, J=6.2Hz), 2.33 (3H, s), 2.44-2.58 (2H, m), 2.87-3.01 (1H, m), 3.61 (3H, s), 4.43-4.62 (3H, m), 5.09-5.18 (1H, m), 5.72 (1H, s), 5.78 (1H, s), 7.40-7.65 (4H, m).
MS m/z: 461(M⁺), 321, 224, 140, 116.

### (Example 169)

### Compound of Compound No. 4-78

¹H-NMR(200MHz, CDCl₃) δppm: 1.09 (3H, d, J=6.6Hz), 2.45-2.62 (2H, m), 2.89-2.97 (1H, m), 3.74 (3H, s), 4.01 (1H, d, J=15.8 Hz), 4.44-4.52 (2H, m), 5.14 (1H, dd, J=4.4, 14.3 Hz), 5.84 (1H, s), 6.23 (1H, d t, J=1.1, 6.9 Hz), 6.57 (1H, d, J=9.2 Hz), 6.57 (1H, d, J=9.2 Hz), 7.22 (1H, d, J=6.9 Hz), 7.37-7.64 (5H, m).
MS m/z: 457(M⁺), 438, 360, 321, 136.

### (Example 170)

### Compound of Compound No. 4-80

¹H-NMR(200MHz, CDCl₃) δppm: 1.08 (3H, d, J=6.2Hz), 2.43-2.58 (2H, m), 2.89-3.00 (1H, m), 3.54 (3H, s), 4.83 (2H, bs), 5.83 (1H, s), 7.08 (2H, d, J=8.1Hz), 7.20-7.65 (7H, m).
MS m/z: 442(M⁺), 349, 227, 168, 116.

### (Example 171)

### Compound of Compound No. 4-81

¹H-NMR(200MHz, CDCl₃) δppm: 1.90 (3H, s), 2.20 (1H, br), 3.46 (3H, s), 4.61 (1H, br), 4.62 (2H, s), 4.91 (1H, br), 5.93 (1H, s), 7.39-7.64 (4H, m).
MS m/z: 284(M⁺), 242, 224, 181, 126.

### (Example 172)

### Compound of Compound No. 4-91

¹H-NMR(200MHz, CDCl₃) δppm: 1.24 (3H, t, J=7.1Hz), 1.90 (3H, s), 3.47 (3H, s), 3.55 (2H, q, J=7.0Hz), 4.42 (2H, s), 4.62 (1H, br), 4.95 (1H, br), 5.95 (1H, s), 7.39-7.63 (4H, m).
MS m/z: 312(M⁺), 293, 268, 226, 116.

### (Example 173)

### Compound of Compound No. 4-101

¹H-NMR(200MHz, CDCl₃) δppm: 1.90 (3H, s), 2.10 (3H, s), 3.49 (3H, s), 4.60 (1H, br), 4.95 (1H, br), 5.02 (2H, s), 5.95 (1H, s), 7.40-7.63 (4H, m).
MS m/z: 326(M⁺), 241, 224, 181, 116.

### (Example 174)

### Compound of Compound No. 4-111

¹H-NMR(200MHz, CDCl₃) δppm: 1.90 (3H, s), 2.25 (6H, s), 3.39 (2H, s), 3.46 (3H, s), 4.58 (1H, br), 4.97 (1H, br), 5.90 (1H, s), 7.38-7.63 (4H, m).
MS m/z: 311(M⁺), 268, 226, 137, 116.

### (Example 175)

### Compound of Compound No. 4-121

¹H-NMR(200MHz, CDCl₃) δppm: 1.78-1.87 (4H, m), 1.90 (3H, s), 2.49-2.60 (4H, m), 3.46 (3H, s), 3.59 (2H, s), 4.57 (1H, br), 4.99 (1H, br), 5.91 (1H, s), 7.39-7.63 (4H, m).
MS m/z: 337(M⁺), 268, 226, 116, 70.

### (Example 176)

### Compound of Compound No. 4-131

¹H-NMR(200MHz, CDCl₃) δppm: 1.40-1.65 (6H, m), 1.90 (3H, s), 2.33-2.42 (4H, m), 3.45 (2H, s), 3.47 (3H, s), 4.51 (1H, br), 5.00 (1H, br), 5.88 (1H, s), 7.38-7.63 (4H, m).
MS m/z: 351(M⁺), 268, 226, 149, 116.

### (Example 177)

### Compound of Compound No. 4-141

¹H-NMR(200MHz, CDCl₃) δppm: 1.88 (3H, s), 2.29-2.40 (2H, m), 2.64 (2H, t, J=7.5Hz), 3.43 (3H, s), 4.55 (1H, br), 4.94-5.05 (3H, m), 5.69 (1H, s), 5.71-5.91 (1H, m), 7.38-7.62 (4H, m).
MS m/z: 308(M⁺), 265, 225, 164, 116.

### (Example 178)

### Compound of Compound No. 4-144

¹H-NMR(200MHz, CDCl₃) δppm: 2.30-2.41 (2H, m), 2.66 (2H, t, J=7.7Hz), 3.36 (3H, s), 3.45 (3H, s), 3.64 (1H, d, J=14.8Hz), 3.87 (1H, d, J=14.8Hz), 4.50 (1H, d, J=13.9Hz), 4.96-5.07 (3H, m), 5.68 (1H, s), 5.71-5.91 (1H, m), 7.39-7.64 (4H, m).
MS m/z: 338(M⁺), 323, 297, 265, 116.

### (Example 179)

### Compound of Compound No. 4-145

¹H-NMR(200MHz, CDCl₃) δppm: 2.28-2.38 (2H, m), 2.65 (2H, t, J=7.5Hz), 3.49 (3H, s), 4.32 (1H, d, J=13.6Hz), 4.42-4.52 (2H, m), 4.95-5.16 (3H, m), 5.68-5.86 (1H, m), 5.69 (1H, s), 6.78 (2H, d, J=7.7Hz), 6.98 (1H, t, J=7.1Hz), 7.22-7.30 (2H, m), 7.40-7.68 (4H, m).
MS m/z: 400(M⁺), 359, 307, 279, 265.

### (Example 180)

### Compound of Compound No. 4-149

¹H-NMR(200MHz, CDCl₃) δppm: 2.30-2.41 (2H, m) , 2.64 (2H, t, J=7.7Hz), 3.41 (3H, s), 3.77 (3H, s), 4.72 (2H, s), 4.96-5.08 (2H, m), 5.68 (1H, s), 5.74-5.94 (1H, m), 7.40-7.48 (2H, m), 7.58-7.65 (2H, m).
MS m/z: 324(M⁺), 283, 265, 222, 176.

### (Example 181)

### Compound of Compound No. 4-150

¹H-NMR(200MHz, CDCl₃) δppm: 2.32-2.43 (2H, m), 2.67 (2H, t, J=7.9Hz), 3.53 (3H, s), 4.81 (2H, bs), 4.96-5.08 (2H, m), 5.75-5.95 (1H, m), 5.81 (1H, s), 7.08 (2H, d, J=7.3Hz), 7.19-7.67 (7H, m).
MS m/z: 386(M⁺), 345, 293, 265, 176.

### (Example 182)

### Compound of Compound No. 4-151

¹H-NMR(200MHz, CDCl₃) δppm: 1.87 (3H, s), 3.45 (3H, s), 3.90 (2H, s), 4.55 (1H, br), 4.93 (1H, br), 5.61 (1H, s), 7.17-7.61 (9H, m).
MS m/z: 344(M⁺), 302, 200, 186, 116.

### (Example 183)

### Compound of Compound No. 4-154

¹H-NMR(200MHz, CDCl₃) δppm: 3.34 (3H, s), 3.46 (3H, s), 3.65 (1H, d, J=15.4Hz), 3.82 (1H, d, J=15.4Hz), 3.90 (2H, s), 4.50 (1H, d, J=15.6Hz), 5.00 (1H, d, J=15.6Hz), 5.59 (1H, s), 7.16-7.78 (9H, m).
MS m/z: 374(M⁺), 359, 345, 301, 258.

### (Example 184)

### Compound of Compound No. 4-155

¹H-NMR(200MHz, CDCl₃) δppm: 3.52 (3H, s), 3.89 (2H, s), 4.27-4.50 (3H, m), 5.08 (1H, d, J=14.7 Hz ), 5.63 (1H, s), 6.74 (2H, d, J=8.1Hz), 6.98 (1H, t, J=7.3Hz), 7.12-7.62 (11H, m).
MS m/z: 436(M⁺), 343, 315, 301, 116.

### (Example 185)

### Compound of Compound No. 4-159

¹H-NMR(200MHz, CDCl₃) δppm: 3.42 (3H, s), 3.76 (3H, s), 3.89 (2H, s), 4.69 (2H, s), 5.59 (1H, s), 7.19-7.62 (9H, m).
MS m/z: 360(M⁺), 329, 301, 244, 116.

### (Example 186)

### Compound of Compound No. 4-160

¹H-NMR(200MHz, CDCl₃) δppm: 3.55 (3H, s), 3.92 (2H, s), 4.78 (2H, bs), 5.72 (1H, s), 7.06 (2H, d, J=7.1Hz), 7.20-7.64 (12H, m).
MS m/z: 422(M⁺), 329, 302, 213, 136.

### (Example 187)

### Compound of Compound No. 5-1

¹H-NMR(200MHz, CDCl₃) δppm: 0.90 (6H, d, J=6.6Hz), 1.84 (1H, m), 2.40 (2H, d, J=7.0Hz), 3.53 (3H, s), 4.79 (2H, s), 5.72 (1H, s), 7.40-7.64 (4H, m), 8.25 (1H, s).
MS m/z: 296(M⁺), 281, 254, 116.

### (Example 188)

### Compound of Compound No. 5-2

¹H-NMR(200MHz, CDCl₃) δppm: 0.90 (6H, d, J=6.6Hz), 1.86 (1H, m), 2.42 (2H, d, J=7.3Hz), 3.45 (3H, s), 4.70 (1H, d, J=14.5Hz), 4.90 (1H, d, J=14.5Hz), 5.74 (1H, s), 7.48-7.70 (4H, s).

### (Example 189)

### Compound of Compound No. 5-3

¹H-NMR(200MHz, CDCl₃) δppm: 0.90 (6H, d, J=6.6Hz), 1.85 (1H, m), 1.89 (3H, s), 2.41 (2H, d, J=7.0Hz), 3.45 (3H, s), 4.42-4.62 (1H, br-d), 4.92-5.10 (1H, br-d), 5.64 (1H, s), 7.38-7.63 (4H, m).
MS m/z: 310(M⁺), 295, 268, 226, 116.

### (Example 190)

### Compound of Compound No. 5-4

¹H-NMR(200MHz, CDCl₃) δppm: 0.90 (6H, d, J=6.6Hz), 1.11 (3H, t, J=7.4Hz), 1.86 (1H, m), 2.04 (2H, m), 2.41 (2H, d, J=7.0Hz), 3.44 (3H, s), 4.50 (1H, d, J=14.0Hz), 5.04 (1H, d, J=14.0Hz), 5.63 (1H, s), 7.38-7.62 (4H, m).
MS m/z: 324(M⁺), 309, 282, 268, 226, 116.

### (Example 191)

### Compound of Compound No. 5-9

¹H-NMR(200MHz, CDCl₃) δppm: 0.70-0.92 (2H, m), 0.91 (6H, d, J=6.6Hz), 1.00-1.30 (3H, m), 1.87 (1H, m), 2.42 (2H, d, J=7.0Hz), 3.47 (3H, s), 4.60 (1H, d, J=14.0Hz), 4.98 (1H, d, J=14.0Hz), 5.73 (1H, s), 7.38-7.62 (4H, m).

### (Example 192)

### Compound of Compound No. 5-10

¹H-NMR(200MHz, CDCl₃) δppm: 0.90 (6H, d, J=6.6Hz), 1.75-1.96 (5H, m), 2.20-2.40 (2H, m), 2.41 (2H, d, J=7.0Hz), 2.88 (1H, m), 3.43 (3H, s), 4.40 (1H, d, J=14.0Hz), 5.08 (1H, d, J=14.0Hz), 5.57 (1H, s), 7.35-7.60 (4H, m).
MS m/z: 350(M⁺), 308, 268, 226, 116.

### (Example 193)

### Compound of Compound No. 5-14

¹H-NMR(200MHz, CDCl₃) δppm: 0.91 (6H, d, J=6.6Hz), 1.80 (2H, dd, J=7.0, 1.8Hz), 1.87 (1H, m), 2.43 (2H, d, J=7.0Hz), 3.38 (3H, s), 4.60 (1H, d, J=14.0Hz), 5.05 (1H, d, J=14.0Hz), 5.58 (1H, dd, J=15.0, 1.5Hz), 5.68 (1H, s), 7.09 (1H, td, J=22.0, 7.0Hz), 7.38-7.61 (4H, m).
MS m/z: 336(M⁺), 321, 294, 268, 226, 116.

### (Example 194)

### Compound of Compound No. 5-19

¹H-NMR(200MHz, CDCl₃) δppm: 0.90 (6H, d, J=6.6Hz), 1.85 (1H, m), 2.41 (2H, d, J=7.0Hz), 3.53 (3H, s), 3.85 (2H, m), 4.44 (1H, d, J=14.0Hz), 5.15 (1H, d, J=14.0Hz), 5.69 (1H, s), 7.40-7.65 (4H, m).

### (Example 195)

### Compound of Compound No. 5-23

¹H-NMR(200MHz, CDCl₃) δppm: 0.88 (6H, d, J=6.6Hz), 1.84 (1H, m) , 2.41 (2H, d, J=7.3Hz), 3.50 (3H, s), 4.46 (1H, d, J=14.0Hz), 5.16 (1H, d, J=14.0Hz), 5.71 (1H, s), 5.82 (1H, t, J=23.0Hz), 7.38-7.67 (4H, m).
MS m/z: 336(M⁺), 321, 294, 268, 226, 116.

### (Example 196)

### Compound of Compound No. 5-25

¹H-NMR(200MHz, CDCl₃) δppm: 0.88 (6H, d, J=6.6Hz), 1.84 (1H, m), 2.41 (2H, d, J=7.0Hz), 3.50 (3H, s), 4.47 (1H, d, J=14.0Hz), 5.17 (1H, d, J=14.0Hz), 5.72 (1H, s), 5.82 (1H, t, J=23.0Hz), 7.46-7.66 (4H, m).
MS m/z: 364(M⁺), 349, 322, 136, 116.

### (Example 197)

### Compound of Compound No. 5-26

¹H-NMR(200MHz, CDCl₃) δppm: 0.94 (6H, d, J=7.0Hz), 1.87 (1H, m), 2.43 (2H, d, J=7.0Hz), 3.06 (3H, s), 3.51 (2H, s), 4.32 (1H, d, J=14.0Hz), 5.15 (1H, d, J=14.0 Hz), 5.63 (1H, s), 7.01 (2H, m), 7.12-7.60 (7H, m).
MS m/z: 386(M⁺), 344, 268, 226, 116.

### (Example 198)

### Compound of Compound No. 5-34

¹H-NMR(200MHz, CDCl₃) δppm: 0.84 (6H, d, J=6.6Hz), 1.80 (1H, m), 2.35 (2H, d, J=7.0Hz), 3.11 (3H, s), 4.99 (2H, br-d, J=9.2Hz), 5.75 (1H, s), 7.19-7.65 (9H, m).
MS m/z: 372(M⁺), 330, 116.

### (Example 199)

### Compound of Compound No. 5-36

¹H-NMR(200MHz, CDCl₃) δppm: 0.84 (6H, d, J=6.6Hz), 1.80 (1H, m), 2.35 (2H, d, J=7.0Hz), 3.13 (3H, s), 3.71 (3H, s), 4.98 (2H, br-d), 5.75 (1H, s), 6.86-6.96 (4H, m), 7.14 (1H, t, J=8.1Hz), 7.43 (1H, t, J=8.1 Hz), 7.53-7.64 (2H, m).
MS m/z: 402(M⁺), 369, 135, 116.

### (Example 200)

### Compound of Compound No. 5-37

¹H-NMR(200MHz, CDCl₃) δppm: 0.88 (6H, d, J=6.6Hz), 1.83 (1H, m), 2.38 (2H, d, J=7.0Hz), 3.08 (3H, s), 3.77 (3H, s), 4.97 (2H, br-s), 5.78 (1H, s), 6.72 (2H, d, J=8.8Hz), 7.37 (2H, d, J=8.8Hz), 7.39-7.65 (4H, m).
MS m/z: 402(M⁺), 369, 135, 116.

### (Example 201)

### Compound of Compound No. 5-41

¹H-NMR(200MHz, CDCl₃) δppm: 0.85 (6H, d, J=6.6Hz), 1.80 (1H, m), 2.36 (2H, d, J=7.0Hz), 3.14 (3H, s), 4.98 (2H, br-d, J=14.0Hz), 5.77 (1H, s), 7.15-7.65 (8H, m).

### (Example 202)

### Compound of Compound No. 5-42

¹H-NMR(200MHz, CDCl₃) δppm: 0.86 (6H, d, J=6.6Hz), 1.81 (1H, m), 2.36 (2H, d, J=7.0Hz), 3.11 (3H, s), 4.98 (2H, br-d, J=14.0Hz), 5.75 (1H, s), 7.19-7.68 (8H, m).
MS m/z: 364(M⁺), 139, 116.

### (Example 203)

### Compound of Compound No. 5-43

¹H-NMR(200MHz, CDCl₃) δppm: 0.85 (6H, d, J=6.6Hz), 1.80 (1H, m), 2.36 (2H, d, J=7.3Hz), 3.14 (3H, s), 4.98 (2H, br-d, J=13.0Hz), 5.78 (1H, s), 7.38 (1H, t, J=7.7Hz), 7.48 (1H, d, J=7.7Hz), 7.52-7.70 (6H, m).
MS m/z: 397(M⁺), 382, 355, 267, 130, 116.

### (Example 204)

### Compound of Compound No. 5-44

¹H-NMR(200MHz, CDCl₃) δppm: 0.83 (6H, d, J=6.6Hz), 1.77 (1H, m), 2.34 (2H, d, J=7.0Hz), 3.17 (3H, s), 4.83-5.18 (2H, br-m), 5.71 (1H, s), 7.41-7.65 (8H, m).
MS m/z: 397(M⁺), 382, 355, 267, 130, 116.

### (Example 205)

### Compound of Compound No. 5-46

¹H-NMR(200MHz, CDCl₃) δppm: 0.89 (6H, d, J=6.6Hz), 1.84 (1H, m), 2.41 (2H, d, J=7.0Hz), 3.09 (1H, t, J=4.8Hz), 3.46 (3H, s), 3.65-3.80 (1H, br-s), 3.85-4.03 (1H, br-s), 4.45-4.65 (1H, br-s), 5.00-5.17 (1H, br-s), 5.65 (1H, s), 7.41-7.65 (4H, m).
MS m/z: 326(M⁺), 311, 284, 267, 226, 116.

### (Example 206)

### Compound of Compound No. 5-47

¹H-NMR(200MHz, CDCl₃) δppm: 0.89 (6H, d, J=6.6Hz), 1.10, 1.24 (3H, d, J=6.0Hz), 1.85 (1H, m), 2.41 (2H, d, J=7.3Hz), 3.18 (1H, d, J=9.2Hz), 3.40, 3.61 (3H, s), 3.86, 4.32 (1H, m), 4.21, 5.38, 4.64, 4.99 (2H, d, J=14.0Hz), 5.64, 5.70 (1H, s), 7.40-7.68 (4H, m).
MS m/z: 340(M⁺), 325, 298, 267, 226, 116.

### (Example 207)

### Compound of Compound No. 5-48

¹H-NMR(200MHz, CDCl₃) δppm: 0.83 (3H, d, J=6.2Hz), 0.84 (3H, d, J=6.6Hz), 0.89 (6H, d, J=7.0Hz), 2.99 (1H, s), 3.41 (3H, s), 4.24 (1H, d, J=14.0Hz), 5.30 (1H, d, J=14.0Hz), 5.63 (1H, s), 7.30-7.61 (4H, m).
MS m/z: 354(M⁺), 339, 312, 267, 226, 116.

### (Example 208)

### Compound of Compound No. 5-51

¹H-NMR(200MHz, CDCl₃) δppm: 0.90 (6H, d, J=6.6Hz), 1.85 (1H, m), 2.41 (2H, d, J=7.0Hz), 3.36 (3H, s), 3.46 (3H, s), 3.66 (1H, d, J=14.0Hz), 3.87 (1H, d, J=14.0Hz), 4.47 (1H, d, J=14.0Hz), 5.08 (1H, d, J=14.0Hz), 5.63 (1H, s), 7.39-7.63 (4H, m).
MS m/z: 340(M⁺), 325, 312, 298, 267, 116.

### (Example 209)

### Compound of Compound No. 5-52

¹H-NMR(200MHz, CDCl₃) δppm: 0.90 (6H, d, J=6.6Hz), 1.28 (3H, d, J=6.6Hz), 1.85 (1H, m), 2.41 (2H, d, J=7.0Hz), 3.14, 3.19 (3H, s), 3.48, 3.51 (3H, s), 3.68, 3.79 (3H, q, J=6.6Hz), 4.31, 4.46 (1H, d, J=14.0Hz), 5.09, 5.26 (1H, d, J=14.0Hz), 5.62 (1H, s), 7.39-7.62 (4H, m).
MS m/z: 354(M⁺), 339, 312, 268, 116.

### (Example 210)

### Compound of Compound No. 5-53

¹H-NMR(200MHz, CDCl₃) δppm: 0.89 (6H, d, J=6.6Hz), 1.84 (1H, m), 2.29 (2H, m), 2.40 (2H, d, J=7.0Hz), 3.32 (3H, s), 3.48 (3H, s), 3.65 (2H, m), 4.46 (1H, d, J=14.0Hz), 5.12 (1H, d, J=14.0Hz), 5.64 (1H, s), 7.37-7.62 (4H, m).

### (Example 211)

### Compound of Compound No. 5-54

¹H-NMR(200MHz, CDCl₃) δppm: 0.90 (6H, d, J=6.6Hz), 1.19 (3H, t, J=7.0Hz), 1.85 (1H, m), 2.41 (2H, d, J=7.0Hz), 3.42-3.54 (2H, m), 3.47 (3H, s), 3.71 (1H, d, J=14.0Hz), 3.92 (1H, d, J=14.0Hz), 4.45 (1H, d, J=14.0Hz), 5.09 (1H, d, J=14.0Hz), 5.63 (1H, s), 7.39-7.62 (4H, m).
MS m/z: 354(M⁺), 339, 312, 284, 267, 116.

### (Example 212)

### Compound of Compound No. 5-55

¹H-NMR(200MHz, CDCl₃) δppm: 0.90, 0.91 (6H, d, J=6.6Hz), 1.84 (1H, m), 2.40, 2.41 (2H, d, J=7.0Hz), 3.38, 3.48 (3H, s), 3.65-3.80 (1H, m), 3.96 (1H, d, J=5.9Hz), 4.42-4.76 (1H, m), 5.01-5.28 (3H, m), 5.64, 5.70 (1H, s), 5.70-5.98, 6.56-6.72 (3H, m), 7.40-7.62 (4H, m).

### (Example 213)

### Compound of Compound No. 5-56

¹H-NMR(200MHz, CDCl₃) δppm: 0.89 (6H, d, J=6.6Hz), 1.84 (1H, m), 2.40 (2H, d, J=7.3Hz), 3.34 (3H, s), 3.46 (3H, s), 3.51-3.72 (4H, m), 3.79 (1H, d, J=14.0Hz), 4.01 (1H, d, J=14.0Hz), 4.46 (1H, d, J=14.0Hz), 5.07 (1H, d, J=14.0Hz), 5.62 (1H, s), 7.38-7.63 (4H, m).
MS m/z: 384(M⁺), 369, 342, 325, 267, 116.

### (Example 214)

### Compound of Compound No. 5-57

¹H-NMR(200MHz, CDCl₃) δppm: 0.88 (6H, d, J=6.6Hz), 1.82 (1H, m), 2.38 (2H, d, J=7.0Hz), 3.36 (3H, s), 3.73 (1H, d, J=15.0Hz), 3.93 (1H, d, J=15.0Hz), 4.45 (1H, d, J=14.0Hz), 4.54 (2H, m), 5.07 (1H, d, J=14.0 Hz), 5.57 (1H, s), 7.23-7.62 (9H, m).
MS m/z: 416(M⁺), 401, 374, 310, 267, 116.

### (Example 215)

### Compound of Compound No. 5-64

¹H-NMR(200MHz, CDCl₃) δppm: 0.88 (6H, d, J=6.6Hz), 1.83 (1H, m), 2.40 (2H, d, J=7.0Hz), 3.50 (3H, s), 4.28-4.55 (3H, m), 5.16 (1H, d, J=13.5 Hz), 5.67 (1H, s), 6.78 (2H, d, J=8.1Hz), 6.97 (1H, t, J=7.3Hz), 7.21 (2H, t, J=8.1Hz), 7.38-7.63 (4H, m).
MS m/z: 402(M⁺), 387, 360, 281, 267, 116.

### (Example 216)

### Compound of Compound No. 5-65

¹H-NMR(200MHz, CDCl₃) δppm: 0.89 (6H, d, J=6.6Hz), 1.84 (1H, m), 2.17 (3H, s), 2.41 (2H, d, J=7.3Hz), 3.45 (3H, s), 4.43 (3H, m), 5.18 (1H, d, J=14.0Hz), 5.68 (1H, s), 7.41-7.65 (8H, m).
MS m/z: 416(M⁺), 401, 310, 282, 267, 116.

### (Example 217)

### Compound of Compound No. 5-66

¹H-NMR(200MHz, CDCl₃) δppm: 0.88 (6H, d, J=6.6Hz), 1.82 (1H, m), 2.40 (2H, d, J=7.0Hz), 3.50 (3H, s), 3.83 (3H, s), 4.34 (1H, d, J=16.0 Hz), 4.36 (1H, d, J=14.0Hz), 4.53 (1H, d, J=16.0Hz), 5.15 (1H, d, J=14.0Hz), 5.65 (1H, s), 6.71-7.02 (4H, m), 7.38-7.64 (4H, m).
MS m/z: 432(M⁺), 417, 390, 310, 281, 267, 116.

### (Example 218)

### Compound of Compound No. 5-67

¹H-NMR(200MHz, CDCl₃) δppm: 0.88 (6H, d, J=6.6Hz), 1.84 (1H, m), 2.41 (2H, d, J=7.0Hz), 3.51 (3H, s), 3.76 (3H, s), 4.25-4.53 (3H, m), 5.13 (1H, d, J=14.0Hz), 5.67 (1H, s), 6.31-6.38 (2H, m), 6.53 (1H, dd, J=9.2, 1.8Hz), 7.15 (1H, t, J=7.7Hz), 7.43-7.64 (4H, m).
MS m/z: 432(M⁺), 417, 390, 310, 281, 267, 116.

### (Example 219)

### Compound of Compound No. 5-68

¹H-NMR(200MHz, CDCl₃) δppm: 0.88 (6H, d, J=6.6Hz), 1.83 (1H, m), 2.40 (2H, d, J=7.0Hz), 3.49 (3H, s), 3.75 (3H, s), 4.23-4.47 (3H, m), 5.15 (1H, d, J=13.5Hz), 5.65 (1H, s), 6.72 (2H, d, J=9.5Hz), 6.80 (2H, d, J=9.5Hz), 7.39-7.64 (4H, m).
MS m/z: 432(M⁺), 417, 390, 310, 281, 267, 116.

### (Example 220)

### Compound of Compound No. 5-69

¹H-NMR(200MHz, CDCl₃) δppm: 0.87 (6H, d, J=6.6Hz), 1.83 (1H, m), 2.39 (2H, d, J=7.3Hz), 3.51 (3H, s), 3.84 (6H, s), 4.32-4.58 (3H, m), 5.15 (1H, d, J=14.0Hz), 5.66 (1H, s), 6.40 (1H, d, J=8.4Hz), 6.61 (1H, d, J=8.4Hz), 6.92 (1H, t, J=8.4Hz), 7.38-7.62 (4H, m).
MS m/z: 462(M⁺), 447, 309, 281, 267, 152, 116.

### (Example 221)

### Compound of Compound No. 5-70

¹H-NMR(200MHz, CDCl₃) δppm: 0.88 (6H, d, J=6.6Hz), 1.82 (1H, m), 2.38 (2H, d, J=7.3Hz), 3.47 (3H, s), 3.78 (6H, s), 4.25-4.58 (3H, m), 5.10 (1H, d, J=14.0Hz), 5.61 (1H, s), 6.53 (2H, d, J=8.4Hz), 6.98 (1H, t, J=8.4Hz), 7.38-7.63 (4H, m).
MS m/z: 462(M⁺), 447, 420, 309, 281, 267, 152, 116.

### (Example 222)

### Compound of Compound No. 5-71

¹H-NMR(200MHz, CDCl₃) δppm: 0.89 (6H, d, J=6.6Hz), 1.84 (1H, m), 2.42 (2H, d, J=7.0Hz), 3.50 (3H, s), 3.82 (3H, s), 3.83 (3H, s), 4.22-4.49 (3H, m), 5.17 (1H, d, J=14.5Hz), 5.67 (1H, s), 6.18 (1H, dd, J=8.8, 2.6Hz), 6.50 (1H, d, J=2.6Hz), 6.72 (1H, d, J=8.8Hz), 7.39-7.65 (4H, m).
MS m/z: 462(M⁺), 309, 281, 267, 152, 116.

### (Example 223)

### Compound of Compound No. 5-72

¹H-NMR(200MHz, CDCl₃) δppm: 0.89 (6H, d, J=6.6Hz), 1.84 (1H, m), 2.41 (2H, d, J=7.0Hz), 3.52 (3H, s), 3.74 (6H, s), 4.21-4.48 (3H, m), 5.16 (1H, d, J=14.0Hz), 5.68 (1H, s), 5.96 (2H, d, J=1.8Hz), 6.09 (1H, t, J=1.8Hz), 7.39-7.64 (4H, m).
MS m/z: 462(M⁺), 447, 420, 309, 281, 267, 116.

### (Example 224)

### Compound of Compound No. 5-73

¹H-NMR(200MHz, CDCl₃) δppm: 0.88 (6H, d, J=6.6Hz), 1.84 (1H, m), 2.42 (2H, d, J=7.3Hz), 3.52 (3H, s), 3.77 (3H, s), 3.79 (6H, s), 4.23-4.48 (3H, m), 5.18 (1H, d, J=13.5Hz), 5.68 (1H, s), 6.04 (2H, s), 7.40-7.64 (4H, m).
MS m/z: 492(M⁺), 477, 462, 309, 281, 267, 116.

### (Example 225)

### Compound of Compound No. 5-74

¹H-NMR(200MHz, CDCl₃) δppm: 0.88 (6H, d, J=6.6Hz), 1.84 (1H, m), 2.41 (2H, d, J=7.0Hz), 3.50 (3H, s), 4.23 (1H, d, J=13.5Hz), 4.40 (1H, m), 4.45 (1H, d, J=14.0Hz), 5.15 (1H, d, J=14.0Hz), 5.66 (1H, s), 5.91 (2H, s), 6.17 (1H, dd, J=8.4, 2.6Hz), 6.40 (1H, d, J=2.6Hz), 6.55 (1H, d, J=8.4Hz), 7.39-7.64 (4H, m).
MS m/z: 446(M⁺), 431, 404, 309, 281, 267, 116.

### (Example 226)

### Compound of Compound No. 5-76

¹H-NMR(200MHz, CDCl₃) δppm: 0.88 (6H, d, J=6.6Hz), 1.83 (1H, m), 2.41 (2H, d, J=7.0Hz), 3.54 (3H, s), 4.29-4.57 (3H, m), 5.18 (1H, d, J=14.0Hz), 5.69 (1H, s), 6.97-7.01 (2H, m), 7.23-7.65 (6H, m).
MS m/z: 470(M⁺), 455, 428, 281, 267, 116.

### (Example 227)

### Compound of Compound No. 5-77

¹H-NMR(200MHz, CDCl₃) δppm: 0.87 (6H, d, J=6.6Hz), 1.82 (1H, m), 2.41 (2H, d, J=7.3Hz), 3.53 (3H, s), 4.31-4.58 (3H, m), 5.16 (1H, d, J=14.5Hz), 5.69 (1H, s), 6.85 (2H, d, J=9.2Hz), 7.40-7.65 (6H, m).
MS m/z: 470(M⁺), 455, 428, 281, 267, 116.

### (Example 228)

### Compound of Compound No. 5-78

¹H-NMR(200MHz, CDCl₃) δppm: 0.87 (6H, d, J=6.6Hz), 1.83 (1H, m), 2.40 (2H, d, J=7.0.Hz), 3.55 (3H, s), 4.29-4.57 (3H, m), 5.16 (1H, d, J=14.0Hz), 5.76 (1H, s), 6.56 (1H, dd, J=8.4, 2.2Hz), 6.90 (1H, d, J=8.2Hz), 7.17 (1H, t, J=8.4Hz), 7.31-7.63 (4H, m).
MS m/z: 459(M⁺), 444, 417, 309, 281, 267, 152, 116.

### (Example 229)

### Compound of Compound No. 5-79

¹H-NMR(200MHz, CDCl₃) δppm: 0.88 (6H, d, J=6.6Hz), 1.83 (1H, m), 2.41 (2H, d, J=7.0Hz), 3.51 (3H, s), 4.25-4.47 (3H, m), 5.16 (1H, d, J=14.0Hz), 5.66 (1H, s), 6.73 (2H, d, J=8.8Hz), 7.38 (2H, d, J=8.8Hz), 7.43-7.64 (4H, m).
MS m/z: 459(M⁺), 444, 417, 309, 281, 267, 164, 116.

### (Example 230)

### Compound of Compound No. 5-81

¹H-NMR(200MHz, CDCl₃) δppm: 0.88 (6H, d, J=6.6Hz), 1.84 (1H, m), 2.41 (2H, d, J=7.0Hz), 3.53 (3H, s), 4.25-4.58 (3H, m), 5.17 (1H, d, J=14.0Hz) 5.68 (1H, s), 6.71-6.76 (2H, m), 6.96 (1H, dd, J=8.1, 1.8Hz), 7.18 (1H, t, J=8.1Hz), 7.44-7.65 (4H, m).
MS m/z: 436(M⁺), 421, 394, 309, 281, 267, 116.

### (Example 231)

### Compound of Compound No. 5-82

¹H-NMR(200MHz, CDCl₃) δppm: 0.88 (6H, d, J=6.6Hz), 1.83 (1H, m), 2.41 (2H, d, J=7.3Hz), 3.51 (3H, s), 4.24-4.49 (3H, m), 5.15 (1H, d, J=14.0Hz), 5.67 (1H, s), 6.72 (2H, d, J=8.8Hz), 7.21 (2H, d, J=8.8Hz), 7.43-7.64 (4H, m).
MS m/z: 436(M⁺), 421, 394, 309, 281, 267, 116.

### (Example 232)

### Compound of Compound No. 5-83

¹H-NMR(200MHz, CDCl₃) δppm: 0.88 (6H, d, J=6.6Hz), 1.83 (1H, m), 2.41 (2H, d, J=7.3Hz), 3.52 (3H, s), 4.35-4.55 (3H, m), 5.16 (1H, d, J=14.0Hz), 5.69 (1H, s), 6.69 (1H, d, J=8.8Hz), 7.15 (1H, dd, J=8.8, 2.6Hz), 7.33 (1H, d, J=2.6Hz), 7.40-7.65 (4H, m).

### (Example 233)

### Compound of Compound No. 5-84

¹H-NMR(200MHz, CDCl₃) δppm: 0.89 (6H, d, J=6.6Hz), 1.85 (1H, m), 2.42 (2H, d, J=7.3Hz), 3.54 (3H, s), 4.23-4.48 (3H, m), 5.17 (1H, d, J=13.0Hz), 5.69 (1H, s), 6.68 (2H, d, J=1.8Hz), 6.99 (1H, t, J=1.8Hz), 7.41-7.66 (4H, m).

### (Example 234)

### Compound of Compound No. 5-85

¹H-NMR(200MHz, CDCl₃) δppm: 0.86 (6H, d, J=6.6Hz), 1.82 (1H, m), 2.39 (2H, d, J=7.0Hz), 3.53 (3H, s), 4.35-4.58 (3H, m), 5.17 (1H, d, J=14.0Hz), 5.66 (1H, s), 6.80-7.13 (4H, m), 7.19 (1H, t, J=7.0Hz), 7.42-7.63 (4H, m).
MS m/z: 420(M⁺), 405, 378, 281, 267, 116.

### (Example 235)

### Compound of Compound No. 5-86

¹H-NMR(200MHz, CDCl₃) δppm: 0.88 (6H, d, J=6.6Hz), 1.84 (1H, m), 2.41 (2H, d, J=7.0Hz), 3.52 (3H, s), 4.26-4.49 (3H, m), 5.17 (1H, d, J=14.0Hz), 5.68 (1H, s), 6.42-6.73 (3H, m), 7.19 (1H, t, J=7.0Hz), 7.44-7.65 (4H, m).
MS m/z: 420(M⁺), 405, 378, 281, 267, 116.

### (Example 236)

### Compound of Compound No. 5-87

¹H-NMR(200MHz, CDCl₃) δppm: 0.88 (6H, d, J=6.6Hz), 1.83 (1H, m), 2.41 (2H, d, J=7.0Hz), 3.50 (3H, s), 4.25-4.49 (3H, m), 5.15 (1H, d, J=14.0Hz), 5.66 (1H, s), 6.70-6.76 (2H, m), 6.90-6.99 (2H, m), 7.40-7.65 (4H, m).

### (Example 237)

### Compound of Compound No. 5-89

¹H-NMR(200MHz, CDCl₃) δppm: 0.89 (6H, d, J=6.6Hz), 1.85 (1H, m), 2.42 (2H, d, J=7.0Hz), 3.54 (3H, s), 4.30-4.55 (3H, m), 5.15 (1H, d, J=14.0Hz), 5.72 (1H, s), 7.00-7.10 (2H, m), 7.27-7.66 (6H, m).

### (Example 238)

### Compound of Compound No. 5-90

¹H-NMR(200MHz, CDCl₃) δppm: 0.88 (6H, d, J=6.6Hz), 1.83 (1H, m), 2.41 (2H, d, J=7.0Hz), 3.53 (3H, s), 4.32-4.61 (3H, m), 5.14 (1H, d, J=14.0Hz), 5.70 (1H, s), 6.85 (2H, d, J=8.8Hz), 7.52 (2H, d, J=8.8Hz), 7.40-7.65 (6H, m).
MS m/z: 427(M⁺), 412, 385, 267, 116.

### (Example 239)

### Compound of Compound No. 5-92

¹H-NMR(200MHz, CDCl₃) δppm: 0.88 (6H, d, J=6.6Hz), 1.83 (1H, m), 2.40 (2H, d, J=7.3Hz), 3.51 (3H, s), 3.88 (3H, s), 4.34-4.58 (3H, m), 5.15 (1H, d, J=14.0Hz), 5.69 (1H, s), 6.74 (1H, d, J=8.8Hz), 7.37 (1H, dd, J=8.8, 2.6Hz), 7.44-7.65 (4H, m), 7.79 (1H, d, J=2.6Hz).
MS m/z: 494(M⁺), 479, 463, 452, 309, 267, 226, 199, 116.

### (Example 240)

### Compound of Compound No. 5-94

¹H-NMR(200MHz, CDCl₃) δppm: 0.89 (6H, d, J=6.6Hz), 0.99 (3H, t, J=7.5Hz), 1.61-1.91 (3H, m), 2.29-2.45 (4H, m), 3.59 (3H, s), 4.32 (2H, m), 4.45 (1H, d, J=14.3Hz), 5.12 (1H, d, J=14.3Hz), 5.71 (1H, s), 7.32-7.65 (4H, m).
MS m/z: 396(M⁺), 354, 325, 284, 268, 226.

### (Example 241)

### Compound of Compound No. 5-95

¹H-NMR(200MHz, CDCl₃) δppm: 0.89 (6H, d, J=6.6Hz), 1.23 (6H, d, J=5.5Hz), 1.78-1.91 (1H, m), 2.41 (2H, d, J=7.0Hz), 2.55-2.76 (1H, m), 3.60 (3H, s), 4.31 (2H, m), 4.44 (1H, d, J=14.3Hz), 5.14 (1H, d, J=14.3Hz), 5.70 (1H, s), 7.32-7.65 (4H, m).
MS m/z: 396(M⁺), 354, 284, 268, 226, 129, 116.

### (Example 242)

### Compound of Compound No. 5-96

¹H-NMR(200MHz, CDCl₃) δppm: 0.89 (6H, d, J=6.6Hz), 1.27 (9H, s), 1.77-1.90 (1H, m), 2.39 (2H, d, J=7.3Hz), 3.59 (3H, s), 4.28 (2H, m), 4.42 (1H, d, J=14.3Hz), 5.13 (1H, d, J=14.3Hz), 5.69 (1H, s), 7.39-7.63 (4H, m).
MS m/z: 410(M⁺), 368, 268, 226, 143, 116.

### (Example 243)

### Compound of Compound No. 5-97

¹H-NMR(200MHz, CDCl₃) δppm: 0.88 (6H, d, J=6.6Hz), 0.85-1.11 (5H, m), 1.67-1.91 (2H, m), 2.40 (2H, d, J=7.0Hz), 3.57 (3H, s), 4.32 (2H, m), 4.44 (1H, d, J=14.3Hz), 5.12 (1H, d, J=14.3Hz), 5.70 (1H, s), 7.33-7.64 (4H, m).
MS m/z: 394(M⁺), 352, 268, 226, 127, 116.

### (Example 244)

### Compound of Compound No. 5-98

¹H-NMR(200MHz, CDCl₃) δppm: 0.89 (6H, d, J=6.6Hz), 1.78-2.43 (7H, m), 2.41 (2H, d, J=7.3Hz), 3.14-3.31 (1H, m), 3.60 (3H, s), 4.30 (2H, m), 4.44 (1H, d, J=14.3Hz), 5.13 (1H, d, J=14.3Hz), 5.70 (1H, s), 7.30-7.64 (4H, m).
MS m/z: 408(M⁺), 366, 284, 268, 226.

### (Example 245)

### Compound of Compound No. 5-99

¹H-NMR(200MHz, CDCl₃) δppm: 0.88 (6H, d, J=6.6Hz), 1.57-1.90 (9H, m), 2.40 (2H, d, J=7.0Hz), 2.75-2.89 (1H, m), 3.58 (3H, s), 4.29 (2H, m), 4.42 (1H, d, J=14.3Hz), 5.12 (1H, d, J=14.3Hz), 5.69 (1H, s), 7.39-7.63 (4H, m).
MS m/z: 422(M⁺), 380, 268, 226, 155, 116.

### (Example 246)

### Compound of Compound No. 5-100

¹H-HMR(200MHz, CDCl₃) δppm: 0.90 (6H, d, J=6.6Hz), 1.76-1.93 (1H, m), 1.93 (3H, s), 2.19 (3H, s), 2.41 (2H, d, J=7.0Hz), 3.59 (3H, s), 4.33 (2H, m), 4.46 (1H, d, J=14.3Hz), 5.11 (1H, s), 5.71 (1H, s), 5.79 (1H, s), 7.39-7.64 (4H, m).
MS m/z: 408(M⁺), 366, 268, 141, 116.

### (Example 247)

### Compound of Compound No. 5-101

¹H-NMR(200MHz, CDCl₃) δppm: 0.89 (6H, d, J=6.6Hz), 1.77-1.93 (1H, m), 1.86 (3H, s), 2.41 (2H, d, J=7.0Hz), 3.16 (2H, s), 3.58 (3H, s), 4.33 (2H, m), 4.46 (1H, d, J=14.3Hz), 4.92 (1H, s), 4.96 (1H, s), 5.12 (1H, d, J=14.3Hz), 5.70 (1H, s), 7.32-7.64 (4H, m).
MS m/z: 408(M⁺), 366, 267, 226, 141, 116.

### (Example 248)

### Compound of Compound No. 5-102

¹H-NMR(200MHz, CDCl₃) δppm: 0.90 (6H, d, J=7.0Hz), 1.78-1.92 (1H, m), 2.41 (2H, d, J=7.3Hz), 3.48 (3H, s), 3.57 (3H, s), 4.17 (2H, s), 4.40 (2H, m), 4.44 (1H, d, J=14.3Hz), 5.10 (1H, d, J=14.3Hz), 5.72 (1H, s), 7.32-7.65 (4H, m).
MS m/z: 398(M⁺), 356, 283, 267, 225, 116.

### (Example 249)

### Compound of Compound No. 5-103

¹H-NMR(200MHz, CDCl₃) δppm: 0.90 (6H, d, J=6.6Hz), 1.78-1.91 (1H, m), 2.41 (2H, d, J=7.0Hz), 3.55 (3H, s), 3.85 (3H, s), 4.34 (2H, m), 4.48 (1H, d, J=14.3Hz), 5.10 (1H, d, J=14.3Hz), 5.70 (1H, s), 7.40-7.65 (4H, m).
MS m/z: 384(M⁺), 342, 267, 226, 116.

### (Example 250)

### Compound of Compound No. 5-104

¹H-NMR(200MHz, CDCl₃) δppm: 0.89 (6H, d, J=7.0Hz), 1.82-1.92 (1H, m), 2.41 (2H, d, J=7.3Hz), 3.39 (3H, s), 3.56 (3H, s), 3.64 (2H, t, J=4.8Hz), 4.31-4.40 (4H, m), 4.57 (1H, d, J=14.3Hz), 5.12 (1H, d, J=14.3Hz), 5.72 (1H, s), 7.36-7.65 (4H, m).
MS m/z: 428(M⁺), 386, 328, 267, 226, 116.

### (Example 251)

### Compound of Compound No. 6-1

¹H-NMR(200MHz, CDCl₃) δppm: 1.62-2.08 (6H, m), 1.88 (3H, s), 2.45-2.65 (3H, m), 3.44 (3H, s), 4.53 (1H, br), 5.00 (1H, br), 5.62 (1H, s), 7.38-7.63 (4H, m).
MS m/z: 322(M⁺), 294, 268, 252, 226.

### (Example 252)

### Compound of Compound No. 6-5

¹H-NMR(200MHz, CDCl₃) δppm: 0.65-0.82 (2H, m), 1.00-1.30 (3H, m), 1.65-2.10 (6H, m), 2.43-2.65 (3H, m), 3.46 (3H, s), 4.58 (1H, d, J=14.0Hz), 4.98 (1H, d, J=14.0Hz), 5.71 (1H, s), 7.38-7.62 (4H, m).
MS m/z: 348(M⁺), 307, 294, 280, 226, 116.

### (Example 253)

### Compound of Compound No. 6-8

¹H-NMR(200MHz, CDCl₃) δppm: 1.65-2.10 (6H, m), 2.50-2.68 (3H, m), 3.04 (3H, s), 3.49 (2H, s), 4.30 (1H, d, J=14.0Hz), 5.13 (1H, d, J=14.0Hz), 5.61 (1H, s), 6.95-6.99 (2H, m), 7.23-7.60 (7H, m).
MS m/z: 398(M⁺), 344, 280, 226, 116.

### (Example 254)

### Compound of Compound No. 6-15

¹H-NMR(200MHz, CDCl₃) δppm: 1.60-2.10 (6H, m), 2.42-2.65 (3H, m), 3.10 (1H, br-t), 3.45 (3H, s), 3.60-4.00 (2H, m), 4.50-4.65 (1H, br-s), 5.00-5.17 (1H, br-s), 5.63 (1H, s), 7.37-7.66 (4H, m).
MS m/z: 338(M⁺), 310, 297, 284, 226, 116.

### (Example 255)

### Compound of Compound No. 6-24

¹H-NMR(200MHz, CDCl₃) δppm: 1.60-2.10 (6H, m), 2.41-2.62 (3H, m), 3.35 (3H, s), 3.72 (1H, d, J=15.0Hz), 3.92 (1H, d, J=15.0Hz), 4.44 (1H, d, J=14.5Hz), 4.53 (2H, m), 5.06 (1H, d, J=14.5Hz), 5.55 (1H, s), 7.28-7.68 (9H, m).
MS m/z: 428(M⁺), 374, 322, 279, 268, 116.

### (Example 256)

### Compound of Compound No. 6-25

¹H-NMR(200MHz, CDCl₃) δppm: 1.58-2.08 (6H, m), 2.44-2.67 (3H, m), 3.52 (3H, s), 4.28-4.53 (3H, m), 5.16 (1H, d, J=12.8Hz), 5.67 (1H, s), 6.79 (2H, d, J=8.4 Hz), 7.00 (1H, t, J=7.3Hz), 7.23-7.33 (2H, m), 7.41-767 (4H, m).
MS m/z: 414(M⁺), 386, 373, 360, 293.

### (Example 257)

### Compound of Compound No. 6-27

¹H-NMR(200MHz, CDCl₃) δppm: 1.60-2.10 (6H, m), 2.42-2.64 (3H, m), 3.48 (3H, s), 3.83 (3H, s), 4.30-4.55 (3H, m), 5.13 (1H, d, J=14.0Hz), 5.63 (1H, s), 6.70-7.01 (4H, m), 7.38-7.63 (4H, m).
MS m/z: 444(M⁺), 416, 403, 390, 321, 293, 279, 137, 116.

### (Example 258)

### Compound of Compound No. 6-28

¹H-NMR(200MHz, CDCl₃) δppm: 1.60-2.10 (6H, m), 2.43-2.65 (3H, m), 3.50 (3H, s), 3.76 (3H, s), 4.22-4.48 (3H, m), 5.14 (1H, d, J=13.5Hz), 5.65 (1H, s), 6.31-6.37 (2H, m), 6.53 (1H, dd, J=8.1, 1.5Hz), 7.15 (1H, t, J=8.1Hz), 7.39-7.63 (4H, m).
MS m/z: 444(M⁺), 416, 403, 390, 321, 293, 279, 137, 116.

### (Example 259)

### Compound of Compound No. 6-29

¹H-NMR(200MHz, CDCl₃) δppm: 1.60-2.10 (6H, m), 2.42-2.64 (3H, m) , 3.48 (3H, s), 3.75 (3H, s), 4.20-4.47 (3H, m), 5.13 (1H, d, J=14.0Hz), 5.63 (1H, s), 6.72 (2H, d, J=9.2Hz), 6.80 (2H, d, J=9.2Hz), 7.39-7.64 (4H, m).
MS m/z: 444(M⁺), 416, 403, 390, 321, 293, 279, 137, 116.

### (Example 260)

### Compound of Compound No. 6-33

¹H-NMR(200MHz, CDCl₃) δppm: 1.60-2.10 (6H, m), 2.41-2.63 (3H, m), 3.51 (3H, s), 4.34-4.56 (3H, m), 5.14 (1H, d, J=14.0Hz), 5.64 (1H, s), 6.80-7.12 (4H, m), 7.39-7.64 (4H, m).
MS m/z: 432(M⁺), 404, 391, 378, 321, 293, 279, 125, 116.

### (Example 261)

### Compound of Compound No. 6-34

¹H-NMR(200MHz, CDCl₃) δppm: 1.60-2.10 (6H, m), 2.42-2.65 (3H, m), 3.51 (3H, s), 4.24-4.48 (3H, m), 5.14 (1H, d, J=14.0Hz), 5.66 (1H, s), 6.46-6.74 (3H, m), 7.19 (1H, t, J=8.4Hz), 7.40-7.65 (4H, m) .
MS m/z: 432(M⁺), 404, 391, 378, 321, 293, 279, 125, 116.

### (Example 262)

### Compound of Compound No. 6-35

¹H-NMR(200MHz, CDCl₃) δppm: 1.60-2.10 (6H, m), 2.42-2.65 (3H, m), 3.49 (3H, s), 4.21-4.48 (3H, m), 5.13 (1H, d, J=13.5Hz), 5.65 (1H, s), 6.69-6.76 (2H, m), 6.91-6.99 (2H, m), 7.39-7.65 (4H, m).
MS m/z: 432(M⁺), 404, 391, 378, 321, 293, 279, 125, 116.

### (Example 263)

### Compound of Compound No. 6-36

¹H-NMR(200MHz, CDCl₃) δppm: 1.60-2.10 (6H, m), 2.42-2.64 (3H, m), 3.56 (3H, s), 4.42-4.62 (3H, m), 5.15 (1H, d, J=14.0Hz), 5.71 (1H, s), 6.76 (1H, d, J=8.8Hz), 7.06 (1H, t, J=7.7Hz), 7.41-7.66 (6H, m).
MS m/z: 439(M⁺), 411, 398, 385, 279, 132, 116.

### (Example 264)

### Compound of Compound No. 6-37

¹H-NMR(200MHz, CDCl₃) δppm: 1.60-2.10 (6H, m), 2.43-2.66 (3H, m), 3.52 (3H, s), 4.28-4.53 (3H, m), 5.13 (1H, d, J=14.0Hz), 5.69 (1H, s), 7.00-7.09 (2H, m), 7.31-7.66 (6H, m).
MS m/z: 439(M⁺), 411, 398, 385, 279, 132, 116.

### (Example 265)

### Compound of Compound No. 6-38

¹H-NMR(200MHz, CDCl₃) δppm: 1.60-2.10 (6H, m), 2.43-2.66 (3H, m), 3.52 (3H, s), 4.30-4.57 (3H, m), 5.12 (1H, d, J=14.0Hz), 5.68 (1H, s), 6.85 (2H, d, J=9.2Hz), 7.41-7.66 (6H, m).
MS m/z: 439(M⁺), 411, 398, 385, 279, 132, 116.

### (Example 266)

### Compound of Compound No. 6-41

¹H-NMR(200MHz, CDCl₃) δppm: 1.60-2.10 (6H, m), 2.45-2.65 (3H, m), 3.67 (3H, s), 4.44 (1H, d, J=14.0Hz), 4.60 (2H, s), 5.13 (1H, d, J=14.0Hz), 5.71 (1H, s), 6.85-6.96 (2H, m), 7.38-7.65 (5H, m), 8.06 (1H, dd, J=5.1, 1.1Hz).
MS m/z: 415(M⁺), 312, 279, 251, 224, 136, 116.

### (Example 267)

### Compound of Compound No. 6-42

¹H-NMR(200MHz, CDCl₃) δppm: 1.60-2.10 (6H, m), 2.43-2.65 (3H, m), 3.52 (3H, s), 4.29-4.57 (3H, m), 5.13 (1H, d, J=14.0Hz), 5.68 (1H, s), 7.11-7.24 (2H, m), 7.41-7.66 (4H, m), 8.10-8.28 (2H, m).
MS m/z: 415(M⁺), 387, 374, 361, 279, 251, 224, 116.

### (Example 268)

### Compound of Compound No. 6-43

¹H-NMR(200MHz, CDCl₃) δppm: 1.60-2.10 (6H, m), 2.32 (3H, s), 2.44-2.64 (3H, m), 3.59 (3H, s), 4.40-4.62 (3H, m), 5.13 (1H, d, J=14.0Hz), 5.68 (1H, s), 5.72 (1H, s), 7.39-7.64 (4H, m).
MS m/z: 419(M⁺), 391, 378, 365, 279, 140, 116.

### (Example 269)

### Compound of Compound No. 6-44

¹H-NMR(200MHz, CDCl₃) δppm: 1.60-2.10 (6H, m), 2.23 (3H, s), 2.43-2.64 (3H, m), 3.59 (3H, s), 4.32-4.65 (3H, m), 5.18 (1H, d, J=15.0Hz), 5.56 (1H, s), 5.69 (1H, s), 7.37-7.62 (4H, m), 7.78 (1H, s).
MS m/z: 418(M⁺), 390, 377, 364, 321, 279, 226, 139, 116.

### (Example 270)

### Compound of Compound No. 6-45

¹H-NMR(200MHz, CDCl₃) δppm: 1.60-2.10 (6H, m), 2.43-2.66 (3H, m), 3.54 (3H, s), 4.40-4.66 (3H, m), 5.18 (1H, d, J=14.0Hz), 5.69 (1H, s), 6.88 (1H, d, J=2.9Hz), 7.30-7.65 (6H, m), 7.97-8.03 (2H, m), 8.79 (1H, dd, J=4.4, 1.8Hz).
MS m/z: 465(M⁺), 437, 424, 411, 321, 293, 279, 226, 158, 128, 116.

### (Example 271)

### Compound of Compound No. 6-46

¹H-NMR(200MHz, CDCl₃) δppm: 1.60-2.10 (6H, m), 2.16 (3H, s), 2.44-2.65 (3H, m), 3.54 (3H, s), 4.23 (1H, d, J=17.0Hz), 4.34 (1H, d, J=17.0Hz), 4.45 (1H, d, J=14.5Hz), 5.07 (1H, d, J=14.5Hz), 5.67 (1H, s), 7.40-7.64 (4H, m).
MS m/z: 380(M⁺), 352, 339, 326, 279, 226, 116.

### (Example 272)

### Compound of Compound No. 6-47

¹H-NMR(200MHz, CDCl₃) δppm: 1.60-2.10 (6H, m), 2.43-2.63 (3H, m), 3.45 (2H, s), 3.46 (3H, s), 4.33 (1H, d, J=14.5Hz), 5.15 (1H, d, J=14.5Hz), 5.52 (1H, s), 7.26-7.63 (9H, m).
MS m/z: 430(M⁺), 402, 389, 376, 279, 226, 123, 116.

### (Example 273)

### Compound of Compound No. 6-54

¹H-NMR(200MHz, CDCl₃) δppm: 1.60-2.10 (6H, m), 2.43-2.65 (3H, m), 3.61 (3H, s), 3.73 (2H, s), 4.45 (1H, d, J=14.5Hz), 5.16 (1H, d, J=14.5Hz), 5.71 (1H, s), 7.04 (1H, t, J=4.8Hz), 7.38-7.64 (4H, m), 8.47 (2H, d, J=4.8Hz).
MS m/z: 432(M⁺), 404, 391, 378, 279, 226, 153, 125, 116.

### (Example 274)

### Compound of Compound No. 6-55

¹H-NMR(200MHz, CDCl₃) δppm: 1.60-2.10 (6H, m), 2.43-2.65 (3H, m), 3.56 (3H, s), 3.62 (2H, s), 3.65 (3H, s), 4.41 (1H, d, J=14.5Hz), 5.10 (1H, d, J=14.5Hz), 5.65 (1H, s), 6.94 (1H, s), 7.03 (1H, s), 7.38-7.50 (2H, m), 7.58-7.65 (2H, m).
MS m/z: 434(M⁺), 406, 380, 321, 279, 226, 155, 127, 116.

### (Example 275)

### Compound of Compound No. 6-56

¹H-NMR(200MHz, CDCl₃) δppm: 1.60-2.10 (6H, m), 2.45-2.65 (3H, m), 2.73 (3H, s), 3.67 (3H, s), 3.79 (1H, d, J=16.0Hz), 3.91 (1H, d, J=16.0Hz), 4.44 (1H, d, J=14.5Hz), 5.15 (1H, d, J=14.5Hz), 5.73 (1H, s), 7.39-7.62 (4H, m).
MS m/z: 452(M⁺), 424, 377, 321, 279, 224, 173, 116.

### (Example 276)

### Compound of Compound No. 6-57

¹H-NMR(200MHz, CDCl₃) δppm: 1.60-2.10 (6H, m), 2.46-2.65 (3H, m), 3.71 (3H, s), 4.04 (1H, d, J=16.0Hz), 4.44 (1H, d, J=14.5Hz), 4.48 (1H, d, J=16.0Hz), 5.14 (1H, d, J=14.5Hz), 5.75 (1H, s), 6.22 (1H, td, J=6.6, 1.5Hz), 6.57 (1H, d, J=9.2Hz), 7.21 (1H, dd, J=6.6, 1.5Hz), 7.36-7.63 (5H, m).
MS m/z: 415(M⁺), 387, 361, 312, 279, 224, 136, 116.

### (Example 277)

### Compound of Compound No. 6-58

¹H-NMR(200MHz, CDCl₃) δppm: 1.60-2.10 (6H, m), 2.43-2.65 (3H, m), 3.10 (3H, s), 4.97 (2H, br-s), 5.73 (1H, s), 7.23-7.64 (9H, m).
MS m/z: 384(M⁺), 356, 343, 330, 279, 224, 116, 105.

### (Example 278)

### Compound of Compound No. 6-60

¹H-NMR(200MHz, CDCl₃) δppm: 1.60-2.10 (6H, m), 2.40-2.60 (3H, m), 3.11 (3H, s), 3.71 (3H, s), 4.96 (2H, br-s), 5.73 (1H, s), 6.85-6.94 (3H, m), 7.14 (1H, t, J=8.1Hz), 7.40-7.64 (4H, m).
MS m/z: 414(M⁺), 386, 373, 360, 279, 226, 135, 116, 107.

### (Example 279)

### Compound of Compound No. 6-65

¹H-NMR(200MHz, CDCl₃) δppm: 1.62-2.09 (6H, m), 2.44-2.64 (3H, m), 3.41 (3H, s), 3.76 (3H, s), 4.71 (2H, s), 5.60 (1H, s), 7.39-7.50 (2H, m) 7.57-7.63 (2H, m).
MS m/z: 338(M⁺), 310, 297, 284, 116.

### (Example 280)

### Compound of Compound No. 6-67

¹H-NMR(200MHz, CDCl₃) δppm: 1.21 (6H, d, J=6.2Hz), 1.60-2.10 (6H, m), 2.44-2.64 (3H, m), 3.40 (3H, s), 4.70 (2H, s), 5.03 (1H, m), 5.59 (1H, s), 7.38-7.50 (2H, m), 7.52-7.64 (2H, m).
MS m/z: 366(M⁺), 338, 325, 312, 279, 270, 226, 178, 116.

### (Example 281)

### Compound of Compound No. 6-72

¹H-NMR(200MHz, CDCl₃) δppm: 1.60-2.10 (6H, m), 2.45-2.64 (3H, m), 3.41 (3H, s), 4.64 (2H, d, J=5.5Hz), 4.72 (2H, s), 5.12-5.25 (2H, m), 5.61 (1H, s), 5.77-5.96 (1H, m), 7.39-7.49 (2H, m), 7.57-7.64 (2H, m).
MS m/z: 364(M⁺), 336, 323, 310, 279, 251, 224, 116.

### (Example 282)

### Compound of Compound No. 6-73

¹H-NMR(200MHz, CDCl₃) δppm: 1.60-2.10 (6H, m), 2.45-2.64 (3H, m), 3.32 (3H, s), 4.71 (2H, s), 5.18 (2H, s), 5.60 (1H, s), 7.20-7.62 (9H, m).
MS m/z: 414(M⁺), 370, 360, 316, 279, 226, 116, 91.

### (Example 283)

### Compound of Compound No. 6-74

¹H-NMR(200MHz, CDCl₃) δppm: 1.61-2.15 (6H, m), 2.48-2.66 (3H, m), 3.53 (3H, s), 4.80 (2H, s), 5.73 (1H, s), 7.07 (2H, d, J=7.7Hz), 7.19-7.66 (7H, m).
MS m/z: 400(M⁺), 359, 346, 307, 116.

### (Example 284)

### Compound of Compound No. 6-76

¹H-NMR(200MHz, CDCl₃) δppm: 1.63-2.12 (6H, m), 2.48-2.67 (3H, m), 3.52 (3H, s), 3.79 (3H, s), 4.79 (2H, s), 5.72 (1H, s), 6.86 (2H, d, J=9.2Hz), 6.99 (2H, d, J=9.2Hz), 7.42-7.67 (4H, m).
MS m/z: 431(M⁺), 390, 377, 307, 279, 239, 224, 123, 116.

### (Example 285)

### Compound of Compound No. 7-1

¹H-NMR(200MHz, CDCl₃) δppm: 0.90 (6H, d, J=6.6Hz), 1.84 (1H, m), 2.39 (2H, d, J=7.3Hz), 3.41 (3H, s), 3.77 (3H, s), 4.72 (2H, s), 5.62 (1H, s), 7.43-7.63 (4H, m).
MS m/z: 326(M⁺), 311, 298, 284, 116.

### (Example 286)

### Compound of Compound No. 7-2

¹H-NMR(200MHz, CDCl₃) δppm: 0.90 (6H, d, J=6.6Hz), 1.23 (3H, t, J=7.0Hz), 1.85 (1H, m), 2.39 (2H, d, J=7.3Hz), 3.42 (3H, s), 4.22 (2H, q, J=7.0Hz), 4.72 (2H, s), 5.63 (1H, s), 7.43-7.63 (4H, m).
MS m/z: 340(M⁺), 325, 298, 116.

### (Example 287)

### Compound of Compound No. 7-3

¹H-NMR(200MHz, CDCl₃) δppm: 0.84 (3H, t, J=7.3Hz), 0.89 (6H, d, J=7.0Hz), 1.60 (2H, m), 1.85 (1H, m), 2.39 (2H, d, J=7.0Hz), 3.42 (3H, S), 4.11 (2H, t, J=6.6Hz), 4.72 (2H, s), 5.62 (1H, s), 7.43-7.63 (4H, m).
MS m/z: 354(M⁺), 339, 312, 116.

### (Example 288)

### Compound of Compound No. 7-4

¹H-NMR(200MHz, CDCl₃) δppm: 0.87 (3H, t, J=7.7Hz), 0.89 (6H, d, J=7.0Hz), 1.15-1.65 (4H, m), 1.85 (1H, m), 2.39 (2H, d, J=7.0Hz), 3.42 (3H, s), 4.15 (2H, t, J=6.6Hz), 4.72 (2H, s), 5.62 (1H, s), 7.43-7.63 (4H, m).
MS m/z: 368(M⁺), 353, 326, 116.

### (Example 289)

### Compound of Compound No. 7-5

¹H-NMR(200MHz, CDCl₃) δppm: 0.90 (6H, d, J=6.6Hz), 1.21 (6H, d, J=6.3Hz), 1.85 (1H, m), 2.39 (2H, d, J=7.0Hz), 3.41 (3H, s), 4.72 (2H, s), 5.00 (1H, m), 5.62 (1H, s), 7.43-7.63 (4H, m).
MS m/z: 354(M⁺), 339, 312, 270, 116.

### (Example 290)

### Compound of Compound No. 7-6

¹H-NMR(200MHz, CDCl₃) δppm: 0.82 (6H, d, J=7.0Hz), 0.89 (6H, d, J=6.6Hz), 1.84 (2H, m), 2.39 (2H, d, J=7.0Hz), 3.42 (3H, s), 3.93 (2H, d, J=6.6Hz), 4.72 (2H, s), 5.62 (1H, s), 7.43-7.63 (4H, m).
MS m/z: 368(M⁺), 353, 326, 270, 116.

### (Example 291)

### Compound of Compound No. 7-8

¹H-NMR(200MHz, CDCl₃) δppm: 0.90 (6H, d, J=6.6Hz), 1.85 (1H, m), 2.40 (2H, d, J=7.0Hz), 3.42 (3H, s), 4.64 (2H, d, J=6.6Hz), 4.73 (2H, s), 5.14-5.27 (2H, m), 5.63 (1H, s), 5.77-5.97 (1H, m), 7.43-7.63 (4H, m).
MS m/z: 352(M⁺), 337, 310, 267, 116.

### (Example 292)

### Compound of Compound No. 7-9

¹H-NMR(200MHz, CDCl₃) δppm: 0.90 (6H, d, J=6.6Hz), 1.85 (1H, m), 2.40 (2H, d, J=7.0Hz), 3.33 (3H, s), 4.72 (2H, s), 5.19 (2H, s), 5.63 (1H, s), 7.20-7.63 (9H, m).
MS m/z: 402(M⁺), 360, 316, 267, 116.

### (Example 293)

### Compound of Compound No. 7-10

¹H-NMR(200MHz, CDCl₃) δppm: 0.90 (6H, d, J=6.6Hz), 1.84 (1H, m), 2.39 (2H, d, J=7.3Hz), 3.30 (3H, br-s), 3.43 (3H, s), 3.54 (2H, br-s), 4.31 (2H, br-s), 4.73 (2H, s), 5.63 (1H, s), 7.43-7.63 (4H, m).

### (Example 294)

### Compound of Compound No. 7-14

¹H-NMR(200MHz, CDCl₃) δppm: 0.90 (6H, d, J=6.6Hz), 1.84 (1H, m), 2.20 (6H, br-s), 2.38 (2H, d, J=7.3Hz), 2.51 (2H, br-s), 3.45 (3H, s), 4.27 (2H, t, J=5.5Hz), 4.73 (2H, s), 5.63 (1H, s), 7.39-7.62 (4H, m).
MS m/z: 383(M⁺), 368, 339, 325, 313, 267, 116.

### (Example 295)

### Compound of Compound No. 7-17

¹H-NMR(200MHz, CDCl₃) δppm: 0.92 (6H, d, J=6.6Hz), 1.88 (1H, m), 2.42 (2H, d, J=7.3Hz), 3.54 (3H, s), 4.82 (2H, s), 5.76 (1H, s), 7.06-7.63 (9H, m).
MS m/z: 388(M⁺), 373, 346, 312, 295, 116.

### (Example 296)

### Compound of Compound No. 7-19

¹H-NMR(200MHz, CDCl₃) δppm: 0.92 (6H, d, J=6.6Hz), 1.87 (1H, m), 2.42 (2H, d, J=7.3Hz), 3.53 (3H, s), 3.79 (3H, s), 4.80 (2H, s), 5.75 (1H, s), 6.86 (2H, d, J=9.2Hz), 7.00 (2H, d, J=9.2Hz), 7.42-7.66 (4H, m).
MS m/z: 418(M⁺), 376, 295, 239, 116.

### (Example 297)

### Compound of Compound No. 7-21

¹H-NMR(200MHz, CDCl₃) δppm: 0.92 (6H, d, J=6.6Hz), 1.87 (1H, m), 2.42 (2H, d, J=7.3Hz), 3.52 (3H, s), 4.81 (2H, s), 5.76 (1H, s), 7.05 (4H, d, J=6.2Hz), 7.42-7.67 (4H, m).

### (Example 298)

### Compound of Compound No. 7-24

¹H-NMR(200MHz, CDCl₃) δppm: 0.89 (6H, d, J=6.6Hz), 1.84 (1H, m), 2.39 (2H, d, J=7.0Hz), 3.34 (3H, s), 4.72 (2H, s), 5.20 (2H, s), 5.63 (1H, s), 7.24-7.63 (6H, m), 8.54-8.59 (2H, m).
MS m/z: 403(M⁺), 388, 361, 267, 225, 116, 92.

### (Example 299)

### Compound of Compound No. 7-25

¹H-NMR(200MHz, CDCl₃) δppm: 0.90 (6H, d, J=7.0Hz), 1.86 (1H, m), 2.42 (2H, d, J=7.3Hz), 3.45 (3H, s), 4.59 (1H, s), 4.62 (1H, s), 4.69 (1H, d, J=14.5Hz), 4.91 (1H, d, J=14.5Hz), 5.74 (1H, s), 6.28-6.35 (2H, m), 7.40-7.70 (5H, m).

### (Example 300)

### Compound of Compound No. 7-26

¹H-NMR(200MHz, CDCl₃) δppm: 0.91 (6H, d, J=6.6Hz), 1.87 (1H, m), 2.42 (2H, d, J=7.0Hz), 3.57 (3H, s), 4.80 (2H, s), 5.82 (1H, s), 6.32 (1H, t, J=2.6Hz), 7.32-7.70 (6H, m).
MS m/z: 378(M⁺), 363, 336, 295, 279, 267, 226, 149, 116.

### (Example 301)

### Compound of Compound No. 7-27

¹H-NMR(200MHz, CDCl₃) δppm: 0.91 (6H, d, J=6.6Hz), 1.87 (1H, m), 2.08 (3H, s), 2.42 (2H, d, J=7.0Hz), 3.56 (3H, s), 4.79 (2H, s), 5.80 (1H, s), 7.16 (2H, s), 7.43-7.69 (4H, m).
MS m/z: 392(M⁺), 377, 350, 295, 267, 226, 213, 179, 137, 116.

### (Example 302)

### Compound of Compound No. 7-29

¹H-NMR(200MHz, CDCl₃) δppm: 0.92 (6H, d, J=6.6Hz), 1.69-1.90 (1H, m), 2.43 (2H, d, J=7.0Hz), 3.54 (3H, s), 4.82 (2H, s), 5.78 (1H, s), 7.30-7.69 (6H, m), 8.44 (1H, d, J=2.6Hz), 8.49 (1H, dd, J=1.9, 4.8Hz).
MS m/z: 389(M⁺), 347, 295, 136, 116.

### (Example 303)

### Compound of Compound No. 7-30

¹H-NMR(200MHz, CDCl₃) δppm: 0.90 (6H, d, J=6.6Hz), 1.71-1.93 (1H, m), 2.15 (3H, s), 2.41 (2H, d, J=7.0Hz), 3.61 (3H, s), 4.83 (2H, s), 5.77 (1H, s), 7.14 (1H, dd, J=5.1, 7.4Hz), 7.43-7.66 (5H, d, J=7.3Hz), 8.21 (1H, d, J=4.0Hz).
MS m/z: 403(M⁺), 361, 295, 267, 252, 224, 178, 136, 116.

### (Example 304)

### Compound of Compound No. 7-31

¹H-NMR(200MHz, CDCl₃) δppm: 0.90 (6H, d, J=6.6Hz), 1.69-1.93 (1H, m), 2.41 (2H, d, J=7.3Hz), 2.52 (3H, s), 3.59 (3H, s), 4.82 (2H, s), 5.77 (1H, s), 6.80 (1H, d, J=7.7Hz), 7.07 (1H, d, J=7.3Hz), 7.46-7.69 (5H, m).
MS m/z: 403(M⁺), 361, 295, 267, 252, 224, 178, 136, 116.

### (Example 305)

### Compound of Compound No. 7-32

¹H-NMR(200MHz, CDCl₃) δppm: 0.90 (6H, d, J=6.6Hz), 1.62-1.91 (1H, m), 2.39 (2H, d, J=7.0Hz), 3.63 (3H, s), 3.88 (3H, s), 4.81 (2H, s), 5.75 (1H, s), 7.26-7.92 (7H, m).
MS m/z: 419(M⁺), 377, 295, 226, 136, 116.

### (Example 306)

### Compound of Compound No. 7-33

¹H-NMR(200MHz, CDCl₃) δppm: 0.90 (6H, d, J=6.6Hz), 1.69-1.89 (1H, m), 2.43 (2H, d, J=7.3Hz), 3.72 (3H, s), 4.49-4.58 (3H, m), 5.08 (1H, d, J=14.3Hz), 5.81 (1H, s), 6.82 (1H, d, J=8.1Hz), 6.95 (1H, d, J=7.7Hz), 7.41-7.64 (5H, m).
MS m/z: 437(M⁺), 395, 267, 224, 170, 142, 116.

### (Example 307)

### Compound of Compound No. 7-34

¹H-NMR(200MHz, CDCl₃) δppm: 0.91 (6H, d, J=6.6Hz), 1.69-1.93 (1H, m), 2.41 (2H, d, J=5.5Hz), 2.42 (3H, s), 3.50 (3H, s), 4.79 (2H, s), 5.75 (1H, s), 6.11 (1H, s), 7.43-7.68 (7H, m).
MS m/z: 393(M⁺), 351, 295, 224, 136, 116.

### (Example 308)

### Compound of Compound No. 7-35

¹H-NMR(200MHz, CDCl₃) δppm: 0.83 (6H, d, J=6.6Hz), 1.67-1.85 (1H, m), 2.27 (3H, s), 2.35 (2H, d, J=7.0Hz), 3.58 (3H, s), 4.60 (1H, bs), 5.10 (1H, bs), 5.60 (1H, s), 7.41-7.68 (4H, m). MS m/z: 410(M⁺), 368, 295, 137, 116.

### (Example 309)

### Compound of Compound No. 7-36

¹H-NMR(200MHz, CDCl₃) δppm: 0.88 (6H, d, J=6.6Hz), 1.74-1.88 (1H, m), 2.40 (2H, d, J=7.3Hz), 3.55 (3H, s), 4.84 (2H, s), 5.76 (1H, s), 7.09 (1H, d, J=5.5Hz), 7.45-7.69 (4H, m), 8.80 (1H, d, J=5.5Hz), 9.02 (1H, s).

### (Example 310)

### Compound of Compound No. 7-37

¹H-NMR(200MHz, CDCl₃) δppm: 0.90 (6H, d, J=6.6Hz), 1.74-1.88 (1H, m), 2.40 (2H, d, J=7.3Hz), 2.80 (3H, d, J=4.7Hz), 3.47 (3H, s), 4.35 (1H, bs), 4.70 (2H, bs), 5.67 (1H, s), 7.37-7.60 (4H, m).
MS m/z: 325(M⁺), 283, 267, 226, 152, 116.

### (Example 311)

### Compound of Compound No. 7-39

¹H-NMR(200MHz, CDCl₃) δppm: 0.81-1.10 (9H, m), 1.40-1.56 (2H, m), 1.82-1.89 (1H, m), 2.41 (2H, d, J=7.0Hz), 3.07-3.27 (2H, m), 3.47 (3H, s), 4.30-4.90 (3H, m), 5.67 (1H, s), 7.41-7.60 (4H, m).
MS m/z: 353(M⁺), 311, 268, 226, 196, 116.

### (Example 312)

### Compound of Compound No. 7-47

¹H-NMR(200MHz, CDCl₃) δppm: 0.89 (6H, d, J=6.6Hz), 1.78-2.05 (1H, m), 2.39 (2H, d, J=7.3Hz), 2.65 (4H, d, J=5.0Hz), 3.23 (4H, d, J=5.0Hz), 3.38 (3H, s), 4.68 (2H, s), 5.70 (1H, s), 7.34-7.60 (4H, m).
MS m/z: 380(M⁺), 312, 267, 221, 116.

### (Example 313)

### Compound of Compound No. 7-48

¹H-NMR(200MHz, CDCl₃) δppm: 0.89 (6H, d, J=6.6Hz), 1.73-1.88 (1H, m), 2.39 (2H, d, J=7.3Hz), 3.24 (4H, d, J=4.8Hz), 3.38 (3H, s), 3.50 (4H, d, J=4.8Hz), 4.69 (2H, s), 5.71 (1H, s), 7.35-7.61 (4H, m).
MS m/z: 381(M⁺), 339, 267, 114.

### (Example 314)

### Compound of Compound No. 7-49

¹H-NMR(200MHz, CDCl₃) δppm: 0.90 (6H, d, J=7.0Hz), 1.06 (6H, d, J=6.2Hz), 1.66-2.05 (1H, m), 2.33-2.45 (4H, m), 3.25-3.64 (5H, m), 4.68 (2H, s), 5.72 (1H, s), 7.35-7.61 (4H, m).
MS m/z: 409(M⁺), 367, 267, 142, 116.

### (Example 315)

### Compound of Compound No. 7-50

¹H-NMR(200MHz, CDCl₃) δppm: 0.86 (6H, d, J=6.6Hz), 1.84 (1H, m), 2.37 (2H, d, J=7.3Hz), 3.24 (3H, s), 4.82 (1H, d, J=14.0Hz), 5.05 (1H, d, J=14.0Hz), 5.77 (1H, s), 7.16 (2H, s), 7.43-7.76 (4H, m).
MS m/z: 441(M⁺), 399, 325, 284, 267, 174, 116.

### (Example 316)

### Compound of Compound No. 7-52

¹H-NMR(200MHz, CDCl₃) δppm: 0.92 (6H, d, J=6.6Hz), 1.88 (1H, m), 2.43 (2H, d, J=7.3Hz), 3.25 (3H, s), 3.96 (3H, s), 4.25 (1H, d, J=17.5Hz), 4.93 (2H, br-s), 5.47 (1H, dd, J=4.0, 1.5Hz), 5.76 (1H, s), 5.89 (1H, dd, J=4.0, 2.5Hz), 6.70 (1H, t, J=2.0Hz), 7.38-7.65 (4H, m).

### (Example 317)

### Compound of Compound No. 7-53

¹H-NMR(200MHz, CDCl₃) δppm: 0.90 (6H, d, J=6.6Hz), 1.86 (1H, m), 2.33 (3H, s), 2.42 (2H, d, J=7.0Hz), 3.46 (3H, s), 4.62 (1H, br-s), 4.95 (1H, br-s), 5.73 (1H, s), 7.40-7.64 (4H, m).
MS m/z: 342(M⁺), 327, 300, 267, 226, 137, 116.

### (Example 318)

### Compound of Compound No. 7-63

¹H-NMR(200MHz, CDCl₃) δppm: 0.92 (6H, d, J=6.6Hz), 1.69-2.05 (1H, m), 2.45 (2H, d, J=7.0Hz), 3.57 (3H, s), 4.60 (1H, d, J=13.8Hz), 4.99 (1H, d, J=13.8Hz), 5.85 (1H, s), 7.28-7.80 (7H, m), 8.63 (1H, d, J=5.5Hz).
MS m/z: 405(M⁺), 363, 295, 267, 138, 116.

### (Example 319)

### Compound of Compound No. 7-64

¹H-NMR(200MHz, CDCl₃) δppm: 0.92 (6H, d, J=6.6Hz), 1.67-1.95 (1H, m), 2.44 (2H, d, J=7.0Hz), 3.58 (3H, s), 4.61 (1H, d, J=14.1Hz), 5.03 (1H, d, J=14.1Hz), 5.86 (1H, s), 7.27-7.65 (5H, m), 8.79 (2H, d, J=4.8Hz).
MS m/z: 406(M⁺), 364, 295, 267, 139, 116.

### (Example 320)

### Compound of Compound No. 7-65

¹H-NMR(200MHz, CDCl₃) δppm: 0.92 (6H, d, J=6.6Hz), 1.73-1.95 (1H, m), 2.45 (2H, d, J=7.3Hz), 2.83 (3H, s), 3.54 (3H, s), 4.64 (1H, d, J=14.1Hz), 5.02 (1H, d, J=14.1Hz), 5.87 (1H, s), 7.47-7.69 (4H, m).
MS m/z: 426(M⁺), 366, 295, 267, 173, 139, 116.

### (Example 321)

### Compound of Compound No. 8-1

¹H-NMR(200MHz, CDCl₃) δppm: 0.90 (6H, d, J=6.6Hz), 1.85 (1H, m), 2.36-2.49 (6H, m), 2.83 (1H, d, J=16.5Hz), 2.96 (1H, d, J=16.5Hz), 3.50 (3H, s), 3.70 (4H, t, J=4.8Hz), 4.41 (1H, d, J=14.0Hz), 5.11 (1H, d, J=14.0Hz), 5.63 (1H, s), 7.38-7.63 (4H, m).
MS m/z: 395(M⁺), 380, 364, 352, 310, 267, 116, 100.

### (Example 322)

### Compound of Compound No. 8-2

¹H-NMR(200MHz, CDCl₃) δppm: 0.90 (6H, d, J=6.6Hz), 1.09-1.27 (6H, m), 1.70-1.85 (3H, m), 2.41 (2H, d, J=7.3Hz), 2.49-2.96 (4H, m), 3.50 (3H, s), 3.60-4.08 (2H, m), 4.40 (1H, d, J=14.5Hz), 5.12 (1H, d, J=14.5Hz), 5.63 (1H, s), 7.38-7.63 (4H, m)(mixture of 2 isomers).
MS m/z: 423(M⁺), 408, 281, 267, 226, 128, 116.

### (Example 323)

### Compound of Compound No. 8-3

¹H-NMR(200MHz, CDCl₃) δppm: 0.88 (6H, d, J=6.6Hz), 1.81 (1H, m), 2.38 (2H, d, J=7.3Hz), 3.41 (3H, s), 3.68 (2H, s), 4.40 (1H, d, J=14.5Hz), 5.23 (1H, d, J=14.5Hz), 5.61 (1H, s), 7.05 (1H, d, J=7.7Hz), 7.18 (1H, dd, J=7.7, 5.1Hz), 7.37-7.66 (5H, m), 8.58 (1H, dd, J=1.8, 1.1Hz).
MS m/z: 387(M+), 349, 268, 226, 120, 92.

### (Example 324)

### Compound of Compound No. 8-4

¹H-NMR(200MHz, CDCl₃) δppm: 0.93 (6H, d, J=6.6Hz), 1.88 (1H, m), 2.44 (2H, d, J=7.3Hz), 3.29 (3H, s), 3.45 (2H, s), 4.43 (1H, d, J=14.0Hz), 5.09 (1H, d, J=14.0Hz), 5.66 (1H, s), 7.25 (1H, dd, J=7.7, 5.0Hz), 7.38-7.62 (5H, m), 8.27 (1H, d, J=1.8Hz), 8.52 (1H, dd, J=4.8, 1.5Hz).
MS m/z: 387(M⁺), 372, 345, 267, 226, 120, 92.

### (Example 325)

### Compound of Compound No. 8-5

¹H-NMR(200MHz, CDCl₃) δppm: 0.93 (6H, d, J=6.6Hz), 1.86 (1H, m), 2.43 (2H, d, J=7.3Hz), 3.23 (3H, s), 3.46 (2H, s), 4.40 (1H, d, J=14.0Hz), 5.09 (1H, d, J=14.0Hz), 5.63 (1H, s), 7.00 (1H, d, J=5.9Hz), 7.38-7.63 (4H, m), 8.53 (1H, d, J=5.9Hz).
MS m/z: 387(M⁺), 372, 345, 267, 226, 120, 92.

### (Example 326)

### Compound of Compound No. 8-6

¹H-NMR(200MHz, CDCl₃) δppm: 0.91 (6H, d, J=6.6Hz), 1.86 (1H, m), 2.08 (3H, s), 2.43 (2H, d, J=7.3Hz), 3.52 (3H, s), 4.47 (1H, d, J=14.0Hz), 4.58 (1H, d, J=17.0Hz), 4.66 (1H, d, J=17.0Hz), 5.08 (1H, d, J=14.0Hz), 5.74 (1H, s), 7.17 (1H, s), 7.34 (1H, s), 7.39-7.64 (4H, m).
MS m/z: 390(M⁺), 375, 348, 284, 267, 231, 226, 116, 95.

### (Example 327)

### Compound of Compound No. 8-7

¹H-NMR(200MHz, CDCl₃) δppm: 0.91 (6H, d, J=6.6Hz), 1.87 (1H, m), 2.43 (2H, d, J=7.0Hz), 3.53 (3H, s), 4.49 (1H, d, J=14.0Hz), 4.53 (1H, d, J=17.0Hz), 4.69 (1H, d, J=17.0Hz), 5.08 (1H, d, J=14.0Hz), 5.75 (1H, s), 7.39-7.65 (6H, m).
MS (APCI) m/z: 456((M+H)⁺).

### (Example 328)

### Compound of Compound No. 8-8

¹H-NMR(200MHz, CDCl₃) δppm: 0.92 (6H, d, J=6.6Hz), 1.88 (1H, m), 2.23 (3H, s), 2.44 (2H, d, J=7.3Hz), 3.43 (3H, s), 4.27 (1H, d, J=16.5Hz), 4.42 (1H, d, J=16.5Hz), 4.48 (1H, d, J=14.0Hz), 5.10 (1H, d, J=14.0Hz), 5.73 (1H, s), 6.67 (1H, d, J=1.1Hz), 6.93 (1H, d, J=1.1Hz), 7.34 (1H, s), 7.41-7.65 (4H, m).

### (Example 329)

### Compound of Compound No. 8-9

¹H-NMR(200MHz, CDCl₃) δppm: 0.92 (6H, d, J=6.6Hz), 1.88 (1H, m), 2.44 (2H, d, J=7.0Hz), 3.57 (3H, s), 4.25 (1H, d, J=17.5Hz), 4.50 (1H, d, J=14.0Hz), 4.52 (1H, d, J=17.5Hz), 4.82 (1H, d, J=14.0Hz), 5.11 (1H, d, J=14.0Hz), 5.77 (1H, s), 7.40 (1H, s), 7.42-7.67 (4H, m).
MS m/z: 444(M⁺), 429, 409, 367, 267, 149, 136, 116.

### (Example 330)

### Compound of Compound No. 8-10

¹H-NMR(200MHz, CDCl₃) δppm: 0.91 (6H, d, J=6.6Hz), 1.87 (1H, m), 2.43 (2H, d, J=7.3Hz), 3.67 (3H, s), 3.91 (3H, s), 3.93 (3H, s), 4.48 (1H, d, J=14.0Hz), 4.65 (1H, d, J=17.0Hz), 4.82 (1H, d, J=17.0Hz), 5.13 (1H, d, J=14.0Hz), 5.82 (1H, s), 7.43-7.64 (5H, m).
MS m/z: 492(M⁺), 477, 461, 450, 413, 359, 302, 267, 197, 116.

### (Example 331)

### Compound of Compound No. 8-12

¹H-NMR(200MHz, CDCl₃) δppm: 0.90 (6H, d, J=6.6Hz), 1.85 (1H, m), 2.41 (3H, s), 2.42 (2H, d, J=7.3Hz), 3.35-3.54 (2H, m), 3.46 (3H, s), 4.48 (1H, d, J=14.5Hz), 5.09 (1H, d, J=14.5Hz), 5.69 (1H, s), 6.03 (1H, s), 7.39-7.63 (4H, m).
MS m/z: 391(M⁺), 376, 349, 267, 226, 116, 96.

### (Example 332)

### Compound of Compound No. 8-13

¹H-NMR(200MHz, CDCl₃) δppm: 0.90 (6H, d, J=6.6Hz), 1.86 (1H, m), 2.14 (3H, s), 2.20 (3H, s), 2.42 (2H, d, J=7.0Hz), 3.54 (3H, s), 4.33-4.63 (3H, m), 5.09 (1H, d, J=14.0Hz), 5.73 (1H, s), 5.85 (1H, s), 7.38-7.64 (4H, m).
MS m/z: 404(M⁺), 389, 267, 245, 228, 149, 109.

### (Example 333)

### Compound of Compound No. 8-14

¹H-NMR(200MHz, CDCl₃) δppm: 0.92 (6H, d, J=6.6Hz), 1.88 (1H, m), 2.44 (2H, d, J=7.0Hz), 3.57 (3H, s), 4.52 (1H, d, J=14.0Hz), 4.62 (1H, d, J=16.5Hz), 4.81 (1H, d, J=16.5Hz), 5.10 (1H, d, J=14.0Hz), 5.79 (1H, s), 7.41-7.66 (4H, m), 7.96 (1H, s), 8.18 (1H, s).
MS m/z: 377(M⁺), 362, 335, 267, 226, 116.

### (Example 334)

### Compound of Compound No. 8-15

¹H-NMR(200MHz, CDCl₃) δppm: 0.91 (6H, d, J=6.6Hz), 1.86 (1H, m), 2.42 (2H, d, J=7.0Hz), 3.73 (3H, s), 4.05 (1H, d, J=15.5Hz), 4.45 (1H, d, J=14.0Hz), 4.49 (1H, d, J=15.5Hz), 5.16 (1H, d, J=14.0Hz), 5.77 (1H, s), 6.23 (1H, td, J=6.6, 1.1Hz), 6.58 (1H, d, J=9.2 Hz), 7.21 (1H, dd, J=6.6, 1.1Hz), 7.36-7.63 (5H, m).
MS m/z: 403(M⁺), 267, 226, 136, 116, 108, 80.

### (Example 335)

### Compound of Compound No. 8-16

¹H-NMR(200MHz, CDCl₃) δppm: 0.88 (6H, d, J=6.6Hz), 1.84 (1H, m), 2.42 (2H, d, J=7.0Hz), 3.54 (3H, s), 4.31-4.60 (3H, m), 5.16 (1H, d, J=14.0Hz), 5.70 (1H, s), 6.70 (2H, d, J=6.2Hz), 7.41-7.66 (4H, m), 8.45 (2H, br-d).

### (Example 336)

### Compound of Compound No. 8-17

¹H-NMR(200MHz, CDCl₃) δppm: 0.90 (6H, d, J=6.6Hz), 1.86 (1H, m), 2.15 (3H, s), 2.42 (2H, d, J=7.3Hz), 3.74 (3H, s), 4.08 (1H, d, J=16.0Hz), 4.45 (1H, d, J=14.5Hz), 4.49 (1H, d, J=16.0Hz), 5.15 (1H, d, J=14.5Hz), 5.78 (1H, s), 6.15 (1H, t, J=6.6Hz), 7.10 (1H, d, J=6.2 Hz), 7.26 (1H, d, J=6.6Hz), 7.39-7.63 (4H, m).
MS m/z: 417(M⁺), 402, 267, 150, 122, 116, 92.

### (Example 337)

### Compound of Compound No. 8-18

¹H-NMR(200MHz, CDCl₃) δppm: 0.90 (6H, d, J=6.6Hz), 1.86 (1H, m), 2.42 (2H, d, J=7.3Hz), 3.72 (3H, s), 4.09 (1H, d, J=16.0Hz), 4.46 (1H, d, J=14.5Hz), 4.51 (1H, d, J=16.0Hz), 5.14 (1H, d, J=14.5Hz), 5.76 (1H, s), 7.27 (1H, d, J=1.1Hz), 7.41-7.64 (5H, m).
MS m/z: 471(M⁺), 431, 429, 402, 267, 204, 176, 116.

### (Example 338)

### Compound of Compound No. 8-19

¹H-NMR(200MHz, CDCl₃) δppm: 0.91 (6H, d, J=6.6Hz), 1.87 (1H, m), 2.43 (2H, d, J=7.3Hz), 3.71 (3H, s), 4.14 (1H, d, J=15.5Hz), 4.45-4.53 (2H, m), 5.15 (1H, d, J=14.5Hz), 5.78 (1H, s), 6.64 (1H, d, J=9.5Hz), 7.41-7.65 (6H, m).
MS m/z: 471(M⁺), 267, 226, 204, 176, 148, 116.

### (Example 339)

### Compound of Compound No. 8-20

¹H-NMR(200MHz, CDCl₃) δppm: 0.90 (6H, d, J=6.6Hz), 1.86 (1H, m), 2.42 (2H, d, J=7.3Hz), 3.70 (3H, s), 4.05 (1H, d, J=16.0Hz), 4.40-4.49 (2H, m), 5.14 (1H, d, J=14.5Hz), 5.77 (1H, s), 6.54 (1H, d, J=9.5Hz), 7.27-7.63 (6H, m).
MS m/z: 437(M⁺), 422, 395, 322, 309, 267, 170, 142, 116.

### (Example 340)

### Compound of Compound No. 8-21

¹H-NMR(200MHz, CDCl₃) δppm: 0.90 (6H, d, J=6.6Hz), 1.86 (1H, m), 2.42 (2H, d, J=7.3Hz), 3.58 (3H, s), 4.41-4.75 (3H, m), 5.09 (1H, d, J=14.0Hz), 5.88 (1H, s), 7.39-7.64 (4H, m), 7.70 (2H, s).
MS m/z: 471(M⁺), 458, 456, 267, 226, 168, 116.

### (Example 341)

### Compound of Compound No. 8-22

¹H-NMR(200MHz, CDCl₃) δppm: 0.90 (6H, d, J=6.6Hz), 1.86 (1H, m), 2.42 (2H, d, J=7.0Hz), 3.76 (3H, s), 3.82 (3H, s), 3.99 (1H, d, J=16.0Hz), 4.46 (1H, d, J=14.5Hz), 4.53 (1H, d, J=16.0Hz), 5.12 (1H, d, J=14.5Hz), 5.77 (1H, s), 6.17 (1H, t, J=7.0Hz), 6.67 (1H, dd, J=7.3, 1.5Hz), 6.85 (1H, dd, J=7.0, 1.5Hz), 7.39-7.63 (4H, m).
MS m/z: 433(M⁺), 418, 228, 166.

### (Example 342)

### Compound of Compound No. 8-23

¹H-NMR(200MHz, CDCl₃) δppm: 0.91 (6H, d, J=6.6Hz), 1.87 (1H, m), 2.43 (2H, d, J=7.0Hz), 3.69 (3H, s), 4.13 (1H, d, J=16.0Hz), 4.42 (1H, d, J=14.5Hz), 4.49 (1H, d, J=16.0Hz), 5.14 (1H, d, J=14.5Hz), 5.79 (1H. s), 6.47 (1H, dd, J=7.3, 0.8Hz), 7.41-7.65 (4H, m), 7.94 (1H, d, J=6.6Hz), 8.04 (1H, s).
MS m/z: 404(M⁺), 389, 362, 267, 226, 137, 116, 109.

### (Example 343)

### Compound of Compound No. 8-24

¹H-NMR(200MHz, CDCl₃) δppm: 0.89 (6H, d, J=6.6Hz), 1.83 (1H, m), 2.39 (2H, d, J=7.3Hz), 3.40 (3H, s), 4.44 (1H, d, J=14.0Hz), 4.59 (1H, d, J=16.0 Hz), 4.71 (1H, d, J=16.0Hz), 5.08 (1H, d, J=14.0Hz), 5.61 (1H, s), 6.18 (1H, t, J=2.2Hz), 7.23 (1H, dd, J=2.5, 1.1Hz), 7.42-7.64 (5H, m).
MS m/z: 392(M⁺), 377, 350, 325, 309, 282, 267, 224, 125, 116.

### (Example 344)

### Compound of Compound No. 8-25

¹H-NMR(200MHz, CDCl₃) δppm: 0.89 (6H, d, J=6.6Hz), 1.83 (1H, m), 2.05 (3H, s), 2.39 (2H, d, J=7.0Hz), 3.41 (3H, s), 4.44 (1H, d, J=14.0Hz), 4.54 (1H, d, J=16.0 Hz), 4.67 (1H, d, J=16.0Hz), 5.09 (1H, d, J=14.0Hz), 5.61 (1H, s), 7.02 (1H, s), 7.30 (1H, s), 7.42-7.61 (4H, m).
MS m/z: 406(M⁺), 391, 364, 325, 309, 282, 267, 139, 116.

### (Example 345)

### Compound of Compound No. 8-26

¹H-NMR(200MHz, CDCl₃) δppm: 0.88 (6H, d, J=6.6Hz), 1.84 (1H, m), 2.22 (3H, s), 2.39 (2H, d, J=7.3Hz), 3.60 (3H, s), 4.36-4.66 (3H, m), 5.19 (1H, d, J=14.5Hz), 5.55 (1H, s), 5.72 (1H, s), 7.37-7.62 (4H, m), 7.76 (1H, s).
MS m/z: 406(M⁺), 391, 364, 267, 226, 139, 116, 111.

### (Example 346)

### Compound of Compound No. 8-27

¹H-NMR(200MHz, CDCl₃) δppm: 0.89 (6H, d, J=6.6Hz), 1.85 (1H, m), 2.41 (2H, d, J=7.3Hz), 3.68 (3H, s), 4.45 (1H, d, J=14.0Hz), 4.62 (2H, s), 5.15 (1H, d, J=14.0Hz), 5.74 (1H, s), 6.87 (1H, d, J=8.1Hz), 6.93 (1H, dd, J=5.8, 1.5Hz), 7.38-7.65 (5H, m), 8.07 (1H, dd, J=5.2, 1.5Hz).
MS m/z: 403(M⁺), 388, 279, 267, 224, 170, 149, 136.

### (Example 347)

### Compound of Compound No. 8-28

¹H-NMR(200MHz, CDCl₃) δppm: 0.90 (6H, d, J=6.6Hz), 1.85 (1H, m), 2.25 (3H, s), 2.41 (2H, d, J=7.0Hz), 3.68 (3H, s), 4.44 (1H, d, J=14.5Hz), 4.63 (2H, s), 5.15 (1H, d, J=14.5Hz), 5.74 (1H, s), 6.84 (1H, dd, J=7.3, 5.1Hz), 7.42-7.62 (5H, m), 7.89 (1H, d, J=4.8Hz).
MS m/z: 417(M⁺), 402, 394, 360, 267, 150, 116.

### (Example 348)

### Compound of Compound No. 8-29

¹H-NMR(200MHz, CDCl₃) δppm: 0.90 (6H, d, J=6.6Hz), 1.86 (1H, m), 2.34 (3H, s), 2.42 (2H, d, J=7.3Hz), 3.68 (3H, s), 4.46-4.70 (3H, m), 5.10 (1H, d, J=14.5Hz), 5.75 (1H, s), 6.67 (1H, d, J=8.1Hz), 6.74 (1H, d, J=7.0Hz), 7.39-7.64 (5H, m).
MS m/z: 417(M⁺), 402, 375, 360, 309, 267, 224, 150, 116.

### (Example 349)

### Compound of Compound No. 8-30

¹H-NMR(200MHz, CDCl₃) δppm: 0.90 (6H, d, J=6.6Hz), 1.86 (1H, m), 2.34 (3H, s), 2.42 (2H, d, J=7.3Hz), 3.68 (3H, s), 4.43 (1H, d, J=14.0Hz), 4.67 (2H, s), 5.16 (1H, d, J=14.0Hz), 5.74 (1H, s), 6.98 (1H, d, J=8.8Hz), 7.40-7.64 (4H, m), 7.83 (1H, dd, J=8.8, 2.6Hz), 8.36 (1H, s).
MS m/z: 471(M⁺), 452, 429, 267, 240, 176, 146, 116.

### (Example 350)

### Compound of Compound No. 8-31

¹H-NMR(200MHz, CDCl₃) δppm: 0.90 (6H, d, J=7.0Hz), 1.85 (1H, m), 2.42 (2H, d, J=7.0Hz), 3.66 (3H, s), 4.43 (1H, d, J=14.0Hz), 4.59 (2H, s), 5.15 (1H, d, J=14.0Hz), 5.73 (1H, s), 6.84 (1H, d, J=8.8Hz), 7.39-7.65 (5H, m), 8.01 (1H, d, J=2.2Hz).
MS m/z: 437(M⁺), 422, 395, 322, 309, 267, 170, 142, 116.

### (Example 351)

### Compound of Compound No. 8-32

¹H-NMR(200MHz, CDCl₃) δppm: 0.88 (6H, d, J=6.6Hz), 1.84 (1H, m), 2.41 (2H, d, J=7.0Hz), 3.55 (3H, s), 4.40-4.60 (3H, m), 5.16 (1H, d, J=14.0Hz), 5.73 (1H, s), 7.09 (1H, dd, J=8.1, 1.5Hz), 7.19 (1H, dd, J=8.1, 4.8Hz), 7.41-7.66 (4H, m), 8.04 (1H, dd, J=4.4, 1.5Hz).
MS m/z: 433(M⁺), 418, 326, 310, 279, 267, 166, 149.

### (Example 352)

### Compound of Compound No. 8-33

¹H-NMR(200MHz, CDCl₃) δppm: 0.89 (6H, d, J=6.6Hz), 1.85 (1H, m), 2.41 (2H, d, J=7.3Hz), 3.62 (3H, s), 3.89 (3H, s), 4.45 (1H, d, J=14.5Hz), 4.68 (2H, s), 5.13 (1H, d, J=14.5Hz), 5.74 (1H, s), 6.90 (1H, dd, J=7.7, 5.1Hz), 7.09 (1H, dd, J=8.1, 1.5Hz), 7.38-7.64 (5H, m).
MS m/z: 456(M⁺), 441, 414, 279, 267, 189, 166, 149, 116.

### (Example 353)

### Compound of Compound No. 8-34

¹H-NMR(200MHz, CDCl₃) δppm: 0.90 (6H, d, J=6.6Hz), 1.86 (1H, m), 2.42 (2H, d, J=7.3Hz), 2.43 (3H, s), 3.65 (3H, s), 4.51 (1H, d, J=14.0Hz), 4.68 (1H, d, J=16.0Hz), 4.77 (1H, d, J=16.0Hz), 5.08 (1H, d, J=14.0Hz), 5.76 (1H, s), 6.88 (1H, d, J=7.7Hz), 7.41-7.65 (4H, m), 7.80 (1H, d, J=7.7Hz).
MS m/z: 442(M⁺), 427, 400, 267, 224, 175, 147, 116.

### (Example 354)

### Compound of Compound No. 8-35

¹H-NMR(200MHz, CDCl₃) δppm: 0.89 (6H, d, J=6.6Hz), 1.85 (1H, m), 2.42 (2H, d, J=7.3Hz), 3.55 (3H, s), 4.30-4.58 (3H, m), 5.15 (1H, d, J=14.5Hz), 5.72 (1H, s), 7.12 (1H, d, J=2.6Hz), 7.41-7.54 (4H, m), 7.63 (1H, td, J=7.0, 1.8Hz), 8.11 (1H, d, J=2.6Hz), 8.22 (1H, d, J=1.8Hz).
MS m/z: 437(M⁺), 422, 395, 267, 225, 142, 116.

### (Example 355)

### Compound of Compound No. 8-36

¹H-NMR(200MHz, CDCl₃) δppm: 0.89 (6H, d, J=6.6Hz), 1.85 (1H, m), 2.41 (2H, d, J=7.0Hz), 3.65 (3H, s), 4.42 (1H, d, J=14.5Hz), 4.62 (1H, d, J=16.0Hz), 4.73 (1H, d, J=16.0Hz), 5.14 (1H, d, J=14.5Hz), 5.73 (1H, s), 7.39-7.64 (4H, m), 7.69 (1H, d, J=2.2Hz), 7.93 (1H, d, J=2.2Hz).
MS m/z: 473(M⁺), 471(M-1+), 431, 429, 309, 267, 224, 204, 176, 149, 116.

### (Example 356)

### Compound of Compound No. 8-37

¹H-NMR(200MHz, CDCl₃) δppm: 0.89 (6H, d, J=6.6Hz), 1.84 (1H, m), 2.41 (2H, d, J=7.3Hz), 3.46 (3H, s), 3.73 (1H, br-s), 3.94 (1H, br-s), 4.58 (1H, br-s), 5.07 (1H, br-s), 5.66 (1H, s), 7.27-7.66 (6H, m), 8.09 (1H, dd, J=4.4, 1.8Hz), 8.27 (1H, d, J=1.8Hz).
MS m/z: 403(M⁺), 388, 361, 353, 329, 309, 284, 267, 226, 116.

### (Example 357)

### Compound of Compound No. 8-38

¹H-NMR(200MHz, CDCl₃) δppm: 0.88 (6H, d, J=6.6Hz), 1.84 (1H, m), 2.41 (2H, d, J=7.0Hz), 3.55 (3H, s), 4.40-4.60 (3H, m), 5.16 (1H, d, J=14.0Hz), 5.73 (1H, s), 7.09 (1H, dd, J=8.1, 1.5Hz), 7.19 (1H, dd, J=8.1, 4.8Hz), 7.41-7.66 (4H, m), 8.04 (1H, dd, J=4.4, 1.5Hz).
MS m/z: 437(M⁺), 422, 395, 281, 267, 224, 116.

### (Example 358)

### Compound of Compound No. 8-39

¹H-NMR(200MHz, CDCl₃) δppm: 0.89 (6H, d, J=6.6Hz), 1.84 (1H, m), 2.33 (3H, t, J=2.6Hz), 2.40 (2H, d, J=7.3Hz), 3.59 (3H, s), 4.45 (1H, d, J=14.0Hz), 4.49 (1H, d, J=15.0Hz), 4.66 (1H, d, J=15.0Hz), 5.14 (1H, d, J=14.0Hz), 5.71 (1H, s), 7.40-7.63 (4H, m).
MS m/z: 425(M⁺), 410, 383, 309, 267, 224, 158, 130, 116.

### (Example 359)

### Compound of Compound No. 8-40

¹H-NMR(200MHz, CDCl₃) δppm: 0.89 (6H, d, J=6.6Hz), 1.84 (1H, m), 2.32 (3H, s), 2.40 (2H, d, J=7.0Hz), 3.61 (3H, s), 4.41-4.63 (3H, m), 5.15 (1H, d, J=14.0Hz), 5.70 (1H, s), 5.72 (1H, s), 7.39-7.64 (4H, m).
MS m/z: 407(M⁺), 392, 365, 267, 226, 149, 116.

### (Example 360)

### Compound of Compound No. 8-41

¹H-NMR(200MHz, CDCl₃) δppm: 0.90 (6H, d, J=6.6Hz), 1.86 (1H, m), 2.42 (2H, d, J=7.3Hz), 3.64 (3H, s), 4.47 (1H, d, J=14.5Hz), 4.57 (1H, d, J=15.0Hz), 4.74 (1H, d, J=15.0Hz), 5.17 (1H, d, J=14.5Hz), 5.71 (1H, s), 7.41-7.65 (7H, m), 7.93-7.98 (2H, m).
MS m/z: 503(M⁺), 488, 461, 309, 267, 236, 224, 116.

### (Example 361)

### Compound of Compound No. 8-42

¹H-NMR(200MHz, CDCl₃) δppm: 0.89 (6H, d, J=6.6Hz), 1.85 (1H, m), 2.41 (2H, d, J=7.0Hz), 3.62 (3H, s), 4.41-4.69 (3H, m), 5.16 (1H, d, J=14.5Hz), 5.71 (1H, s), 6.07 (1H, d, J=1.8Hz), 7.40-7.64 (4H, m), 8.14 (1H, d, J=1.8Hz).
MS m/z: 393(M⁺), 378, 351, 267, 224, 126, 116, 98.

### (Example 362)

### Compound of Compound No. 8-43

¹H-NMR(200MHz, CDCl₃) δppm: 0.89 (6H, d, J=6.6Hz), 1.83 (1H, m), 1.85 (3H, s), 2.23 (3H, s), 2.40 (2H, d, J=7.3Hz), 3.62 (3H, s), 4.43 (1H, d, J=14.5Hz), 4.47 (1H, d, J=16.0Hz), 4.61 (1H, d, J=16.0Hz), 5.15 (1H, d, J=14.5Hz), 5.71 (1H, s), 7.39-7.63 (4H, m).
MS m/z: 421(M⁺), 406, 379, 309, 267, 224, 154, 126, 116.

### (Example 363)

### Compound of Compound No. 8-44

¹H-NMR(200MHz, CDCl₃) δppm: 0.89 (6H, d, J=6.6Hz), 1.84 (1H, m), 2.34 (3H, s), 2.41 (2H, d, J=7.3Hz), 3.61 (3H, s), 4.41-4.60 (3H, m), 5.15 (1H, d, J=14.5Hz), 5.71 (1H, s), 7.39-7.63 (4H, m).
MS m/z: 441(M⁺), 426, 399, 309, 267, 224, 174, 146, 116.

### (Example 364)

### Compound of Compound No. 8-45

¹H-NMR(200MHz, CDCl₃) δppm: 0.90 (6H, d, J=6.6Hz), 1.85 (1H, m), 2.41 (2H, d, J=7.0Hz), 3.61 (3H, s), 3.95 (3H, s), 4.42-4.71 (3H, m), 5.14 (1H, d, J=14.5Hz), 5.72 (1H, s), 6.64 (1H, s), 7.341-7.64 (4H, m).
MS m/z: 451 (M⁺), 436, 409, 293, 267, 224, 184, 156, 116.

### (Example 365)

### Compound of Compound No. 8-46

¹H-NMR (200MHz, CDCl₃) δppm: 0.89 (6H, d, J=6.6Hz), 1.27 (6H, d, J=6.9Hz), 1.84 (1H, m), 2.40 (2H, d, J=7.3Hz), 2.95 (1H, m), 3.62 (3H, s), 4.41-4.64 (3H, m), 5.16 (1H, d, J=14.5Hz), 5.68 (1H, d, J=1.1Hz), 5.70 (1H, s), 7.39-7.64 (4H, m).
MS m/z: 435(M⁺), 420, 393, 309, 267, 224, 168, 140, 116.

### (Example 366)

### Compound of Compound No. 8-47

¹H-NMR(200MHz, CDCl₃) δppm: 0.86 (6H, d, J=6.6Hz), 1.84 (1H, m), 2.39 (2H, d, J=7.3Hz), 3.55 (3H, s), 4.47 (1H, d, J=14.0Hz), 4.68 (1H, d, J=14.5Hz), 4.83 (1H, d, J=14.5Hz), 5.18 (1H, d, J=14.0Hz), 5.70 (1H, s), 6.92 (1H, dd, J=6.6, 1.8Hz), 7.36-7.63 (7H, m), 8.13 (1H, dd, J=8.4, 1.4Hz), 8.91 (1H, dd, J=4.4, 1.8Hz).
MS m/z: 453(M⁺), 438, 395, 309, 267, 226, 186, 158, 116.

### (Example 367)

### Compound of Compound No. 8-48

¹H-NMR(200MHz, CDCl₃) δppm: 0.88 (6H, d, J=6.6Hz), 1.84 (1H, m), 2.42 (2H, d, J=7.0Hz), 3.55 (3H, s), 4.42-4.65 (3H, m), 5.20 (1H, d, J=14.0Hz), 5.72 (1H, s), 6.88 (1H, d, J=2.6Hz), 7.30-7.65 (6H, m), 7.96-8.03 (2H, m), 8.79 (1H, dd, J=4.4, 1.8Hz).
MS m/z: 453(M⁺), 438, 411, 309, 278, 226, 186, 176, 158, 149, 116.

### (Example 368)

### Compound of Compound No. 8-49

¹H-NMR(200MHz, CDCl₃) δppm: 0.90 (6H, d, J=6.6Hz), 1.86 (1H, m), 2.42 (3H, s), 2.42 (2H, d, J=7.0Hz), 3.56 (3H, s), 4.18 (1H, d, J=16.5Hz), 4.47 (1H, d, J=16.5Hz), 4.80 (1H, d, J=14.5Hz), 5.07 (1H, d, J=14.5Hz), 5.75 (1H, s), 6.88 (1H, d, J=2.6Hz), 7.40-7.65 (4H, m).
MS m/z: 424(M⁺), 409, 382, 267, 226, 168, 157, 129, 116.

### (Example 369)

### Compound of Compound No. 8-5

¹H-NMR(200MHz, CDCl₃) δppm: 0.89 (6H, d, J=6.6Hz), 1.85 (1H, m), 2.41 (2H, d, J=7.3Hz), 2.44 (3H, s), 3.65 (3H, s), 4.49 (1H, d, J=14.5Hz), 4.60 (1H, d, J=16.5Hz), 4.71 (1H, d, J=16.5Hz), 5.09 (1H, d, J=14.5Hz), 5.74 (1H, s), 6.85 (1H, d, J=5.0Hz), 7.39-7.64 (4H, m), 8.34 (1H, d, J=5.0Hz).
MS m/z: 418(M⁺), 403, 381, 309, 267, 224, 151, 123, 116.

### (Example 370)

### Compound of Compound No. 8-51

¹H-NMR(200MHz, CDCl₃) δppm: 0.90 (6H, d, J=6.6Hz), 1.86 (1H, m), 2.37 (6H, s), 2.42 (2H, d, J=7.0Hz), 3.65 (3H, s), 4.53 (1H, d, J=14.0Hz), 4.63 (2H, s), 5.05 (1H, d, J=14.0Hz), 5.75 (1H, s), 6.70 (1H, s), 7.38-7.63 (4H, m).
MS m/z: 432(M⁺), 417, 390, 309, 267, 224, 165, 137, 116, 107.

### (Example 371)

### Compound of Compound No. 8-52

¹H-NMR(200MHz, CDCl₃) δppm: 0.90 (6H, d, J=6.6Hz), 1.86 (1H, m), 2.41 (3H, s), 2.42 (2H, d, J=7.3Hz), 2.49 (3H, s), 3.67 (3H, s), 4.47 (1H, d, J=14.0Hz), 4.57 (1H, d, J=16.5Hz), 4.69 (1H, d, J=16.5Hz), 5.11 (1H, d, J=14.5Hz), 5.74 (1H, s), 6.54 (1H, s), 7.39-7.64 (4H, m).
MS m/z: 432(M⁺), 417, 390, 309, 267, 224, 165, 137, 116, 107.

### (Example 372)

### Compound of Compound No. 8-53

¹H-NMR(200MHz, CDCl₃) δppm: 0.91 (6H, d, J=6.6Hz), 1.21 (6H, d, J=7.0Hz), 1.87 (1H, m), 2.42 (2H, d, J=7.3Hz), 2.43 (3H, s), 2.97 (1H, m), 3.60 (3H, s), 4.51 (1H, d, J=14.5Hz), 4.62 (1H, d, J=15.0Hz), 4.76 (1H, d, J=15.0Hz), 5.04 (1H, d, J=14.5Hz), 5.76 (1H, s), 6.51 (1H, s), 7.38-7.63 (4H, m).
MS m/z: 460(M⁺), 445, 418, 309, 267, 224, 193, 165, 135, 116.

### (Example 373)

### Compound of Compound No. 8-54

¹H-NMR(200MHz, CDCl₃) δppm: 0.90 (6H, d, J=6.6Hz), 1.86 (1H, m), 2.42 (2H, d, J=7.0Hz), 3.68 (3H, s), 4.44 (1H, d, J=14.0Hz), 4.67 (2H, s), 5.16 (1H, d, J=14.0Hz), 5.74 (1H, s), 6.89 (1H, dd, J=5.9, 1.1Hz), 7.40-7.65 (4H, m), 8.50 (1H, d, J=5.9Hz), 8.72 (1H, s).
MS m/z: 404(M⁺), 389, 362, 309, 267, 226, 137, 116.

### (Example 374)

### Compound of Compound No. 8-55

¹H-NMR(200MHz, CDCl₃) δppm: 0.92 (6H, d, J=6.6Hz), 1.88 (1H, m), 2.44 (2H, d, J=7.3Hz), 3.73 (3H, s), 4.23 (1H, d, J=16.5Hz), 4.49 (1H, d, J=16.5Hz), 4.51 (1H, d, J=14.0Hz), 5.13 (1H, d, J=14.0Hz), 5.83 (1H, s), 7.41-8.02 (8H, m), 8.28 (1H, d, J=7.3Hz).
MS m/z: 454(M⁺), 439, 412, 267, 226, 187, 159, 116.

### (Example 375)

### Compound of Compound No. 8-56

¹H-NMR(200MHz, CDCl₃) δppm: 0.90 (6H, d, J=6.6Hz), 1.85 (1H, m), 2.42 (2H, d, J=7.3Hz), 3.67 (3H, s), 4.47 (1H, d, J=14.5Hz), 4.68 (1H, d, J=15.0Hz), 4.82 (1H, d, J=15.0Hz), 5.18 (1H, d, J=14.0Hz), 5.75 (1H, s), 7.25-7.73 (8H, m).
MS m/z: 443(M⁺), 428, 401, 385, 309, 267, 224, 176, 148, 116.

### (Example 376)

### Compound of Compound No. 8-58

¹H-NMR(200MHz, CDCl₃) δppm: 0.90 (6H, d, J=6.6Hz), 1.08 (3H, t, J=7.3Hz), 1.85 (1H, m), 2.41 (2H, d, J=7.0Hz), 2.57 (2H, q, J=7.3Hz), 2.90-3.30 (2H, m), 3.46 (3H, s), 4.49 (1H, d, J=14.5Hz), 5.08 (1H, d, J=14.5Hz), 5.64 (1H, s), 7.39-7.63 (4H, m).
MS m/z: 353(M⁺), 338, 323, 310, 296, 268, 226, 180, 166, 149, 116.

### (Example 377)

### Compound of Compound No. 8-67

¹H-NMR(200MHz, CDCl₃) δppm: 0.93 (6H, d, J=6.6Hz), 1.89 (1H, m), 2.45 (2H, d, J=7.3Hz), 3.48 (3H, s), 3.40-3.80 (2H, m), 4.40-4.60 (2H, m), 5.15 (1H, d, J=13.5Hz), 5.70 (1H, s), 6.49 (2H, dd, J=8.4, 1.1Hz), 6.73 (1H, t, J=7.3Hz), 7.16 (2H, td, J=7.3, 1.1Hz), 7.39-7.64 (4H, m).
MS m/z: 401(M⁺), 386, 373, 268, 226, 180, 166, 116, 106.

### (Example 378)

### Compound of Compound No. 8-68

¹H-NMR(200MHz, CDCl₃) δppm: 0.90 (6H, d, J=6.6Hz), 1.85 (1H, m), 2.24 (6H, s), 2.41 (2H, d, J=7.0Hz), 2.79 (1H, d, J=15.0Hz), 2.91 (1H, d, J=15.0Hz), 3.46 (3H, s), 4.42 (1H, d, J=14.0Hz), 5.11 (1H, d, J=14.0Hz), 5.60 (1H, s), 7.38-7.62 (4H, m).
MS m/z: 353(M⁺), 338, 310, 282, 267, 226, 116.

### (Example 379)

### Compound of Compound No. 8-69

¹H-NMR(200MHz, CDCl₃) δppm: 0.91 (12H, m), 1.84 (1H, m), 2.40 (2H, d, J=7.3Hz), 2.47-2.59 (4H, m), 3.02 (2H, s), 3.48 (3H, s), 4.39 (1H, d, J=14.5Hz), 5.10 (1H, d, J=14.5Hz), 5.60 (1H, s), 7.37-7.66 (4H, m).
MS m/z: 381(M⁺), 366, 293, 279, 265, 167, 149, 86.

### (Example 380)

### Compound of Compound No. 8-70

¹H-NMR(200MHz, CDCl₃) δppm: 0.91 (6H, d, J=6.6Hz), 1.87 (1H, m), 2.43 (2H, d, J=7.3Hz), 2.83-2.95 (5H, m), 3.47 (3H, s), 3.74 (1H, d, J=17.0Hz), 3.96 (1H, d, J=17.0Hz), 4.39 (1H, d, J=14.5Hz), 5.11 (1H, d, J=14.5Hz), 5.60 (1H, s), 7.37-7.66 (4H, m).
MS m/z: 415(M⁺), 400, 372, 281, 267, 226, 120.

### (Example 381)

### Compound of Compound No. 8-81

¹H-NMR(200MHz, CDCl₃) δppm: 0.89 (6H, d, J=6.6Hz), 1.83 (1H, m), 2.39 (2H, d, J=7.3Hz), 3.46 (2H, s), 3.47 (3H, s), 4.33 (1H, d, J=14.5Hz), 5.17 (1H, d, J=14.5Hz), 5.53 (1H, s), 7.25-7.63 (9H, m).
MS m/z: 418(M⁺), 403, 376, 309, 281, 268, 226, 123, 116.

### (Example 382)

### Compound of Compound No. 8-83

¹H-NMR(200MHz, CDCl₃) δppm: 0.89 (6H, d, J=6.6Hz), 1.84 (1H, m), 2.40 (2H, d, J=7.0Hz), 3.50 (5H, s), 4.38 (1H, d, J=14.5Hz), 5.16 (1H, d, J=14.5Hz), 5.63 (1H, s), 7.38-7.63 (8H, m).
MS m/z: 486(M⁺), 471, 444, 309, 281, 268, 226, 191.

### (Example 383)

### Compound of Compound No. 8-86

¹H-NMR(200MHz, CDCl₃) δppm: 0.90 (6H, d, J=6.6Hz), 1.84 (1H, m), 2.40 (2H, d, J=7.0Hz), 3.43 (2H, s), 3.50 (3H, s), 4.34 (1H, d, J=14.5Hz), 5.16 (1H, d, J=14.5Hz), 5.57 (1H, s), 7.25 (2H, d, J=8.8Hz), 7.32 (2H, d, J=8.8Hz), 7.37-7.64 (4H, m).
MS m/z: 453(M⁺), 410, 281, 279, 268, 226, 149.

### (Example 384)

### Compound of Compound No. 8-90

¹H-NMR(200MHz, CDCl₃) δppm: 0.89 (6H, d, J=6.6Hz), 1.84 (1H, m), 2.41 (2H, d, J=7.3Hz), 3.47 (2H, s), 3.61 (3H, s), 4.55 (1H, br-s), 5.08 (1H, br-s), 5.65 (1H, s), 6.69-6.72 (2H, m), 7.40-7.63 (4H, m).
MS m/z: 442(M⁺), 382, 325, 284, 267, 226, 155, 154, 125, 116.

### (Example 385)

### Compound of Compound No. 8-91

¹H-NMR(200MHz, CDCl₃) δppm: 0.90 (6H, d, J=6.6Hz), 1.85 (1H, m), 2.40 (2H, d, J=7.0Hz), 3.60 (3H, s), 3.74 (2H, s), 4.46 (1H, d, J=14.0Hz), 5.17 (1H, d, J=14.0Hz), 5.73 (1H, s), 7.01 (1H, t, J=5.0Hz), 7.38-7.64 (4H, m), 8.47 (2H, d, J=5.0Hz).

### (Example 386)

### Compound of Compound No. 8-92

¹H-NMR(200MHz, CDCl₃) δppm: 0.90 (6H, d, J=6.6Hz), 1.85 (1H, m), 2.41 (2H, d, J=7.3Hz), 2.73 (3H, s), 3.68 (3H, s), 3.86 (2H, s), 4.44 (1H, d, J=14.0Hz), 5.17 (1H, d, J=14.0Hz), 5.76 (1H, s), 7.39-7.63 (4H, m).

### (Example 387)

### Compound of Compound No. 9-1

¹H-NMR(200MHz, CDCl₃) δppm: 0.89 (6H, d, J=6.6Hz), 1.29 (3H, t, J=7.3Hz), 1.78-1.88 (1H, m), 1.90 (3H, s), 2.42 (2H, d, J=7.3Hz), 3.75 (2H, q, J=7.3Hz), 4.41 (1H, d, J=14.1Hz), 5.11 (1H, d, J=14.1Hz), 5.60 (1H, s), 7.38-7.69 (4H, m).
MS m/z: 324(M⁺), 282, 240, 166, 116.

### (Example 388)

### Compound of Compound No. 9-3

¹H-NMR(200MHz, CDCl₃) δppm: 0.72-1.43 (3H, m), 0.90 (6H, d, J=6.6Hz), 1.29 (3H, t, J=7.3Hz), 1.79-1.96 (1H, m), 1.90 (3H, s), 2.43 (2H, d, J=7.3Hz), 3.78 (2H, q, J=7.3Hz), 4.49 (1H, d, J=14.3Hz), 5.09 (1H, d, J=14.3Hz), 5.69 (1H, s), 7.38-7.62 (4H, m).
MS m/z: 350(M⁺), 308, 282, 240, 116.

### (Example 389)

### Compound of Compound No. 9-7

¹H-NMR(200MHz, CDCl₃) δppm: 0.90 (6H, d, J=6.6Hz), 1.32 (3H, t, J=7.3Hz), 1.78-1.93 (1H, m), 2.42 (2H, d, J=7.3Hz), 3.37 (3H, s), 3.63-3.93 (4H, m), 4.39 (1H, d, J=14.1Hz), 5.18 (1H, d, J=14.1Hz), 5.59 (1H, s), 7.39-7.64 (4H, m).
MS m/z: 354(M⁺), 312, 281, 116.

### (Example 399)

### Compound of Compound No. 9-8

¹H-NMR(200MHz, CDCl₃) δppm: 0.88 (6H, d, J=6.6Hz), 1.34 (3H, t, J=7.3Hz), 1.77-1.91 (1H, m), 2.43 (2H, d, J=7.3Hz), 3.74-3.87 (2H, m), 4.30 (1H, d, J=14.8Hz), 4.38 (1H, d, J=14.3Hz), 4.49 (1H, d, J=14.8Hz), 5.23 (1H, d, J=14.3Hz), 5.64 (1H, s), 6.78 (2H, d, J=8.8Hz), 6.97 (1H, t, J=7.3Hz), 7.20-7.64 (6H, m).
MS m/z: 416(M⁺), 374, 295, 116.

### (Example 391)

### Compound of Compound No. 9-9

¹H-NMR(200MHz, CDCl₃) δppm: 0.90 (6H, d, J=6.6Hz), 1.24 (3H, t, J=7.2Hz), 1.78-1.95 (1H, m), 2.41 (2H, d, J=7.3Hz), 3.69 (2H, q, J=7.2Hz), 3.76 (3H, s), 4.72 (2H, s), 5.59 (1H, s), 7.39-7.64 (4H, m).
MS m/z: 340(M⁺), 325, 298, 116.

### (Example 392)

### Compound of Compound No. 9-21

¹H-NMR(200MHz, CDCl₃) δppm: 0.86 (6H, d, J=6.9Hz), 1.63 (9H, s), 1.63-1.87 (1H, m), 1.93 (3H, s), 2.37 (2H, d, J=7.0Hz), 3.80 (1H, d, J=14.3Hz), 5.33 (1H, s), 5.59 (1H, d, J=14.3Hz), 7.39-7.67 (4H, m).
MS m/z: 352(M⁺), 296, 254, 212, 116.

### (Example 393)

### Compound of Compound No. 9-23

¹H-NMR(200MHz, CDCl₃) δppm: 0.72-1.09 (4H, m), 0.87 (6H, d, J=6.6Hz), 1.34-1.59 (1H, m), 1.64 (9H, s), 1.65-1.83 (1H, m), 2.38 (2H, d, J=7.0Hz), 3.85 (1H, d, J=14.3Hz), 5.42 (1H, s), 5.58 (1H, d, J=14.3Hz), 7.43-7.67 (4H, m).
MS m/z: 378(M⁺), 322, 280, 254, 116.

### (Example 394)

### Compound of Compound No. 9-27

¹H-NMR(200MHz, CDCl₃) δppm: 0.87 (6H, d, J=6.6Hz), 1.64 (9H, s), 1.67-1.87 (1H, m), 2.37 (2H, d, J=7.0Hz), 3.38 (3H, s), 3.74 (1H, d, J=15.4Hz), 3.79 (1H, d, J=14.1Hz), 3.91 (1H, d, J=15.4Hz), 5.32 (1H, s), 5.64 (1H, d, J=14.1Hz), 7.39-7.63 (4H, m).
MS m/z: 382(M⁺), 326, 311, 284, 253, 116.

### (Example 395)

### Compound of Compound No. 9-28

¹H-NMR(200MHz, CDCl₃) δppm: 0.87 (6H, d, J=6.6Hz), 1.65 (9H, s), 1.68-1.88 (1H, m), 2.21 (2H, d, J=7.0Hz), 3.85 (1H, d, J=13.9Hz), 4.35 (1H, d, J=14.8Hz), 4.51 (1H, d, J=14.8Hz), 5.40 (1H, s), 5.66 (1H, d, J=13.9Hz), 6.76-7.64 (9H, m).
MS m/z: 444(M⁺), 388, 346, 295, 253, 116.

### (Example 396)

### Compound of Compound No. 9-29

¹H-NMR(200MHz, CDCl₃) δppm: 0.87 (6H, d, J=6.6Hz), 1.47-1.82 (1H, m), 1.57 (9H, s), 2.36 (2H, d, J=6.6Hz), 3.74 (3H, s), 4.00 (1H, d, J=13.6Hz), 5.30 (2H, m), 7.44-7.69 (4H, m).
MS m/z: 368(M⁺), 312, 270, 253, 116.

### (Example 397)

### Compound of Compound No. 4-39

¹H-NMR(200MHz, CDCl₃) δppm: 0.11-0.19 (2H, m), 0.46-0.55 (2H, m), 0.85-1.02 (1H, m), 2.46 (2H, d, J=7.0Hz), 3.42 (3H, s), 3.78 (3H, s), 4.73 (2H, s), 5.76 (1H, s), 7.41-7.64 (4H, m).
MS m/z: 324(M⁺), 283, 265, 222, 208.

### (Example 398)

### Compound of Compound No. 4-115

¹H-NMR(200MHz, CDCl₃) δppm: 2.20 (6H, s), 3.38 (2H, s), 3.52 (3H, s), 4.31-4.53 (3H, m), 5.14 (1H, d, J=13.6Hz), 5.91 (1H, s), 6.75-6.79 (2H, m), 6.97 (1H, t, J=7.3Hz), 7.22-7.29 (2H, m), 7.39-7.64 (4H, m).
MS m/z: 403(M⁺), 360, 244, 224, 137.

### (Example 399)

### Compound of Compound No. 5-5

¹H-NMR(200MHz, CDCl₃) δppm: 0.87 (3H, t, J=7.7Hz), 0.90 (6H, d, J=6.6Hz), 1.54-2.07 (5H, m), 2.41 (2H, d, J=7.3Hz), 3.45 (3H, s), 4.47 (1H, d, J=14.0Hz), 5.07 (1H, d, J=14.0Hz), 5.61 (1H, s), 7.37-7.62 (4H, m).
MS m/z: 338(M⁺), 323, 296, 268, 226, 166, 116.

### (Example 400)

### Compound of Compound No. 7-45

¹H-NMR(200MHz, CDCl₃) δppm: 0.85 (6H, d, J=6.6Hz), 1.82 (1H, m), 2.37 (2H, d, J=7.0Hz), 3.44 (3H, s), 4.41 (2H, d, J=5.9Hz), 4.72 (2H, t, J=5.5Hz), 4.75 (1H, bs), 5.66 (1H, s), 7.19-7.61 (9H, m).
MS m/z: 401(M⁺), 359, 268, 253, 226, 152, 116, 91.

### (Example 401)

### Compound of Compound No. 10-1

¹H-NMR(200MHz, CDCl₃) δppm: 1.04-1.51 (8H, m), 1.60-1.72 (1H, m), 1.89 (1H, s), 1.95 (1H, bs), 2.21 (1H, bs), 2.32 (1H, dd, J=8.1Hz, 14.3Hz), 2.50 (1H, dd, J=8.1Hz, 14.3Hz), 3.45 (3H, s), 4.53 (1H, bs), 5.01 (1H, bs), 5.65 (1H, s), 7.38-7.63 (4H, m). MS m/z: 362(M⁺), 333, 319, 294, 268.

### (Example 402)

### Compound of Compound No. 10-7

¹H-NMR(200MHz, CDCl₃) δppm: 1.02-1.75 (9H, m), 1.95 (1H, bs), 2.22 (1H, bs), 2.32 (1H, dd, J=7.9Hz, 14.5Hz), 2.50 (1H, dd, J=7.9Hz, 14.5Hz), 3.36 (3H, s), 3.46 (3H, s), 3.66 (1H, bd), 3.85 (1H, bd), 4.46 (1H, bd), 5.08 (1H, bd), 5.63 (1H, s), 7.39-7.64 (4H, m).
MS m/z: 392(M⁺), 363, 298, 268, 226.

### (Example 403)

### Compound of Compound No. 10-8

¹H-NMR(200MHz, CDCl₃) 1.02-1.72 (9H, m), 1.92 (1H, bs), 2.20 (1H, bs), 2.32 (1H, dd, J=8.1Hz, 14.3Hz), 2.50 (1H, dd, J=8.1Hz, 14.3Hz), 3.51 (3H, s), 4.27-4.54 (3H, m), 5.15 (1H, bd), 5.68 (1H, s), 6.76-6.80 (2H, m), 6.98 (1H, t, J=7.5Hz), 7.22-7.64 (6H, m).
MS m/z: 392(M⁺), 363, 298, 268, 226.

### (Example 404)

### Compound of Compound No. 10-10

¹H-NMR(200MHz, CDCl₃) δppm: 1.06-1.54 (8H, m), 1.62-1.76 (1H, m), 1.97 (1H, bs), 2.21 (1H, bs), 2.31 (1H, dd, J=8.1Hz, 14.3Hz), 2.49 (1H, dd, J=8.1Hz, 14.3Hz), 3.41 (3H, s), 3.77 (3H, s), 4.72 (2H, s), 5.63 (1H, s), 7.39-7.64 (4H, m).
MS m/z: 454(M⁺), 425, 386, 360,116.

### (Example 405)

### Compound of Compound No. 10-11

¹H-NMR(200MHz, CDCl₃) δppm: 1.39-1.56 (1H, m), 1.89 (3H, s), 1.91-2.09 (1H, m), 2.26-2.34 (2H, m), 2.47-2.68 (2H, m), 2.89-2.98 (1H, m), 3.45 (3H,s), 4.56 (1H, bs), 4.98 (1H, bs), 5.60-5.65 (1H, m), 5.68 (1H, s), 5.72-5.79 (1H, m), 7.39-7.63 (4H, m).
MS m/z: 334(M⁺), 291, 268, 226, 152.

### (Example 406)

### Compound of Compound No. 10-16

¹H-NMR(200MHz, CDCl₃) δppm: 1.42-1.60 (1H, m), 1.95-2.11 (1H, m), 2.27-2.38 (2H, m), 2.47-2.69 (2H, m), 2.90-3.04 (1H, m), 3.54 (3H, s), 4.82 (2H, s), 5.62-5.68 (1H, m), 5.74-5.80 (2H, m), 7.10-7.67 (9H, m).
MS m/z: 412(M⁺), 347, 253, 136, 116.

### (Example 407)

### Compound of Compound No. 10-17

¹H-NMR(200MHz, CDCl₃) δppm: 1.38-1.55 (1H, m), 1.91-2.09 (1H, m), 2.26-2.34 (2H, m), 2.47-2.68 (2H, m), 2.89-2.98 (1H, m), 3.37 (3H, s), 3.47 (3H, s), 3.67 (1H, bd), 3.85 (1H, bd), 4.47 (1H, d, J=14.1Hz), 5.08 (1H, d, J=14.1Hz), 5.59-5.64 (1H, m), 5.67 (1H, s), 5.72-5.78 (1H, m), 7.39-7.64 (4H, m).
MS m/z: 364(M⁺), 298, 225, 182, 136.

### (Example 408)

### Compound of Compound No. 10-19

¹H-NMR(200MHz, CDCl₃) δppm: 1.40-1.53 (1H, m), 1.87-2.05 (1H, m), 2.25-2.33 (2H, m), 2.47-2.68 (2H, m), 2.86-2.98 (1H, m), 3.51 (3H, s), 4.28-4.52 (3H, m), 5.16 (1H, d, J=14.3Hz), 5.54-5.60 (1H, m), 5.70 (1H, s), 5.70-5.75 (1H, m), 6.71-6.80 (2H, m), 6.98 (1H, t, J=7.3Hz), 7.22-7.27 (2H, m), 7.40-7.64 (4H, m).
MS m/z: 426 (M⁺), 360, 291, 244, 150.

### (Example 409)

### Compound of Compound No. 10-20

¹H-NMR(200MHz, CDCl₃) δppm: 1.39-1.56 (1H, m), 1.92-2.10 (1H, m), 2.26-2.35 (2H, m), 2.45-2.66 (2H, m), 2.86-3.02 (1H, m), 3.42 (3H, s), 3.77 (3H, s), 4.73 (2H, s), 5.61-5.66 (1H, m), 5.66 (1H, s), 5.72-5.78 (1H, m), 7.40-7.64 (4H, m).
MS m/z: 350 (M⁺), 284, 168, 136, 116.

### (Example 410)

### Compound of Compound No. 10-21

¹H-NMR(200MHz, CDCl₃) δppm: 1.89 (3H, s), 2.01-2.07 (2H, m), 2.43-2.59 (5H, m), 3.45 (3H, s), 4.52 (1H, bs), 5.00 (1H ,bs), 5.66-5.67 (3H, m), 7.39-7.63 (4H, m).
MS m/z: 334 (M⁺), 268, 226, 137, 116.

### (Example 411)

### Compound of Compound No. 10-30

¹H-NMR(200MHz, CDCl₃) δppm: 2.01-2.07 (2H, m), 2.43-2.59 (5H, m), 3.36 (3H, s), 3.46 (3H, s), 3.65 (1H, d, J=14.5Hz), 3.88 (1H, d, J=14.5Hz), 4.47 (1H, d, J=14.1Hz), 5.09 (1H, d, J=14.1Hz), 5.66 (3H, s), 7.39-7.64 (4H, m).
MS m/z: 364 (M⁺), 298, 225, 182, 137.

### (Example 412)

### Compound of Compound No. 10-34

¹H-NMR(200MHz, CDCl₃) δppm: 2.02-2.08 (2H, m), 2.50-2.58 (5H, m), 3.41 (3H, s), 3.77 (3H, s), 4.72 (2H, s), 5.66 (3H, s), 7.43-7.64 (4H, m).
MS m/z: 350 (M⁺), 284, 168, 137, 116.

### (Example 413)

### Compound of Compound No. 10-35

¹H-NMR(200MHz, CDCl₃) δppm: 0.91 (3H, s), 1.25-1.67 (10H, m), 1.89 (3H, s), 2.52 (2H, s), 3.47 (3H, s), 4.49 (1H, d, J=13.0Hz), 5.26 (1H, d, J=13.0Hz), 5.65 (1H, s), 7.38-7.62 (4H, m).
MS m/z: 350(M⁺), 335, 307, 268, 226.

### (Example 414)

### Compound of Compound No. 10-41

¹H-NMR(200MHz, CDCl₃) δppm: 0.90 (3H, s), 1.23-1.64 (10H, m), 2.51 (2H, s), 3.36 (3H, s), 3.48 (3H, s), 3.65 (1H, d, J=14.5Hz), 3.88 (1H, d, J=14.5Hz), 4.43 (1H, d, J=13.9Hz), 5.13 (1H, d, J=13.9Hz) 5.62 (1H, s), 7.39-7.63 (4H, m).
MS m/z: 380(M⁺), 365, 298, 268, 116.

### (Example 415)

### Compound of Compound No. 10-44

¹H-NMR(200MHz, CDCl₃) δppm: 0.91 (3H, s), 1.22-1.67 (10H, m), 2.50 (2H, s), 3.42 (3H, s), 3.77 (3H, s), 4.72 (2H, s), 5.62 (1H, s), 7.38-7.64 (4H, m).
MS m/z: 366(M⁺), 351, 297, 284, 168.

### (Example 416)

### Compound of Compound No. 10-45

¹H-NMR(200MHz, CDCl₃) δppm: 0.84-0.96 (3H, m), 1.12-2.15 (8H, m), 1.89 (3H, s), 2.29-2.43 (1H, m), 2.56-2.76 (1H, m), 3.45 (3H, s), 4.55 (1H, bs), 4.98 (1H, bs), 5.67 (1H, s), 7.39-7.62 (4H, m).
MS m/z: 350(M⁺), 335, 307, 268, 226.

### (Example 417)

### Compound of Compound No. 10-51

¹H-NMR(200MHz, CDCl₃) δppm: 0.84-0.96 (3H, m), 1.12-2.15 (8H, m), 2.32-2.43 (1H, m), 2.56-2.76 (1H, m), 3.37 (3H, s), 3.46 (3H, s), 3.65 (1H, d, J=13.0Hz), 3.88 (1H, d, J=13.0Hz), 4.46 (1H, d, J=14.1Hz), 5.09 (1H, d, J=14.1Hz), 5.65 (1H, s), 7.39-7.60(4H, m).
MS m/z: 380(M⁺), 365, 298, 226, 116.

### (Example 418)

### Compound of Compound No. 10-52

¹H-NMR(200MHz, CDCl₃) δppm: 0.82-0.95 (3H, m), 1.12-2.12 (8H, m), 2.32-2.43 (1H, m), 2.56-2.77 (1H, m), 3.51 (3H, s), 4.27-4.52 (3H, m), 5.17 (1H, d, J=13.9Hz), 5.68 (1H, s), 6.76-6.80 (2H, m), 6.98 (1H, t, J=7.3Hz), 7.22-7.70 (6H, m).
MS m/z: 442(M⁺), 427, 360, 349, 268.

### (Example 419)

### Compound of Compound No. 10-54

¹H-NMR(200MHz, CDCl₃) δppm: 0.84-0.95 (3H, m), 1.11-2.15 (8H, m), 2.27-2.42 (1H, m), 2.55-2.76 (1H, m), 3.41 (3H, s), 3.77 (3H, s), 4.72 (2H, s), 5.64 (1H, s), 7.39-7.63 (4H, m).
MS m/z: 366(M⁺), 351, 311, 284, 168.

### (Example 420)

### Compound of Compound No. 10-55

¹H-NMR(200MHz, CDCl₃) δppm: 0.94-1.00 (3H, m), 1.06-2.28 (8H, m), 1.89 (3H, s), 2.49-2.56 (2H, m), 3.44 (3H, s), 4.54 (1H, bs), 4.98 (1H, bs), 5.65 (1H, s), 7.38-7.63 (4H, m).
MS m/z: 350(M⁺), 335, 307, 268, 226.

### (Example 421)

### Compound of Compound No. 10-61

¹H-NMR(200MHz, CDCl₃) δppm: 0.94-1.00 (3H, m), 1.12-2.28 (8H, m), 2.49-2.56 (2H, m), 3.37 (3H, s), 3.45 (3H, s), 3.65 (1H, d, J=13.7), 3.88 (1H, d, J=13.7), 4.46 (1H, d, J=14.7), 5.09 (1H, d, J=14.7), 5.64 (1H, s), 7.39-7.64 (4H, m).
MS m/z: 380(M⁺), 365, 325, 298, 270.

### (Example 422)

### Compound of Compound No. 10-62

¹H-NMR(200MHz, CDCl₃) δppm: 0.92-0.98 (3H, m), 1.06-2.26 (8H, m), 2.49-2.56 (2H, m), 3.56 (3H, s), 4.27-4.56 (3H, m), 5.16 (1H, d, J=13.2Hz), 5.68 (1H, s), 6.75-6.80 (2H, m), 6.97 (1H, t, J=7.3Hz), 7.22-7.68 (6H, m).
MS m/z: 442(M⁺), 427, 387, 360, 116.

### (Example 423)

### Compound of Compound No. 10-64

¹H-NMR(200MHz, CDCl₃) δppm: 0.94-0.99 (3H, m), 1.06-2.28 (8H, m), 2.48-2.55 (2H, m), 3.41 (3H, s), 3.77 (3H, s), 4.72 (2H, s), 5.63 (1H, s), 7.39-7.64 (4H, m).
MS m/z: 366(M⁺), 351, 311, 284, 168.

### (Example 424)

### Compound of Compound No. 10-65

¹H-NMR(200MHz, CDCl₃) δppm: 1.53-1.94 (8H, m), 1.89 (3H, s), 2.96 (2H, d, J=22.3Hz), 3.45 (3H, s), 4.59 (1H, bs), 4.95 (1H, bs), 5.82 (1H, s), 7.38-7.62 (4H, m).
MS m/z: 354(M⁺), 334, 291, 268, 190.

### (Example 425)

### Compound of Compound No. 10-73

¹H-NMR(200MHz, CDCl₃) δppm: 1.38-2.61 (8H, m), 1.88 (3H, s), 2.88 (1H, dd, J=6.2Hz, 14.3Hz), 3.43 (3H, s), 4.57 (1H, bs), 4.97 (1H, bs), 5.72 (1H, s), 7.39-7.62 (4H, m).
MS m/z: 372(M⁺), 352, 330, 309, 268.

### (Example 426)

### Compound of Compound No. 10-79

¹H-NMR(200MHz, CDCl₃) δppm: 1.38-2.62 (8H, m), 2.87 (1H, dd, J=6.4Hz, 14.5Hz), 3.36 (3H, s), 3.45 (3H, s), 3.65 (1H, d, J=14.7Hz), 3.87 (1H, d, J=14.7Hz), 4.48 (1H, d, J=12.5Hz), 5.06 (1H, d, J=12.5Hz), 5.71 (1H, s), 7.39-7.63 (4H, m).
MS m/z: 402(M⁺), 382, 329, 298, 270.

### (Example 427)

### Compound of Compound No. 10-81

¹H-NMR(200MHz, CDCl₃) δppm: 1.38-2.58 (8H, m), 2.89 (1H, dd, J=5.5Hz, 14.3Hz), 3.40 (3H, s), 3.77 (3H, s), 4.72 (2H, s), 5.69 (1H, s), 7.40-7.64 (4H, m).
MS m/z: 388(M⁺), 368, 284, 272, 168.

### (Example 428)

### Compound of Compound No. 10-82

¹H-NMR(200MHz, CDCl₃) δppm: 1.05 (3H, s), 1.63-1.90 (6H, m), 1.89 (3H, s), 2.60 (2H, s), 4.50 (1H, bs), 5.04 (1H, bs), 5.62 (1H, s), 7.38-7.63 (4H, m).
MS m/z: 336 (M⁺), 321, 308, 293, 281.

### (Example 429)

### Compound of Compound No. 10-88

¹H-NMR(200MHz, CDCl₃) δppm: 1.05 (3H, s), 1.63-1.90 (6H, m), 2.60 (2H, s), 3.36 (3H, s), 3.48 (3H, s), 3.66 (1H, d, J=14.7Hz), 3.86 (1H, d, J=14.7Hz), 4.44 (1H, d, J=14.3Hz), 5.11 (1H, d, J=14.3Hz), 5.61 (1H, s), 7.38-7.63 (4H, m).
MS m/z: 366 (M⁺), 351, 338, 311, 298.

### (Example 430)

### Compound of Compound No. 10-90

¹H-NMR(200MHz, CDCl₃) δppm: 1.05 (3H, s), 1.63-1.94 (6H, m), 2.59 (2H, s), 3.42 (3H, s), 3.77 (3H, s), 4.72 (2H, s), 5.60 (1H, s), 7.39-7.63 (4H, m).
MS m/z: 352 (M⁺), 337, 324, 309, 297.

### (Example 431)

### Compound of Compound No. 10-91

¹H-NMR(200MHz, CDCl₃) δppm: 1.53-2.05 (4H, m), 1.90 (3H, s), 2.67-2.87 (2H, m), 3.45 (3H, s), 3.69-3.92 (2H, m), 4.02-4.12 (1H, m), 4.58 (1H, bs), 4.95 (1H, bs), 5.80 (1H, s), 7.39-7.62 (4H, m).
MS m/z: 338(M⁺), 295, 268, 226, 116.

### (Example 432)

### Compound of Compound No. 10-97

¹H-NMR(200MHz, CDCl₃) δppm: 1.53-2.02 (4H, m), 2.71-2.79 (2H, m), 3.36 (3H, s), 3.41 (3H, s), 3.61-3.91 (4H, m), 4.01-4.14 (1H, m), 4.51 (1H, bs), 5.04 (1H, bs), 5.78 (1H, s), 7.39-7.64 (4H, m).
MS m/z: 368(M⁺), 325, 298, 182, 116.

### (Example 433)

### Compound of Compound No. 10-100

¹H-NMR(200MHz, CDCl₃) δppm: 1.51-1.68 (2H, m), 1.89 (3H, s), 1.94-2.10 (1H, m), 2.44-2.65 (3H, m), 3.45 (3H, s), 3.71-3.93 (3H, m), 4.56 (1H, bs), 4.98 (1H, bs), 5.68 (1H, s), 7.39-7.63 (4H, m).
MS m/z: 338(M⁺), 310, 295, 268, 226.

### (Example 434)

### Compound of Compound No. 10-105

¹H-NMR(200MHz, CDCl₃) δppm: 1.53-1.70 (1H, m), 1.95-2.12 (1H, m), 2.46-2.66 (3H, m), 3.41-3.49 (1H, m), 3.54 (3H, s), 3.71-3.93 (3H, m), 4.82 (2H, s), 5.79 (1H, s), 7.06-7.10 (2H, m), 7.20-7.67 (7H, m).
MS m/z: 416(M⁺), 388, 373, 346, 137.

### (Example 435)

### Compound of Compound No. 10-109

¹H-NMR(200MHz, CDCl₃) δppm: 1.21-1.41 (2H, m), 1.52-2.00 (3H, m), 1.89 (3H, s), 2.48 (2H, d, J=7.3Hz), 3.36 (2H, dt, J=2.2, 11.7Hz), 3.46 (3H, s), 3.96 (2H, dd, J=2.6, 11.7Hz), 4.40-4.60 (1H, m), 4.90-5.10 (1H, m), 5.65 (1H, s), 7.43-7.63 (4H, m).
MS m/z: 352(M⁺), 268, 116.

### (Example 436)

### Compound of Compound No. 10-114

¹H-NMR(200MHz, CDCl₃) δppm: 1.22-1.43 (2H, m), 1.55-1.62 (2H, m), 1.73-1.86 (1H, m), 2.49 (2H, d, J=7.0Hz), 3.36 (2H, dt, J=2.2, 11.5Hz), 3.55 (3H, s), 3.95 (2H, dd, J=2.9, 11.5Hz), 4.82 (2H, s), 5.77 (1H, s), 7.06-7.67 (9H, m).
MS m/z: 430(M⁺), 387, 346, 116.

### (Example 437)

### Compound of Compound No. 10-115

¹H-NMR(200MHz, CDCl₃) δppm: 1.21-1.39 (2H, m), 1.41-1.57 (2H, m), 1.69-1.85 (1H, m), 2.48 (2H, d, J=7.0Hz), 3.25-3.45 (2H, m), 3.37 (3H, s), 3.48 (3H, s), 3.60-4.00 (4H, m), 4.46 (1H, d, J=14.8Hz), 5.10 (1H, d, J=14.8Hz), 5.64 (1H, s), 7.40-7.65 (4H, m) .
MS m/z: 382(M⁺), 339, 298, 116.

### (Example 438)

### Compound of Compound No. 10-116

¹H-NMR(200MHz, CDCl₃) δppm: 1.16-1.36 (2H, m), 1.44-1.50 (2H, m), 1.65-1.78 (1H, m), 2.46 (2H, d, J=7.0Hz), 3.30 (2H, dt, J=2.2, 11.3Hz), 3.53 (3H, s), 3.90 (2H, dd, J=2.9, 11.3Hz), 4.30-4.55 (3H, m), 5.19 (1H, d, J=14.3Hz), 5.67 (1H, s), 6.75 (1H, d, J=7.5Hz), 6.97 (1H, t, J=7.5Hz), 7.25 (1H, t, J=7.5Hz), 7.43-7.64 (4H, m).
MS m/z: 444(M⁺), 401, 360, 116.

### (Example 439)

### Compound of Compound No. 10-117

¹H-NMR(200MHz, CDCl₃) δppm: 1.21-1.41 (2H, m), 1.53-1.60 (2H, m), 1.70-1.80 (1H, m), 2.46 (2H, d, J=7.0Hz), 3.36 (2H, dt, J=2.2, 11.5Hz), 3.43 (3H, s), 3.78 (3H, s), 3.95 (2H, dd, J=2.6, 11.5Hz), 4.73 (2H, s), 5.63 (1H, s), 7.40-7.65 (4H, m).
MS m/z: 368(M⁺), 284, 247.

### (Example 440)

### Compound of Compound No. 10-118

¹H-NMR(200MHz, CDCl₃) δppm: 1.22-1.41 (2H, m), 1.51-1.57 (2H, m), 1.72-1.83 (1H, m), 2.48 (2H, d, J=7.0Hz), 3.35 (2H, dt, J=2.2, 11.7Hz), 3.69 (3H, s), 3.94 (2H, dd, J=2.9, 11.7Hz), 4.45 (1H, d, J=14.1Hz), 4.61 (2H, s), 5.16 (1H, d, J=14.1Hz), 5.75 (1H, s), 6.85-6.95 (2H, m), 7.39-7.66 (5H, m), 8.05-8.09 (1H, m).
MS m/z: 445(M⁺), 382, 136, 116.

### (Example 441)

### Compound of Compound No. 10-119

¹H-NMR(200MHz, CDCl₃) δppm: 1.23-1.41 (2H, m), 1.51-1.57 (2H, m), 1.70-1.80 (1H, m), 2.17 (3H, s), 2.48 (2H, d, J=7.0Hz), 3.36 (2H, dt, J=2.2, 11.5Hz), 3.56 (3H, s), 3.95 (2H, dd, J=2.6, 11.5Hz), 4.25 (1H, d, J=12.7Hz), 4.35 (1H, d J=12.7Hz), 4.46 (1H, d, J=14.1Hz), 5.10 (1H, d, J=14.1Hz), 5.70 (1H, s), 7.40-7.65 (9H, m).
MS m/z: 418(M⁺), 326, 116.

### (Example 442)

### Compound of Compound No. 10-120

¹H-NMR(200MHz, CDCl₃) δppm: 1.23-1.42 (2H, m), 1.54-1.60 (2H, m), 1.71-1.84 (1H, m), 2.50 (2H, d, J=7.3Hz), 3.09 (3H, s), 3.38 (2H, dt, J=2.2, 11.7Hz), 3.50 (2H, s), 3.97 (2H, dd, J=2.6, 11.7Hz), 4.33 (1H, d, J=14.3Hz), 5.15 (1H, d, J=14.3Hz), 5.62 (1H, s), 6.96-7.01 (2H, m), 7.23-7.61 (7H, m).
MS m/z: 428(M⁺), 385, 344, 226, 116.

### (Example 443)

### Compound of Compound No. 10-121

¹H-NMR(200MHz, CDCl₃) δppm: 1.24-1.43 (2H, m), 1.54-1.60 (2H, m), 1.73-1.84 (1H, m), 2.49 (2H, d, J=7.0Hz), 3.12 (3H, s), 3.37 (2H, dt, J=2.2, 11.4Hz), 3.47 (2H, s), 3.76 (3H, s), 3.97 (2H, dd, J=3.1, 11.4Hz), 4.32 (1H, d, J=14.1Hz), 5.15 (1H, d, J=14.1Hz), 5.63 (1H, s), 6.54-6.56 (2H, m), 6.76-6.81 (1H, t, m), 7.18 (1H, t, J=8.1Hz), 7.41-7.61 (4H, m).
MS m/z: 458(M⁺), 415, 374, 226, 124.

### (Example 444)

### Compound of Compound No. 10-122

¹H-NMR(200MHz, CDCl₃) δppm: 1.17-1.38 (2H, m), 1.45-1.50 (2H, m), 1.66-1.81 (1H, m), 2.47 (2H, d, J=7.0Hz), 3.32 (2H, dt, J=2.2, 11.7Hz), 3.53 (3H, s), 3.92 (2H, dd, J=2.6, 11.7Hz), 4.20-4.50 (3H, m), 5.17 (1H, d, J=14.7Hz), 5.67 (1H, s), 6.72 (2H, dd, J=4.5, 9.5Hz), 6.94 (2H, t, J=4.5Hz), 7.40-7.65 (4H, m).
MS m/z: 462(M⁺), 419, 378, 116.

### (Example 445)

### Compound of Compound No. 10-123

¹H-NMR(200MHz, CDCl₃) δppm: 1.18-1.38 (2H, m), 1.44-1.50 (2H, m), 1.67-1.80 (1H, m), 2.47 (2H, d, J=7.3Hz), 3.31 (2H, dt, J=2.2, 11.7Hz), 3.54 (3H, s), 3.92 (2H, dd, J=2.2, 11.7Hz), 4.25-4.50 (3H, m), 5.18 (1H, d, J=15.4Hz), 5.67 (1H, s), 6.40-6.80 (3H, m), 7.19 (1H, t, J=7.5Hz), 7.41-7.65 (4H, m).
MS m/z: 462(M⁺), 378, 116.

### (Example 446)

### Compound of Compound No. 10-124

¹H-NMR(200MHz, CDCl₃) δppm: 1.17-1.38 (2H, m), 1.46-1.52 (2H, m), 1.67-1.80 (1H, m), 2.47 (2H, d, J=7.0Hz), 3.32 (2H, dt, J=2.2, 11.7Hz), 3.53 (3H, s), 3.92 (2H, dd, J=2.9, 11.7Hz), 4.30-4.60 (3H, m), 5.16 (1H, d, J=13.9Hz), 5.67 (1H, s), 6.70-6.90 (3H, m), 7.38-7.65 (4H, m).
MS m/z: 480(M⁺), 396, 116.

### (Example 447)

### Compound of Compound No. 10-125

¹H-NMR(200MHz, CDCl₃) δppm: 1.17-1.38 (2H, m), 1.45-1.50 (2H, m), 1.67-1.84 (1H, m), 2.47 (2H, d, J=7.0Hz), 3.32 (2H, dt, J=2.2, 11.7Hz), 3.55 (3H, s), 3.92 (2H, dd, J=3.7, 11.7Hz), 4.27-4.50 (3H, m), 5.19 (1H, d, J=13.9Hz), 5.69 (1H, s), 6.65-6.74 (2H, m), 6.93-6.98 (1H, m), 7.18 (1H, t, J=8.1Hz), 7.41-7.65 (4H, m).
MS m/z: 480(M⁺+2), 478(M⁺), 394, 116.

### (Example 448)

### Compound of Compound No. 10-126

¹H-NMR(200MHz, CDCl₃) δppm: 1.19-1.39 (2H, m), 1.49-1.56 (2H, m), 1.68-1.81 (1H, m), 2.46 (2H, d, J=7.0Hz), 3.35 (2H, dt, J=2.2, 11.7Hz), 3.43-3.54 (2H, m), 3.49 (3H, s), 3.94 (2H, dd, J=2.6, 11.7Hz), 4.34 (1H, d, J=14.3Hz), 5.18 (1H, d, J=14.3Hz), 5.55 (1H, s), 7.22-7.63 (9H, m).
MS m/z: 460(M⁺), 417, 376, 116.

### (Example 449)

### Compound of Compound No. 10-127

¹H-NMR(200MHz, CDCl₃) δppm: 1.19-1.88 (5H, m), 2.45 (2H, d, J=7.0Hz), 3.29-3.42 (2H, m), 3.34 (3H, bs), 3.77-3.97 (2H, m), 3.83 (3H, s), 3.88 (3H, s), 4.72 (2H, s), 5.13 (2H, s), 5.62 (1H, s), 6.78 (1H, bs), 6.83 (2H, bs), 7.41-7.62 (4H, m).
MS m/z: 504(M⁺), 460, 376, 310, 226, 151, 116.

### (Example 450)

### Compound of Compound No. 10-128

¹H-NMR(200MHz, CDCl₃) δppm: 1.18-1.85 (5H, m), 2.44 (2H, d, J=7.0Hz), 3.27-3.39 (2H, m), 3.44 (3H, s), 3.85 (3H, s), 3.86 (3H, s), 3.88-3.97 (2H, m), 4.34 (2H, d, J=5.5Hz), 4.65 (2H, t, J=5.5Hz), 4.70 (1H, bs), 5.66 (1H, s), 6.72-6.82 (3H, m), 7.38-7.61 (4H, m).
MS m/z: 503(M⁺), 460, 419, 309, 253, 226, 193, 166, 151, 136, 116, 107, 83.

### (Example 451)

### Compound of Compound No. 10-129

¹H-NMR(200MHz, CDCl₃) δppm: 1.19-1.90 (5H, m), 2.46 (2H, d, J=7.0Hz), 3.28-3.41 (2H, m), 3.34 (3H, s), 3.91-4.09 (2H, m), 4.72 (2H, s), 5.14 (2H, s), 5.62 (1H, s), 6.97-7.64 (8H, m).
MS m/z: 462(M⁺), 419, 378, 334, 309, 109, 83.

### (Example 452)

### Compound of Compound No. 10-130

¹H-NMR(200MHz, CDCl₃) δppm: 1.18-1.85 (5H, m), 2.45 (2H, d, J=7.0Hz), 3.27-3.40 (2H, m), 3.44 (3H, s), 3.89-3.97 (2H, m), 4.36 (2H, d, J=5.9Hz), 4.69 (2H, t, J=5.9Hz), 4.75 (1H, bs), 5.66 (1H, s), 6.96-7.62 (8H, m).
MS m/z: 461(M⁺), 418, 404, 377, 309, 267, 253, 226, 136, 117, 109, 83, 66, 55.

### (Example 453)

### Compound of Compound No. 10-131

¹H-NMR(200MHz, CDCl₃) δppm: 1.19-1.90 (5H, m), 2.46 (2H, d, J=7.0Hz), 3.29-3.41 (2H, m), 3.35 (3H, s), 3.91-3.98 (2H, m), 4.72 (2H, s), 5.21 (2H, s), 5.63 (1H, s), 7.24-7.64 (6H, m), 8.54-8.59 (2H, m).
MS m/z: 445(M⁺), 402, 388, 374, 361, 353, 309, 225, 116, 92, 83.

### (Example 454)

### Compound of Compound No. 10-132

¹H-NMR(200MHz, CDCl₃) δppm: 1.22-1.90 (5H, m), 2.50 (2H, d, J=7.0Hz), 3.36 (2H, dt, J=2.2, 11.7Hz), 3.51 (3H, s), 3.95 (2H, dd, J=2.9, 11.4Hz), 4.52 (2H, d, J=4.8Hz), 4.75 (1H, bs), 5.74 (2H, m), 5.74 (1H, s), 7.13-7.69 (7H, m), 8.38 (1H, dd, J=0.7, 4.0Hz).
MS m/z: 444(M⁺), 402, 387, 360, 310, 226, 135, 116, 92.

### (Example 455)

### Compound of Compound No. 10-133

¹H-NMR(200MHz, CDCl₃) δppm: 1.23-1.90 (5H, m), 2.48 (2H, d, J=7.0Hz), 3.36 (2H, dt, J=2.2, 11.7Hz), 3.60 (3H, s), 3.95 (2H, dd, J=2.9, 11.4Hz), 4.83 (2H, bs), 5.22 (1H, s), 7.00-8.40 (8H, m).
MS m/z: 431(M⁺), 388, 374, 347, 293, 220, 211, 164, 136, 116, 89.

### (Example 456)

### Compound of Compound No. 10-134

¹H-NMR(200MHz, CDCl₃) δppm: 1.23-1.92 (5H, m), 2.52 (2H, d, J=7.0Hz), 3.37 (2H, dt, J=2.2, 11.7Hz), 3.58 (3H, s), 3.95 (2H, dd, J=2.9, 11.4Hz), 4.80 (2H, m), 5.86 (1H, s), 7.29-7.81 (7H, m), 8.61-8.64 (1H, m).
MS m/z: 447(M⁺), 404, 390, 363, 309, 253, 226, 193, 136, 116, 89.

### (Example 457)

### Compound of Compound No. 10-135

¹H-NMR(200MHz, CDCl₃) δppm: 1.21-1.90 (5H, m), 2.49 (2H, d, J=7.0Hz), 3.35 (2H, dt, J=2.2, 11.7Hz), 3.61 (3H, s), 3.94 (2H, dd, J=2.6, 11.4Hz), 4.82 (2H, bs), 5.76 (1H, s), 7.16-7.68 (8H, m).
MS m/z: 466(M⁺+2), 464(M⁺), 421, 407, 393, 380, 164, 137, 116.

### (Example 458)

### Compound of Compound No. 10-136

¹H-NMR(200MHz, CDCl₃) δppm: 1.22-1.91 (5H, m), 2.49 (2H, d, J=7.0Hz), 3.37 (2H, dt, J=2.2, 11.7Hz), 3.54 (3H, s), 3.96 (2H, dd, J=2.9, 11.7Hz), 4.81 (2H, bs), 5.76 (1H, s), 7.05 (4H, d, J=6.2Hz), 7.43-7.67 (4H, m).
MS m/z: 448(M⁺), 405, 391, 377, 364, 164, 136, 116, 83.

### (Example 459)

### Compound of Compound No. 10-137

¹H-NMR(200MHz, CDCl₃) δppm: 1.22-1.90 (5H, m), 2.47 (2H, d, J=7.0Hz), 3.34 (2H, dt, J=2.2, 11.7Hz), 3.85 (3H, bs), 3.92 (2H, dd, J=2.9, 11.7Hz), 4.85-5.18 (2H, m), 5.85 (1H, s), 7.43-8.21 (9H, m), 9.01 (1H, bs).
MS m/z: 481(M⁺), 438, 424, 397, 353, 308, 295, 259, 220, 172, 158.

### (Example 460)

### Compound of Compound No. 10-138

¹H-NMR(200MHz, CDCl₃) δppm: 1.21-1.91 (5H, m), 2.43 (3H, s), 2.48 (2H, d, J=7.3Hz), 3.36 (2H, dt, J=1.8, 11.7Hz), 3.51 (3H, s), 3.94 (2H, dd, J=3.3, 11.7Hz), 4.80 (2H, bs), 5.76 (1H, s), 6.13 (1H, bs), 7.44-7.69 (4H, m).
MS m/z: 435(M⁺), 392, 378, 364, 351, 337, 309, 293, 251, 226, 190.

### (Example 461)

### Compound of Compound No. 10-139

¹H-NMR(200MHz, CDCl₃) δppm: 1.89 (3H, s), 3.46 (3H, s), 4.10 (2H, s), 4.55 (1H, bs), 4.95 (1H, bs), 5.73 (1H, s), 6.82-6.84 (1H, m), 6.91-6.96 (1H, m), 7.15-7.19 (1H, m), 7.37-7.62 (4H, m).
MS m/z: 350(M⁺), 308, 206, 192, 149.

### (Example 462)

### Compound of Compound No. 10-144

¹H-NMR(200MHz, CDCl₃) δppm: 3.55 (3H, s), 4.12 (2H, s), 4.80 (2H, s), 5.84 (1H, s), 6.84-6.86 (1H, m), 6.92-6.96 (1H, m), 7.05-7.09 (2H, m), 7.16-7.65 (8H, m).
MS m/z: 428(M⁺), 335, 219, 204, 136.

### (Example 463)

### Compound of Compound No. 10-145

¹H-NMR(200MHz, CDCl₃) δppm: 3.35 (3H, s), 3.48 (3H, s), 3.66 (1H, bd), 3.85 (1H, bd), 4.11 (2H, s), 4.47 (1H, d, J=15.2Hz), 5.04 (1H, d, J=15.2Hz), 5.71 (1H, s), 6.82-6.84 (1H, m), 6.91-6.96 (1H, m), 7.16-7.19 (1H, m), 7.37-7.62 (4H, m).
MS m/z: 380(M⁺), 350, 319, 307, 218.

### (Example 464)

### Compound of Compound No. 10-146

¹H-NMR(200MHz, CDCl₃) δppm: 3.52 (3H, s), 4.10 (2H, s), 4.28-4.52 (3H, m), 5.13 (1H, d, J=13.9Hz), 5.74 (1H, s), 6.75-6.80 (3H, m), 6.89-7.02 (2H, m), 7.14-7.63 (7H, m).
MS m/z: 442(M⁺), 380, 321, 136, 116.

### (Example 465)

### Compound of Compound No. 10-147

¹H-NMR(200MHz, CDCl₃) δppm: 3.43 (3H, s), 3.77 (3H, s), 4.09 (2H, s), 4.70 (2H, s), 5.70 (1H, s), 6.82-6.84 (1H, m), 6.91-6.95 (1H, m), 7.15-7.18 (1H, m), 7.38-7.63 (4H, m).
MS m/z: 366(M⁺), 250, 218, 149, 116.

### (Example 466)

### Compound of Compound No. 10-148

¹H-NMR(200MHz, CDCl₃) δppm: 1.89 (3H, s), 3.46 (3H, s), 3.91 (2H, s), 4.56 (1H, bs), 4.93 (1H, bs), 5.65 (1H, s), 6.90-6.98 (2H, m), 7.26-7.30 (1H, m), 7.37-7.63 (4H, m).
MS m/z: 350(M⁺), 308, 233, 219, 206.

### (Example 467)

### Compound of Compound No. 10-154

¹H-NMR(200MHz, CDCl₃) δppm: 3.35 (3H, s), 3.48 (3H, s), 3.67 (1H, bd), 3.85 (1H, bd), 3.91 (2H, s), 4.48 (1H, d, J=14.7Hz), 5.04 (1H, d, J=14.7Hz), 5.64 (1H, s), 6.91 (1H, d, J=5.1Hz), 6.96-6.97 (1H, m), 7.27-7.30 (1H, m), 7.37-7.62 (4H, m).
MS m/z: 380(M⁺), 365, 350, 319, 307.

### (Example 468)

### Compound of Compound No. 10-155

¹H-NMR(200MHz, CDCl₃) δppm: 3.53 (3H, s), 3.90 (2H, s), 4.28-4.49 (3H, m), 5.13 (1H, d, J=14.3Hz), 5.66 (1H, s), 6.73-6.78 (2H, m), 6.83-6.89 (2H, m), 6.99 (1H, t, J=7.3Hz), 7.23-7.63 (7H, m).
MS m/z: 442(M⁺), 380, 349, 307, 218.

### (Example 469)

### Compound of Compound No. 10-165

¹H-NMR(200MHz, CDCl₃) δppm: 1.06 (6H, s), 2.69 (2H, s), 3.43 (3H, s), 3.78 (3H, s), 4.73 (2H, s), 5.67 (1H, s), 7.44-7.64 (4H, m).
MS m/z: 394(M⁺), 284, 137, 116.

### (Example 470)

### Compound of Compound No. 10-171

¹H-NMR(200MHz, CDCl₃) δppm: 0.93 (3H, d, J=6.8Hz), 1.05 (3H, d, J=6.8Hz), 2.10-2.25 (1H, m), 3.59 (3H, s), 4.84 (2H, s), 5.47 (1H, d, J=7.3Hz), 6.02 (1H, s), 7.06-7.66 (9H, m).
MS m/z: 387, 116.

### (Example 471)

### Compound of Compound No. 10-172

¹H-NMR(200MHz, CDCl₃) δppm: 0.86 (3H, d, J=6.8Hz), 0.95 (3H, d, J=6.8Hz), 2.10-2.25 (1H, m), 3.34 (3H, s), 3.50 (3H, s), 3.61-3.70 (1H, m), 3.82-3.85 (1H, m), 4.40-4.55 (1H, m), 5.05-5.10 (1H, m), 5.60 (1H, d, J=7.0Hz), 5.78 (1H, s), 7.43-7.63 (4H, m).
MS m/z: 339, 265, 116.

### (Example 472)

### Compound of Compound No. 10-173

¹H-NMR(200MHz, CDCl₃) δppm: 0.86 (3H, d, J=6.8Hz), 0.94 (3H, d, J=6.8Hz), 2.10-2.25 (1H, m), 2.16 (3H, s), 3.58 (3H, s), 4.27 (2H, s), 4.48 (1H, d, J=14.7Hz), 5.02-5.12 (1H, m), 5.57 (1H, d, J=7.0Hz), 5.87 (1H, s), 7.40-7.65 (4H, m).
MS m/z: 367, 116.

### (Example 473)

### Compound of Compound No. 10-175

¹H-NMR(200MHz, CDCl₃) δppm: 1.20 (6H, d, J=7.0Hz), 1.90 (3H, s), 3.55 (3H, s), 3.55-3.70 (1H, m), 4.68 (1H, bs), 4.91 (1H, bs), 6.47 (1H, s), 7.40-7.65 (4H, m).
MS m/z: 324(M⁺), 281, 239, 227, 210.

### (Example 474)

### Compound of Compound No. 10-184

¹H-NMR(200MHz, CDCl₃) δppm: 1.53 (6H, d, J=7.0Hz), 1.90 (3H, s), 2.35-2.56 (1H, m), 3.52 (3H, s), 4.64 (1H, bs), 4.95 (1H, bs), 6.03 (1H, s), 7.43-7.46 (2H, m), 7.54 (1H, s), 7.60-7.65 (1H, m).
MS m/z: 346(M⁺), 304, 284, 262, 226.

### (Example 475)

### Compound of Compound No. 10-201

¹H-NMR(200MHz, CDCl₃) δppm: 3.04 (2H, d, J=6.6Hz), 3.40 (3H, s), 3.40-3.63 (1H, m), 3.78 (3H, s), 4.72 (2H, s), 5.74 (1H, s), 7.42-7.61 (4H, m).
MS m/z: 434(M⁺), 415, 375, 318, 274.

### (Example 476)

### Compound of Compound No. 10-202

¹H-NMR(200MHz, CDCl₃) δppm: 1.55-1.78 (8H, m), 1.90 (3H, s), 2.62-2.90 (1H, m), 3.51 (3H, s), 4.64 (1H, bs), 4.94 (1H, bs), 6.04 (1H, s), 7.43-7.49 (2H, m), 7.54 (1H, s), 7.58-7.65 (1H, m).
MS m/z: 372(M⁺), 352, 330, 304, 289.

### (Example 477)

### Compound of Compound No. 10-211

¹H-NMR(200MHz, CDCl₃) δppm: 1.52-2.06 (8H, m), 1.89 (3H, s), 2.90-3.08 (1H, m), 3.44 (3H, s), 4.58 (1H, bs), 4.95 (1H, bs), 5.69 (1H, s), 7.39-7.63 (4H, m).
MS m/z: 322(M⁺), 294, 281, 252, 239.

### (Example 478)

### Compound of Compound No. 10-216

¹H-NMR(200MHz, CDCl₃) δppm. 1.55-1.76 (6H, m), 1.96-2.10 (2H, m), 2.93-3.08 (1H, m), 3.54 (3H, s), 4.81 (2H, s), 5.80 (1H, s), 7.06-7.68 (9H, m).
MS m/z: 400 (M⁺), 372, 359, 307, 239.

### (Example 479)

### Compound of Compound No. 10-217

¹H-NMR(200MHz, CDCl₃) δppm: 1.52-1.76 (6H, m), 1.95-2.08 (2H, m), 2.89-3.06 (1H, m), 3.37 (3H, s), 3.44 (3H, s), 3.68 (1H, d, J=15.8Hz), 3.88 (1H, d, J=15.8Hz), 4.50 (1H, d, J=14.7Hz), 5.03 (1H, d, J=14.7Hz), 5.67 (1H, s), 7.39-7.63 (4H, m).
MS m/z: 352(M⁺), 324, 311, 279, 237.

### (Example 480)

### Compound of Compound No. 10-218

¹H-NMR(200MHz, CDCl₃) δppm: 1.45-1.73 (6H, m), 1.93-2.07 (2H, m), 2.90-3.06 (1H, m), 3.50 (3H, s), 4.29-4.53 (3H, m), 5.10 (1H, d, J=13.4Hz), 5.70 (1H, s), 6.76-6.80 (2H, m), 6.93-6.97 (1H, m), 7.21-7.68 (6H, m).
MS m/z: 414 (M⁺), 373, 321, 293, 279.

### (Example 481)

### Compound of Compound No. 10-219

¹H-NMR(200MHz, CDCl₃) δppm: 1.52-1.82 (6H, m), 1.95-2.05 (2H, m), 2.93-3.06 (1H, m), 3.42 (3H, s), 3.77 (3H, s), 4.72 (2H, s), 5.66 (1H, s), 7.40-7.64 (4H, m).
MS m/z: 338 (M⁺), 310, 297, 150, 116.

### (Example 482)

### Compound of Compound No. 11-3

¹H-NMR(200MHz, CDCl₃) δppm: 0.92-1.13 (4H, m), 1.07 (3H, d, J=5.1Hz), 1.68-1.78 (1H, m), 2.40-2.62 (2H, m), 2.86-3.00 (1H, m), 3.56 (3H, s), 4.29 (2H, s), 4.42-4.50 (1H, m), 5.04-5.11 (1H, m), 5.75 (1H, s), 7.40-7.65 (4H, m).
MS m/z: 448(M⁺), 352, 322, 224, 137.

### (Example 483)

### Compounds of Compound No. 11-10

¹H-NMR(200MHz, CDCl₃) δppm: 1.05-1.25 (2H, m), 1.45-1.80 (6H, m), 1.92-2.05 (1H, m), 2.16 (3H, s), 2.53 (2H, d, J=7.7Hz), 3.56 (3H, s), 4.25 (1H, d, J=11.2Hz), 4.35 (1H, d, J=11.2Hz), 4.45 (1H, d, J=14.5Hz), 5.39 (1H, d, J=14.5Hz), 5.72 (1H, s), 7.40-7.64 (4H, m).
MS m/z: 394(M⁺), 326, 116.

### (Example 484)

### Compound of Compound No. 11-11

¹H-NMR(200MHz, CDCl₃) δppm: 1.05-1.25 (2H, m), 1.45-1.80 (6H, m), 2.10-2.25 (1H, m), 2.52 (2H, d, J=7.3Hz), 3.23 (1H, bs), 3.45 (3H, s), 3.72 (1H, bs), 3.93 (1H, bs), 4.56 (1H, bs), 5.06 (1H, bs), 5.66 (1H, s), 7.40-7.65 (4H, m).
MS m/z: 352(M⁺), 284, 226, 116.

### (Example 485)

### Compound of Compound No. 11-12

¹H-NMR(200MHz, CDCl₃) δppm: 0.90-1.00 (2H, m), 1.02-1.25 (4H, m), 1.45-1.80 (7H, m), 2.10-2.25 (1H, m), 2.53 (2H, d, J=7.3Hz), 3.55 (3H, s), 4.31 (1H, s), 4.44 (1H, d, J=14.3Hz), 5.11 (1H, d, J=14.3Hz), 5.71 (1H, s), 7.40-7.65 (4H, m).
MS m/z: 420(M⁺), 352, 116.

### (Example 486)

### Compound of Compound No. 11-16

¹H-NMR(200MHz, CDCl₃) δppm: 1.05-1.25 (2H, m), 1.45-1.80 (6H, m), 2.10-2.25 (1H, m), 2.53 (2H, d, J=7.7Hz), 3.59 (3H, s), 4.83 (2H, s), 5.80 (1H, s), 7.00-7.04 (1H, m), 7.20-7.28 (1H, m), 7.43-7.68 (4H, m), 7.74-7.83 (1H, m), 8.37-8.40 (1H, m). MS m/z: 415(M⁺), 347, 136, 116.

### (Example 487)

### Compound of Compound No. 11-19 (mixture of 2 diastereomers)

¹H-NMR(200MHz, CDCl₃) δppm: 0.90 (6H, d, J=6.6Hz), 0.98 (2H, t, J=6.2Hz), 1.20-1.35 (2H, m), 1.35-1.50 (1H, m), 1.78-1.97 (1H, m), 2.43 (2H, d, J=7.3Hz), 3.45 (3H, s), 4.59, 4.62 (1H, d, J=14.0Hz), 4.95, 4.97 (1H, d, J=14.0Hz), 5.73 (1H, s), 7.37-7.62 (4H, m).
MS m/z: 350(M⁺), 335, 308, 268, 226, 116.

### (Example 488)

### Compound of Compound No. 11-20

¹H-NMR(200MHz, CDCl₃) δppm: 0.80-0.97 (9H, m), 1.82-1.97 (1H, m), 2.25-2.48 (2H, m), 3.71 (3H, s), 4.09 (1H, d, J=14.0Hz), 6.04 (1H, d, J=14.0Hz), 5.99, 6.08 (1H, s), 7.34-7.58 (4H, m).
MS m/z: 420(M⁺+2), 418(M⁺), 405, 403, 378, 376, 322, 280, 267, 116.

### (Example 489)

### Compound of Compound No. 11-21

¹H-NMR(200MHz, CDCl₃) δppm: 0.90 (6H, d, J=6.6Hz), 1.79-1.92 (1H, m), 2.42 (2H, d, J=7.0Hz), 3.58 (3H, s), 4.21 (2H, s), 4.39-4.50 (3H, m), 5.10 (1H, d, J=14.0Hz), 5.72 (1H, s), 7.40-7.62 (4H, m).
MS m/z: 402(M⁺), 387, 360, 268, 226, 116.

### (Example 490)

### Compound of Compound No. 11-22

¹H-NMR(200MHz, CDCl₃) δppm: 0.90 (6H, d, J=6.2Hz), 1.78-1.92 (1H, m), 2.42 (2H, d, J=7.0Hz), 3.59 (3H, s), 4.35-4.60 (3H, m), 5.12 (1H, d, J=14.0Hz), 6.08 (1H, s), 7.45-7.64 (4H, m).
MS m/z: 439(M⁺+2), 437(M⁺), 397, 395, 285, 268, 116.

### (Example 491)

### Compound of Compound No. 11-23

¹H-NMR(200MHz, CDCl₃) δppm: 0.90 (6H, d, J=6.6Hz), 1.85 (1H, m), 2.37 (3H, s), 2.41 (2H, d, J=7.3Hz), 3.43 (3H, s), 3.59 (3H, s), 4.45 (1H, d, J=14.5Hz), 5.11 (1H, d, J=14.5Hz), 5.72 (1H, s), 7.38-7.62 (4H, m).
MS m/z: 384(M⁺), 369, 342, 309, 300, 268, 253, 226.

### (Example 492)

### Compound of Compound No. 11-24

¹H-NMR(200MHz, CDCl₃) δppm: 0.90 (6H, d, J=6.6Hz), 1.85 (1H, m), 2.40 (2H, d, J=7.0Hz), 3.24 (2H, s), 3.54 (3H, s), 3.58 (2H, m), 4.46 (1H, d, J=14.3Hz), 5.12 (1H, d, J=14.3Hz), 5.71 (1H, s), 7.41-7.65 (4H, m).
MS m/z: 381(M⁺), 366, 339, 267, 226, 191, 136, 116.

### (Example 493)

### Compound of Compound No. 11-25

¹H-NMR(200MHz, CDCl₃) δppm: 0.91 (6H, d, J=6.6Hz), 1.87 (1H, m), 2.41 (2H, d, J=7.0Hz), 3.38 (3H, s), 4.66 (1H, d, J=13.9Hz), 5.04 (1H, d, J=13.9Hz), 5.64-5.98 (2H, m), 5.70 (1H, s), 5.72 (1H, dd, J=1.8, 10.3Hz), 5.91 (1H, dd, J=10.3, 16.5Hz), 6.51 (1H, dd, J=1.8, 16.5Hz), 7.39-7.62 (4H, m).
MS m/z: 322(M⁺), 280, 267, 226, 149, 116, 89.

### (Example 494)

### Compound of Compound No. 11-26

¹H-NMR(200MHz, CDCl₃) δppm: 0.90 (6H, d, J=7.0Hz), 1.58-1.78 (3H, m), 1.74-1.92 (1H, m), 2.42 (2H, d, J=7.0Hz), 3.46-3.62 (3H, m), 3.92-4.35 (1H, m), 4.34-4.46 (1H, m), 5.12-5.26 (1H, m), 5.61-5.84 (1H, m), 7.35-7.64 (4H, m).
MS m/z: 404(M⁺+2), 402(M⁺), 389, 360, 343, 316, 295, 267, 226, 180, 152.

### (Example 495)

### Compound of Compound No. 11-27

¹H-NMR(200MHz, CDCl₃) δppm: 0.90 (6H, d, J=6.6Hz), 1.85 (1H, m), 2.40 (2H, d, J=7.1Hz), 3.33 (3H, bs), 4.73 (2H, s), 5.35 (2H, s), 5.65 (1H, s), 7.31-7.85 (11H, m).
MS m/z: 452(M⁺), 408, 141, 116.

### (Example 496)

### Compound of Compound No. 11-28

¹H-NMR(200MHz, CDCl₃) δppm: 0.90 (6H, d, J=6.6Hz), 1.86 (1H, m), 2.40 (2H, d, J=7.0Hz), 3.37 (3H, s), 4.74 (2H, s), 5.24 (2H, s), 5.66 (1H, s), 7.28-7.64 (8H, m).
MS m/z: 470(M⁺), 455, 428, 267, 159, 116, 89.

### (Example 497)

### Compound of Compound No. 11-29

¹H-NMR(200MHz, CDCl₃) δppm: 0.90 (6H, d, J=6.6Hz), 1.85 (1H, m), 2.40 (2H, d, J=7.3Hz), 3.35 (3H, bs), 4.72 (2H, s), 5.12 (2H, s), 5.64 (1H, s), 6.99-7.64 (7H, m).
MS m/z: 438(M⁺), 396, 267, 127, 116.

### (Example 498)

### Compound of Compound No. 11-30

¹H-NMR(200MHz, CDCl₃) δppm: 0.90 (6H, d, J=6.6Hz), 1.85 (1H, m), 2.40 (2H, d, J=7.3Hz), 3.36 (3H, s), 4.73 (2H, s), 5.15 (2H, s), 5.64 (1H, s), 7.12-7.64 (8H, m).
MS m/z: 438(M⁺+2), 436(M⁺), 394, 267, 226, 125, 116, 89.

### (Example 499)

### Compound of Compound No. 11-31

¹H-NMR(200MHz, CDCl₃) δppm: 0.90 (6H, d, J=6.6Hz), 1.85 (1H, m), 2.40 (2H, d, J=7.3Hz), 3.34 (3H, bs), 4.72 (2H, s), 5.14 (2H, s), 5.63 (1H, s), 7.16 (2H, d, J=8.4Hz), 7.29 (2H, d, J=8.4Hz), 7.38-7.63 (4H, m).
MS m/z: 438(M⁺+2), 436(M⁺), 394, 350, 267, 125, 116, 89.

### (Example 500)

### Compound of Compound No. 11-32

¹H-NMR(200MHz, CDCl₃) δppm: 0.90 (6H, d, J=6.6Hz), 1.85 (1H, m), 2.40 (2H, d, J=7.3Hz), 3.37 (3H, bs), 4.72 (2H, s), 5.12 (2H, s), 5.64 (1H, s), 7.04-7.61 (7H, m).
MS m/z: 474(M⁺+4), 472(M⁺+2), 470(M⁺), 428, 267, 181, 159.

### (Example 501)

### Compound of Compound No. 11-33

¹H-NMR(200MHz, CDCl₃) δppm: 0.90 (6H, d, J=6.6Hz), 1.85 (1H, m), 2.32 (3H, s), 2.39 (2H, d, J=7.3Hz), 3.34 (3H, bs), 4.73 (2H, s), 5.15 (2H, s), 5.64 (1H, s), 7.04-7.63 (8H, m).
MS m/z: 416(M⁺), 401, 372, 330, 267, 224, 116, 105, 89, 77.

### (Example 502)

### Compound of Compound No. 11-34

¹H-NMR(200MHz, CDCl₃) δppm: 0.89 (6H, d, J=6.6Hz), 1.84 (1H, m), 2.34 (3H, s), 2.39 (2H, d, J=7.0Hz), 3.32 (3H, bs), 4.71 (2H, s), 5.14 (2H, s), 5.62 (1H, s), 7.13 (4H, s), 7.36-7.62 (4H, m).
MS m/z: 416(M⁺), 372, 330, 267, 226, 116, 105, 89, 77.

### (Example 503)

### Compound of Compound No. 11-35

¹H-NMR(200MHz, CDCl₃) δppm: 0.89 (6H, d, J=6.8Hz), 1.84 (1H, m), 2.38 (2H, d, J=7.3Hz), 3.35 (3H, bs), 3.76 (3H, s), 4.73 (2H, s), 5.16 (2H, s), 5.64 (1H, s), 5.65 (1H, s), 6.75-6.85 (3H, m), 7.20-7.63 (5H, m).
MS m/z: 432(M⁺), 388, 346, 267, 226, 121, 91.

### (Example 504)

### Compound of Compound No. 11-36

¹H-NMR(200MHz, CDCl₃) δppm: 0.89 (6H, d, J=6.6Hz), 1.84 (1H, m), 2.38 (2H, d, J=7.3Hz), 3.30 (3H, bs), 3.80 (3H, s), 4.71 (2H, s), 5.12 (2H, s), 5.61 (1H, s), 6.83-7.62 (8H, m).
MS m/z: 432(M⁺), 431, 417, 388, 373, 345, 268, 226, 195, 121, 89.

### (Example 505)

### Compound of Compound No. 11-37

¹H-NMR(200MHz, CDCl₃) δppm: 0.89 (6H, d, J=6.6Hz), 1.84 (1H, m), 2.38 (2H, d, J=7.0Hz), 3.31 (3H, s), 3.82 (3H, s), 3.87 (3H, s), 4.72 (2H, s), 5.13 (2H, s), 5.62 (1H, s), 6.77 (1H, bs), 6.83 (2H, s), 7.37-7.62 (4H, m).
MS m/z: 462(M⁺), 418, 268, 226, 210, 151, 116.

### (Example 506)

### Compound of Compound No. 11-38

¹H-NMR(200MHz, CDCl₃) δppm: 0.89 (6H, d, J=6.6Hz), 1.83 (1H, m), 2.38 (2H, d, J=7.0Hz), 3.36 (3H, bs), 3.80 (6H, s), 3.83 (3H, s), 4.74 (2H, s), 5.12 (2H, s), 5.64 (1H, s), 6.44 (2H, bs), 7.38-7.64 (4H, m).
MS m/z: 492(M⁺), 226, 181, 148, 116.

### (Example 507)

### Compound of Compound No. 11-39

¹H-NMR(200MHz, CDCl₃) δppm: 0.90 (6H, d, J=6.6Hz), 1.85 (1H, m), 2.40 (2H, d, J=7.3Hz), 3.35 (3H, s), 4.73 (2H, s), 5.18 (2H, s), 5.64 (1H, s), 7.14-7.64 (8H, m).
MS m/z: 486(M⁺), 444, 400, 267, 175, 116.

### (Example 508)

### Compound of Compound No. 11-40

¹H-NMR(200MHz, CDCl₃) δppm: 0.90 (6H, d, J=6.6Hz), 1.84 (1H, m), 2.39 (2H, d, J=7.3Hz), 2.47 (3H, s), 3.32 (3H, bs), 4.72 (2H, s), 5.13 (2H, s), 5.63 (1H, s), 7.18 (4H, bs), 7.37-7.62 (4H, m).
MS m/z: 448(M⁺), 404, 267, 226, 203, 137, 116.

### (Example 509)

### Compound of Compound No. 11-41

¹H-NMR(200MHz, CDCl₃) δppm: 0.91 (6H, d, J=6.6Hz), 1.86 (1H, m), 2.39 (2H, d, J=7.3Hz), 3.38 (3H, s), 4.74 (2H, s), 5.27 (2H, s), 5.66 (1H, s), 7.33-8.20 (8H, m).
MS m/z: 447(M⁺), 432, 405, 267, 226, 181, 136, 116, 106, 89, 78.

### (Example 510)

### Compound of Compound No. 11-42

¹H-NMR(200MHz, CDCl₃) δppm: 0.87 (6H, d, J=6.6Hz), 1.82 (1H, m), 2.37 (2H, d, J=7.0Hz), 3.43 (3H, s), 4.36 (2H, d, J=5.9Hz), 4.78 (2H, t, J=5.5Hz), 4.78 (1H, bs), 5.67 (1H, s), 6.95-7.61 (8H, m). MS m/z: 419(M⁺), 377, 268, 253, 226, 152, 137, 117, 109, 89, 66.

### (Example 511)

### Compound of Compound No. 11-43

¹H-NMR(200MHz, CDCl₃) δppm: 0.88 (6H, d, J=7.0Hz), 1.83 (1H, m), 2.38 (2H, d, J=7.0Hz), 3.46 (3H, s), 4.37 (2H, d, J=6.2Hz), 4.75 (1H, bs), 4.84 (2H, t, J=5.9Hz), 5.68 (1H, s), 7.07-7.62 (8H, m).
MS m/z: 437(M⁺+2), 435(M⁺), 393, 268, 253, 226, 152, 137, 125.

### (Example 512)

### Compound of Compound No. 11-44

¹H-NMR(200MHz, CDCl₃) δppm: 0.87 (6H, d, J=7.0Hz), 1.82 (1H, m), 2.37 (2H, d, J=7.0Hz), 3.43 (3H, s), 4.36 (2H, d, J=5.5Hz), 4.80 (3H, bs), 5.67 (1H, s), 7.15 (2H, d, J=8.4Hz), 7.27 (2H, d, J=8.4Hz), 7.28-7.61 (4H, m).
MS m/z: 437(M⁺+2), 435(M⁺), 393, 268, 226, 152, 137, 125, 116.

### (Example 513)

### Compound of Compound No. 11-45

¹H-NMR(200MHz, CDCl₃) δppm: 0.88 (6H, d, J=7.0Hz), 1.83 (1H, m), 2.38 (2H, d, J=7.0Hz), 3.45 (3H, s), 4.34 (2H, d, J=6.2Hz), 4.70 (1H, bs), 4.91 (2H, t, J=5.9Hz), 5.68 (1H, s), 7.04-7.62 (7H, m).
MS m/z: 473(M⁺+4), 471(M⁺+2), 469(M⁺), 427, 268, 253, 226.

### (Example 514)

### Compound of Compound No. 11-46

¹H-NMR(200MHz, CDCl₃) δppm: 0.87 (6H, d, J=6.6Hz), 1.82 (1H, m), 2.32 (3H, s), 2.36 (2H, d, J=7.3Hz), 3.43 (3H, s), 4.36 (2H, d, J=5.5Hz), 4.70 (2H, t, J=5.5Hz), 4.75 (1H, bs), 5.66 (1H, s), 7.10 (4H, s), 7.37-7.60 (4H, m).
MS m/z: 415(M⁺), 373, 268, 253, 226, 152, 116, 105.

### (Example 515)

### Compound of Compound No. 11-47

¹H-NMR(200MHz, CDCl₃) δppm: 0.88 (6H, d, J=7.0Hz), 1.83 (1H, m), 2.38 (2H, d, J=7.0Hz), 3.45 (3H, s), 4.45 (2H, d, J=5.9Hz), 4.80 (1H, bs), 4.85 (2H, t, J=5.9Hz), 5.68 (1H, s), 7.31-7.61 (8H, m).
MS m/z: 469(M⁺), 427, 268, 253, 226, 159, 137, 116, 89, 66.

### (Example 516)

### Compound of Compound No. 11-48

¹H-NMR(200MHz, CDCl₃) δppm: 0.87 (6H, d, J=6.6Hz), 1.81 (1H, m), 2.36 (2H, d, J=7.3Hz), 3.36 (3H, s), 3.42 (3H, s), 4.33 (2H, d, J=5.9Hz), 4.67 (2H, t, J=5.5Hz), 4.75 (1H, bs), 5.65 (1H, s), 6.98 (4H, dd, J=8.6, 60.2Hz s), 7.37-7.60 (4H, m).
MS m/z: 431(M⁺), 389, 268, 226, 162, 136, 121, 77.

### (Example 517)

### Compound of Compound No. 11-49

¹H-NMR(200MHz, CDCl₃) δppm: 0.87 (6H, d, J=7.0Hz), 1.82 (1H, m), 2.37 (2H, d, J=7.3Hz), 3.43 (3H, s), 3.85 (3H, s), 3.86 (3H, s), 4.34 (2H, d, J=5.9Hz), 4.67 (2H, t, J=5.5Hz), 4.75 (1H, bs), 5.65 (1H, s), 6.72-6.83 (3H, m), 7.37-7.61 (4H, m).
MS m/z: 461(M⁺), 268, 226, 210, 193, 166, 151, 116, 107.

### (Example 518)

### Compound of Compound No. 11-50

¹H-NMR(200MHz, CDCl₃) δppm: 0.89 (6H, d, J=7.0Hz), 1.84 (1H, m), 2.39 (2H, d, J=7.3Hz), 3.47 (3H, s), 4.49 (2H, d, J=6.2Hz), 4.75 (1H, bs), 4.97 (2H, t, J=5.9Hz), 5.70 (1H, s), 7.37-8.21 (8H, m).
MS m/z: 446(M⁺), 416, 404, 268, 253, 226, 152, 136, 116, 106, 89.

### (Example 519)

### Compound of Compound No. 11-51

¹H-NMR(200MHz, CDCl₃) δppm: 0.88 (6H, d, J=6.6Hz), 1.82 (1H, m), 2.31 (2H, m), 2.37 (2H, d, J=7.0Hz), 2.90 (2H, t, J=7.3Hz), 3.30 (3H, s), 3.82 (3H, s), 3.85 (3H, s), 4.42 (1H, d, J=14.3Hz), 5.06 (1H, d, J=14.3Hz), 5.42 (1H, s), 6.62-6.78 (3H, m), 7.33-7.61 (4H, m).
MS m/z: 460(M⁺), 268, 226, 193, 151, 116.

### (Formulation Example 1)

### Dust formulation

After mixing the compound (0.5 parts by mass) obtained in Example 1, hymexazol (free acid, 4 parts by mass), "CARPLEX #80-D" (product of Shionogi & Co., Ltd., 5 parts by mass) and "Keiwa Kure fuhi" (product of Keiwa Rozai Co., Ltd., 90.5 parts by mass), the mixture was pulverized in "Eck Sample Mill Type KII-1" (product of Fuji Paudal Co., Ltd.), whereby a dust formulation was obtained.

### (Formulation Example 2)

### Emulsion

The compound (10 parts by mass) obtained in Example 2 was dissolved in a mixed solution of xylene (product of Wako Pure Chemicals, 40 parts by mass) and DMSO (product of Wako Pure Chemicals, 35 parts by mass). The resulting solution was added to "Parakol KPS" (product of Nippon Nyukazai Co., Ltd., 25 parts by mass), followed by mixing, whereby an emulsifiable concentrate was obtained.

### (Formulation Example 3)

### Wettable powder

The compound (10 parts by mass) obtained in Example 3, "CARPLEX #80-D" (product of Shionogi & Co., Ltd., 10 parts by mass), "Gohsenol GL05" (product of the Nippon Synthetic Chemical Industry Co., Ltd., 2 parts by mass), "Newcole 291PG" (product of Nippon Nyukazai Co., Ltd., 0.5 part by mass), "Neogen Powder" (product of Daiichi Kogyo Seiyaku Co., Ltd., 5 parts by mass), "Radiolite #200" (product of Showa Chemical Industry, 10 parts by mass) and "H bifun" (product of Keiwa Rozai Co., Ltd., 62.5 parts by mass) were mixed sufficiently, followed by pulverization in "Eck Sample Mill Type KII-1" (product of Fuji Paudal), whereby a wettable powder was obtained.

### (Formulation Example 4)

### Granules

The compound (2 parts by mass) obtained in Example 4, sodium tripolyphosphate (product of Mitsui Chemical, 2 parts by mass), "AMYCOL No. 1" (product of Nippon Starch Chemical, 1.5 parts by mass), bentonite (product of Houjun Kogyo, 25 parts by mass) and "Calcium Carbonate Karuhin 600" (product of Adachi Sekkai, 69.5 parts by mass) were mixed. The resulting mixture was subjected to extrusion granulation by using "Dome Gran" (product of Fuji Paudal, screen: 0.9 mmφ). The granulate was then dried in a shelf type drier (product of TABAI, "PERFECT OVEN PS-222", 60°C) and sieved to the granule size between 600 and 1180 µm, whereby granules were obtained.

### (Test 1)

### Control test of tomato late blight (curative effect)

A spore suspension of fungus was sprayed and inoculated to the 3 to 5 leaf stage of a test plant (tomato: Oogata-fukuju) cultivated in a pot. The plant was placed in an inoculation chamber having a room temperature set at 20 to 22°C to promote the onset of the disease. An emulsifiable concentrate prepared in accordance with Formulation Example 2 was diluted with water to prepare a spray solution containing 300 ppm of the invention compound. One day after inoculation, the spray solution was applied uniformly to the test plant in the pot by a spray gun. The degree of disease incidence 7 days after inoculation was studied. The test was conducted in two pots. As a comparative product, 2-chloro-N-(3-methylbenzyl)-N-(1-methylpyrazol-5-yl)acetamide (Comparative compound 1) and 5-{(chloroacetyl)(3-methylbenzyl)amino}-3-ethyl-1-methylpyrazole-4-carboxylate (Comparative compound 2) (both described in Japanese Patent Application Kokai No. Sho 57-167972) were also tested. They have the following structural formulae:

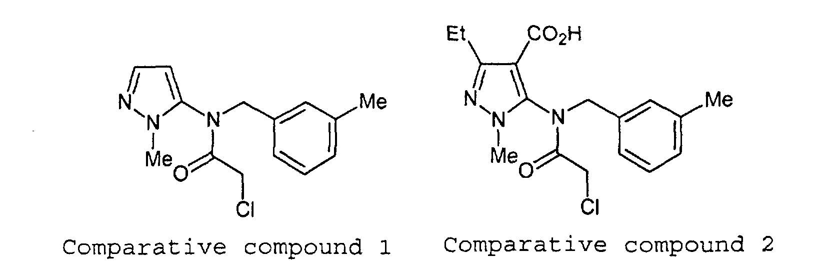

The degree of disease incidence of the test plant was observed by the naked eye, judged in accordance with the below-described criteria and rated on a scale of 0 to 3.
0: no disease incidence was observed.
1: the degree of disease incidence was less than 40% of that of the untreated plant.
2: the degree of disease incidence was 40%-80% of that of the untreated plant.
3: the degree of disease incidence was 80% or more of the untreated plant.

As a result of the test, the degree of disease incidence was 0 when the below-described compounds were applied: Example 53 (Compound No. 2-21), Example 60 (Compound No. 2-31), Example 67 (Compound No. 2-38), Example 75 (Compound No. 2-61), Example 78 (Compound No. 2-67), Example 79 (Compound No. 2-73), Example 81 (Compound No. 2-75), Example 93 (Compound No. 3-17), Example 98 (Compound No. 3-27), Example 103 (Compound No. 3-37), Example 133 (Compound No. 4-1), Example 134 (Compound No. 4-4), Example 136 (Compound No. 4-9), Example 138 (Compound No. 4-11), Example 141 (Compound No. 4-19), Example 143 (Compound No. 4-21), Example 144 (Compound No. 4-23), Example 145 (Compound No. 4-24), Example 147 (Compound No. 4-29), Example 153 (Compound No. 4-41), Example 154 (Compound No. 4-43), Example 155 (Compound No. 4-44), Example 157 (Compound No. 4-49), Example 158 (Compound No. 4-51), Example 159 (Compound No. 4-54), Example 164 (Compound No. 4-71), Example 165 (Compound No. 4-74), Example 167 (Compound No. 4-76), Example 168 (Compound No. 4-77), Example 2 (Compound No. 4-79), Example 178 (Compound No. 4-144), Example 189 (Compound No. 5-3), Example 190 (Compound No. 5-4), Example 191 (Compound No. 5-9), Example 192 (Compound No. 5-10), Example 205 (Compound No. 5-46), Example 206 (Compound No. 5-47), Example 208 (Compound No. 5-51), Example 209 (Compound No. 5-52), Example 210 (Compound No. 5-53), Example 211 (Compound No. 5-54), Example 4 (Compound No. 5-93), Example 240 (Compound No. 5-94), Example 241 (Compound No. 5-95), Example 242 (Compound No. 5-96), Example 243 (Compound No. 5-97), Example 244 (Compound No. 5-98), Example 245 (Compound No. 5-99), Example 246 (Compound No. 5-100), Example 247 (Compound No. 5-101), Example 248 (Compound No. 5-102), Example 249 (Compound No. 5-103), Example 250 (Compound No. 5-104), Example 251 (Compound No. 6-1), Example 252 (Compound No. 6-5), Example 254 (Compound No. 6-15), Example 1 (Compound No. 6-20), Example 266 (Compound No. 6-41), Example 268 (Compound No. 6-43), Example 271 (Compound No. 6-46), Example 274 (Compound No. 6-55), Example 275 (Compound No. 6-56), Example 276 (Compound No. 6-57), Example 279 (Compound No. 6-65), Example 280 (Compound No. 6-67), Example 285 (Compound No. 7-1), Example 293 (Compound No. 7-10), Example 298 (Compound No. 7-24), Example 4 (Compound No. 7-28), Example 302 (Compound No. 7-29), Example 303 (Compound No. 7-30), Example 304 (Compound No. 7-31), Example 311 (Compound No. 7-39), Example 317 (Compound No. 7-53), Example 323 (Compound No. 8-3), Example 324 (Compound No. 8-4), Example 326 (Compound No. 8-6), Example 327 (Compound No. 8-7), Example 331 (Compound No. 8-12), Example 332 (Compound No. 8-13), Example 333 (Compound No. 8-14), Example 334 (Compound No. 8-15), Example 336 (Compound No. 8-17), Example 343 (Compound No. 8-24), Example 344 (Compound No. 8-25), Example 346 (Compound No. 8-27), Example 347 (Compound No. 8-28), Example 348 (Compound No. 8-29), Example 351 (Compound No. 8-32), Example 354 (Compound No. 8-35), Example 356 (Compound No. 8-37), Example 357 (Compound No. 8-38), Example 359 (Compound No. 8-40), Example 361 (Compound No. 8-42), Example 362 (Compound No. 8-43), Example 367 (Compound No. 8-48), Example 368 (Compound No. 8-49), Example 369 (Compound No. 8-50), Example 373 (Compound No. 8-54), Example 379 (Compound No. 8-69), Example 385 (Compound No. 8-91), Example 386 (Compound No. 8-92), Example 399 (Compound No. 5-5), Example 401 (Compound No. 10-1), Example 402 (Compound No. 10-7), Example 405 (Compound No. 10-11), Example 407 (Compound No. 10-17), Example 409 (Compound No. 10-20), Example 410 (Compound No. 10-21), Example 411 (Compound No. 10-30), Example 417 (Compound No. 10-51), Example 420 (Compound No. 10-55), Example 421 (Compound No. 10-61), Example 424 (Compound No. 10-65), Example 425 (Compound No. 10-73), Example 426 (Compound No. 10-79), Example 427 (Compound No. 10-81), Example 428 (Compound No. 10-82), Example 429 (Compound No. 10-88), Example 431 (Compound No. 10-91), Example 432 (Compound No. 10-97), Example 433 (Compound No. 10-100), Example 434 (Compound No. 10-105), Example 435 (Compound No. 10-109), Example 436 (Compound No. 10-114), Example 437 (Compound No. 10-115), Example 438 (Compound No. 10-116), Example 439 (Compound No. 10-117), Example 440 (Compound No. 10-118), Example 441 (Compound No. 10-119), Example 443 (Compound No. 10-121), Example 445 (Compound No. 10-123), Example 446 (Compound No. 10-124), Example 469 (Compound No. 10-165), Example 473 (Compound No. 10-175), Example 474 (Compound No. 10-184), Example 475 (Compound No. 10-201), Example 476 (Compound No. 10-202), Example 479 (Compound No. 10-217), Example 482 (Compound No. 11-3), Example 483 (Compound No. 11-10), Example 484 (Compound No. 11-11), Example 488 (Compound No. 11-20), Example 489 (Compound No. 11-21), Example 490 (Compound No. 11-22) and Example 492 (Compound No. 11-24). On the other hand, the degree of disease incidence was 3 when the comparative compounds 1 and 2 were applied.

### (Test 2)

### Control test of downy mildew on cucumber (curative effect) A spore suspension of fungus was sprayed and inoculated to the 3 to 5 leaf stage of a test plant (cucumber: Sagami-hanpaku) cultivated in a pot. The plant was placed in an inoculation chamber having a room temperature set at 20 to 22°C to promote the onset of the disease. An emulsifiable concentrate prepared in accordance with Formulation Example 2 was diluted with water to prepare a spray solution containing 300 ppm of the invention compound. One day after inoculation, the spray solution was applied uniformly to the test plant in the pot by a spray gun. The degree of disease incidence 7 days after inoculation was studied. The test was conducted in 2 pots. As comparative products, Comparative compounds 1 and 2 described in Test 1 were employed.

The degree of disease incidence of the test plant was observed by the naked eye, judged in accordance with the below-described criteria and rated on a scale of 0 to 3. 0: no damage was observed.
1: the degree of disease incidence was less than 40% of that of the untreated plant.
2: the degree of disease incidence was 40-80% of that of the untreated plant.
3: the degree of damage was 80% or more of the untreated plant.

As a result of the test, the degree of disease incidence was 0 when the below-described compounds were applied: Example 10 (Compound No. 1-11), Example 11 (Compound No. 1-12), Example 3 (Compound No. 1-14), Example 17 (Compound No. 1-19), Example 23 (Compound No. 1-25), Example 32 (Compound No. 1-34), Example 37 (Compound No. 1-62), Example 40 (Compound No. 1-72), Example 41 (Compound No. 1-73), Example 47 (Compound No. 1-86), Example 49 (Compound No. 1-89), Example 54 (Compound No. 2-22), Example 56 (Compound No. 2-24), Example 57 (Compound No. 2-26), Example 60 (Compound No. 2-31), Example 62 (Compound No. 2-33), Example 64 (Compound No. 2-35), Example 66 (Compound No. 2-37), Example 67 (Compound No. 2-38), Example 69 (Compound No. 2-40), Example 74 (Compound No. 2-50), Example 75 (Compound No. 2-61), Example 76 (Compound No. 2-63), Example 77 (Compound No. 2-64), Example 78 (Compound No. 2-67), Example 79 (Compound No. 2-73), Example 81 (Compound No. 2-75), Example 82 (Compound No. 2-76), Example 83 (Compound No. 2-78), Example 84 (Compound No. 2-82), Example 86 (Compound No. 2-84), Example 95 (Compound No. 3-19), Example 96 (Compound No. 3-20), Example 97 (Compound No. 3-21), Example 98 (Compound No. 3-27), Example 99 (Compound No. 3-28), Example 100 (Compound No. 3-29), Example 101 (Compound No. 3-30), Example 102 (Compound No. 3-31), Example 103 (Compound No. 3-37), Example 126 (Compound No. 3-87), Example 130 (Compound No. 3-97), Example 133 (Compound No. 4-1), Example 134 (Compound No. 4-4), Example 135 (Compound No. 4-5), Example 138 (Compound No. 4-11), Example 139 (Compound No. 4-14) Example 141 (Compound No. 4-19), Example 142 (Compound No. 4-20), Example 145 (Compound No. 4-24), Example 150 (Compound No. 4-34), Example 151 (Compound No. 4-35), Example 152 (Compound No. 4-40), Example 153 (Compound No. 4-41), Example 154 (Compound No. 4-43), Example 155 (Compound No. 4-44), Example 156 (Compound No. 4-45), Example 157 (Compound No. 4-49), Example 158 (Compound No. 4-51), Example 159 (Compound No. 4-54), Example 164 (Compound No. 4-71), Example 165 (Compound No. 4-74), Example 167 (Compound No. 4-76), Example 168 (Compound No. 4-77), Example 169 (Compound No. 4-78), Example 2 (Compound No. 4-79), Example 170 (Compound No. 4-80), Example 175 (Compound No. 4-121), Example 176 (Compound No. 4-131), Example 181 (Compound No. 4-150), Example 183 (Compound No. 4-154), Example 189 (Compound No. 5-3), Example 190 (Compound No. 5-4), Example 191 (Compound No. 5-9), Example 192 (Compound No. 5-10), Example 195 (Compound No. 5-23), Example 197 (Compound No. 5-26), Example 199 (Compound No. 5-36), Example 203 (Compound No. 5-43), Example 207 (Compound No. 5-48), Example 208 (Compound No. 5-51), Example 209 (Compound No. 5-52), Example 210 (Compound No. 5-53), Example 211 (Compound No. 5-54), Example 213 (Compound No. 5-56), Example 214 (Compound No. 5-57), Example 215 (Compound No. 5-64), Example 217 (Compound No. 5-66), Example 218 (Compound No. 5-67), Example 219 (Compound No. 5-68), Example 220 (Compound No. 5-69), Example 221 (Compound No. 5-70), Example 225 (Compound No. 5-74), Example 228 (Compound No. 5-78), Example 229 (Compound No. 5-79), Example 234 (Compound No. 5-85), Example 235 (Compound No. 5-86), Example 236 (Compound No. 5-87), Example 237 (Compound No. 5-89), Example 238 (Compound No. 5-90), Example 240 (Compound No. 5-94), Example 241 (Compound No. 5-95), Example 242 (Compound No. 5-96), Example 243 (Compound No. 5-97), Example 246 (Compound No. 5-100), Example 247 (Compound No. 5-101), Example 249 (Compound No. 5-103), Example 250 (Compound No. 5-104), Example 251 (Compound No. 6-1), Example 1 (Compound No. 6-20), Example 256 (Compound No. 6-25), Example 257 (Compound No. 6-27), Example 258 (Compound No. 6-28), Example 259 (Compound No. 6-29), Example 260 (Compound No. 6-33), Example 261 (Compound No. 6-34), Example 262 (Compound No. 6-35), Example 263 (Compound No. 6-36), Example 264 (Compound No. 6-37), Example 267 (Compound No. 6-42), Example 268 (Compound No. 6-43), Example 269 (Compound No. 6-44), Example 270 (Compound No. 6-45), Example 272 (Compound No. 6-47), Example 273 (Compound No. 6-54), Example 274 (Compound No. 6-55), Example 275 (Compound No. 6-56), Example 276 (Compound No. 6-57), Example 279 (Compound No. 6-65), Example 280 (Compound No. 6-67), Example 281 (Compound No. 6-72), Example 285 (Compound No. 7-1), Example 291 (Compound No. 7-8), Example 294 (Compound No. 7-14), Example 295 (Compound No. 7-17), Example 296 (Compound No. 7-19), Example 297 (Compound No. 7-21), Example 298 (Compound No. 7-24), Example 4 (Compound No. 7-28), Example 302 (Compound No. 7-29), Example 303 (Compound No. 7-30), Example 304 (Compound No. 7-31), Example 305 (Compound No. 7-32), Example 310 (Compound No. 7-37), Example 318 (Compound No. 7-63), Example 321 (Compound No. 8-1), Example 322 (Compound No. 8-2), Example 323 (Compound No. 8-3), Example 324 (Compound No. 8-4), Example 325 (Compound No. 8-5), Example 326 (Compound No. 8-6), Example 327 (Compound No. 8-7), Example 328 (Compound No. 8-8), Example 331 (Compound No. 8-12), Example 332 (Compound No. 8-13), Example 333 (Compound No. 8-14), Example 334 (Compound No. 8-15), Example 336 (Compound No. 8-17), Example 337 (Compound No. 8-18), Example 338 (Compound No. 8-19), Example 339 (Compound No. 8-20), Example 341 (Compound No. 8-22), Example 342 (Compound No. 8-23), Example 343 (Compound No. 8-24), Example 344 (Compound No. 8-25), Example 345 (Compound No. 8-26), Example 346 (Compound No. 8-27), Example 347 (Compound No. 8-28), Example 348 (Compound No. 8-29), Example 350 (Compound No. 8-31), Example 351 (Compound No. 8-32), Example 354 (Compound No. 8-35), Example 357 (Compound No. 8-38), Example 358 (Compound No. 8-39), Example 359 (Compound No. 8-40), Example 361 (Compound No. 8-42), Example 362 (Compound No. 8-43), Example 366 (Compound No. 8-47), Example 367 (Compound No. 8-48), Example 368 (Compound No. 8-49), Example 369 (Compound No. 8-50), Example 370 (Compound No. 8-51), Example 371 (Compound No. 8-52), Example 373 (Compound No. 8-54), Example 374 (Compound No. 8-55), Example 378 (Compound No. 8-68), Example 383 (Compound No. 8-86), Example 384 (Compound No. 8-90), Example 385 (Compound No. 8-91), Example 386 (Compound No. 8-92), Example 389 (Compound No. 9-7), Example 397 (Compound 4-39), Example 400 (Compound No. 7-45), Example 410 (Compound No. 10-21), Example 411 (Compound No. 10-30), Example 412 (Compound No. 10-34), Example 424 (Compound No. 10-65), Example 425 (Compound No. 10-73), Example 426 (Compound No. 10-79), Example 427 (Compound No. 10-81), Example 428 (Compound No. 10-82), Example 429 (Compound No. 10-88), Example 430 (Compound No. 10-90), Example 433 (Compound No. 10-100), Example 434 (Compound No. 10-105), Example 436 (Compound No. 10-114), Example 438 (Compound No. 10-116), Example 440 (Compound No. 10-118), Example 443 (Compound No. 10-121), Example 445 (Compound No. 10-123), Example 454 (Compound No. 10-132), Example 455 (Compound No. 10-133), Example 459 (Compound No. 10-137), Example 461 (Compound No. 10-139), Example 463 (Compound No. 10-145), Example 464 (Compound No. 10-146), Example 467 (Compound No. 10-154), Example 473 (Compound No. 10-175), Example 474 (Compound No. 10-184), Example 475 (Compound No. 10-201), Example 477 (Compound No. 10-211), Example 478 (Compound No. 10-216), Example 479 (Compound No. 10-217), Example 481 (Compound No. 10-219), Example 482 (Compound No. 11-3), Example 486 (Compound No. 11-16), Example 493 (Compound No. 11-25), Example 497 (Compound No. 11-29), Example 509 (Compound No. 11-41), Example 516 (Compound No. 11-48), Example 517 (Compound No. 11-49) and Example 518 (Compound No. 11-50). On the other hand, the degree of disease incidence was 3 when the comparative compounds 1 and 2 were applied.

### [Industrial Applicability]

The compounds of the present invention are usable as agricultural fungicides. Since they exhibit excellent effects against plant diseases caused by various fungi, particularly late blight on tomatoes and downy mildew on cucumbers, without giving a damage to host plants, they are excellent as agricultural or horticultural fungicides.

Examples of the plant diseases against which the compounds of the invention are markedly effective include late blight on tomatoes (*Phytophthora infestans*), downy mildew on cucumbers (*Pseudoperonospora cubensis*), downy mildew on grape vines (*Plasmopara viticola*) and late blight on potatoes (*Phytophthora infestans*). The fungicidal spectrum of the compounds of the invention are not limited to them.

## Claims

1. A 5-(m-cyanobenzylamino)pyrazole derivative represented by the following formula (I): 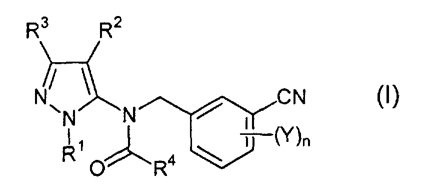 wherein:
R¹ represents a C₁₋₆ alkyl group, a C₃₋₇ cycloalkyl group, or a phenyl group;
R² represents a hydrogen atom or a C₁₋₆ alkyl group;
R³ represents a C₁₋₆ alkyl group, a cyano-C₁₋₆ alkyl group, a hydroxy-C₁₋₆ alkyl group, a (C₁₋₆ alkoxy)-C₁₋₆ alkyl group, a (C₂₋₇ aliphatic acyloxy)-C₁₋₆ alkyl group, a (C₁₋₆ alkylamino)-C₁₋₆ alkyl group, a di(C₁₋₆ alkyl)amino-C₁₋₆ alkyl group, a (5- or 6-membered nitrogen-containing saturated heterocyclyl)-C₁₋₆ alkyl group (the heterocyclic moiety of said heterocyclylalkyl group may additionally include one ring oxygen atom or NH group), a substituted or unsubstituted C₃₋₇-cycloalkyl-C₁₋₆ alkyl group (the substituent(s) of the cycloalkyl moiety of said cycloalkylalkyl group is (are) one C₁₋₆ alkyl group or 1 to 3 halogen atoms which may be the same or different, and the cycloalkyl moiety of said cycloalkylalkyl group may be interrupted by one oxygen atom), a C₃₋₇-cycloalkenyl-C₁₋₆-alkyl group, a halo-C₁₋₆ alkyl group (said halogen substituent(s) is (are) 1 to 6 halogen atoms which may be the same or different), a substituted or unsubstituted C₇₋₉ aralkyl group (the substituent(s) of the aryl moiety of said aralkyl group is (are) 1 to 5 substituents which are selected from the below-described substituent group A and may be the same or different), a substituted or unsubstituted heteroaralkyl group (the substituent(s) of the aryl moiety of said heteroaralkyl group is (are) 1 to 5 substituents which are selected from the below-described substituent group B and may be the same or different), a C₃₋₇ cycloalkyl group, a C₃₋₆ alkenyl group, a C₂₋₇ aliphatic acyl group or a substituted or unsubstituted phenyl group (the substituent(s) of said phenyl group is (are) 1 to 5 substituents which are selected from the below-described substituent group A and which may be the same or different),
R⁴ represents a hydrogen atom, a halogen atom, a C₁₋₆ alkyl group, a cyano-C₁₋₆ alkyl group, a hydroxy-C₁₋₆ alkyl group, a halo-C₁₋₆ alkyl group (said halogen substituent(s) is (are) 1 to 3 halogen atoms which may be the same or different), a (C₁₋₆-alkoxy)-C₁₋₆ alkyl group, a {(C₁₋₆ alkoxy)-C₁₋₆ alkoxy}-C₁₋₆ alkyl group, a (C₃₋₆ alkenyloxy)-C₁₋₆ alkyl group, a (substituted or unsubstituted C₂₋₇ aliphatic acyloxy)-C₁₋₆ alkyl group (said substituent is a C₁₋₆ alkoxy group), a (substituted or unsubstituted C₂₋₇ alkoxycarbonyloxy)-C₁₋₆ alkyl group (said substituent is a C₁₋₆ alkoxy group), a (substituted or unsubstituted phenoxy)-C₁₋₆ alkyl group (said substituent(s) is (are) 1 to 5 substituents which are selected from the below-described substituent group A and may be the same or different), a (substituted or unsubstituted benzyloxy)-C₁₋₆ alkyl group (said substituent(s) is (are) 1 to 5 substituents which are selected from the below-described substituent group A and may be the same or different), a (substituted or unsubstituted heteroaryloxy)-C₁₋₆ alkyl group (said substituent(s) is (are) 1 to 3 substituents which are selected from the below-described substituent group B and may be the same or different), a (substituted or unsubstituted C₁₋₆ alkylamino)-C₁₋₆ alkyl group (said substituent is a phenyl group or a C₁₋₆ alkoxy group), a (C₃₋₆ alkenylamino)-C₁₋₆ alkyl group, a (phenylamino)-C₁₋₆ alkyl group, an {N-(C₁₋₆ alkyl)anilino}-C₁₋₆ alkyl group, a di(C₁₋₆ alkyl)amino-C₁₋₆ alkyl group, a (substituted or unsubstituted 5- or 6-membered nitrogen-containing saturated heterocyclyl)-C₁₋₆ alkyl group (said substituent(s) is (are) 1 or 2 C₁₋₆ alkyl groups which may be the same or different and the heterocyclic moiety of said heterocyclylalkyl group may additionally include one ring oxygen atom or NH group), a (substituted or unsubstituted C₁₋₆-alkylthio)-C₁₋₆ alkyl group (said substituent is a phenyl group or a C₁₋₆ alkoxy group), a (C₃₋₆ alkenylthio)-C₁₋₆ alkyl group, a (substituted or unsubstituted phenylthio)-C₁₋₆ alkyl group (said substituent(s) is (are) 1 to 5 substituents which are selected from the below-described substituent group A and may be the same or different), a (substituted or unsubstituted heteroarylthio)-C₁₋₆ alkyl group (said substituent(s) is (are) 1 to 3 substituents which are selected from the below-described substituent group B and may be the same or different), a (C₂₋₇ alkoxycarbonyl)-C₁₋₆ alkyl group, a substituted or unsubstituted C₇₋₉ aralkyl group (said substituent(s) is (are) 1 to 5 substituents which are selected from the below-described substituent group A and may be the same or different), a substituted or unsubstituted heteroaralkyl group (said substituent(s) is (are) 1 to 3 substituents which are selected from the below-described substituent group B and may be the same or different), a C₃₋₇ cycloalkyl group, a substituted or unsubstituted phenyl group (the substituent(s) of said phenyl group is (are) 1 to 5 substituents which are selected from the below-described substituent group A and may be the same or different), a substituted or unsubstituted heteroaryl group (said substituent(s) is (are) 1 to 3 substituents which are selected from the below-described substituent group B and may be the same or different), a C₃₋₆ alkenyl group, a C₂₋₇ alkoxycarbonyl group, a C₁₋₆ alkoxy group, a (C₁₋₆ alkoxy)-C₁₋₆ alkoxy group, a (C₁₋₆ alkylamino)-C₁₋₆ alkoxy group, a di(C₁₋₆ alkyl)amino-C₁₋₆ alkoxy group, a substituted or unsubstituted heteroaralkyloxy group (said substituent(s) is (are) 1 to 3 substituents which are selected from the below-described substituent group B and may be the same or different), a C₃₋₇ cycloalkoxy group, a C₃₋₆ alkenyloxy group, a phenoxy group (said substituent(s) is(are) 1 to 5 substituents which are selected from the below-described substituent group A and may be the same or different), a substituted or unsubstituted benzyloxy group (said substituent(s) is (are) 1 to 5 substituents which are selected from the below-described substituent group A and may be the same or different), a substituted or unsubstituted heteroaryloxy group (the substituent(s) is (are) 1 to 3 substituents which are selected from the below-described substituent group B and may be the same or different), a substituted or unsubstituted C₁₋₆ alkylamino group (said substituent is a phenyl group or a C₁₋₆ alkoxy group), a C₃₋₆ alkenylamino group, a di(C₁₋₆ alkyl)amino group, a substituted or unsubstituted 5- or 6-membered nitrogen-containing saturated heterocyclic group (said substituent(s) is (are) 1 or 2 C₁₋₆ alkyl groups which may be the same or different, and said heterocyclic group may additionally include one ring oxygen atom or NH group), a substituted or unsubstituted C₁₋₆ alkylthio group (said substituent is a phenyl group or a C₁₋₆ alkoxy group), a C₃₋₆ alkenylthio group, a substituted or unsubstituted phenylthio group (said substituent(s) is (are) 1 to 5 substituents which are selected from the below-described substituent group A and may be the same or different), or a substituted or unsubstituted heteroarylthio group (the substituent(s) is (are) 1 to 3 substituents which are selected from the below-described substituent group B and may be the same or different);
Y represents a C₁₋₆ alkyl group, a C₁₋₆ alkoxy group, a cyano group, a nitro group, a halogen atom, or a C₂₋₇ alkoycarbonyl group;
n is an integer of 0 to 4, with the proviso that when n is 2 to 4, the groups Y may be the same or different;
substituent group A consists of C₁₋₆ alkyl groups, halo-C₁₋₆ alkyl groups (said halogen substituent(s) is (are) 1 to 3 halogen atoms which may be the same or different), C₁₋₆ alkoxy groups, a cyano group, a nitro group, halogen atoms, C₂₋₇ alkoxycarbonyl groups, C₂₋₇ alkylcarbonylamino groups, and C₁₋₃ alkylenedioxy groups; and
substituent group B consists of C₁₋₆ alkyl groups, halo-C₁₋₆ alkyl groups (said halogen substituent(s) is (are) 1 to 3 halogen atoms which may be the same or different), C₁₋₆ alkoxy groups, a cyano group, a phenyl group, halogen atoms, C₂₋₇ alkoxycarbonyl groups, and an oxo group; or a salt thereof.

2. The 5-(m-cyanobenzylamino)pyrazole derivative of Claim 1, wherein R¹ represents a C₁₋₄ alkyl group, a cyclohexyl group or a phenyl group; or a salt thereof.

3. The 5-(m-cyanobenzylamino)pyrazole derivative of Claim 1, wherein R¹ represents a C₁₋₂ alkyl group; or a salt thereof.

4. The 5-(m-cyanobenzylamino)pyrazole derivative of Claim 1, wherein R¹ represents a methyl group; or a salt thereof.

5. The 5-(m-cyanobenzylamino)pyrazole derivative of any one of Claims 1 to 4, wherein R² represents a hydrogen atom or a C₁₋₂ alkyl group; or a salt thereof.

6. The 5-(m-cyanobenzylamino)pyrazole derivative of any one of Claims 1 to 4, wherein R² represents a hydrogen atom or a methyl group; or a salt thereof.

7. The 5-(m-cyanobenzylamino)pyrazole derivative of any one of Claims 1 to 4, wherein R² represents a hydrogen atom; or a salt thereof.

8. The 5-(m-cyanobenzylamino)pyrazole derivative of any one of Claims 1 to 7, wherein R³ represents a C₁₋₅ alkyl group, a cyanomethyl group, a 2-cyanoethyl group, a hydroxymethyl group, a 2-hydroxyethyl group, a methoxymethyl group, an ethoxymethyl group, a 2-methoxyethyl group, an acetoxymethyl group, a methylaminomethyl group, an ethylaminomethyl group, a propylaminomethyl group, an isopropylaminomethyl group, a butylaminomethyl group, an isobutylaminomethyl group, a t-butylaminomethyl group, a dimethylaminomethyl group, a diethylaminomethyl group, an ethylmethylaminomethyl group, a 1-pyrrolidinylmethyl group, a piperidinomethyl group, a (C₁₋₂ alkyl-C₃₋₆ cycloalkyl)-C₁₋₂ alkyl group, a (fluoro-C₃₋₆ cycloalkyl)-C₁₋₂ alkyl group, a (difluoro-C₃₋₆ cycloalkyl)-C₁₋₂ alkyl group, a tetrahydrofuranyl-C₁₋₂ alkyl group, a tetrahydropyranyl-C₁₋₂ alkyl group, a halo-C₁₋₄ alkyl group (said halogen substituent(s) is (are) 1 to 3 fluorine atoms or chlorine atoms which are the same), a benzyl group, a methylbenzyl group, an isopropylbenzyl group, a methoxybenzyl group, a cyanobenzyl group, a nitrobenzyl group, a chlorobenzyl group, a methoxycarbonylbenzyl group, a 3-thienylmethyl group, an acetyl group, a cyclohexyl group, a 3-butenyl group, a phenyl group, a methylphenyl group, an isopropylphenyl group, a methoxyphenyl group, a cyanophenyl group, a nitrophenyl group, a chlorophenyl group, a fluorophenyl group or a methoxycarbonylphenyl group; or a salt thereof.

9. The 5-(m-cyanobenzylamino)pyrazole derivative of any one of Claims 1 to 7, wherein R³ represents a C₄₋₅ alkyl group, a cyclobutylmethyl group, a (methylcyclobutyl)methyl group, a tetrahydrofuranylmethyl group, a cyclopentylmethyl group, a (methylcyclopentyl)methyl group, a (fluorocyclopentyl)methyl group, a (difluorocyclopentyl)methyl group, a tetrahydropyranylmethyl group, a cyclohexylmethyl group, a cyclopentenylmethyl group, a chloromethyl group, a 1,1-difluoroisobutyl group, a 3,3,3-trifluoro-2-trifluoromethylpropyl group, a 3,3,3-trifluoro-2-methylpropyl group, a 3,3,3-trifluoro-2,2-dimethylpropyl group, or a cyclopentyl group; or a salt thereof.

10. The 5-(m-cyanobenzylamino)pyrazole derivative of any one of Claims 1 to 7, wherein R³ represents a C₄₋₅ branched alkyl group, a cyclobutylmethyl group, a (methylcyclobutyl)methyl group, a tetrahydrofuranyl group, a cyclopentylmethyl group, a (fluorocyclopentyl)methyl group, a (difluorocyclopentyl)methyl group, a tetrahydropyranylmethyl group, a cyclohexylmethyl group, a cyclopent-2-enylmethyl group, a cyclopent-3-enylmethyl group, a 1,1-difluoroisobutyl group, a 3,3,3-trifluoro-2-trifluoromethylpropyl group, a 3,3,3-trifluoro-2-methylpropyl group, or a 3,3,3-trifluoro-2,2-dimethylpropyl group; or a salt thereof.

11. The 5-(m-cyanobenzylamino)pyrazole derivative of any one of Claims 1 to 7, wherein R³ represents an isobutyl group, a cyclobutylmethyl group, a tetrahydrofuranylmethyl group, a cyclopentylmethyl group, a 3,3,3-trifluoro-2-methylpropyl group or a 3,3,3-trifluoro-2,2,-dimethylpropyl group; or a salt thereof.

12. The 5-(m-cyanobenzylamino)pyrazole derivative of any one of Claims 1 to 11, wherein R⁴ represents a hydrogen atom, a fluorine atom, a chlorine atom, a C₁₋₄ alkyl group, a cyanomethyl group, a 2-cyanoethyl group, a hydroxymethyl group, a hydroxyethyl group, a hydroxypropyl group, a trifluoromethyl group, a trichloromethyl group, a difluoromethyl group, a dichloromethyl group, a fluoromethyl group, a chloromethyl group, a bromomethyl group, a (C₁₋₄ alkoxy)-C₁₋₂ alkyl group, a methoxymethoxymethyl group, a (2-methoxy)ethoxymethyl group, a 2-(methoxymethoxy)ethyl group, an allyloxymethyl group, a 2-allyloxyethyl group, a butyryloxymethyl group, an isobutyryloxymethyl group, a pivaloyloxymethyl group, a cyclopropylcarbonyloxymethyl group, a 2-methyl-1-butenoyloxymethyl group, a 2-methyl-2-butenoyloxymethyl group, a methoxyacetoxymethyl group, a 2-methoxybutyryloxymethyl group, an ethoxyacetoxymethyl group, a methoxycarbonyloxymethyl group, an ethoxycarbonyloxymethyl group, a 2-(methoxycarbonyloxy)ethyl group, a methoxymethoxycarbonyloxymethyl group, a 2-methoxyethoxycarbonyloxymethyl group, a phenoxymethyl group, a 2-fluorophenoxymethyl group, a 3-fluorophenoxymethyl group, a 4-fluorophenoxymethyl group, a 2,4-dichlorophenoxymethyl group, a 3-trifluoromethylphenoxymethyl group, a 2-methylphenoxymethyl group, a 3-methoxyphenoxymethyl group, a 4-methoxyphenoxymethyl group, a 3-cyanophenoxymethyl group, a 4-cyanophenoxymethyl group, a benzyloxy group, a methylbenzyloxy group, an isopropylbenzyloxy group, a cyanobenzyloxy group, a nitrobenzyloxy group, a chlorobenzyloxy group, a methoxycarbonylbenzyloxy group, a 2-pyridyloxymethyl group, a (3-methyl-2-pyridyl)oxymethyl group, a (6-methyl-2-pyridyl)oxymethyl group, a (3-methoxy-2-pyridyl)oxymethyl group, a (2-chloro-2-pyridyl)oxymethyl group, a (5-chloro-3-pyridyl)oxymethyl group, a 1-pyrazolyloxymethyl group, a (4-bromo-1-pyrazolyl)oxymethyl group, a 3-isoxazolyloxymethyl group, a (5-methyl-3-isoxazolyl)oxymethyl group, a (4,5-dimethyl-3-isoxazolyl)oxymethyl group, a {5-methyl-2-(1,3,4-thiadiazolyl)}oxymethyl group, a 6-quinolyloxymethyl group, a (4-methyl-2-pyrimidinyl)oxymethyl group, a 4-pyrimidinyloxymethyl group, a methylaminomethyl group, a benzylaminomethyl group, a methoxymethylaminomethyl group, an ethylaminomethyl group, a propylaminomethyl group, an isopropylaminomethyl group, a butylaminomethyl group, an isobutylaminomethyl group, a t-butylaminomethyl group, an allylaminomethyl group, a phenylaminomethyl group, an N-methylanilinomethyl group, a dimethylaminomethyl group, a diethylaminomethyl group, an ethylmethylaminomethy group, a piperidinomethyl group, a morpholinomethyl group, a 2,6-dimethylmorpholinomethyl group, a methylthiomethyl group, a benzylthiomethyl group, a methoxymethylthiomethyl group, an ethylthiomethyl group, a propylthiomethyl group, an isopropylthiomethyl group, a butylthiomethyl group, an isobutylthiomethyl group, a t-butylthiomethyl group, an allylthiomethyl group, a phenylthiomethyl group, a methylphenylthiomethyl group, an isopropylphenylthiomethyl group, a methoxyphenylthiomethyl group, a cyanophenylthiomethyl group, a nitrophenylthiomethyl group, a chlorophenylthiomethyl group, a methoxycarbonylphenylthiomethyl group, a (2-oxo-1-pyridyl)thiomethyl group, a 2-pyridylthiomethyl group, a (1-methyl-2-imidazolyl)thiomethyl group, a 2-pyrimidylthiomethyl group, a {5-methyl-2-(1,3,4-thiadiazolyl)}thiomethyl group, a methoxycarbonylmethyl group, a 2-(methoxycarbonyl)ethyl group, an ethoxycarbonylmethyl group, a 2-(ethoxycarbonyl)ethyl group, a benzyl group, a methylbenzyl group, an isopropylbenzyl group, a methoxybenzyl group, a cyanobenzyl group, a nitrobenzyl group, a chlorobenzyl group, a fluorobenzyl group, a methoxycarbonylbenzyl group, a 2-pyridylmethyl group, a (2-oxo-1-pyridyl)methyl group, a (3-methyl-2-oxo-1-pyridyl)methyl group, a (5-methyl-2-oxo-1-pyridyl)methyl group, a (3-methoxy-2-oxo-1-pyridyl)methyl group, a (3,5-dichloro-2-oxo-1-pyridyl)methyl group, a (5-trifluoromethyl-2-oxo-1-pyridyl)methyl group, a (4-oxo-1-pyridyl)methyl group, a (3,5-dichloro-4-oxo-1-pyridyl)methyl group, a 2-pyridylmethyl group, a 3-pyridylmethyl group, a 4-pyridylmethyl group, a 1-pyrazolylmethyl group, a (4-methyl-1-pyrazolyl)methyl group, a (3,5-dimethyl-1-pyrazolyl)methyl group, a (4-bromo-1-pyrazolyl)methyl group, a 1-imidazolylmethyl group, a (2-methyl-1-imidazolyl)methyl group, a (4,5-dichloro-1-imidazolyl)methyl group, a {4,5-di(methoxycarbonyl)-1-imidazolyl}methyl group, a 3-isoxazolylmethyl group, a (5-methyl-3-isoxazolyl)methyl group, a 1-(1,2,4-triazolyl)methyl group, a 2-pyrimidinylmethyl group, a (2-oxo-1-pyrimidinyl)methyl group, a 2-furylmethyl group, a C₃₋₆ cycloalkyl group, a phenyl group, a methylphenyl group, an isopropylphenyl group, a methoxyphenyl group, a cyanophenyl group, a nitrophenyl group, a chlorophenyl group, a methoxycarbonylphenyl group, a 2-furyl group, a 2-thienyl group, a 2,6-dichloro-4-pyridyl group, a 1-methyl-2-pyrrolyl group, a C₃₋₅ alkenyl group, a methoxycarbonyl group, an ethoxycarbonyl group, a C₁₋₄ alkoxy group, a methoxymethoxy group, a (2-methoxy)ethoxy group, an ethoxymethoxy group, a methylaminomethoxy group, an ethylaminomethoxy group, a dimethylaminomethoxy group, a diethylaminomethoxy group, an ethylmethylaminomethoxy group, a 2-pyridylmethoxy group, a 3-pyridylmethoxy group, a 4-pyridylmethoxy group, a 1-pyrazolylmethoxy group, a 1-imidazolylmethoxy group, a 3-isoxazolylmethoxy group, a 1-(1,2,4-triazolyl)methoxy group, a 2-pyrimidinylmethoxy group, a 2-furylmethoxy group, a (6-trifluoromethyl-2-pyridyl)methoxy group, a (5-phenyl-2-methyl-3-furyl)methoxy group, a (6-chloro-2-pyridyl)methoxy group, a (5-cyano-3-methyl-1-pyrazolyl)methoxy group, a (5-methoxycarbonyl-1-pyrazolyl)methoxy group, a cyclopropoxy group, a cyclobutoxy group, a cyclohexyloxy group, an allyloxy group, a phenoxy group, a 2-fluorophenoxy group, a 3-fluorophenoxy group, a 4-fluorophenoxy group, a 2,4-dichlorophenoxy group, a 3-trifluoromethylphenoxy group, a 2-methylphenoxy group, a 3-methoxyphenoxy group, a 4-methoxyphenoxy group, a 3-cyanophenoxy group, a 4-cyanophenoxy group, a benzyloxy group, a methylbenzyloxy group, an isopropylbenzyloxy group, a cyanobenzyloxy group, a nitrobenzyloxy group, a chlorobenzyloxy group, a methoxycarbonylbenzyloxy group, a 2-pyridyloxy group, a (3-methyl-2-pyridyl)oxy group, a (6-methyl-2-pyridyl)oxy group, a (3-methoxy-2-pyridyl)oxy group, a (6-chloro-2-pyridyl)oxy group, a 3-pyridyloxy group, a 1-pyrazolyloxy group, a (4-methyl-1-pyrazolyl)oxy group, a 3-isoxazolyloxy group, a (5-methyl-3-isoxazolyl)oxy group, a (4,5-dimethyl-3-isoxazolyl)oxy group, a (4-chloro-5-phenyl-3-isoxazolyl)oxy group, a (5-methoxycarbonyl-3-isoxazolyl)oxy group, a {5-methyl-2-(1,3,4-thiadiazolyl)}oxy group, a 2-pyrazinyloxy group, a 6-quinolyloxy group, a 8-quinolyloxy group, a 4-quinazolyloxy group, a 2-pyrimidinyloxy group, a (4-methyl-2-pyrimidinyl)oxy group, a (3,5-dimethyl-2-pyrimidinyl)oxy group, a 4-pyrimidinyloxy group, a (2,6-dimethyl-4-pyrimidinyl)oxy group, a (6-methyl-2-isopropyl-4-pyrimidinyl)oxy group, a 3-benzoisoxazolyloxy group, a methylaminomethyl group, a benzylaminomethyl group, a methoxymethylaminomethyl group, an ethylaminomethyl group, a propylaminomethyl group, an isopropylaminomethyl group, a butylaminomethyl group, an isobutylaminomethyl group, a t-butylaminomethyl group, an allylamino group, a dimethylamino group, a diethylamino group, an ethylmethylamino group, a piperidino group, a morpholino group, a 2,6-dimethylmorpholino group, a methylthio group, a benzylthio group, a methoxymethylthio group, an ethylthio group, a propylthio group, an isopropylthio group, a butylthio group, an isobutylthio group, a t-butylthio group, an allylthio group, a phenylthio group, a methylphenylthio group, an isopropylphenylthio group, a methoxyphenylthio group, a cyanophenylthio group, a nitrophenylthio group, a chlorophenylthio group, a methoxycarbonylphenylthio group, a 2-oxo-1-pyridylthio group, a 2-pyridylthio group, a 1-methyl-2-imidazolylthio group or a 5-methyl-2-(1,3,4-thiadiazolyl)thio group; or a salt thereof.

13. The (5-m-cyanobenzylamino)pyrazole derivative of any one of Claims 1 to 11, wherein R⁴ represents a C₁₋₂ alkyl group, a methoxymethyl group, an ethoxymethyl group, a 2-methoxyethyl group, a butyryloxymethyl group, an isobutyryloxymethyl group, a pivaloyloxymethyl group, a cyclopropylcarbonyloxymethyl group, a methoxyacetoxymethyl group, a methoxycarbonyloxymethyl group, a 2-methoxyethoxycarbonyloxymethyl group, a phenoxymethyl group, a 2-pyridyloxymethyl group, a 1-pyrazolyloxymethyl group, a 3-isoxazolyloxymethyl group, a (5-methyl-3-isoxazolyl)oxymethyl group, a 6-quinolyloxymethyl group, a (1-methyl-2-imidazolyl)thiomethyl group, a 2-pyrimidylthiomethyl group, a {5-methyl-2-(1,3,4-thiadiazolyl)}thiomethyl group, a methoxybenzyl group, fluorobenzyl group, a (2-oxo-1-pyridyl)methyl group, a 2-pyridylmethyl group, a 3-pyridylmethyl group, a (3,5-dimethyl-1-pyrazolyl)methyl group, a (4-bromo-1-pyrazolyl)methyl group, a (5-methyl-3-isoxazolyl)methyl group, a cyclopropyl group, a cyclobutyl group, a methoxy group, an ethoxy group, an isopropoxy group, a 2-pyridylmethoxy group, a phenoxy group, a 2-pyridyloxy group, a (3-methyl-2-pyridyl)oxy group, a (6-methyl-2-pyridyl)oxy group, a 3-pyridyloxy group or a (4-methyl-1-pyrazolyl)oxy group; or a salt thereof.

14. The 5-(m-cyanobenzylamino)pyrazole derivative of any one of Claims 1 to 11, wherein R⁴ represents a C₁₋₂ alkyl group, a methoxymethyl group, a cyclopropylcarbonyloxymethyl group, a phenoxymethyl group, a 2-pyridyloxymethyl group, a (1-methyl-2-imidazolyl)thiomethyl group, a {5-methyl-2-(1,3,4-thiadiazolyl)}thiomethyl group, a fluorobenzyl group, a 2-pyridylmethyl group, a 3-pyridylmethyl group, a (5-methyl-3-isoxazolyl)methyl group, a cyclopropyl group, a cyclobutyl group, a methoxy group, an ethoxy group, an isopropoxy group, a 2-pyridylmethoxy group, a phenoxy group, a 2-pyridyloxy group, a (3-methyl-2-pyridyl)oxy group, or a 3-pyridyloxy group; or a salt thereof.

15. The 5-(m-cyanobenzylamino)pyrazole derivative of any one of Claims 1 to 11, wherein R⁴ represents a methyl group, a methoxymethyl group, a 2-pyridyloxymethyl group, a methoxy group, a phenoxy group, or a 2-pyridyloxy group; or a salt thereof.

16. The 5-(m-cyanobenzylamino)pyrazole derivative of any one of Claims 1 to 15, wherein Y represents a methyl group, an ethyl group, a methoxy group, an ethoxy group, a cyano group, a nitro group, a fluorine atom, a chlorine atom, a bromine atom, a methoxycarbonyl group or an ethoxycarbonyl group, and n is 0 or 1; or a salt thereof.

17. The 5-(m-cyanobenzylamino)pyrazole derivative of any one of Claims 1 to 15, wherein n is 0; or a salt thereof.

18. An agricultural chemical comprising, as an effective ingredient, a 5-(m-cyanobenzylamino)pyrazole derivative or a salt thereof as claimed in any one of Claims 1 to 17.
